(19) Europäisches Patentamt
European Patent Office
Office européen des brevets

(11) EP 3 940 069 A1

(12) EUROPEAN PATENT APPLICATION

(43) Date of publication:
**19.01.2022 Bulletin 2022/03**

(21) Application number: **20185475.9**

(22) Date of filing: **13.07.2020**

(51) International Patent Classification (IPC):
***C12N 9/22*** *(2006.01)* ***C12N 15/113*** *(2010.01)*

(52) Cooperative Patent Classification (CPC):
**C12N 9/22; C12N 15/111;** C12N 2310/113;
C12N 2310/20

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **University of Copenhagen**
**1165 Copenhagen K (DK)**

(72) Inventors:
- **Pinilla-Redondo, Rafael**
  **1165 Copenhagen K (DK)**
- **Camara-Wilpert, Sarah**
  **1165 Copenhagen K (DK)**
- **Sørensen, Søren Johannes**
  **1165 Copenhagen K (DK)**
- **Russel, Jakop**
  **1165 Copenhagen K (DK)**

(74) Representative: **Birkeland, Morten**
**IPTector Consulting ApS**
**Diplomvej 377, 222**
**2800 Kgs. Lyngby (DK)**

# (54) METHODS FOR MODULATING CAS-EFFECTOR ACTIVITY

(57) The present invention relates to methods of modulating an activity of a Cas-effector on a target polynucleotide comprising contacting the Cas-effector with an inhibitor component, wherein the inhibitor component comprises an anti-CRISPR ribonucleotide sequence (acrRNA) capable of inhibiting the Cas-effector from (i) associating with a target nucleotide sequence; and/or (ii) associating with a CRISPR guide RNA, and thereby inhibiting the Cas-effector from forming an active RNA-guided Cas-effector complex.

EP 3 940 069 A1

## Description

### Field of the invention

The present invention relates to methods for modulating an activity of a Cas-effector on a target nucleotide using newly discovered inhibitor components comprising an anti-CRISPR ribonucleotide sequence (acrRNA). The invention also encompasses acrRNA's and compositions comprising such acrRNA's as well applications of such acrRNA's for therapy and/or diagnostics as well as for research and development purposes such as gene editing and the like.

### Background of the invention

Protein based technologies for modulation and control of CRISPR-Cas systems have emerged over the recent years, such as described in WO2018/197520 disclosing anti-CRISPR polypeptides modulating the activity of a Cas endonuclease or described in Marino, N. D. et al. (2020) 'Anti-CRISPR protein applications: natural brakes for CRISPRCas technologies', Nature Methods. Another recent patent application, WO2018/009822, discloses inhibiting CRISPR genome editing systems using chemically modified complementary guide RNA. Further, Meeske & Marraffini, Molecular Cell. 71, 791-801, September 6, 2018 disclose that in type VI-A CRISPR-Cas system in its natural host, *Listeria seeligeri,* a guide RNA modified by adding a tag extending the complementarity to the target sequence of the flanking sequence between the protospacer and the repeat sequence of the guide RNA prevents the VI-A nuclease Cas13 from cleaving the target. Accordingly, there remains a tremendous need for providing further technology for modulation and control of CRISPR-Cas systems.

### Summary of the invention

New ribonucleotide structures, notably (poly)ribonucleotides, have been identified, which surprisingly are capable of inhibiting or even preventing activities of Cas-effectors on target DNA. Accordingly, in a first aspect the invention provides a method for modulating an activity of a Cas-effector on a target polynucleotide comprising contacting the Cas-effector with an inhibitor component, wherein the inhibitor component comprises an anti-CRISPR ribonucleotide sequence (acrRNA) capable of inhibiting the Cas-effector from (i) associating with a target nucleotide sequence; and/or (ii) associating with a CRISPR guide RNA, and thereby inhibiting the Cas-effector from forming an active RNA-guided Cas-effector complex, wherein the arcRNA lacks a sequence (spacer) recognizing the target nucleotide sequence.

In a second aspect the invention provides acrRNAs capable of inhibiting a Cas-effector from (i) associating with a target nucleotide sequence; and/or (ii) associating with a CRISPR guide RNA, and thereby inhibiting the Cas-effector from forming an active RNA-guided Cas-effector complex. wherein the arcRNA lacks a spacer sequence of the guide RNA recognizing the target nucleotide sequence.

In a further aspect the invention provides genetically modified host cells comprising a gene encoding the acrRNA of the invention, operably linked to a controllable or constitutive regulatory expression element.

In a further aspect the invention provides compositions comprising the acrRNA of the invention.

In a further aspect the invention provides the use of acrRNA of the invention as a medicament for treating a disease.

### Description of figures

Figure 1 shows the results of a phage spotting assay where lanes of bacterial lawns are spotted with phage serial dilutions

### Incorporation by reference

All publications, patents, and patent applications referred to herein are incorporated by reference to the same extent as if each individual publication, patent, or patent application was specifically and individually indicated to be incorporated by reference. In the event of a conflict between a term herein and a term in an incorporated reference, the term herein prevails and controls.

### Detailed Description of the invention

### Definitions

Any EC numbers used herein refers to Enzyme Nomenclature 1992 from NC-IUBMB, Academic Press, San Diego, California, including 30 supplements 1-5 published in Eur. J. Bio-chem. 1994, 223, 1-5; Eur. J. Biochem. 1995, 232, 1-6; Eur. J. Biochem. 1996, 237, 1-5; Eur. J. Biochem. 1997, 250, 1-6; and Eur. J. Biochem. 1999, 264, 610-650; respectively. The nomenclature is regularly supplemented and updated; see e.g. http://enzyme.expasy.org/.

The term "CRISPR RNA", "crRNA", or "guide RNA" are used interchangeably herein and refer to a polynucleotide sequence that can form a complex with a Cas protein or multi-Cas-protein complex and guide the ribonucleoprotein complex to recognize, and potentially bind to, and optionally cleave, a target nucleotide sequence site. The guide polynucleotide sequence may be an RNA sequence, a DNA sequence, or a combination thereof, optionally including modified nucleotide bases.

The term "trans-activating CRISPR RNA" or "tracrRNA" as used herein refers to an RNA species that interacts with the crRNA to form the crRNA-tracrRNA chimeric guide employed by some CRISPR-Cas systems (e.g. all type II subtypes and some type V subtypes). The tracrRNA serves an important scaffold function for the recognition and coupling by Cas proteins. The tracrRNA-crRNA interaction is essential for pre-crRNA processing, target recognition and cleavage, as well as transcriptional autoregulation of expression in native type II systems. The tracrRNA an anti-repeat, named as such because it forms an imperfect hybrid (partially complementary) with the repeat in the crRNA repeat region.

The term "crRNA-tracrRNA fusion" as used herein refers to the fusion of two RNA molecules comprising a crRNA fused to a tracrRNA. The so-called single guide (sgRNA) or crRNA-tracrRNA fusion may comprise a complete crRNA and tracrRNA, or fragments thereof, that form a complex with a Cas effector protein (e.g. Cas9 or modified versions and homolog variants), wherein the resultant RNA-protein complex is guided by the crRNA portion to recognize a complementary, or partially complementary (Watson-Crick base-pairing), target site to the spacer, allowing the complex to optionally bind to, and potentially cleave or nick (double or single stranded DNA breaks, respectively) the target nucleic acid.

"CRISPR-Cas system" refers to Clustered regularly interspaced short palindromic repeats and their CRISPR-associated (Cas) proteins. These systems comprise a plurality of diverse RNA guided prokaryotic adaptive immune systems employed by these organisms to defend against foreign parasitic nucleic acids. CRISPR-Cas systems include type I to VI types, each of which includes multiple subtypes and variants. CRISPR-Cas systems typically comprise a CRISPR array (updatable memory bank of the immune system that includes sequences of former genetic intruders) and a cluster of Cas protein involved in different stages of immunity. It is clear to a person in the art field that CRISPR and Cas loci can be functionally linked despite not being co-localized within a genome and that diverse CRISPR-Cs systems may overlap in Cas protein homolog content. A more extensive structural, functional, evolutionary classification can be found in Makarova, K. S. et al. (2015) 'An updated evolutionary classification of CRISPR-Cas systems', Nature reviews. Microbiology., 13, p. 722; and Makarova, K. S. et al. (2020) 'Evolutionary classification of CRISPR-Cas systems: a burst of class 2 and derived variants', Nature reviews. Microbiology, 18(2), pp. 67-83.

The term "CRISPR repeat" or "repeat sequence" as used herein refers to their conventional meaning as used in the art, that is, multiple, short, direct repeat nucleotide sequences showing reduced or no sequence variation. These sequences originate from, or are homologous to, the sequences in between the spacer sequences found within a given CRISPR array. Many repeat sequences are partially/semi-palindromic, thus potentially leading to the formation or partial adoption of stable, conserved secondary structure arrangements, e.g. stem-loop folds or hairpins.

The term "palindromic repeat sequence" as used herein refers to a repeat nucleotide sequence for which at least a portion of the repeat is equal to its reverse complement. Due to the natural Watson-Crick base-pairing properties of nucleic acids, palindromic nucleotide sequences are capable of (partially) folding over themselves forming hairpin and stem-loop secondary structures.

The term "semipalindromic repeat sequence" as used herein refers to repeat sequences embedded within CRISPR-derived repeats which do not comprise perfect or full-length palindromes, meaning that, either portions or certain punctual nucleotides across the predicted palindrome, are not functionally predisposed to base-pair.

The term "cognate" as used herein refers to interacting pairs of functional entities. More specifically, that each protein or protein effector complex having RNA guided activity has a cognate crRNA and/or crRNA-tracrRNA fusion, that are required for their activity upon recognition of the targeted site.

The term "spacer" as used herein refers to sequences interspersed among the direct repeat sequences of CRISPR arrays and which, after CRISPR array transcription and processing into mature crRNAs, comprise a portion of the crRNAs that guide the Cas effector protein(s) to a complementary site (so-called the protospacer). In nature, spacer sequences within CRISPR arrays are known to derive from the genomes of viral and other invading genetic elements, thus comprising the memory-basis for the adaptive immune response against recidivist threats. Note that each crRNA contains only one repeat sequence and a variable portion of one of the adjacent repeats in the CRISPR array from which it was transcribed.

The terms "type X CRISPR-Cas system", where X refers to either I, II, III, IV, V, or VI, as used herein refer to the different 6 types of CRISPR-Cas systems hitherto described in literature. A thorough description of their specific functional components and evolutionary relationships is detailed in Makarova et al 2015 and Makarova et al 2020.

The terms "CasX", where X refers to numbers 1 to 14, DinG, RecD, and LS as used herein refer to the Cas protein components (and homologs thereof, or modified versions thereof) involved in the functioning of the different CRISPR-Cas systems. A thorough description of their properties, classification and evolution is described in Makarova et al 2015 and Makarova et al 2020. In some embodiments, Cas protein nucleases (e.g. Cas9, Cas12, etc.) can be defective. For instance, the Cas nuclease can perform nicks in the target DNA, rather than a double strand breakage or have been modified to have deactivated nuclease domains (catalytically dead Cas variants), thus allowing for programmable nucleic acid recognition and potentially binding, without nuclease activity. In other embodiments, Cas proteins which retain the activity to be RNA-guided to a given target site can additionally comprise additional functionalities, such as for example through the fusion of, or conjugation/linkage with, other proteins and/or functional moieties, including fluorophores or fluorescent proteins, transcription factors (activators or repressors), DNA/chromatin remodelling effectors, epigenetic modifiers (methyltransferases, acetylases, etc.), prime/base editors (cytidine/(deoxy)adenosine deaminases), affinity tags, retrons, polymerases (reverse transcriptases or error-prone DNA polymerases), among others.

The terms "heterologous" or "recombinant" or "genetically modified" and its grammatical equivalents as used herein interchangeably refers to entities "derived from a different species or cell". For example, a heterologous or recombinant polynucleotide gene is a gene in a host cell not naturally containing that gene, i.e. the gene is from a different species or cell type than the host cell. The terms as used herein about microbial host cells refers to microbial host cells comprising and expressing heterologous or recombinant polynucleotide genes.

The term "in vivo", as used herein refers to within a living cell, including, for example, a microorganism or a human cell or a plant cell.

The term "ex vivo", as used herein refers to when a given experiment or procedure is conducted outside a given biological organism, cell or tissue (e.g. a bacterium, human organs, mammalian cell lines), and are thus conducted directly in a laboratory environment, with attention to minimally altering the organism or cell natural in vivo conditions.

The term "in vitro", as used herein refers to outside a living cell, including, without limitation, for example, in a microwell plate, a tube, a flask, a beaker, a tank, a reactor and the like.

The terms "substantially" or "approximately" or "about", as used herein refers to a reasonable deviation around a value or parameter such that the value or parameter is not significantly changed. These terms of deviation from a value should be construed as including a deviation of the value where the deviation would not negate the meaning of the value deviated from. For example, in relation to a reference numerical value the terms of degree can include a range of values plus or minus 10% from that value. For example, using these deviating terms can also include a range deviation plus or minus such as plus or minus 9%, 8%, 7%, 6%, 5%, 4%, 3%, 2%, or 1% from a specified value.

The term "and/or" as used herein is intended to represent an inclusive "or". The wording X and/or Y is meant to mean both X or Y and X and Y. Further the wording X, Y and/or Z is intended to mean X, Y and Z alone or any combination of X, Y, and Z.

The term "isolated" or "recovered" as used herein about a compound, refers to any compound, which by means of human intervention, has been put in a form or environment that differs from the form or environment in which it is found in nature. Isolated compounds include but is no limited to compounds of the disclosure for which the ratio of the compounds relative to other constituents with which they are associated in nature is increased or decreased. In an important embodiment the amount of compound is increased relative to other constituents with which the compound is associated in nature. In an embodiment the compound of the disclosure may be isolated into a pure or substantially pure form. In this context a substantially pure compound means that the compound is separated from other extraneous or unwanted material present from the onset of producing the compound or generated in the manufacturing process. Such a substantially pure compound preparation contains less than 10%, such as less than 8%, such as less than 6%, such as less than 5%, such as less than 4%, such as less than 3%, such as less than 2%, such as less than 1 %, such as less than 0.5% by weight of other extraneous or unwanted material usually associated with the compound when expressed natively or recombinantly. In an embodiment the isolated compound is at least 90% pure, such as at least 91% pure, such as at least 92% pure, such as at least 93% pure, such as at least 94% pure, such as at least 95% pure, such as at least 96% pure, such as at least 97% pure, such as at least 98% pure, such as at least 99% pure, such as at least 99.5% pure, such as 100 % pure by weight.

The term "% identity" is used herein about the relatedness between two amino acid sequences or between two nucleotide sequences. "% identity" as used herein about amino acid sequences refers to the degree of identity in percent between two amino acid sequences obtained when using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$\frac{identical\ amino\ acid\ residues}{\text{Length of alignment} - \text{total number of gaps in alignment}}\ x\ 100$$

"% identity" as used herein about nucleotide sequences refers to the degree of identity in percent between two nucleotide sequences obtained when using the Needleman-Wunsch algorithm (Needleman and Wunsch, 1970, supra) as implemented in the Needle program of the EMBOSS package (EMBOSS: The European Molecular Biology Open Software Suite, Rice et al., 2000, supra), preferably version 5.0.0 or later. The parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

$$\frac{\text{identical deoxyribonucleotides}}{\text{Length of alignment} - \text{total number of gaps in alignment}}\ x\ 100$$

The protein sequences of the present disclosure can further be used as a "query sequence" to perform a search against sequence databases, for example to identify other family members or related sequences. Such searches can be performed using the BLAST programs. Software for performing BLAST analyses is publicly available through the National Center for Biotechnology Information (http://www.ncbi.nlm.nih.gov). BLASTP is used for amino acid sequences and BLASTN for nucleotide sequences. The BLAST program uses as defaults:

- Cost to open gap: default= 5 for nucleotides/ 11 for proteins
- Cost to extend gap: default = 2 for nucleotides/ 1 for proteins
- Penalty for nucleotide mismatch: default = -3
- Reward for nucleotide match: default= 1
- Expect value: default = 10
- Wordsize: default = 11 for nucleotides/ 28 for megablast/ 3 for proteins.

Furthermore, the degree of local identity between the amino acid sequence query or nucleic acid sequence query and the retrieved homologous sequences is determined by the BLAST program. However only those sequence segments are compared that give a match above a certain threshold. Accordingly, the program calculates the identity only for these matching segments. Therefore, the identity calculated in this way is referred to as local identity.

The term "coding sequence" refers to a nucleotide sequence, which directly specifies the amino acid sequence of a polypeptide. The boundaries of the coding sequence are generally determined by an open reading frame, which begins with a start codon such as ATG, GTG, or TTG and ends with a stop codon such as TAA, TAG, or TGA. The coding sequence may be a genomic DNA, cDNA, synthetic DNA, or a combination thereof.

The term "control sequence" as used herein refers to a nucleotide sequence necessary for expression of a polynucleotide encoding a polypeptide. A control sequence may be native (i.e., from the same gene) or heterologous or foreign (i.e., from a different gene) to the polynucleotide encoding the polypeptide. Control sequences include, but are not limited to leader sequences, polyadenylation sequence, pro-peptide coding sequence, promoter sequences, signal peptide coding sequence, translation terminator (stop) sequences and transcription terminator (stop) sequences. To be operational control sequences usually must include promoter sequences, transcriptional and translational stop signals. Control sequences may be provided with linkers for the purpose of introducing specific restriction sites facilitating ligation of the control sequences with a coding region of a polynucleotide encoding a polypeptide.

The term "expression" includes any step involved in the production of a polypeptide including, but not limited to, transcription, post-transcriptional modification, translation, post- translational modification, and secretion.

The term "host cell" refers to any cell type that is susceptible to transformation, transfection, transduction, or the like with a polynucleotide construct or expression vector comprising a polynucleotide of the present disclosure. The term "host cell" encompasses any progeny of a parent cell that is not identical to the parent cell due to mutations that occur during replication.

The term "polynucleotide construct" refers to a polynucleotide, either single- or double stranded, which is isolated from a naturally occurring gene or is modified to contain segments of nucleic acids in a manner that would not otherwise exist in nature or which is synthetic, and which comprises a polynucleotide encoding a polypeptide and one or more control sequences.

The term "expression vector" refers to a DNA molecule, either single- or double stranded, either linear or circular, which comprises a polynucleotide encoding a polypeptide and is operably linked to control sequences that provide for its expression. Expression vectors include expression cassettes for the integration of genes into a host cell as well as plasmids and/or chromosomes comprising such genes.

The term "operably linked" refers to a configuration in which a control sequence is placed at an appropriate position relative to the coding polynucleotide such that the control sequence directs expression of the coding polynucleotide.

The terms "nucleotide sequence and "polynucleotide" are used herein interchangeably.

The term "comprise" and "include" as used throughout the specification and the accompanying claims as well as variations such as "comprises", "comprising", "includes" and "including" are to be interpreted inclusively. These words are intended to convey the possible inclusion of other elements or integers not specifically recited, where the context allows.

The articles "a" and "an" are used herein refers to one or to more than one (i.e. to one or at least one) of the grammatical object of the article. By way of example, "an element" may mean one element or more than one element.

Terms like "preferably", "commonly", "particularly", and "typically" are not utilized herein to limit the scope of the claimed disclosure or to imply that certain features are critical, essential, or even important to the structure or function of the claimed disclosure. Rather, these terms are merely intended to highlight alternative or additional features that can or cannot be utilized in a particular embodiment of the present disclosure.

**Methods provided by invention**

As described, *supra*, the present invention evolves from the inventor's discovery of ribonucleotide sequences, which can interact with CRISPR-Cas systems both *in vivo* in a living cell and *ex vivo* and modulate the activity of the Cas effector. Accordingly, in a first aspect the in the methods provided invention comprise modulating an activity of a Cas-effector on a target polynucleotide comprising contacting the Cas-effector with an inhibitor component, wherein the inhibitor component comprises an anti-CRISPR ribonucleotide sequence (acrRNA) capable of inhibiting the Cas-effector from (i) associating with a target nucleotide sequence; and/or (ii) associating with a CRISPR guide RNA for the Cas-effector, and thereby inhibiting the Cas-effector from forming an active RNA-guided Cas-effector complex. The acrRNA may inhibit the Cas-Effector to a varying degree from weak to moderate to strong to even completely prevent the Cas-effector from (i) associating with the target nucleotide sequence; and/or (ii) associating with the CRISPR guide RNA, and thereby prevents the Cas-effector from forming an active RNA-guided Cas effector complex. The guide RNA can in particular be a CRISPR RNA (crRNA), include a trans-activating CRISPR RNA (tracrRNA); and/or be a fusion of a crRNA and a tracrRNA (crRNA-tracrRNA fusion).

In a preferred embodiment the modulating property of the acrRNA is accomplished by the acrRNA comprising a ribonucleotide sequence having a high similarity to the structural moiety of the CRISPR guide RNA, which binds to one or more components of a given Cas-effector, but where the arcRNA lacks one or more spacer sequences of the guide RNA recognizing the target nucleotide sequence. In some embodiments the acrRNA comprises a ribonucleotide sequence having at least 70% identity to a sequence of the structural moiety of the CRISPR guide RNA, which binds to one or more components of the corresponding Cas-effector, but wherein the arcRNA lacks one or more spacer sequences of the guide RNA recognizing the target nucleotide sequence. In more specific embodiments the acrRNA comprises a ribonucleotide sequence that is at least 70%, such as at least 75%, such as at least 80%, such as at least 85%, such as at least 90%, such as at least 95%, such as at least 97%, such as at least 98%, such as at least 99%, such as 100% identical to any one of SEQ ID NO: 10 through 13 all sequences separately included.

In further embodiments the acrRNA may comprise at least one repeat sequence of the structural moiety of the CRISPR guide RNA, which binds to the one or more components of the corresponding Cas-effector. Such repeat sequences can be palindromic, semi-palindromic and/or cognate repeat sequences. Moreover, such repeat sequences is selected from a type I, type III, type IV, type V, type VI CRISPR-Cas system repeat sequence. More specifically the repeat sequence has at least 70%, such as at least 75%, such as at least 80%, such as at least 85%, such as at least 90%, such as at least 95%, such as at least 97%, such as at least 98%, such as at least 99%, such as 100% identity to the repeat sequence comprised in SEQ ID NO: 14 to 929 all sequences separately included.

In still further embodiments the acrRNA may comprise one or more moieties hybridizing to the CRISPR guide RNA and thereby inhibit the CRISPR guide RNA from associating with the Cas-effector. Such hybridizing moieties may include anti-repeat ribonucleotide sequences complementary to a repeat sequence of the CRISPR guide RNA. Particularly, such an anti-repeat sequence can be at least 70%, such as at least 75%, such as at least 80%, such as at least 85%, such as at least 90%, such as at least 95%, such as at least 97%, such as at least 98%, such as at least 99%, such as 100% identical to the sequence complementary to the repeat sequence comprised in SEQ ID NO: 14 to 929 all sequences separately included.

In still further embodiments the acrRNA can modulate Cas-effectors which are selected from type I, type III, type IV, type V and/or type VI Cas-effectors. Such Cas-effectors may comprise a Cas3, Cas5, Cas6, Cas7, Cas8, Cas10, DinG, RecD, LS, Cas11, Cas9, Cas12, Cas12f, Cas13 and/or Cas14 protein complex. These protein complexes may have RNA-guided nuclease activity, they may be catalytically inactive (dCas) or have single stranded nickase function (nCas), instead of a double stranded nuclease activity. More specifically the protein complex can comprise an amino acid sequence which is at least 70% identical to SEQ ID NO: 1146 to 1184 all sequences separately included.

Where the guide RNA is a crRNA, the crRNA may be a type I, type III, type IV, type V and/or type VI CRISPR-Cas system crRNA.

Where the guide RNA includes a tracrRNA, the tracrRNA may be a type II and/or type V CRISPR-Cas system tracrRNA. More specifically the tracrRNA can have has at least 70%, such as at least 75%, such as at least 80%, such as at least 85%, such as at least 90%, such as at least 95%, such as at least 97%, such as at least 98%, such as at least 99%, such as 100% identity to the tracrRNA comprised in SEQ ID NO: 930 to 1145 all sequences separately included.

Where the guide RNA is a crRNA-tracrRNA fusion, the crRNA-tracrRNA fusion may be a type II or type V CRISPR-Cas system crRNA-tracrRNA fusion.

The methods provided for herein can be performed by contacting of the Cas-effector with the inhibitor component *in vivo* in a living cell or ex vivo. When performing the method in vivo in a living cell, such cell can be an eukaryotic cell, a prokaryotic cell or an archaeal cell. Particularly the cell can be eukaryotic, such as a mammalian cell, a plant cell, an insect cell, or a fungal cell. In some embodiments the mammalian cell can be an animal cell or human cell, optionally a blood or an induced pluripotent stem cell.

When performing the methods provided for herein *in vivo* in a living cell, may by encoded by a transgene comprised in the cell. In an embodiment the transgene can be comprised in a self-replicating genetic element. The transgene encoding the acrRNA is preferably operably linked to a native or heterologous regulatory expression element, which in some embodiments may be controllable in response to selected conditions. Such conditions can be selected from one or more of temperature, presence or absence of a molecule/ligand, activation or suppression of an endogenous gene, light, sound, cell cycle, organism phase, tissue, cell type and/or environmental stress. The regulatory expression element may also be constitutive.

In an embodiment alternative to having the cell express the acrRNA, the acrRNA can also be fed exogenously to the cell, optionally by contacting the cell with the acrRNA and/or a delivery vehicle comprising the acrRNA. Suitable delivery forms include lipofection, nucleofection, microinjection, biolistics, virosomes, liposomes, immunoliposomes, polycation or lipid-nucleic acid conjugates, naked DNA, and artificial or phage-based virions.

The methods provided for herein may also be performed *ex vivo,* where the Cas-effector is contacted with the inhibitor component outside a living cell. In such *ex vivo* methods the contacting of the Cas-effector with the inhibitor component can be performed by preparing a medium comprising an extract of the cells provided for herein comprising the Cas-effector and the acrRNA or genes encoding them and providing for cell-free transcription-translation protein synthesis in the medium. Optionally, the medium may also provide for DNA and/or RNA synthesis.

**acrRNAs and compositions provided by the invention**

In a further aspect the invention provides acrRNA's capable of inhibiting a Cas-effector from (i) associating with a target nucleotide sequence; and/or (ii) associating with a CRISPR guide RNA, and thereby inhibiting the Cas-effector from forming an active RNA-guided Cas-effector complex. The acrRNA may inhibit the Cas-Effector to a varying degree from weak to moderate to strong to even completely prevent the Cas-effector from (i) associating with the target nucleotide sequence; and/or (ii) associating with the CRISPR guide RNA, and thereby prevents the Cas-effector from forming an active RNA-guided Cas effector complex. The guide RNA can in particular be a CRISPR RNA (crRNA), include a trans-activating CRISPR RNA (tracrRNA); and/or be a fusion of a crRNA and a tracrRNA (crRNA-tracrRNA fusion). In a particular embodiment the acrRNA comprises a ribonucleotide sequence having at least 70% identity to a sequence of the structural moiety of the CRISPR guide RNA, which binds to one or more components of the corresponding Cas-effector, and wherein the arcRNA lacks a spacer sequence of the guide RNA recognizing the target nucleotide sequence. Suitable acrRNA's comprises a ribonucleotide sequence that is at least 70%, such as at least 75%, such as at least 80%, such as at least 85%, such as at least 90%, such as at least 95%, such as at least 97%, such as at least 98%, such as at least 99%, such as 100% identity to any one of SEQ ID NO: 10 to 13 all sequences separately included. In further embodiments, the acrRNA comprises at least one repeat sequence of the structural moiety of the CRISPR guide RNA, which binds to the one or more components of the corresponding Cas-effector. Said repeat sequence may be palindromic, semi-palindromic and/or cognate and may be selected from a type I, type III, type IV, type V, type VI CRISPR-Cas system repeat sequence. Suitable repeat sequences has at least 70%, such as at least 75%, such as at least 80%, such as at least 85%, such as at least 90%, such as at least 95%, such as at least 97%, such as at least 98%, such as at least 99%, such as 100% identity to the repeat sequence comprised in SEQ ID NO: 10 to 929 all sequences separately included. In still further embodiments the acrRNA comprises a moiety hybridizing to the CRISPR guide RNA and thereby inhibits the CRISPR guide RNA from associating with the corresponding Cas-effector. The hybridizing moiety can be an anti-repeat sequence complementary to a repeat sequence of the CRISPR guide RNA. Suitable anti-repeat sequences are at least 70%, such as at least 75%, such as at least 80%, such as at least 85%, such as at least 90%, such as at least 95%, such as at least 97%, such as at least 98%, such as at least 99%, such as 100% identical to a sequence complementary to the repeat sequence comprised in SEQ ID NO: 10 to 929 all sequences separately included. The Cas-effectors modulated by the acrRNA of the invention may be selected from a type I, type III, type IV, type V and/or type VI Cas-effector. Such Cas-effectors may comprise a Cas3, Cas5, Cas6, Cas7, Cas8, Cas10, DinG, RecD, LS, Cas11, Cas9, Cas12, Cas12f, Cas13 and/or Cas14 protein complex and such protein complex may have RNA-guided nuclease activity or may be catalytically inactive (dCas). For catalytically inactive Cas-effectors one activity modulated by the acrRNA of the invention is chromatin remodelling, prime/base editing, recruitment of other effector proteins or molecules, optionally via fusion, potentially including a linker sequence connecting them. Examples of potential functional domains that may be fused to a Cas protein include, without limitation, epitope tags (e.g. histidine tags, V5 tags, FLAG tags, influenza hemagglutinin tags, Myc tags, VSV-G tags, and thioredoxin tags), reporters (e.g. horseradish peroxidase, chloramphenicol acetyltransferase, beta-galactosidase, beta-glucuronidase, luciferase, glutathione-5-transferase, green fluorescent protein, HcRed, DsRed, cyan fluorescent protein, yellow fluorescent protein, flavin mononucleotide-based fluorescent proteins and autofluorescent proteins including blue fluorescent protein) and protein domains with one or more of the following functions: methylase or demethylase activity, transcription activation or transcription repression activity, transcription release factor activity, polymerases, histone modification activity, RNA cleavage activity and nucleic acid binding activity. The modulated Cas-effector protein complex can comprise an amino acid sequence which at least 70%, such as at least 75%, such as at least 80%, such as at least 85%, such as at least 90%, such as at least 95%, such as at least 97%, such as at least 98%, such as at least 99%, such as 100% identical to SEQ ID NO: 1146 to 1184 all sequences separately included. In a particular embodiment the acrRNA of the invention inhibits the Cas-effector from forming an active RNA-guided Cas-effector complex with crRNA of type I, type III, type IV, type V and/or type VI CRISPR-Cas systems. In another particular embodiment the acrRNA of the invention inhibits the Cas-effector from forming an active RNA-guided Cas-effector complex with tracrRNA of type II and/or type V CRISPR-Cas systems, such as those tracrRNA's that have at least 70%, such as at least 75%, such as at least 80%, such as at least 85%, such as at least 90%, such as at least 95%, such as at least 97%, such as at least 98%, such as at least 99%, such as 100% identity to the tracrRNA comprised in SEQ ID NO: 930 to 1145 all sequences separately included. In another particular embodiment the acrRNA of the invention inhibits the Cas-effector from forming an active RNA-guided Cas-effector complex with a crRNA-tracrRNA fusion of type II and/or type V CRISPR-Cas systems.

The present invention also provides compositions comprising the acrRNA of the invention the delivery comprising the acrRNA. Such compositions may further include suitable carriers, excipients, agents, additives and/or adjuvants and in particular the composition is a pharmaceutical composition comprising one or more pharmaceutical grade carriers, excipients, agents, additives and/or adjuvants.

**Genes and constructs provided by the invention**

In a further aspect the invention provides genes encoding the acrRNA of the invention. In particular such genes comprises a nucleotide sequence which is at least 70%, such as at least 75%, such as at least 80%, such as at least 85%, such as at least 90%, such as at least 95%, such as at least 97%, such as at least 98%, such as at least 99%, such as 100% identical to the gene encoding the acrRNA comprised in anyone of SEQ ID NO: 10 to 13 all sequences separately included or genomic DNA thereof. The gene encoding the acrRNA, may be operably linked to one or more regulatory expression elements. Such regulatory expression elements may be controllable or constitutive. Controllable regulatory expression elements may respond to various conditions such as one or more conditions selected from: temperature, presence or absence of a molecule/ligand, activation or suppression of an endogenous gene, light, sound, cell cycle, organism phase, tissue, cell type or environmental stress.

**Vehicles and cells provided by the invention**

In a further aspect the invention provides delivery vehicles or cells comprising the acrRNA of the invention. In one embodiment the delivery vehicle optionally comprising a liposome, nanoparticle or a phage particle. In another embodiment the cell is a genetically modified host cell comprising the gene or the nucleotide construct of the invention providing for the expression of acrRNA.

**Applications of acrRNAs provided by the invention**

In a further aspect the invention provides applications and uses of acrRNA of the invention.

Particularly the arRNA may be used as a medicament for treatment of a disease or malfunction in a living organism or for diagnosing such malfunctions.

**Sequence listings**

The present application contains a Sequence Listing prepared in PatentIn version 3.5.1, which is also submitted electronically in ST25 format which is hereby incorporated by reference in its entirety.

**Items of the invention**

The present invention further provides the following embodiments and items:

1. A method of modulating an activity of a Cas-effector on a target polynucleotide comprising contacting the Cas-effector with an inhibitor component, wherein the inhibitor component comprises an anti-CRISPR ribonucleotide sequence (acrRNA) capable of inhibiting the Cas-effector from (i) associating with a target nucleotide sequence; and/or (ii) associating with a CRISPR guide RNA, and thereby inhibiting the Cas-effector from forming an active RNA-guided Cas-effector complex.

2. The method of item 1, wherein the guide RNA is a CRISPR RNA (crRNA), includes a trans-activating CRISPR RNA (tracrRNA); and/or is a fusion of a crRNA and a tracrRNA (crRNA-tracrRNA fusion)

3. The method of item 1, wherein the arcRNA prevents the Cas-effector from (i) associating with a target nucleotide sequence; and/or (ii) associating with the CRISPR guide RNA, and thereby prevents the Cas-effector from forming an active RNA-guided Cas effector complex.

4. The method of item 1 or 3, wherein the acrRNA comprises a ribonucleotide sequence having at least 70% identity to a sequence of the structural moiety of the CRISPR guide RNA, which binds to one or more components of the Cas-effector, and wherein the arcRNA lacks a spacer sequence of the guide RNA recognizing the target nucleotide sequence.

5. The method of item 4, wherein the acrRNA comprises a ribonucleotide sequence that is at least 70% identical to any one of SEQ ID NO: 10 to 13.

6. The method of item 4 or 5, wherein the acrRNA comprises at least one repeat sequence of the structural moiety of the CRISPR guide RNA, which binds to the one or more components of the Cas-effector.

7. The method of item 6 wherein the said repeat sequence is palindromic, semi-palindromic and/or cognate.

8. The method of item 7, wherein the said repeat sequence is selected from a type I, type III, type IV, type V, type VI CRISPR-Cas system repeat sequence.

9. The method of items 6 to 8, wherein the said repeat sequence has at least 70% identity to the repeat sequence comprised in SEQ ID NO: 14 to 929.

10. The method of item 1, wherein acrRNA comprises a moiety hybridizing to the CRISPR guide RNA and thereby inhibits the CRISPR guide RNA from associating with the Cas-effector.

11. The method of item 10, wherein said moiety is a ribonucleotide sequence which is an anti-repeat sequence complementary to a repeat sequence of the CRISPR guide RNA.

12. The method of item 11, wherein the anti-repeat is at least 70% identical to the sequence complementary to the repeat sequence comprised in SEQ ID NO: 14 to 929.

13. The method of any preceding item, wherein the Cas-effector is selected from a type I, type III, type IV, type V and/or type VI Cas-effector.

14. The method of item 13, wherein the Cas-effector comprises a Cas3, Cas5, Cas6, Cas7, Cas8, Cas10, DinG, RecD, LS, Cas11, Cas9, Cas12, Cas12f, Cas13 and/or Cas14 protein complex.

15. The method of item 14, wherein the protein complex has RNA-guided nuclease activity.

16. The method of item 14, wherein the protein complex is catalytically inactive (dCas).

17. The method of item 10, wherein the protein complex comprises an amino acid sequence which is at least 70% identical to SEQ ID NO: 1146 to 1184.

18. The method of item 2, wherein the crRNA is a type I, type III, type IV, type V and/or type VI CRISPR-Cas system crRNA.

19. The method of item 2, wherein the tracrRNA is a type II and/or type V CRISPR-Cas system tracrRNA.

20. The method of item 19, wherein the tracrRNA has at least 70% identity to the tracrRNA comprised in SEQ ID NO: 930 to 1145 .

21. The method of item 2, wherein the crRNA-tracrRNA fusion is a type II or type V CRISPR-Cas system crRNA-tracrRNA fusion.

22. The method of any preceding item, wherein the said contacting of the Cas-effector with the inhibitor component is performed in vivo in a living cell.

23. The method of item 22, wherein the cell is an eukaryotic cell, animal cell, mammalian cell, human cell, blood or an induced pluripotent stem cell, prokaryotic (bacteria or archaea) cell, plant cell, insect cell, fungal cell.

24. The method of items 22 to 23, wherein the cell comprises a transgene encoding the acrRNA.

25. The method of items 22 to 24, wherein the cell comprises a self-replicating genetic element comprising the transgene encoding the acrRNA.

26. The method of item 24 to 25, wherein the transgene is operably linked to a heterologous regulatory expression element.

27. The method of item 26, wherein said heterologous regulatory expression element is controllable in response to a condition selected from the group consisting of: temperature, presence or absence of a molecule/ligand, activation or suppression of an endogenous gene, light, sound, cell cycle, organism phase, tissue, cell type or environmental stress.

28. The method of item 26, wherein said heterologous regulatory expression element is constitutive.

29. The method of items 22 to 28, wherein the acrRNA is fed to the cell exogenously, optionally by contacting the cell with a delivery vehicle comprising the acrRNA.

30. The method of items 29, wherein the delivery vehicle comprises a liposome, nanoparticle or a phage particle.

31. The method of items 1 to 21, wherein the contacting of the Cas-effector with the inhibitor component is performed ex vivo.

32. The method of items 31, wherein the contacting of the Cas-effector with the inhibitor component is performed in a medium comprising an extract of a cell of items 22 to 28 and providing for cell-free transcription-translation protein synthesis in a medium.

33. The method of item 31, wherein the contacting of the Cas-effector with the inhibitor component is performed in a medium comprising an extract of a cell of items 22 to 28 comprising the Cas-effector.

34. The method of item 31, wherein the contacting of the Cas-effector with the inhibitor component is performed in a medium providing for DNA or RNA synthesis.

35. An acrRNA capable of inhibiting a Cas-effector from (i) associating with a target nucleotide sequence; and/or (ii) associating with a CRISPR guide RNA, and thereby inhibiting the Cas-effector from forming an active RNA-guided Cas-effector complex.

36. The acrRNA of item 35, wherein the CRISPR guide RNA is a CRISPR RNA (crRNA), include a trans-activating CRISPR RNA (tracrRNA); and/or a fusion of a crRNA and a tracrRNA (crRNA-tracrRNA fusion).

37. The acrRNA of item 35 or 35, wherein arcRNA prevents the Cas-effector from (i) associating with the target nucleotide sequence; and/or (ii) associating with the CRISPR guide RNA, and thereby prevents the Cas-effector from forming an active RNA-guided Cas effector complex.

38. The acrRNA of item 35, wherein the acrRNA comprises a ribonucleotide sequence having at least 70% identity to a sequence of the structural moiety of the CRISPR guide RNA, which binds to one or more components of the Cas-effector, and wherein the arcRNA lacks a spacer sequence of the guide RNA recognizing the target nucleotide sequence.

39. The acrRNA of item 35, wherein the acrRNA comprises a ribonucleotide sequence that is at least 70% identical to any one of SEQ ID NO: 10 to 13

40. The acrRNA of item 38 or 39, wherein the acrRNA comprises at least one repeat sequence of the structural moiety of the CRISPR guide RNA, which binds to the one or more components of the Cas-effector.

41. The acrRNA of item 40, wherein the said repeat sequence is palindromic, semi-palindromic and/or cognate.

42. The acrRNA of item 41, wherein the said repeat sequence is selected from a type I, type III, type IV, type V, type VI CRISPR-Cas system repeat sequence.

43. The acrRNA of items 40 to 42, wherein the said repeat sequence has at least 70% identity to the repeat sequence comprised in SEQ ID NO: 14 to 929.

44. The acrRNA of item 35, wherein acrRNA comprises a moiety hybridizing to the CRISPR guide RNA and thereby inhibits the CRISPR guide RNA from associating with the Cas-effector.

45. The acrRNA of item 44, wherein said moiety is a ribonucleotide sequence which is an anti-repeat sequence complementary to a repeat sequence of the CRISPR guide RNA.

46. The acrRNA of item 45, wherein the anti-repeat is at least 70% identical to the sequence complementary to the repeat sequence comprised in SEQ ID NO: 14 to 929.

47. The acrRNA of items 35 to 46, wherein the Cas-effector is selected from a type I, type III, type IV, type V and/or type VI Cas-effector.

48. The acrRNA of item 47, wherein the Cas-effector comprises a Cas3, Cas5, Cas6, Cas7, Cas8, Cas10, DinG, RecD, LS, Cas11, Cas9, Cas12, Cas12f, Cas13 and/or Cas14 protein complex.

49. The acrRNA of item 48, wherein the protein complex has RNA-guided nuclease activity.

50. The acrRNA of item 48, wherein the protein complex is catalytically inactive (dCas).

51. The acrRNA of item 48, wherein the protein complex comprises an amino acid sequence which is at least 70% identical to SEQ ID NO: 1146 to 1184.

52. The acrRNA of item 36, wherein the crRNA is a type I, type III, type IV, type V and/or type VI CRISPR-Cas system crRNA.

53. The acrRNA of item 36, wherein the tracrRNA is a type II and/or type V CRISPR-Cas system tracrRNA.

54. The acrRNA of item 53, wherein the tracrRNA has at least 70% identity to the tracrRNA comprised in SEQ ID NO: 930 to 1145.

55. The acrRNA of item 36, wherein the crRNA-tracrRNA fusion is a type II or type V CRISPR-Cas system crRNA-tracrRNA fusion.

56. A gene encoding the acrRNA of items 35 to 55.

57. A nucleotide construct comprising the gene of item 56, operably linked to a regulatory expression element.

58. The nucleotide construct of item 57, wherein said regulatory expression element is controllable in response to a condition selected from the group consisting of: temperature, presence or absence of a molecule/ligand, activation or suppression of an endogenous gene, light, sound, cell cycle, organism phase, tissue, cell type or environmental stress.

59. The nucleotide construct of item 57, wherein said regulatory expression element is constitutive.

60. A delivery vehicle comprising the acrRNA of items 35 to 55, said delivery vehicle optionally comprising a liposome, nanoparticle or a phage particle.

61. A genetically modified host cell comprising the gene or the nucleotide construct of items 56 to 59.

62. A composition comprising the acrRNA of items 35 to 55 or the delivery vehicle of item 60

63. The composition of item 62, wherein the composition is a pharmaceutical composition further comprising one or more pharmaceutical grade carriers, excipients, agents, additives and/or adjuvants.

64. The acrRNA of items 35 to 55 for use as a medicament.

## Examples

### Materials and methods

Chemicals used in the examples herein, e.g. for buffers and substrates, are commercial products of at least reagent grade. Water utilized in the examples was de-ionized, MilliQ water.

*Chemicals used:*

| Compound | Abbr. | Use |
|---|---|---|
| Sucrose | | Used in the preparation of competent cells of PA14 and PAO1. This special treatment allows these cells to be in a state of competency, that is, permitting the uptake of DNA from their immediate surroundings (transformation). |
| L-Arabinose | L-ara | For induction of gene expression under pBad promoter |
| Isopropyl β-d-1-thiogalactopyranoside | IPTG | For induction of gene expression under ptac promoter |
| Magnesium sulfate | MgSO4 | For supplementation of solid LB-media used for phage-related assays |
| Sodium Chloride | NaCl | For buffer solutions |
| Calcium chloride | CaCl2 | Used in the preparation of competent cells of *E.coli.* This special treatment allows these cells to be in a state of competency, permitting the pick up DNA through transformation at higher efficiencies. |
| Gentamycin sulfate | Genta | For antibiotic selection of strains carrying pHerd30T and/or variants |
| Carbenicillin | Carb | For antibiotic selection of strains carrying pMMbHE67 and/or variants |
| Hydrochloric acid | HCl | For buffer solutions |
| Agar | | For supplementation of solid growth medium |
| Lysogenic broth | LB | For growth media |
| Deionized Water | ddH2O | Solvent for antibiotics and buffer/media |

(continued)

| Compound | Abbr. | Use |
|---|---|---|
| Tris-base | | For SM-Buffer |
| Chloroform | | For phage propagation |

*Medias and buffers*

| Name | Composition |
|---|---|
| SM-Buffer | 5.8g NaCl; 2g MgSo4*7H2O; 50mL Tris-Cl (1M, pH 7.5); volume adjusted to 1 liter with ddH2O; autoclaved |
| Tris-Cl | 121.1g Tris base dissolved in 800mL ddH2O; pH adjusted to 7.5 by adding concentrated HCl |
| Liquid bacterial growth media | 8g LB; 400mL ddH2O; if needed supplemented with antibiotics and inducers |
| Solid bacterial growth media | 8g LB; 6g Agar; 400mL ddH2O; if needed supplemented with antibiotics and/or inducers |

*Antibiotic and inducer solutions*

| | Stock conc. | Conc. in growth media for E.coli | Conc. in growth media for Pseudomonas |
|---|---|---|---|
| Carb | 50mg/mL | 100ug/mL | 100ug/m L |
| Genta | 10mg/mL | 15ug/mL | 50ug/mL |
| L-ara | 10% w/v | - | 0.3% w/v |
| IPTG | 1M | - | 1mM |

Cells and phages

The following microbial strains were prepared for use in the examples

| Alias | Strain | Strain | genotype | Description | Reference |
|---|---|---|---|---|---|
| | E. coli | genehogs | | Used for enrichment of vector DNA and assembly of constructs | Thermo Fisher catalog nos. C800-05 |
| RPR 145 | P. aeroguinosa | PA14 | WT; I-F WT | PA14 harboring the native I-F CRISPR-Cas system, targeting phage DMSm | NCBI database: NC_008463.1 |
| RPR 146 | P. aeroguinosa | PA14 | I-F ΔCRISPR1 | PA14 harboring the native I-F | Cady et al., |
| | | | | CRISPR-Cas system, lacking the CRISPR array which includes the spacer targeting phage DMSm | 2012* |
| RPR 212 | P.aeruginosa | PAO1 | tn7::mbCpf1, ctx2::crRNA23 | PAO1 harboring MbCpf1 and a crRNA targeting phage JBD30 | Marino N.D., et al 2018 - see below |

(continued)

| Alias | Strain | Strain | genotype | Description | Reference |
|---|---|---|---|---|---|
| RPR 213 | P.aeruginosa | PAO1 | tn7::mbCpf1, ctx2:: no crRNA23 | PA01 harboring MbCpf1, lacking the crRNA targeting phage JBD30 | Marino N.D., et al 2018 - see below |
| * Cady, K. C. et al. (2012) 'The CRISPR/Cas adaptive immune system of Pseudomonas aeruginosa mediates resistance to naturally occurring and engineered phages', Journal of bacteriology, 194(21), pp. 5728-5738. | | | | | |

The following phages were prepared for use in the examples

| Name | Description | Reference |
|---|---|---|
| DMS3m | Pseudomonas phage capable of infecting PA14 carrying a protospacer that is targeted by the PA14 I-F CRISPR-Cas system | NCBI database (DMS3): NC_ 008717.1 |
| JBD30 | Pseudomonas phage capable of infecting PA01 carrying a protospacer that is targeted by the engineered PA01 strain carrying mbCpf1 and crRNA23 | NCBI database: NC_ 020298.1 |

*Primers and oligonucleotides*

| Name | Sequence 5'→3' | Description |
|---|---|---|
| Prs1 | AAATTATUTCTAGCCCAAAAAAACGG | pHerd30t backbone amplification |
| Prs2 | ACTGGCCGUCGTTTTACAACGTCG | pHerd30t backbone amplification |
| Prs3 | AGATTAGCGGATCCTACCTG | sequencing of repeat/acrRNA site |
| Prs4 | GCTGCAAGGCGATTAAGTTGG | sequencing of repeat/acrRNA site |
| Prs9 | ACGGCCAGUTGATACGATTAGGACAATGGTCACCGACG | amplification of acrRNAs ordered from twist bioscience for insertion into pHerd30T |
| Scp14 | AGTCCGAUCCCAACTATTTTGTCCGCCCAC | amplification of acrRNAs ordered from twist bioscience for insertion into pHerd30T |
| Prs31 | AATAATTUTCCGGGGCCTGCTCTC | amplification of acrRNA865 without predicted native promoter for insertion behind pBad |
| PRS40 | AATAATTUGGAGTATATATGCAACTACATAACGCC | amplification of acrRNA1792 without predicted native promoter for insertion behind pBad |
| PRS41 | AATAATTUATTGCAGGTAAGATGGCATCTATG | amplification of acrRNA1794 without predicted native promoter for insertion behind pBad |
| Ors15 | GTTCACTGCCGTATAGGCAGCTAAGAAAAACGGCCGACGCTT | full repeat I-F |

(continued)

| Name | Sequence 5'→3' | Description |
|---|---|---|
| Ors16 | CGGCCGTTTTTCTTAGCTGCCTATACGGCAGTGAACAAATTATT | full repeat I-F |
| Ors23 | TCTCGTTCACTGCCGGATAGGCAGCCAAGGAAATC | "Synthetic" acrRNA865 (palindromic repeat only) |
| Ors24 | CAGTGATTTCCTTGGCTGCCTATCCGGCAGTGAAC | "Synthetic" acrRNA865 (palindromic repeat only) |

*DNA Fragments comprising acrRNA's*

Active moieties in bold; primer sequences underlined and do not form part of the original sequence

| Name | Sequence 5'→3' | Description |
|---|---|---|
| Frs acrRNA 773 | AGTCCGATCCCAACTATTTTGTCCGCCCACTGGTGTTGCTGGACTACCTGTCCAACATCTTCCGGGTTGGCGGTGAAGACCAGTTATCCAAGTACCGGAAGAATATCGGTCAGGCATAGGGACTAGCTCCAATGGAATGAGCCGCCAGGACGGCAAACCGAAGCCCCGCCATCGTGCGGGGCTTCTTTTTTTCCAGTCAGCAAACTTGAAATACACCCTTAAGGGTGTATTATTGTTTCCACGGAAGGCGAATCGCCAACC**CCTCACTGCCGTATAGGCAGCCCAGAA**TGTACGGAGATCACCACACGTCGGTGACCATTGTCCTAATCGTATCAACTGGCCGT | DNA Fragment for amplification of I-F acrRNA773 sequence from NCBI database: CP011110.1 |
| Frs acrRNA 865 | AGTCCGATCCCAACTATTTTGTCCGCCCACGAGACAAGGTCGCCTTGTCTCGACAACGCCCCCGCGAAAACCGCCCCTGTGAGCCGTCCGCCACCAGACCCGCCACCGTCCTACCGCCCAAACCGCCCCGCGCGCGTCTACCCGCGTTAGACCCGCGTTAGATTCGATTTCCGGGGCCTGCTCTCGCCGCCCCCTTCGGCTTCGCTCAGGGCAGGCTTGCGACCGTCGCCCGATTGCGCGACAGTAGAGACAGGGCGCCGCCTTGTCTGATGCTCTC**GTTCACTGCCGGATAGGCAGCCAAGGAAATC**ATGCTCGGCCACGGACGGTCTTCCCCCTGCGCCCCCGCAACCTATTGTGCACATCGCCCGCCGTCGATCATGGCGGCCGTCGGTGACCATTGTCCTAATCGTATCAACTGGCCGT | DNA Fragment for amplification of I-F acrRNA865 sequence from NCBI database: NC_018012.1 |
| Frs acrRNA 1792 | AGTCCGATCCCAACTATTTTGTCCGCCCACCACAGCCCTTAGCATTTCCATGCTATACAAGTCGCACCTTAAGCAGTCTGGCAAACTGCCTTTTGGGATTACCAGTCCCAAACCTTGAACGCCTCTAAGGATTTAAAACCTTAGGGGTGTTTATTTTATCTGGGAGTATATATGCAACTACATAACGCCATATTTGTTGATGGTCGCATCACAGCAAATAGCA**GTCTAACGACCTTTTAAATTTCTACTGTTTGTAGAT**TTCCTGTTGGGGATTATCAGCGGCTTATTGGTTATTTGCGCTTTGACAGATTTTGTGTGAAATATCCTGACAGCAAGGCGGATTTTAACTGCTTTGAGCCGTCGGTGACCATTGTCCTAATCGTATCAACTGGCCGT | DNA Fragment for amplification of V-A acrRNA1792 sequence from NCBI database: CP011377.1 |
| Frs acrRNA 1794 | AGTCCGATCCCAACTATTTTGTCCGCCCACATGCTACATCGTTATGCATCAGTATTGATGGATGCAGGTTTTAGCTTTGAAAGTATTAAAGAGAAAACTAT | DNA Fragment for amplification of V-A acrRNA1794 |

(continued)

| Name | Sequence 5'→3' | Description |
|---|---|---|
| | TGCTTTAAATAATAAGTTAATAGATAAACTTGATGAACTGGAGTTAGCTA<br>ATACAATATTCCATACCATTGCAGGTAAGATGGCATCTATGGGCAGAATG<br>TAGCTTTTAACCATAACATCGCATTCTGCGGTGTTATGGTTTTGTTTTTAA<br>ACCATGTCAATT**GTCTAACAACTTTTTAAATTTCTACTGTTTGTAGAT**TAG<br>ATTAGATAACCATGTCAATTAATCATAGGAGACTTTATGAGTCAAGTTAA<br>TGACCATTTAGTACTTATCTGTGGTGAATCTAGTACAGGTAAATCAGCGT<br>CGGTGACCATTGTCCTAATCGTATCAACTGGCCGT | sequence from NCBI database: CP011376.1 |

**Example 1 Construction of vectors for acrRNA's and genes of interest**

*Plasmid extractions*

Plasmid DNA was extracted from an overnight culture of the according host strain with either the Plasmid Mini AX kit (A&A Biotechnology) for plasmids larger than 15kb and the QIAprep Spin Miniprep Kit (Qiagen) for plasmids smaller than that. Concentrations of the DNA extract were determined by means of the Qubit ® Fluorometer (Invitrogen) as instructed by the manufacturer.

*Vector construction*

Expression vectors utilized for testing effect of acrRNAs

| Alias | Backbone | Promoter | Expressed gene of interest | Description |
|---|---|---|---|---|
| pHerd 30T-ev | pHerd30T | pBad | - | empty vector. NCBI database: EU603326.1 |
| pRS0 | pHerd30T | native phage promoter (pNative) | I-F acrRNA773 | I-F-repeat like acrRNA773 identified on a phage genome (NCBI database: CP011110.1) expressed under control of the predicted wild type promoter |
| pSR1 | pHerd30T | native phage promoter (pNative) | I-F acrRNA865 | I-F-repeat like acrRNA865 identified on a phage genome (NCBI database: NC_018012.1) expressed under control of the predicted wild type promoter |
| pSR2 | pHerd30T | pBad | I-F acrRNA865 | I-F-repeat like acrRNA865 identified on a phage genome (NCBI database: NC_018012.1) expressed under control of the L-ara. induced pBad promoter |
| pSR3 | pHerd30T | pBad | I-F repeat native to PA14 | Native I-F-repeat expressed under control of the L-ara. induced pBad |
| | | | | promoter (seq: GTTCACTGCCGTATAGGCAGCTAAGAAA) |
| pSR4 | pHerd30T | pBad | "synthetic" acrRNA865 | Exclusively the palindromic repeat identified in the phage genome under control of the L-ara. induced pBad promoter (seq: gttcactgccggataggcagccaaggaaatc) |

(continued)

| Alias | Backbone | Promoter | Expressed gene of interest | Description |
|---|---|---|---|---|
| pSR5 | pHerd30T | pBad | V-A acrRNA1792 | V-A-repeat like acrRNA1792 identified on a phage genome (NCBI database: CP011377.1) expressed under control of the L-ara. induced pBad promoter |
| pSR6 | pHerd30T | pBad | V-A acrRNA1794 | V-A-repeat like acrRNA1792 identified on a phage genome (NCBI database: CP011376.1) expressed under control of the L-ara. induced pBad promoter |
| pSR7 | pHerd30T | pBad | V-A repeat native to Moraxella bovoculi | Native V-A-repeat expressed under control of the L-ara. induced pBad promoter.<br><br>(seq: GTCTAACGACCTTTTAAATTTCTACTGTTTGTAGAT) |

Constructing of the expression vectors expressing genes of interest was done using USER® cloning (NEB, USA). DNA amplification for all inserts and backbones was done by using the Phusion U Hot Start DNA Polymerase (Thermo Scientific) as instructed by the provider. Melting temperatures of the primers were calculated with the help of the Tm calculator from ThermoFisher Scientific. PCR products were segregated after size via gel electrophoresis and the product with the correct size was subsequently purified from the gel using the QIAquick Gel Extraction Kit (Qiagen). The gel electrophoresis was performed on solidified (1% Biotechnology grade Agarose I; VWR International) 1x Modified Tris-Acetate EDTA (TAE) buffer, supplemented with 1 drop of 0.07% ethidium bromide/100μl TAE buffer. Segregation of DNA has been conducted at 120V for 20min in 1x TAE buffer. The bands were visualized with the help of the G:box F3 (Syngene) equipped with a UV transilluminator, controlled by Genesys v. 1.5 software (Syngene).

Assembly of the fragments (USER® assembly) was conducted with the help of the USER ® enzyme (New England Biolabs) as recommended by the manufacturer. The ratio between backbone and insert DNA was chosen to be approximately 0.015pmol to approximately 0.15pmol, with the DNA of the smaller insert fragment exceeding the DNA of the backbone fragment 10-fold. The assembled product was subsequently transformed into chemically competent E. coli genehogs.

Positive clones were screened for by colony-PCRs using primers flanking the insert region. Colony PCRs were conducted by picking one single colony and its dilution in a PCR reaction mix including the PCRBIO HiFi Polymerase. The PCR reactions were prepared as recommended by PCRBIOSYSTEMS. The amplified products were sequenced to confirm correctness of the construct. All primers were ordered as oligonucleotides from from a commercial provider. Molecular design of DNA sequences as well as mapping of fragment sequences to reference sequences was done using the software SnapGene® 1.1.3 with default

**Example 2** - **Preparation of strains with expression vectors encoding acrRNA's and genes of interest** *Electro competent transformed P. aeroguinosa cells*

Electrocompetent *P. aeroguinosa* cells were prepared by (i) streaking out the *P. aeroguinosa* cells on a selective medium; (ii) a single colony was picked and utilized to prepare an overnight culture of *P. aeroguinosa.* Cells were harvested (5000g; 10min; 4°C), the supernatant removed, and the pellet was washed in the same volume of room-temperature 300mM succrose twice. The cells were harvested again and subsequently diluted in 1/10 of the original culture volume. Glycerol was added to a final concentration of around 15%, aliquots a 100μl were prepared, and finally frozen at -80°C.

For preparation of functional testing of acrRNAs, the pHerd30T plasmids with different candidate acrRNAs were electroporated into the different *P. aeruginosa* strains. Briefly, the electrocompent cells were carefully thawed when needed and incubated with around 2ul (>500ug) of DNA of interest for 30min on ice. The cells were then transferred to a 2mm electroporation cuvette and exposed to 2500 V. Recovery of the cells was conducted in 600ul LB broth at 30°C for 1h.

*Chemically competent transformed E. coli genehogs cells*

Chemically competent *E. coli* genehogs cells were prepared by (i) streaking out the *E. coli* genehogs on a selective medium; (ii) a single colony was picked and utilized to prepare an overnight culture of the *E. coli* genehogs, which (iii) was used to inoculate 100mL LB Miller broth in an Ehrlenmeyer flasks to develop an OD600=0.02. The cultures were then grown up to an OD600=0.6. Cells were harvested (5000g; 10min; 4°C), the supernatant removed, and the pellet was carefully suspended in 10mL (0.1x of the original culture volume) of ice-cold 0.1M CaCl2 (Sigma Aldrich). After 10min of incubation on ice, the cells were harvested again (5000g; 10min; 4°C), carefully dissolved in 4mL (0.04x of the original culture) of ice cold 0.1M CaCl2 and incubated on ice for 1h. Afterwards, 0.5mL of ice-cold 80% glycerol was added, carefully mixed and aliquots a 50μl competent cells were prepared. The aliquots were then frozen at -80°C. All steps from (iii) onwards were conducted on ice or on 4°C and the cells were not vortexed at any point.

The chemically competent cells were carefully thawed on ice, DNA was added (<500ng) and incubated for 20 to 30min. The cells were then exposed to 42°C for 45s and subsequently placed on ice for 2 min. 500μl LB Miller broth was added and the cells were recovered for 1h at 30°C and 250rpm. The transformed cells were then spread out on solid media with the according antibiotic resistances (100μl on one plate, rest of the cells on another plate).

**Example 3 - Cultivation of cells expressing of acrRNA's and genes of interest**

Samples of cells from example 2 were grown on/in LB media. Overnight cultures were grown in 5mL LB broth at 30°C and 350rpm for 15-16h. LB agar plates have been prepared with 1.5% agar and bacterial growth on the solid media was conducted at 30°C as well. Antibiotics and inducers were added to the according growth media if necessary.

**Example 4 - Screening for and designing acrRNAs**

*Screen for acrRNAs*

BLAST searches using known CRISPR repeats (specific for each CRISPR-Cas system subtype/variant) across NCBI public prokaryotic genome sequence databases were carried out (95% identity and sequence coverage). Sequences matching a known CRISPR repeat were selected as potential acrRNAs, except for those within a distance of 100bp, which are disregarded in order to avoid false-positive detection of true CRISPR arrays. Potential acrRNAs were screened for their association to phage/MGE sequences with virsorter and PHASTER (integrated or extrachromosomal). Candidates present on an MGE genome were selected for being likely true acrRNAs. The identification of potential promoter sequence regions in front of the putative acrRNA were predicted via pBrom. When possible, the presence of host CRISPR-Cas targeting was assessed and stable coexistence of the targeted MGE inside the host cell was considered is a good indicator of CRISPR-Cas inhibition. (Watters, K. E. et al. (2020) 'Potent CRISPR-Cas9 inhibitors from Staphylococcus genomes', PNAS; Rauch, B. J. et al. (2017) 'Inhibition of CRISPR-Cas9 with Bacteriophage Proteins', Cell, 168(1-2), pp. 150-158; Marino, N. D. et al. (2018) 'Discovery of widespread type I and type V CRISPR-Cas inhibitors', Science, 362(6411), pp. 240-242.; Borges, A. L., Davidson, A. R. and Bondy-Denomy, J. (2018) 'The Discovery, Mechanisms, and Evolutionary Impact of Anti-CRISPRs', Annual review of virology, 4(1), pp. 37-59).

*Design of acrRNAs*

AcrRNAs identified on phage genomes were ordered as gene fragments from a commercial provider (IDT) and cloned into pHerd30t under (i) the native promoter, and (ii) under the L-arabinose inducible pBad promoter. The repeat sequence native to the corresponding system was designed and cloned as a "synthetic" acrRNA under expression regulation of pBad.

**Example 5 - Testing effect of acrRNAs**

*Phage propagation*

Pseudomonas phages DMS3m, and JBD30 derivatives were propagated on PA14 ΔCRISPR or PAO1 WT. Pseudomonas phages were stored at 4°C in SM-buffer over chloroform.

*Phage spotting assay*

The functionality of the acrRNAs was assessed through phage spotting assays. Bacterial lawns of the model organism PA14 (carrying the CRISPR-Cas system type I-F) or the engineered model organism PAO1 (carrying MbfCpf1/Cas12a and a respective synthetic crRNA) were challenged with a CRISPR-Cas targeted phage (DMS3m passed through PA14 for I-F; JBD30 passed through PAO1 for V-A). These tests evaluate the replication of CRISPR-targeted phages DMS3m (I-F) and JBD30 (V-A) in bacterial lawns expressing the acrRNA from the vector pHerd30T relative to the empty vector control. Briefly, 150µL of bacterial overnight cultures were combined with 4mL of molten top agar (0.7%) supplemented with 10mM $MgSO_4$ and the according inducers. The mix was poured onto LB agar (1.5%) plates containing the according inducers and antibiotics, 10mM $MgSO_4$ and 0.3% w/v arabinose (induction). Phage dilutions 2.4µL of ten-fold serial dilutions of the respective phage lysates were spotted onto the plate surface containing the bacterial lawn in the top agar. The plates were incubated at 30°C ON and pictures were taken the next day.

*Results*

The results of the phage spotting assay are displayed in figure 1, where lane 1 to 15 shows bacterial lawns on which the phage serial dilution was spotted. Figure 1A shows the assaying of synthetic acrRNAs designed by the present inventors inhibiting the wild type CRISPR-Cas I-F system in *P. aeruginosa* PA14. Figure 1B shows the assaying of natural acrRNAs isolated by the present inventors inhibiting the wild type CRISPR-Cas I-F system in *P. aeruginosa* PA14. In both 1A and 1B, X Indicates a 10-fold serial dilution of phage DMS3m, which were targeted by the CRISPR-Cas I-F system harbored in the *P. aeruginosa* PA14. Figure 1C shows the assaying of synthetic and isolated natural acrRNAs inhibiting MbCpf1 activity in PAO1. Here, Y indicates a 10-fold serial dilution of phage DMS3m, which were targeted by the CRISPR-Cas I-F system harbored in PAO1. The individual lanes were characterized as follows:

**Lane 1** - **bacterial lawn of RPR145 harboring pHerd30T-ev challenged by phage DMS3m.**
This lane shows a bacterial lawn of PA14 with an active CRISPR-Cas type I-F system, targeting the phage DMS3m. Phage replication is inhibited.
**Lane 2** - **bacterial lawn of RPR146 harboring pHerd30T-ev challenged by phage DMS3m.**
This lane shows a bacterial lawn of PA14 deletion strain CRISPR-Cas type I-F ΔCRISPR1, not targeting the phage DMS3m. Phage replication is not prohibited by the CRISPR-Cas system.
**Lane 3** - **bacterial lawn of RPR145 harboring pSR2 challenged by phage DMS3m.**
This lane shows a bacterial lawn of PA14 with an active CRISPR-Cas type I-F system, targeting the phage DMS3m. The expression of the acrRNA865 under the pBad promoter inhibits the I-F CRISPR-Cas system and thereby enables phage replication.
**Lane 4** - **bacterial lawn of RPR145 harboring pSR3 challenged by phage DMS3m.**
This lane shows a bacterial lawn of PA14 with an active CRISPR-Cas type I-F system, targeting the phage DMS3m. The expression of the native I-F PA14 repeat sequence under the pBad promoter inhibits the I-F CRISPR-Cas system and thereby enables phage replication.
**Lane 5** - **bacterial lawn of RPR145 harboring pSR4 challenged by phage DMS3m.**
This lane shows a bacterial lawn of PA14 with an active CRISPR-Cas type I-F system, targeting the phage DMS3m. The expression of the "synthetic" acrRNA865 under the pBad promoter inhibits the I-F CRISPR-Cas system and thereby enables phage replication.
**Lane 6** - **bacterial lawn of RPR145 harboring pHerd30T-ev challenged by phage DMS3m.**
This lane shows a bacterial lawn of PA14 deletion strain CRISPR-Cas type I-F ΔCRISPR1, not targeting the phage DMS3m. Phage replication is not prohibited by the CRISPR-Cas system.
**Lane 7** - **bacterial lawn of RPR146 harboring pHerd30T-ev challenged by phage DMS3m.**
This lane shows a bacterial lawn of PA14 with an active CRISPR-Cas type I-F system, targeting the phage DMS3m. The expression of the acrRNA865 under the pBad promoter inhibits the I-F CRISPR-Cas system and thereby enables phage replication.
**Lane 9** - **bacterial lawn of RPR145 harboring pSR0 challenged by phage DMS3m.**
This lane shows a bacterial lawn of PA14 with an active CRISPR-Cas type I-F system, targeting the phage DMS3m. The expression of the acrRNA773 under the native promoter inhibits the I-F CRISPR-Cas system and thereby enables phage replication.
**Lane 10** - **bacterial lawn of RPR145 harboring pSR1 challenged by phage DMS3m.**
This lane shows a bacterial lawn of PA14 with an active CRISPR-Cas type I-F system, targeting the phage DMS3m. The expression of the acrRNA865 under the native promoter inhibits the I-F CRISPR-Cas system and thereby enables phage replication.
**Lane 11: bacterial lawn of RPR212 harboring pHerd30T-ev challenged by phage JBD30.**
This lane shows a bacterial lawn of PAO1 genetically engineered with MbCpf1 and a crRNA, targeting the phage JBD30. Phage replication is inhibited.
**Lane 12: bacterial lawn of RPR213 harboring pHerd30T-ev challenged by phage JBD30.**
This lane shows a bacterial lawn of PAO1 genetically engineered with MbCpf1, lacking a crRNA, not targeting the phage JBD30. Phage replication is not prohibited.
**Lane 13: bacterial lawn of RPR212 harboring pSR7 challenged by phage JBD30.**
This lane shows a bacterial lawn of PAO1 genetically engineered with MbCpf1 and a crRNA, targeting the phage JBD30. The expression of the native V-A repeat under the pBad promoter inhibits the targeting by MbCpf1 and thereby enables phage replication.

**Lane 14: bacterial lawn of RPR212 harboring pSR5 challenged by phage JBD30.**

This lane shows a bacterial lawn of PAO1 genetically engineered with MbCpf1 and a crRNA, targeting the phage JBD30. The expression of the acrRNA1792 under the pBad promoter inhibits the targeting by MbCpf1 and thereby enables phage replication.

**Lane 15: bacterial lawn of RPR212 harboring pSR6 challenged by phage JBD30.**

This lane shows a bacterial lawn of PAO1 genetically engineered with MbCpf1 and a crRNA, targeting the phage JBD30. The expression of the acrRNA1794 under the pBad promoter inhibits the targeting by MbCpf1 and thereby enables phage replication.

## Claims

1. A method of modulating an activity of a Cas-effector on a target polynucleotide comprising contacting the Cas-effector with an inhibitor component, wherein the inhibitor component comprises an anti-CRISPR ribonucleotide sequence (acrRNA) capable of inhibiting the Cas-effector from (i) associating with a target nucleotide sequence; and/or (ii) associating with a CRISPR guide RNA, and thereby inhibiting the Cas-effector from forming an active RNA-guided Cas-effector complex, wherein the arcRNA lacks a sequence (spacer) recognizing the target nucleotide sequence.

2. The method of claim 1, wherein the acrRNA comprises a ribonucleotide sequence that is at least 90% identical to any one of SEQ ID NO: 10 to 13.

3. The method of claim 1 or 2, wherein the acrRNA comprises at least one repeat sequence of the structural moiety of the CRISPR guide RNA, which binds to the one or more components of the Cas-effector.

4. The method of claims 3, wherein the said repeat sequence has at least 90% identity to the repeat sequence comprised in SEQ ID NO: 14 to 929 or to a sequence complementary thereto.

5. The method of claim 1 to 4, wherein the CRISPR guide RNA comprises a tracrRNA which has at least 90% identity to the tracrRNA comprised in SEQ ID NO: 930 to 1145.

6. An acrRNA capable of inhibiting a Cas-effector from (i) associating with a target nucleotide sequence; and/or (ii) associating with a CRISPR guide RNA, and thereby inhibiting the Cas-effector from forming an active RNA-guided Cas-effector complex. wherein the arcRNA lacks a spacer sequence of the guide RNA recognizing the target nucleotide sequence.

7. The acrRNA of claim 6, wherein the acrRNA comprises a ribonucleotide sequence that is at least 90% identical to any one of SEQ ID NO: 10 to 13.

8. The acrRNA of claim 6 or 7, wherein the acrRNA comprises at least one repeat sequence of the structural moiety of the CRISPR guide RNA, which binds to the one or more components of the Cas-effector.

9. The acrRNA of claims 6 to 8 , wherein the said repeat sequence has at least 90% identity to the repeat sequence comprised in SEQ ID NO: 14 to 929 or to a sequence complementary thereto.

10. The acrRNA of claims 6 to 9, wherein the CRISPR guide RNA comprises a tracrRNA which has at least 90% identity to the tracrRNA comprised in SEQ ID NO: 930 to 1145.

11. A genetically modified host cell comprising a gene encoding the acrRNA of claims 6 to 10, operably linked to a controllable or constitutive regulatory expression element.

12. A composition comprising the acrRNA of claims 6 to 10.

13. The composition of claim 12, wherein the composition is a pharmaceutical composition further comprising one or more pharmaceutical grade carriers, excipients, agents, additives and/or adjuvants.

14. The acrRNA of claims 6 to 10 for use as a medicament.

Figure 1

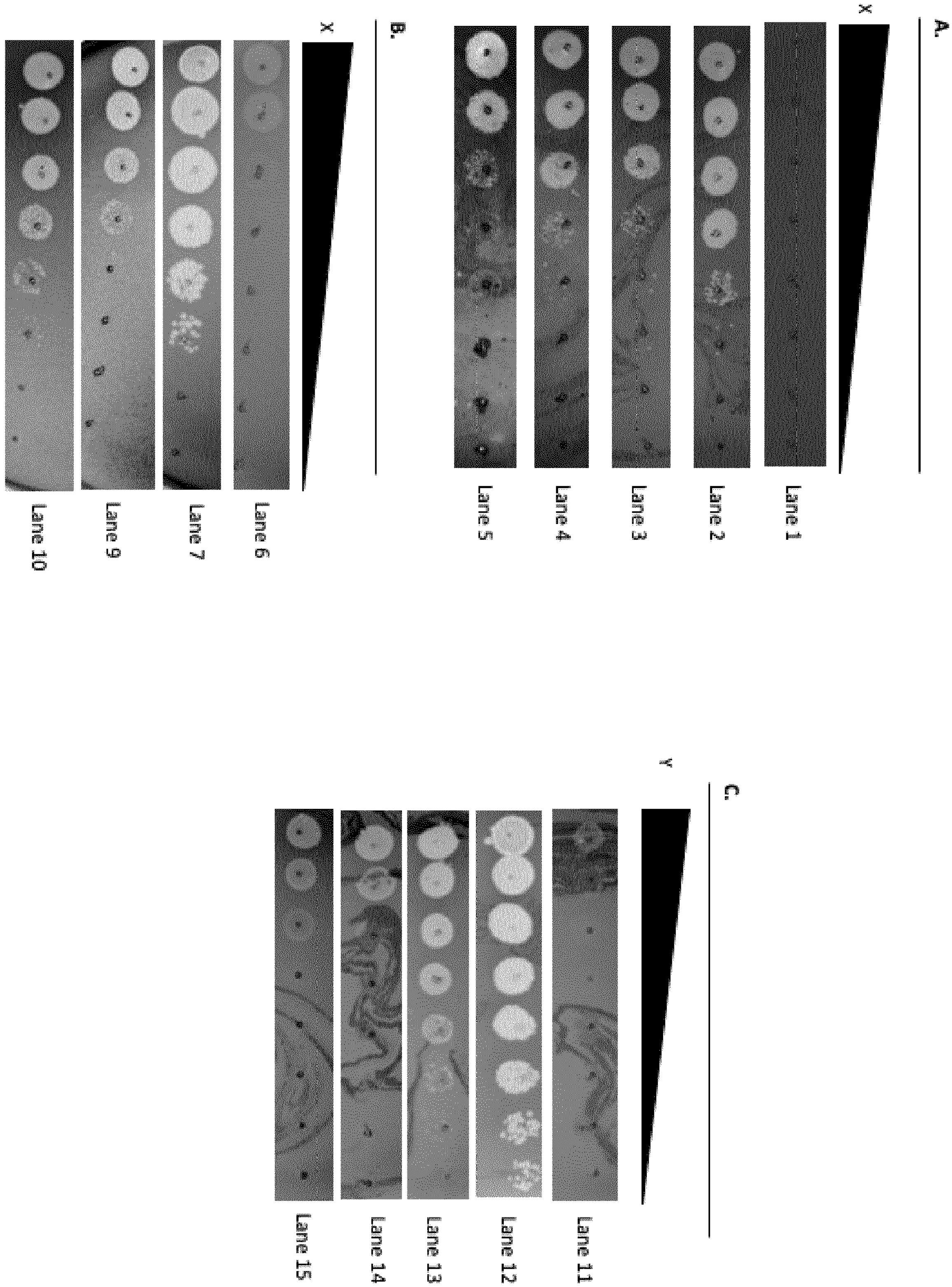
A.
X
Lane 1
Lane 2
Lane 3
Lane 4
Lane 5
B.
X
Lane 6
Lane 7
Lane 9
Lane 10
C.
Y
Lane 11
Lane 12
Lane 13
Lane 14
Lane 15

Europäisches Patentamt
European Patent Office
Office européen des brevets

# EUROPEAN SEARCH REPORT

Application Number
EP 20 18 5475

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BIN LI ET AL: "Synthetic Oligonucleotides Inhibit CRISPR-Cpf1-Mediated Genome Editing", CELL REPORTS, vol. 25, no. 12, 18 December 2018 (2018-12-18), pages 3262-3272, XP055759227, US ISSN: 2211-1247, DOI: 10.1016/j.celrep.2018.11.079 * the whole document * ----- | 1,6 | INV. C12N9/22 C12N15/113 |
| X | CHRISTOPHER L. BARKAU ET AL: "Rationally Designed Anti-CRISPR Nucleic Acid Inhibitors of CRISPR-Cas9", NUCLEIC ACID THERAPEUTICS, vol. 29, no. 3, 30 May 2019 (2019-05-30), pages 136-147, XP055652596, US ISSN: 2159-3337, DOI: 10.1089/nat.2018.0758 * the whole document * ----- | 1-14 | |
| A | SHAO-RU WANG ET AL: "Conditional control of RNA-guided nucleic acid cleavage and gene editing", NATURE COMMUNICATIONS, vol. 11, no. 1, 3 January 2020 (2020-01-03), XP055757252, DOI: 10.1038/s41467-019-13765-3 * the whole document * ----- -/-- | 1-14 | TECHNICAL FIELDS SEARCHED (IPC) C12N |

The present search report has been drawn up for all claims

| Place of search | Date of completion of the search | Examiner |
|---|---|---|
| The Hague | 17 December 2020 | Kools, Patrick |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 20 18 5475

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | NICOLE D. MARINO ET AL: "Discovery of widespread type I and type V CRISPR-Cas inhibitors", SCIENCE, vol. 362, no. 6411, 12 October 2018 (2018-10-12), pages 240-242, XP055715056, US ISSN: 0036-8075, DOI: 10.1126/science.aau5174 * the whole document * ----- | 1-14 | |
| | | | TECHNICAL FIELDS SEARCHED (IPC) |

The present search report has been drawn up for all claims

2

| Place of search | Date of completion of the search | Examiner |
|---|---|---|
| The Hague | 17 December 2020 | Kools, Patrick |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018197520 A
- WO 2018009822 A


### Non-patent literature cited in the description


- **MARINO, N. D. et al.** Anti-CRISPR protein applications: natural brakes for CRISPRCas technologies. *Nature Methods,* 2020
- **MEESKE ; MARRAFFINI.** *Molecular Cell,* 06 September 2018, vol. 71, 791-801
- *Eur. J. Bio-chem.,* 1994, vol. 223, 1-5
- *Eur. J. Biochem.,* 1995, vol. 232, 1-6
- *Eur. J. Biochem.,* 1996, vol. 237, 1-5
- *Eur. J. Biochem.,* 1997, vol. 250, 1-6
- *Eur. J. Biochem.,* 1999, vol. 264, 610-650
- **MAKAROVA, K. S. et al.** An updated evolutionary classification of CRISPR-Cas systems. *Nature reviews. Microbiology,* 2015, vol. 13, 722
- **MAKAROVA, K. S. et al.** Evolutionary classification of CRISPR-Cas systems: a burst of class 2 and derived variants. *Nature reviews. Microbiology,* 2020, vol. 18 (2), 67-83
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **RICE et al.** *EMBOSS: The European Molecular Biology Open Software Suite,* 2000
- **CADY, K. C. et al.** The CRISPR/Cas adaptive immune system of Pseudomonas aeruginosa mediates resistance to naturally occurring and engineered phages. *Journal of bacteriology,* 2012, vol. 194 (21), 5728-5738
- **WATTERS, K. E. et al.** Potent CRISPR-Cas9 inhibitors from Staphylococcus genomes. *PNAS,* 2020
- **RAUCH, B. J. et al.** Inhibition of CRISPR-Cas9 with Bacteriophage Proteins. *Cell,* 2017, vol. 168 (1-2), 150-158
- **MARINO, N. D. et al.** Discovery of widespread type I and type V CRISPR-Cas inhibitors. *Science,* 2018, vol. 362 (6411), 240-242
- **BORGES, A. L. ; DAVIDSON, A. R. ; BONDY-DENOMY, J.** The Discovery, Mechanisms, and Evolutionary Impact of Anti-CRISPRs. *Annual review of virology,* 2018, vol. 4 (1), 37-59

SEQUENCE LISTING

<110> University of Copenhagen

<120> Methods for modulating Cas-effector activity.

<130> P20-059EPPR01

<160> 1184

<170> PatentIn version 3.5

<210> 1
<211> 26
<212> DNA
<213> Artificial

<220>
<223> Artificial sequence

<400> 1
aaattatutc tagcccaaaa aaacgg 26

<210> 2
<211> 24
<212> DNA
<213> Artificial

<220>
<223> Artificial sequence

<400> 2
actggccguc gttttacaac gtcg 24

<210> 3
<211> 20
<212> DNA
<213> Artificial

<220>
<223> Artificial sequence

<400> 3
agattagcgg atcctacctg 20

<210> 4
<211> 21
<212> DNA
<213> Artificial

<220>
<223> Artificial sequence

<400> 4
gctgcaaggc gattaagttg g 21

<210> 5
<211> 38
<212> DNA

<213>  Artificial

<220>
<223>  Artificial sequence

<400>  5
acggccagut gatacgatta ggacaatggt caccgacg    38

<210>  6
<211>  30
<212>  DNA
<213>  Artificial

<220>
<223>  Artificial sequence

<400>  6
agtccgaucc caactatttt gtccgcccac    30

<210>  7
<211>  24
<212>  DNA
<213>  Artificial

<220>
<223>  Artificial sequence

<400>  7
aataattutc cggggcctgc tctc    24

<210>  8
<211>  35
<212>  DNA
<213>  Artificial

<220>
<223>  Artificial sequence

<400>  8
aataattugg agtatatatg caactacata acgcc    35

<210>  9
<211>  32
<212>  DNA
<213>  Artificial

<220>
<223>  Artificial sequence

<400>  9
aataattuat tgcaggtaag atggcatcta tg    32

<210>  10
<211>  344
<212>  DNA
<213>  Artificial

<220>

<223> Artificial sequence

<400> 10
agtccgatcc caactatttt gtccgcccac tggtgttgct ggactacctg tccaacatct 60

tccgggttgg cggtgaagac cagttatcca agtaccggaa gaatatcggt caggcatagg 120

gactagctcc aatggaatga gccgccagga cggcaaaccg aagccccgcc atcgtgcggg 180

gcttcttttt ttccagtcag caaacttgaa atacaccctt aagggtgtat tattgtttcc 240

acggaaggcg aatcgccaac ccctcactgc cgtataggca gcccagaatg tacggagatc 300

accacacgtc ggtgaccatt gtcctaatcg tatcaactgg ccgt 344

<210> 11
<211> 423
<212> DNA
<213> Artificial

<220>
<223> Artificial sequence

<400> 11
agtccgatcc caactatttt gtccgcccac gagacaaggt cgccttgtct cgacaacgcc 60

cccgcgaaaa ccgcccctgt gagccgtccg ccaccagacc cgccaccgtc ctaccgccca 120

aaccgccccg cgcgcgtcta cccgcgttag acccgcgtta gattcgattt ccggggcctg 180

ctctcgccgc cccctcggc ttcgctcagg gcaggcttgc gaccgtcgcc cgattgcgcg 240

acagtagaga cagggcgccg ccttgtctga tgctctcgtt cactgccgga taggcagcca 300

aggaaatcat gctcggccac ggacggtctt ccccctgcgc ccccgcaacc tattgtgcac 360

atcgcccgcc gtcgatcatg gcggccgtcg gtgaccattg tcctaatcgt atcaactggc 420

cgt 423

<210> 12
<211> 396
<212> DNA
<213> Artificial

<220>
<223> Artificial sequence

<400> 12
agtccgatcc caactatttt gtccgcccac cacagccctt agcatttcca tgctatacaa 60

gtcgcacctt aagcagtctg gcaaactgcc ttttgggatt accagtccca aaccttgaac 120

gcctctaagg atttaaaacc ttaggggtgt ttattttatc tgggagtata tatgcaacta 180

cataacgcca tatttgttga tggtcgcatc acagcaaata gcagtctaac gacctttttaa 240

atttctactg tttgtagatt tcctgttggg gattatcagc ggcttattgg ttatttgcgc 300

tttgacagat tttgtgtgaa atatcctgac agcaaggcgg attttaactg ctttgagccg 360

tcggtgacca ttgtcctaat cgtatcaact ggccgt 396

<210> 13
<211> 438
<212> DNA
<213> Artificial

<220>
<223> Artificial sequence

<400> 13
agtccgatcc caactatttt gtccgcccac atgctacatc gttatgcatc agtattgatg 60

gatgcaggtt ttagctttga aagtattaaa gagaaaacta ttgctttaaa taataagtta 120

atagataaac ttgatgaact ggagttagct aatacaatat tccataccat tgcaggtaag 180

atggcatcta tgggcagaat gtagctttta accataacat cgcattctgc ggtgttatgg 240

ttttgttttt aaaccatgtc aattgtctaa caacttttta aatttctact gtttgtagat 300

tagattagat aaccatgtca attaatcata ggagacttta tgagtcaagt taatgaccat 360

ttagtactta tctgtggtga atctagtaca ggtaaatcag cgtcggtgac cattgtccta 420

atcgtatcaa ctggccgt 438

<210> 14
<211> 42
<212> DNA
<213> Artificial

<220>
<223> Artificial sequence

<400> 14
gttcactgcc gtataggcag ctaagaaaaa cggccgacgc tt 42

<210> 15
<211> 44
<212> DNA
<213> Artificial

<220>
<223> Artificial sequence

<400> 15
cggccgtttt tcttagctgc ctatacggca gtgaacaaat tatt 44

<210> 16
<211> 35
<212> DNA
<213> Artificial

<220>
<223> Artificial sequence

<400> 16
tctcgttcac tgccggatag gcagccaagg aaatc 35

<210> 17
<211> 35
<212> DNA
<213> Artificial

<220>
<223> Artificial sequence

<400> 17
cagtgatttc cttggctgcc tatccggcag tgaac 35

<210> 18
<211> 36
<212> DNA
<213> Artificial

<220>
<223> Artificial sequence

<400> 18
gtctaacgac cttttaaatt tctactgttt gtagat 36

<210> 19
<211> 44
<212> DNA
<213> Artificial

<220>
<223> Artificial sequence

<400> 19
atctacaaac agtagaaatt taaaaggtcg ttagacaaat tatt 44

<210> 20
<211> 24
<212> DNA
<213> Artificial

<220>
<223> Artificial sequence

<400> 20
gataatctct tatagaattg aaag 24

<210> 21
<211> 36
<212> DNA
<213> Artificial

<220>
<223> Artificial sequence

<400> 21
gtgctcaacg cctaacggca tcaaagttat attcag 36

<210> 22

<211> 25
<212> DNA
<213> Artificial

<220>
<223> Artificial sequence

<400> 22
ctttcaattc tctttgagtt gcaac 25

<210> 23
<211> 37
<212> DNA
<213> Artificial

<220>
<223> Artificial sequence

<400> 23
gcttcacatc cttcgatccg ataaaggata ctgaaag 37

<210> 24
<211> 25
<212> DNA
<213> Artificial

<220>
<223> Artificial sequence

<400> 24
gattaatcct aaaaggaatt gaaag 25

<210> 25
<211> 25
<212> DNA
<213> Artificial

<220>
<223> Artificial sequence

<400> 25
gcatatccct aaagggaatt gaaag 25

<210> 26
<211> 37
<212> DNA
<213> Artificial

<220>
<223> Artificial sequence

<400> 26
ctttcagtac cctccaagcg ggtcgagtcg ctgcaac 37

<210> 27
<211> 37
<212> DNA
<213> Artificial

<220>
<223> Artificial sequence

<400> 27
cttgcagtcc cctcctacgg ggcgatggcc ctgaaac 37

<210> 28
<211> 36
<212> DNA
<213> Artificial

<220>
<223> Artificial sequence

<400> 28
gtgctcaacg cctttcggca tcatcggaat tctcac 36

<210> 29
<211> 30
<212> DNA
<213> Artificial

<220>
<223> Artificial sequence

<400> 29
gtttcaattc cacactggtt cgattaaaag 30

<210> 30
<211> 30
<212> DNA
<213> Artificial

<220>
<223> Artificial sequence

<400> 30
atttcaattc cactatggtg cgattagaag 30

<210> 31
<211> 30
<212> DNA
<213> Artificial

<220>
<223> Artificial sequence

<400> 31
ctttcaattc tcctagagtc ttattgcaac 30

<210> 32
<211> 37
<212> DNA
<213> Artificial

<220>
<223> Artificial sequence

<400> 32
cttgcagtac cctcagacgg gtcgaaccca atgcaac 37

<210> 33
<211> 24
<212> DNA
<213> Artificial

<220>
<223> Artificial sequence

<400> 33
gttaatctac tatagaattg aaag 24

<210> 34
<211> 30
<212> DNA
<213> Artificial

<220>
<223> Artificial sequence

<400> 34
gtttcaattc cttataggta cagtgaaaac 30

<210> 35
<211> 37
<212> DNA
<213> Artificial

<220>
<223> Artificial sequence

<400> 35
gttgcaccga tctccccgat gaggagggga ctgaaac 37

<210> 36
<211> 25
<212> DNA
<213> Artificial

<220>
<223> Artificial sequence

<400> 36
ctttcaattc tttctgagtt gcatc 25

<210> 37
<211> 25
<212> DNA
<213> Artificial

<220>
<223> Artificial sequence

<400> 37
gaatctccct aaaggagta gaaag 25

<210> 38
<211> 24
<212> DNA
<213> Artificial

<220>
<223> Artificial sequence

<400> 38
gcatctcaaa aagaggattg aaag 24

<210> 39
<211> 25
<212> DNA
<213> Artificial

<220>
<223> Artificial sequence

<400> 39
ccttctattc tctttagaga atatc 25

<210> 40
<211> 24
<212> DNA
<213> Artificial

<220>
<223> Artificial sequence

<400> 40
gatgatctac tatagaattg aaag 24

<210> 41
<211> 37
<212> DNA
<213> Artificial

<220>
<223> Artificial sequence

<400> 41
gtttcaatcc ttgttttaat ggatcttgct ctcgaat 37

<210> 42
<211> 30
<212> DNA
<213> Artificial

<220>
<223> Artificial sequence

<400> 42
atttacattc caatctggtt agattacaat 30

<210> 43

<211> 29
<212> DNA
<213> Artificial

<220>
<223> Artificial sequence

<400> 43
atttacattt cataataagt agttaaaac 29

<210> 44
<211> 30
<212> DNA
<213> Artificial

<220>
<223> Artificial sequence

<400> 44
atttcaatcc caatatggtc tgattttaac 30

<210> 45
<211> 30
<212> DNA
<213> Artificial

<220>
<223> Artificial sequence

<400> 45
gtttcaattc cttataggta ctttataaac 30

<210> 46
<211> 30
<212> DNA
<213> Artificial

<220>
<223> Artificial sequence

<400> 46
gttaaaaagt aactatatta gtttttaaat 30

<210> 47
<211> 30
<212> DNA
<213> Artificial

<220>
<223> Artificial sequence

<400> 47
gtattaatag tagcaaagtg taatgtaaat 30

<210> 48
<211> 30
<212> DNA
<213> Artificial

<220>
<223> Artificial sequence

<400> 48
atttaagtag caccatattg gaatgtaaat 30

<210> 49
<211> 30
<212> DNA
<213> Artificial

<220>
<223> Artificial sequence

<400> 49
atttcaatac atctaatgtt actattcaac 30

<210> 50
<211> 29
<212> DNA
<213> Artificial

<220>
<223> Artificial sequence

<400> 50
gtattaattg taccttatgg aattgaaat 29

<210> 51
<211> 37
<212> DNA
<213> Artificial

<220>
<223> Artificial sequence

<400> 51
cttccagagt aacatccact agaataagga ttgaaac 37

<210> 52
<211> 30
<212> DNA
<213> Artificial

<220>
<223> Artificial sequence

<400> 52
gtattaattt aaatatatta gaatgtaaat 30

<210> 53
<211> 30
<212> DNA
<213> Artificial

<220>
<223> Artificial sequence

<400>  53
gtttcaaact ccaacggagt aaattctaat                                        30

<210>  54
<211>  30
<212>  DNA
<213>  Artificial

<220>
<223>  Artificial sequence

<400>  54
atttcaatac atcaaatgtg ttggttccgc                                        30

<210>  55
<211>  30
<212>  DNA
<213>  Artificial

<220>
<223>  Artificial sequence

<400>  55
atttcaaaac ttttcaagtt tatagttcat                                        30

<210>  56
<211>  30
<212>  DNA
<213>  Artificial

<220>
<223>  Artificial sequence

<400>  56
gtcctcatcg cccctacgag gggtagcaac                                        30

<210>  57
<211>  30
<212>  DNA
<213>  Artificial

<220>
<223>  Artificial sequence

<400>  57
gttaaacaat aacatgagat gttttgaaat                                        30

<210>  58
<211>  37
<212>  DNA
<213>  Artificial

<220>
<223>  Artificial sequence

<400>  58
ctttgaatta attgtccaac agaacaagga ttgaaac                                37

<210> 59
<211> 30
<212> DNA
<213> Artificial

<220>
<223> Artificial sequence

<400> 59
gtttcagacg aacccttgtg gggttgaagc 30

<210> 60
<211> 30
<212> DNA
<213> Artificial

<220>
<223> Artificial sequence

<400> 60
gtttgtagcc tgcctatgag ggattgaaac 30

<210> 61
<211> 30
<212> DNA
<213> Artificial

<220>
<223> Artificial sequence

<400> 61
gtttcaattc caatatggtt cgattaaaag 30

<210> 62
<211> 29
<212> DNA
<213> Artificial

<220>
<223> Artificial sequence

<400> 62
atttccatac tattgagtct attttaaac 29

<210> 63
<211> 30
<212> DNA
<213> Artificial

<220>
<223> Artificial sequence

<400> 63
gtttcaattg taccatacag gaattgaaac 30

<210> 64

<211> 30
<212> DNA
<213> Artificial

<220>
<223> Artificial sequence

<400> 64
ctagaaccga cacaaaagtt gtattgaaac 30

<210> 65
<211> 30
<212> DNA
<213> Artificial

<220>
<223> Artificial sequence

<400> 65
ctataaatct atccatattg gattttaaat 30

<210> 66
<211> 30
<212> DNA
<213> Artificial

<220>
<223> Artificial sequence

<400> 66
atttcaatcc tattatagtc tgattttaac 30

<210> 67
<211> 30
<212> DNA
<213> Artificial

<220>
<223> Artificial sequence

<400> 67
gtttccatcc cttataggaa gtttaaaaac 30

<210> 68
<211> 31
<212> DNA
<213> Artificial

<220>
<223> Artificial sequence

<400> 68
agtttcaata caccatgtgt tactgtcaaa c 31

<210> 69
<211> 30
<212> DNA
<213> Artificial

<220>
<223> Artificial sequence

<400> 69
gtttcaattc taccttagtt caattaaaag 30

<210> 70
<211> 29
<212> DNA
<213> Artificial

<220>
<223> Artificial sequence

<400> 70
ctttatatcc cactacgttc agataaaac 29

<210> 71
<211> 30
<212> DNA
<213> Artificial

<220>
<223> Artificial sequence

<400> 71
gttaaaatca gactatttta ggattgaaat 30

<210> 72
<211> 30
<212> DNA
<213> Artificial

<220>
<223> Artificial sequence

<400> 72
gtttttagcc tacctatgag gaattgaaac 30

<210> 73
<211> 30
<212> DNA
<213> Artificial

<220>
<223> Artificial sequence

<400> 73
atttacattt ctcttaagtt aaataaaaac 30

<210> 74
<211> 30
<212> DNA
<213> Artificial

<220>
<223> Artificial sequence

<400> 74
atttcaattc cactatggtt cgattaaaag 30

<210> 75
<211> 30
<212> DNA
<213> Artificial

<220>
<223> Artificial sequence

<400> 75
attaaagtag aaccatattg gaatgtaaag 30

<210> 76
<211> 29
<212> DNA
<213> Artificial

<220>
<223> Artificial sequence

<400> 76
gtcgtcatca gccctggagg gttcgcaac 29

<210> 77
<211> 30
<212> DNA
<213> Artificial

<220>
<223> Artificial sequence

<400> 77
gtttgctaat gacaatattt gtgttgaaac 30

<210> 78
<211> 30
<212> DNA
<213> Artificial

<220>
<223> Artificial sequence

<400> 78
atcttaatcg taccagagtg gaattgaaat 30

<210> 79
<211> 37
<212> DNA
<213> Artificial

<220>
<223> Artificial sequence

<400> 79
gtttcaatcc ttcttatcat ggatggtggt cagtaag 37

<210> 80
<211> 30
<212> DNA
<213> Artificial

<220>
<223> Artificial sequence

<400> 80
gattaacttt aacatgagat gtatttaaat 30

<210> 81
<211> 30
<212> DNA
<213> Artificial

<220>
<223> Artificial sequence

<400> 81
gtttgtgacc cgcatatagg ggattgaaac 30

<210> 82
<211> 30
<212> DNA
<213> Artificial

<220>
<223> Artificial sequence

<400> 82
atttaaattc taatatagaa atacataaat 30

<210> 83
<211> 30
<212> DNA
<213> Artificial

<220>
<223> Artificial sequence

<400> 83
gtttgtatcg tacctatgag gaattgaaac 30

<210> 84
<211> 30
<212> DNA
<213> Artificial

<220>
<223> Artificial sequence

<400> 84
ctcataatcg aagcagactg aaatttaaat 30

<210> 85

<211> 30
<212> DNA
<213> Artificial

<220>
<223> Artificial sequence

<400> 85
cttaaagtat tatcatattg gaatttaaat 30

<210> 86
<211> 30
<212> DNA
<213> Artificial

<220>
<223> Artificial sequence

<400> 86
gataaacaat aacaagagtt gtattgaaat 30

<210> 87
<211> 30
<212> DNA
<213> Artificial

<220>
<223> Artificial sequence

<400> 87
attagagtat tactaaagta gaatgtaaat 30

<210> 88
<211> 30
<212> DNA
<213> Artificial

<220>
<223> Artificial sequence

<400> 88
gttgcaataa gactctagga gaattgaaag 30

<210> 89
<211> 30
<212> DNA
<213> Artificial

<220>
<223> Artificial sequence

<400> 89
atttacattc tactgtagtt ctattaaagg 30

<210> 90
<211> 30
<212> DNA
<213> Artificial

<220>
<223> Artificial sequence

<400> 90
gttttgatcg tacctatgag ggattgaaac 30

<210> 91
<211> 30
<212> DNA
<213> Artificial

<220>
<223> Artificial sequence

<400> 91
gttataatcg tactaatgta gaattgaaac 30

<210> 92
<211> 30
<212> DNA
<213> Artificial

<220>
<223> Artificial sequence

<400> 92
cttgaaccaa aacatatgat gtattgaaat 30

<210> 93
<211> 30
<212> DNA
<213> Artificial

<220>
<223> Artificial sequence

<400> 93
atttacattc cactcttttt agatttaaac 30

<210> 94
<211> 30
<212> DNA
<213> Artificial

<220>
<223> Artificial sequence

<400> 94
gttgttagct tacctataag gaattgaaac 30

<210> 95
<211> 30
<212> DNA
<213> Artificial

<220>
<223> Artificial sequence

<400> 95
gttgaacctt aacaagggtt gtatttaaat 30

<210> 96
<211> 30
<212> DNA
<213> Artificial

<220>
<223> Artificial sequence

<400> 96
cttttaatcg caccatatag gaattgaaat 30

<210> 97
<211> 29
<212> DNA
<213> Artificial

<220>
<223> Artificial sequence

<400> 97
atttacatac cacttagtta atataaaac 29

<210> 98
<211> 30
<212> DNA
<213> Artificial

<220>
<223> Artificial sequence

<400> 98
gtattaatag atacattgtg gaatgtaaag 30

<210> 99
<211> 30
<212> DNA
<213> Artificial

<220>
<223> Artificial sequence

<400> 99
atttatattt aacaagcatt gtttttaaag 30

<210> 100
<211> 30
<212> DNA
<213> Artificial

<220>
<223> Artificial sequence

<400> 100
gttacagacg aaccctagtt gggttgaagc 30

<210> 101
<211> 30
<212> DNA
<213> Artificial

<220>
<223> Artificial sequence

<400> 101
gttgaaatcg gaccaatgtg ggattgaaac 30

<210> 102
<211> 29
<212> DNA
<213> Artificial

<220>
<223> Artificial sequence

<400> 102
gttctaatcg aactatatag aattgaaat 29

<210> 103
<211> 30
<212> DNA
<213> Artificial

<220>
<223> Artificial sequence

<400> 103
ctttacattc cactctggta cgattctaat 30

<210> 104
<211> 29
<212> DNA
<213> Artificial

<220>
<223> Artificial sequence

<400> 104
atttataact aacttagtgt aatttaaac 29

<210> 105
<211> 30
<212> DNA
<213> Artificial

<220>
<223> Artificial sequence

<400> 105
gtttttagtc tacctatgag gaattgaaac 30

<210> 106

<211> 30
<212> DNA
<213> Artificial

<220>
<223> Artificial sequence

<400> 106
atttaaatac atcctatgtt aaggttcaac 30

<210> 107
<211> 30
<212> DNA
<213> Artificial

<220>
<223> Artificial sequence

<400> 107
ctttgaatct aaccatattg gaatgtaaat 30

<210> 108
<211> 30
<212> DNA
<213> Artificial

<220>
<223> Artificial sequence

<400> 108
attcaaaaac aacttttgtt actgttaatc 30

<210> 109
<211> 29
<212> DNA
<213> Artificial

<220>
<223> Artificial sequence

<400> 109
cctttaattg taccttatgg aattgaaac 29

<210> 110
<211> 30
<212> DNA
<213> Artificial

<220>
<223> Artificial sequence

<400> 110
gtttaaattc cacactggtt caatgctcac 30

<210> 111
<211> 37
<212> DNA
<213> Artificial

<220>
<223> Artificial sequence

<400> 111
gtttaaatcc taaatccatt aaaacaagga ttgaaac 37

<210> 112
<211> 30
<212> DNA
<213> Artificial

<220>
<223> Artificial sequence

<400> 112
ctcccaatcg caccatactg gaattgaaat 30

<210> 113
<211> 30
<212> DNA
<213> Artificial

<220>
<223> Artificial sequence

<400> 113
atttcaaaac atcccatgtt aaagttaaac 30

<210> 114
<211> 30
<212> DNA
<213> Artificial

<220>
<223> Artificial sequence

<400> 114
gtttgtagcc ttccctttgg ggattgaaac 30

<210> 115
<211> 37
<212> DNA
<213> Artificial

<220>
<223> Artificial sequence

<400> 115
gtcgaagagc cacatccacg aaaacaagga ttgaaac 37

<210> 116
<211> 30
<212> DNA
<213> Artificial

<220>
<223> Artificial sequence

<400>  116
atttcaaaac atttcatgtt tacggttaat                                        30

<210>  117
<211>  37
<212>  DNA
<213>  Artificial

<220>
<223>  Artificial sequence

<400>  117
ctttcagtcc cctctaacgg ggcaatggcc ctgcaac                                37

<210>  118
<211>  29
<212>  DNA
<213>  Artificial

<220>
<223>  Artificial sequence

<400>  118
gtttttatct tcctaagagg aatatgaac                                         29

<210>  119
<211>  29
<212>  DNA
<213>  Artificial

<220>
<223>  Artificial sequence

<400>  119
ctttataact aacatagttc tattaatac                                         29

<210>  120
<211>  29
<212>  DNA
<213>  Artificial

<220>
<223>  Artificial sequence

<400>  120
gttcaaattc ctctatggtc gatggtcac                                         29

<210>  121
<211>  29
<212>  DNA
<213>  Artificial

<220>
<223>  Artificial sequence

<400>  121
gtatcaatcg caccttcggg tattgaaat                                         29

<210> 122
<211> 29
<212> DNA
<213> Artificial

<220>
<223> Artificial sequence

<400> 122
ctttaaatcc cacaattggg ggctaaaac 29

<210> 123
<211> 30
<212> DNA
<213> Artificial

<220>
<223> Artificial sequence

<400> 123
gtttcaattc ccaacgggat ggaattctac 30

<210> 124
<211> 29
<212> DNA
<213> Artificial

<220>
<223> Artificial sequence

<400> 124
gttttatctg aactatgagg gatgtaaac 29

<210> 125
<211> 29
<212> DNA
<213> Artificial

<220>
<223> Artificial sequence

<400> 125
ctttccacac aattctgttc tacggaaac 29

<210> 126
<211> 37
<212> DNA
<213> Artificial

<220>
<223> Artificial sequence

<400> 126
gtttcaatcc ttgttgttct ggatatagct ctgaaag 37

<210> 127

<211> 27
<212> DNA
<213> Artificial

<220>
<223> Artificial sequence

<400> 127
ggggaattaa tagatacata gtgggat 27

<210> 128
<211> 29
<212> DNA
<213> Artificial

<220>
<223> Artificial sequence

<400> 128
gtttctaatt aaccgtgtgg agttgaaag 29

<210> 129
<211> 37
<212> DNA
<213> Artificial

<220>
<223> Artificial sequence

<400> 129
gttaaagagt aagttccatt aaaacaagga ttaaaac 37

<210> 130
<211> 37
<212> DNA
<213> Artificial

<220>
<223> Artificial sequence

<400> 130
gtttcaatgc ttgttatttt gcaagttact cttttag 37

<210> 131
<211> 37
<212> DNA
<213> Artificial

<220>
<223> Artificial sequence

<400> 131
gtttcagtgc tctaccgtga gccgaaattg ttgaaag 37

<210> 132
<211> 30
<212> DNA
<213> Artificial

<220>
<223> Artificial sequence

<400> 132
gttttaataa tacctatgag ggattgaaac 30

<210> 133
<211> 30
<212> DNA
<213> Artificial

<220>
<223> Artificial sequence

<400> 133
gtttcaatcc ttattgggtt cgattaggac 30

<210> 134
<211> 36
<212> DNA
<213> Artificial

<220>
<223> Artificial sequence

<400> 134
gtgctcaacg cctttcggca tcagaggttt ttgcac 36

<210> 135
<211> 30
<212> DNA
<213> Artificial

<220>
<223> Artificial sequence

<400> 135
gttaaaatca gaccttagag ggattgaaac 30

<210> 136
<211> 36
<212> DNA
<213> Artificial

<220>
<223> Artificial sequence

<400> 136
ccgcaccgat ctccgatgcc gaaaggcgtt gagcac 36

<210> 137
<211> 37
<212> DNA
<213> Artificial

<220>
<223> Artificial sequence

<400> 137
gttttaatct ccgttggagc gacgttgggg tgggaag 37

<210> 138
<211> 37
<212> DNA
<213> Artificial

<220>
<223> Artificial sequence

<400> 138
gtcaaaatga caaatccatc ataacaagga ttgaaac 37

<210> 139
<211> 35
<212> DNA
<213> Artificial

<220>
<223> Artificial sequence

<400> 139
attgcaattt aaaaatccct attagggatt gaaac 35

<210> 140
<211> 36
<212> DNA
<213> Artificial

<220>
<223> Artificial sequence

<400> 140
ctgaatataa ctttgatgcc gttaggcgtt gagcac 36

<210> 141
<211> 30
<212> DNA
<213> Artificial

<220>
<223> Artificial sequence

<400> 141
atttcaattc cactaaggtt caattataac 30

<210> 142
<211> 34
<212> DNA
<213> Artificial

<220>
<223> Artificial sequence

<400> 142
atttcaaaac atcacgacca agaggatact aaaa 34

<210> 143
<211> 30
<212> DNA
<213> Artificial

<220>
<223> Artificial sequence

<400> 143
gtttcaaaat ccataggatc aaattgaaac 30

<210> 144
<211> 34
<212> DNA
<213> Artificial

<220>
<223> Artificial sequence

<400> 144
tagcaacgcc taacggcata aagccacgaa tcac 34

<210> 145
<211> 36
<212> DNA
<213> Artificial

<220>
<223> Artificial sequence

<400> 145
gtgctcaacg cctttcggca tcattggaat cctcac 36

<210> 146
<211> 36
<212> DNA
<213> Artificial

<220>
<223> Artificial sequence

<400> 146
gtttcaatcc tctacgaggc taacgaggtt tgcaac 36

<210> 147
<211> 37
<212> DNA
<213> Artificial

<220>
<223> Artificial sequence

<400> 147
cttccagatg catccaaata gaaactagga ttgaaac 37

<210> 148

<211> 36
<212> DNA
<213> Artificial

<220>
<223> Artificial sequence

<400> 148
gtgctcaacg ccttccggca tcacagcggg cgtcac 36

<210> 149
<211> 36
<212> DNA
<213> Artificial

<220>
<223> Artificial sequence

<400> 149
gtgttttaac cttagatgcc ataaggcgtt gatcac 36

<210> 150
<211> 30
<212> DNA
<213> Artificial

<220>
<223> Artificial sequence

<400> 150
ctttacattc cactatgttt ctattaattc 30

<210> 151
<211> 32
<212> DNA
<213> Artificial

<220>
<223> Artificial sequence

<400> 151
gtcgcccccc gcgcgggggc gtggattgaa ac 32

<210> 152
<211> 33
<212> DNA
<213> Artificial

<220>
<223> Artificial sequence

<400> 152
atttcaatcc acgctctcca tgaggagagc gac 33

<210> 153
<211> 37
<212> DNA
<213> Artificial

<220>
<223> Artificial sequence

<400> 153
gtagcgcccg gcctcaaagc cgggcgagga ttgaaac 37

<210> 154
<211> 32
<212> DNA
<213> Artificial

<220>
<223> Artificial sequence

<400> 154
atttcaatcc acgcacccgc aaagggtgcg ac 32

<210> 155
<211> 33
<212> DNA
<213> Artificial

<220>
<223> Artificial sequence

<400> 155
atttcaatcc acgcgcccct tgcggggcgc gac 33

<210> 156
<211> 32
<212> DNA
<213> Artificial

<220>
<223> Artificial sequence

<400> 156
atttaaatcc actagcctca gggaggctag ac 32

<210> 157
<211> 33
<212> DNA
<213> Artificial

<220>
<223> Artificial sequence

<400> 157
atttcaaccc actccgcccg cgaggacgga gac 33

<210> 158
<211> 32
<212> DNA
<213> Artificial

<220>
<223> Artificial sequence

<400> 158
gtttcaacac acagccgccc gaaggcggct gc 32

<210> 159
<211> 32
<212> DNA
<213> Artificial

<220>
<223> Artificial sequence

<400> 159
atttcaactc acagcctcac gaagaggctg ac 32

<210> 160
<211> 33
<212> DNA
<213> Artificial

<220>
<223> Artificial sequence

<400> 160
gtcacacctc gcatgaggtg tgtgagtaga aat 33

<210> 161
<211> 33
<212> DNA
<213> Artificial

<220>
<223> Artificial sequence

<400> 161
gtttcaattc acgcagccca agcgggctgc gac 33

<210> 162
<211> 32
<212> DNA
<213> Artificial

<220>
<223> Artificial sequence

<400> 162
gtcactccct tcgcgggagt gtggattgaa at 32

<210> 163
<211> 32
<212> DNA
<213> Artificial

<220>
<223> Artificial sequence

<400> 163
gtttcaatcc gcaggctcgc gagagcctga tc 32

<210> 164
<211> 32
<212> DNA
<213> Artificial

<220>
<223> Artificial sequence

<400> 164
gtttcaatcc acacacccac gtagggtgtg ac 32

<210> 165
<211> 32
<212> DNA
<213> Artificial

<220>
<223> Artificial sequence

<400> 165
gtcgtacctt atataggtac gtggattgaa ac 32

<210> 166
<211> 32
<212> DNA
<213> Artificial

<220>
<223> Artificial sequence

<400> 166
atttcaattc acgcctctct gcgagaggct ac 32

<210> 167
<211> 33
<212> DNA
<213> Artificial

<220>
<223> Artificial sequence

<400> 167
atttcaatcc actagctcta aacagagcta gac 33

<210> 168
<211> 33
<212> DNA
<213> Artificial

<220>
<223> Artificial sequence

<400> 168
atttcaatcc ccgccttccg tgaggaaggc gac 33

<210> 169

<211> 32
<212> DNA
<213> Artificial

<220>
<223> Artificial sequence

<400> 169
gtttcaattc acgcacccgc gaggggtgcg ac 32

<210> 170
<211> 33
<212> DNA
<213> Artificial

<220>
<223> Artificial sequence

<400> 170
gtcgcacccc acacgggttg cgtggattga aat 33

<210> 171
<211> 37
<212> DNA
<213> Artificial

<220>
<223> Artificial sequence

<400> 171
gattcgcccg ccagaaatgg cgggcgcgga ttgaaac 37

<210> 172
<211> 32
<212> DNA
<213> Artificial

<220>
<223> Artificial sequence

<400> 172
gtcacttccc gtgcgggagt gtggattgaa at 32

<210> 173
<211> 32
<212> DNA
<213> Artificial

<220>
<223> Artificial sequence

<400> 173
gtcaccctcc gtgcgagggt gtggattgaa at 32

<210> 174
<211> 32
<212> DNA
<213> Artificial

<220>
<223> Artificial sequence

<400> 174
gtttcaattc acggtcgcgc gtgaggcgac cg 32

<210> 175
<211> 33
<212> DNA
<213> Artificial

<220>
<223> Artificial sequence

<400> 175
gtcactcccc gcatggggag tgcgggttga aat 33

<210> 176
<211> 32
<212> DNA
<213> Artificial

<220>
<223> Artificial sequence

<400> 176
gtttcaattc gcgctcccgc gtggggagcg ac 32

<210> 177
<211> 33
<212> DNA
<213> Artificial

<220>
<223> Artificial sequence

<400> 177
atttcaatcc acagccccat cgcagagact gac 33

<210> 178
<211> 32
<212> DNA
<213> Artificial

<220>
<223> Artificial sequence

<400> 178
atttcaatcc acaccactgc atcagtggtg ac 32

<210> 179
<211> 32
<212> DNA
<213> Artificial

<220>
<223> Artificial sequence

<400> 179
atttcaactc acacgctcac gtggagcgtg ac 32

<210> 180
<211> 33
<212> DNA
<213> Artificial

<220>
<223> Artificial sequence

<400> 180
atttcaatcc acgcacctcg tgtgaggtgc gac 33

<210> 181
<211> 32
<212> DNA
<213> Artificial

<220>
<223> Artificial sequence

<400> 181
gtttcgatcc acaggctctt acgagcctga cc 32

<210> 182
<211> 31
<212> DNA
<213> Artificial

<220>
<223> Artificial sequence

<400> 182
cagccaccta cgcgtggctg tgaattgaaa c 31

<210> 183
<211> 33
<212> DNA
<213> Artificial

<220>
<223> Artificial sequence

<400> 183
gtcgaggtct gcgaaggcct cgtggattga aat 33

<210> 184
<211> 33
<212> DNA
<213> Artificial

<220>
<223> Artificial sequence

<400> 184
atttctactc acacgcccct cgcggggcgt gac 33

<210> 185
<211> 33
<212> DNA
<213> Artificial

<220>
<223> Artificial sequence

<400> 185
atttcaatcc acactctcta tgtagagagt gac 33

<210> 186
<211> 32
<212> DNA
<213> Artificial

<220>
<223> Artificial sequence

<400> 186
ttttcaactc acgacctctc gtggaggtcg ac 32

<210> 187
<211> 32
<212> DNA
<213> Artificial

<220>
<223> Artificial sequence

<400> 187
atttcaattc atgcccctgc atgaggggca gc 32

<210> 188
<211> 30
<212> DNA
<213> Artificial

<220>
<223> Artificial sequence

<400> 188
atttcaactc acgccctcac ggagggcgac 30

<210> 189
<211> 32
<212> DNA
<213> Artificial

<220>
<223> Artificial sequence

<400> 189
cttaaatcca ccccgcccat gcgggcgggg ac 32

<210> 190

<211> 32
<212> DNA
<213> Artificial

<220>
<223> Artificial sequence

<400> 190
gtcgttcccc acgtgggaac gtggattgaa at 32

<210> 191
<211> 33
<212> DNA
<213> Artificial

<220>
<223> Artificial sequence

<400> 191
gtttcaactc gcgcgccccg gggggggtgc gac 33

<210> 192
<211> 33
<212> DNA
<213> Artificial

<220>
<223> Artificial sequence

<400> 192
gtcgcactcc gcaaggagtg cgtggattga aac 33

<210> 193
<211> 37
<212> DNA
<213> Artificial

<220>
<223> Artificial sequence

<400> 193
gtttcaatca gcccccgagg gtgaggtcgg gggagac 37

<210> 194
<211> 32
<212> DNA
<213> Artificial

<220>
<223> Artificial sequence

<400> 194
ccagctacgt acgcgtagct gtgagttgaa ac 32

<210> 195
<211> 32
<212> DNA
<213> Artificial

<220>
<223>  Artificial sequence

<400>  195
gatgcccccc acacgggggc acgacttgaa ac                                    32

<210>  196
<211>  32
<212>  DNA
<213>  Artificial

<220>
<223>  Artificial sequence

<400>  196
gtcgcactct atgtgagtgc gtggattgaa at                                    32

<210>  197
<211>  32
<212>  DNA
<213>  Artificial

<220>
<223>  Artificial sequence

<400>  197
gtttcgatcc gcgctcccgc gtggggagcg ac                                    32

<210>  198
<211>  37
<212>  DNA
<213>  Artificial

<220>
<223>  Artificial sequence

<400>  198
gtttcaatcc gcatcccgac cggaggtcgg aagtaac                               37

<210>  199
<211>  30
<212>  DNA
<213>  Artificial

<220>
<223>  Artificial sequence

<400>  199
atttcaattc cacaaaggtg cgattaaaag                                       30

<210>  200
<211>  30
<212>  DNA
<213>  Artificial

<220>
<223>  Artificial sequence

<400> 200
atttcaattg caccatacag gaattaaaac 30

<210> 201
<211> 30
<212> DNA
<213> Artificial

<220>
<223> Artificial sequence

<400> 201
gtttcaattc cacattggtt cgattaaaag 30

<210> 202
<211> 29
<212> DNA
<213> Artificial

<220>
<223> Artificial sequence

<400> 202
cccttaatcg caccgtatgg aattgaaat 29

<210> 203
<211> 37
<212> DNA
<213> Artificial

<220>
<223> Artificial sequence

<400> 203
gtagcacccc ccctcacggg ggggtgagga ttgaaat 37

<210> 204
<211> 37
<212> DNA
<213> Artificial

<220>
<223> Artificial sequence

<400> 204
gtcgcacccg gccgcaaggc cgggtgagga ttgaaac 37

<210> 205
<211> 37
<212> DNA
<213> Artificial

<220>
<223> Artificial sequence

<400> 205
gtttcaatcc tcacccggcg cgctgaccgg gtgcaac 37

<210> 206
<211> 37
<212> DNA
<213> Artificial

<220>
<223> Artificial sequence

<400> 206
gtggcggtcg ccctccgggg cggccgagga tcgcaac 37

<210> 207
<211> 32
<212> DNA
<213> Artificial

<220>
<223> Artificial sequence

<400> 207
gtttcaatcc acgctcccgc gaggggagcg ac 32

<210> 208
<211> 37
<212> DNA
<213> Artificial

<220>
<223> Artificial sequence

<400> 208
gtttcaaccc acccccgct tcgtcagcga ggggaac 37

<210> 209
<211> 32
<212> DNA
<213> Artificial

<220>
<223> Artificial sequence

<400> 209
gtttcaatcc acgcgcccgc gtggggcgcg ac 32

<210> 210
<211> 37
<212> DNA
<213> Artificial

<220>
<223> Artificial sequence

<400> 210
gtttcaatcc tcgcccgccc cgaaggacgg gcgctac 37

<210> 211

<211> 33
<212> DNA
<213> Artificial

<220>
<223> Artificial sequence

<400> 211
gtttcaatcc actccccccg agcgagggga gat 33

<210> 212
<211> 37
<212> DNA
<213> Artificial

<220>
<223> Artificial sequence

<400> 212
gttgcgatcc tcaccggccc cggagggccg gtgccac 37

<210> 213
<211> 33
<212> DNA
<213> Artificial

<220>
<223> Artificial sequence

<400> 213
gtttcaatcc actcccgtat caccaggggg aac 33

<210> 214
<211> 32
<212> DNA
<213> Artificial

<220>
<223> Artificial sequence

<400> 214
gtcgcacccc tcgcgggtgc gtggattgaa ac 32

<210> 215
<211> 33
<212> DNA
<213> Artificial

<220>
<223> Artificial sequence

<400> 215
gtttcaatcc acgccccccg cgggggcgc gac 33

<210> 216
<211> 33
<212> DNA
<213> Artificial

<220>
<223> Artificial sequence

<400> 216
gtttctatcc gcacgccccg ggcggggcgc gac 33

<210> 217
<211> 32
<212> DNA
<213> Artificial

<220>
<223> Artificial sequence

<400> 217
gtcgccccct tcgcgggggc gtggattgaa ac 32

<210> 218
<211> 37
<212> DNA
<213> Artificial

<220>
<223> Artificial sequence

<400> 218
gtttcaatcc tcacccggct ttatagccgg gtgcaac 37

<210> 219
<211> 33
<212> DNA
<213> Artificial

<220>
<223> Artificial sequence

<400> 219
gtcgctcccc gcaaggaggg cgtggattga aac 33

<210> 220
<211> 37
<212> DNA
<213> Artificial

<220>
<223> Artificial sequence

<400> 220
gttgcgatcc tcgcccggcg cgatggccgg gcgcagc 37

<210> 221
<211> 32
<212> DNA
<213> Artificial

<220>
<223> Artificial sequence

<400> 221
atttcaatcc acgctcccgt gaggggagcg ac 32

<210> 222
<211> 37
<212> DNA
<213> Artificial

<220>
<223> Artificial sequence

<400> 222
gcagcgccgg gcctccgcgc ccggcgagga tcgcaac 37

<210> 223
<211> 37
<212> DNA
<213> Artificial

<220>
<223> Artificial sequence

<400> 223
gtttcaatcc tcaattacct taaatagtaa ttgtcac 37

<210> 224
<211> 32
<212> DNA
<213> Artificial

<220>
<223> Artificial sequence

<400> 224
gtcgcccccc acacgggggc gtggattgaa ac 32

<210> 225
<211> 35
<212> DNA
<213> Artificial

<220>
<223> Artificial sequence

<400> 225
atcgcacctc ctccgggccg ggtgaggatt gaaac 35

<210> 226
<211> 37
<212> DNA
<213> Artificial

<220>
<223> Artificial sequence

<400> 226
gtttcaatcc acgcccgtta ttgctaacgg gcgaatc 37

<210> 227
<211> 32
<212> DNA
<213> Artificial

<220>
<223> Artificial sequence

<400> 227
attttcaattc acactctcgt aaagagagtg ac 32

<210> 228
<211> 32
<212> DNA
<213> Artificial

<220>
<223> Artificial sequence

<400> 228
gtcgcgcccc gcgtgggcgc gtggattgaa ac 32

<210> 229
<211> 31
<212> DNA
<213> Artificial

<220>
<223> Artificial sequence

<400> 229
gtcgcgcccc cacgggcgcg tggattgaaa c 31

<210> 230
<211> 33
<212> DNA
<213> Artificial

<220>
<223> Artificial sequence

<400> 230
atttctatcc acgccctccg cgaggagggc gac 33

<210> 231
<211> 37
<212> DNA
<213> Artificial

<220>
<223> Artificial sequence

<400> 231
gtttcaatcc tcgcccagcc cgttggccgg gcgctac 37

<210> 232

<211> 32
<212> DNA
<213> Artificial

<220>
<223> Artificial sequence

<400> 232
gtcaccttcc aaagggaggt gtggattgaa at 32

<210> 233
<211> 37
<212> DNA
<213> Artificial

<220>
<223> Artificial sequence

<400> 233
gtttcaatcc tcaccggccc tttcgggccg gtgctac 37

<210> 234
<211> 33
<212> DNA
<213> Artificial

<220>
<223> Artificial sequence

<400> 234
gtcgccccct gcgagggagg cgtggattga aac 33

<210> 235
<211> 33
<212> DNA
<213> Artificial

<220>
<223> Artificial sequence

<400> 235
atttcaatcc acgcccccg cgagggggc gac 33

<210> 236
<211> 35
<212> DNA
<213> Artificial

<220>
<223> Artificial sequence

<400> 236
gtttcaatcc acgcccgtca tcctgacggg cgagc 35

<210> 237
<211> 33
<212> DNA
<213> Artificial

<220>
<223>  Artificial sequence

<400>  237
cgcccgcccc tacacgcggg cgcggattga aac                                    33

<210>  238
<211>  33
<212>  DNA
<213>  Artificial

<220>
<223>  Artificial sequence

<400>  238
gttgcactcc actcggagtg catgaattga aat                                    33

<210>  239
<211>  31
<212>  DNA
<213>  Artificial

<220>
<223>  Artificial sequence

<400>  239
gtttcaatcc acgcgcctgt gaaggcgcga c                                      31

<210>  240
<211>  32
<212>  DNA
<213>  Artificial

<220>
<223>  Artificial sequence

<400>  240
atttcaatcc acaccaccgt gtagggggtg ac                                     32

<210>  241
<211>  32
<212>  DNA
<213>  Artificial

<220>
<223>  Artificial sequence

<400>  241
gtcgctcccc tcgtgggagc gtggattgaa at                                     32

<210>  242
<211>  34
<212>  DNA
<213>  Artificial

<220>
<223>  Artificial sequence

<400> 242
tgtagttccc ggttattact tggtatgtta taat 34

<210> 243
<211> 32
<212> DNA
<213> Artificial

<220>
<223> Artificial sequence

<400> 243
atttcaatcc actcacccat gaagggtgag ac 32

<210> 244
<211> 33
<212> DNA
<213> Artificial

<220>
<223> Artificial sequence

<400> 244
gtttcaatcc acgcactccg tgcagagtgc gac 33

<210> 245
<211> 37
<212> DNA
<213> Artificial

<220>
<223> Artificial sequence

<400> 245
gtttcaatcc ctatcgggtt tttttatccg ttgcgac 37

<210> 246
<211> 37
<212> DNA
<213> Artificial

<220>
<223> Artificial sequence

<400> 246
gtttcaatcc ctaataggga ttaagattaa ttgcaac 37

<210> 247
<211> 28
<212> DNA
<213> Artificial

<220>
<223> Artificial sequence

<400> 247
ctcacaatcc ctcatgggga tagataac 28

<210> 248
<211> 28
<212> DNA
<213> Artificial

<220>
<223> Artificial sequence

<400> 248
gttccgaatc cccatgaggg gttatgag 28

<210> 249
<211> 28
<212> DNA
<213> Artificial

<220>
<223> Artificial sequence

<400> 249
gtcctctctc cccatgaggg gtcaggag 28

<210> 250
<211> 24
<212> DNA
<213> Artificial

<220>
<223> Artificial sequence

<400> 250
gtaacaacga caagaaacta aaac 24

<210> 251
<211> 37
<212> DNA
<213> Artificial

<220>
<223> Artificial sequence

<400> 251
gtttcaatcc ctatcgggtt ttcttatccg ttgcgac 37

<210> 252
<211> 37
<212> DNA
<213> Artificial

<220>
<223> Artificial sequence

<400> 252
gtggcaaagg cattgagcgc gactgagcga ttgaaag 37

<210> 253

<211> 37
<212> DNA
<213> Artificial

<220>
<223> Artificial sequence

<400> 253
gcgaaaatcc ccaataatcc ctttgaggga ttgaaac 37

<210> 254
<211> 37
<212> DNA
<213> Artificial

<220>
<223> Artificial sequence

<400> 254
gttgcagcgc acttgaaaac ccgacaggga ttgaaac 37

<210> 255
<211> 37
<212> DNA
<213> Artificial

<220>
<223> Artificial sequence

<400> 255
gtttcaatct ctaacgagtt ttctaaccga ttgcgat 37

<210> 256
<211> 37
<212> DNA
<213> Artificial

<220>
<223> Artificial sequence

<400> 256
gtttcagtcc ccttgcgggg attaggtaag tggaaac 37

<210> 257
<211> 37
<212> DNA
<213> Artificial

<220>
<223> Artificial sequence

<400> 257
gttggagagg caattgtcgc gcttgagcga ttgaaac 37

<210> 258
<211> 37
<212> DNA
<213> Artificial

<220>
<223> Artificial sequence

<400> 258
gttgaaatta cctttaatcc ctatgaggga ttgaaac 37

<210> 259
<211> 37
<212> DNA
<213> Artificial

<220>
<223> Artificial sequence

<400> 259
gtcggaatgg cgctaagccc gaatgaggga ttgaaac 37

<210> 260
<211> 37
<212> DNA
<213> Artificial

<220>
<223> Artificial sequence

<400> 260
gtttcaatcc ctgaaagggt ttcaggttcg ttgaagc 37

<210> 261
<211> 37
<212> DNA
<213> Artificial

<220>
<223> Artificial sequence

<400> 261
ctttcaatcc cgcactcggg cttatccatc ttctgac 37

<210> 262
<211> 37
<212> DNA
<213> Artificial

<220>
<223> Artificial sequence

<400> 262
gttgcaatcc ctcaatcggg ctttggcctc ttctgac 37

<210> 263
<211> 37
<212> DNA
<213> Artificial

<220>
<223> Artificial sequence

<400> 263
gtttcaatcc cgttctgggt tttctgggtg tcgcgac 37

<210> 264
<211> 37
<212> DNA
<213> Artificial

<220>
<223> Artificial sequence

<400> 264
gtttcaatcc gccgggcgga tttttggctt ttggaac 37

<210> 265
<211> 37
<212> DNA
<213> Artificial

<220>
<223> Artificial sequence

<400> 265
gtttcaatcc cgcgaacggg tttcatttat ttgaaag 37

<210> 266
<211> 37
<212> DNA
<213> Artificial

<220>
<223> Artificial sequence

<400> 266
gttgcaactt ttattaatcc ctattaggga ttgaaac 37

<210> 267
<211> 37
<212> DNA
<213> Artificial

<220>
<223> Artificial sequence

<400> 267
gtcagaaggg ccctaagccc gaacgcggga ttgaaac 37

<210> 268
<211> 37
<212> DNA
<213> Artificial

<220>
<223> Artificial sequence

<400> 268
gtttcaatta acataaatcc ctatcaggga ttgaaac 37

<210> 269
<211> 37
<212> DNA
<213> Artificial

<220>
<223> Artificial sequence

<400> 269
gtttcaatcc cattactagg attcattaaa aagaaac 37

<210> 270
<211> 37
<212> DNA
<213> Artificial

<220>
<223> Artificial sequence

<400> 270
gtttaaatta ttattaatac ctatcaggga ttgaaac 37

<210> 271
<211> 37
<212> DNA
<213> Artificial

<220>
<223> Artificial sequence

<400> 271
gtcgcgacgc ggtagaaacc cagaacggga ttgaaac 37

<210> 272
<211> 37
<212> DNA
<213> Artificial

<220>
<223> Artificial sequence

<400> 272
gtttcaatcc ctaataggga gtaagtgaaa ttgcaat 37

<210> 273
<211> 37
<212> DNA
<213> Artificial

<220>
<223> Artificial sequence

<400> 273
gttcaaattt agacttatct ccataggaga ttgaaac 37

<210> 274

<211> 37
<212> DNA
<213> Artificial

<220>
<223> Artificial sequence

<400> 274
gtttcaatct caaccgagat gccaggcccc tggcgac 37

<210> 275
<211> 37
<212> DNA
<213> Artificial

<220>
<223> Artificial sequence

<400> 275
ctttccaacc actaaatccc gattacggga ctgaaac 37

<210> 276
<211> 37
<212> DNA
<213> Artificial

<220>
<223> Artificial sequence

<400> 276
ctttcagtcc ccttgtcatc ggggaaagta gtgcaac 37

<210> 277
<211> 37
<212> DNA
<213> Artificial

<220>
<223> Artificial sequence

<400> 277
gtttcagtcc cgcaagcagg attattttaa ttgaaag 37

<210> 278
<211> 30
<212> DNA
<213> Artificial

<220>
<223> Artificial sequence

<400> 278
gttaaaatag aaccttaata ggattgaaag 30

<210> 279
<211> 28
<212> DNA
<213> Artificial

<220>
<223>  Artificial sequence

<400>  279
ctcataaccc cttataggga ttcgtaac                                          28

<210>  280
<211>  28
<212>  DNA
<213>  Artificial

<220>
<223>  Artificial sequence

<400>  280
ggaccatccc cgcgggcgcg gggccgac                                          28

<210>  281
<211>  29
<212>  DNA
<213>  Artificial

<220>
<223>  Artificial sequence

<400>  281
gtgttccccg tacccacggg gctgaaccg                                         29

<210>  282
<211>  29
<212>  DNA
<213>  Artificial

<220>
<223>  Artificial sequence

<400>  282
gtaatcccca cgcacgtggg gatggaccg                                         29

<210>  283
<211>  28
<212>  DNA
<213>  Artificial

<220>
<223>  Artificial sequence

<400>  283
gtattcccca cgtatgtggg ggtgatcc                                          28

<210>  284
<211>  29
<212>  DNA
<213>  Artificial

<220>
<223>  Artificial sequence

<400> 284
attctccccg tattcgcggg gatgatccc 29

<210> 285
<211> 29
<212> DNA
<213> Artificial

<220>
<223> Artificial sequence

<400> 285
cggttcatcc ccacgggtgt ggggaacgc 29

<210> 286
<211> 29
<212> DNA
<213> Artificial

<220>
<223> Artificial sequence

<400> 286
ggtttccccg cccgcgcggg gatggtccc 29

<210> 287
<211> 29
<212> DNA
<213> Artificial

<220>
<223> Artificial sequence

<400> 287
gtcctccccg cccacgcggg ggtgttccg 29

<210> 288
<211> 29
<212> DNA
<213> Artificial

<220>
<223> Artificial sequence

<400> 288
gtgctctccg cgcgagcgga ggtggtccg 29

<210> 289
<211> 29
<212> DNA
<213> Artificial

<220>
<223> Artificial sequence

<400> 289
cttttctccg cgtatgcgga ggtagttcc 29

<210> 290
<211> 28
<212> DNA
<213> Artificial

<220>
<223> Artificial sequence

<400> 290
ggctcatccc cgcgggtgcg gggcggac 28

<210> 291
<211> 28
<212> DNA
<213> Artificial

<220>
<223> Artificial sequence

<400> 291
ggatcatccc cgcatacgcg gggagcac 28

<210> 292
<211> 29
<212> DNA
<213> Artificial

<220>
<223> Artificial sequence

<400> 292
gtccgctccg cgcaagcgga ggtgagccg 29

<210> 293
<211> 29
<212> DNA
<213> Artificial

<220>
<223> Artificial sequence

<400> 293
gggaccatcc ccgcgggcgc ggggagcag 29

<210> 294
<211> 29
<212> DNA
<213> Artificial

<220>
<223> Artificial sequence

<400> 294
cggttcatcc ccgcgcctgc ggggaacgg 29

<210> 295

<211> 29
<212> DNA
<213> Artificial

<220>
<223> Artificial sequence

<400> 295
cggtttatcc ccgtgcatac ggggaacac 29

<210> 296
<211> 29
<212> DNA
<213> Artificial

<220>
<223> Artificial sequence

<400> 296
gtgttcccca cacacgtggg gatgaaccg 29

<210> 297
<211> 29
<212> DNA
<213> Artificial

<220>
<223> Artificial sequence

<400> 297
gggcctatcc ccgctcgcgc gggggaacc 29

<210> 298
<211> 28
<212> DNA
<213> Artificial

<220>
<223> Artificial sequence

<400> 298
ggtcgatccg cgcgtacgcg gggggcac 28

<210> 299
<211> 28
<212> DNA
<213> Artificial

<220>
<223> Artificial sequence

<400> 299
ggatcacccc cgcatacgcg gggaacac 28

<210> 300
<211> 28
<212> DNA
<213> Artificial

<220>
<223>  Artificial sequence

<400>  300
ggttcctccg cgtgcgcgga gatagacc                                            28

<210>  301
<211>  29
<212>  DNA
<213>  Artificial

<220>
<223>  Artificial sequence

<400>  301
ctattcccca cgcatgtggg ggtgaaccg                                           29

<210>  302
<211>  29
<212>  DNA
<213>  Artificial

<220>
<223>  Artificial sequence

<400>  302
ggaaatacct ccgcgtgtgc ggagaagag                                           29

<210>  303
<211>  29
<212>  DNA
<213>  Artificial

<220>
<223>  Artificial sequence

<400>  303
cggttcatcc ccacgcgcgt ggggaacac                                           29

<210>  304
<211>  29
<212>  DNA
<213>  Artificial

<220>
<223>  Artificial sequence

<400>  304
cggttcaccc ccacgtgcgt ggggacaac                                           29

<210>  305
<211>  28
<212>  DNA
<213>  Artificial

<220>
<223>  Artificial sequence

<400> 305
ggatcatccc cgctcgcgcg gggtttac 28

<210> 306
<211> 29
<212> DNA
<213> Artificial

<220>
<223> Artificial sequence

<400> 306
cggttcatcc ccacgcgtgt ggggaatgc 29

<210> 307
<211> 28
<212> DNA
<213> Artificial

<220>
<223> Artificial sequence

<400> 307
ggaacacccc cgcacgcgcg gggacgac 28

<210> 308
<211> 29
<212> DNA
<213> Artificial

<220>
<223> Artificial sequence

<400> 308
gttctcccca cgcgcgtggg gatggtccg 29

<210> 309
<211> 28
<212> DNA
<213> Artificial

<220>
<223> Artificial sequence

<400> 309
cttttccccg catacgcggg ggtgatcc 28

<210> 310
<211> 28
<212> DNA
<213> Artificial

<220>
<223> Artificial sequence

<400> 310
ggatcacccc cgcatacgcg ggacaaat 28

<210> 311
<211> 29
<212> DNA
<213> Artificial

<220>
<223> Artificial sequence

<400> 311
cggttcatcc ccgcgcatgc ggggaacac 29

<210> 312
<211> 29
<212> DNA
<213> Artificial

<220>
<223> Artificial sequence

<400> 312
gtgctctccg cgcgagcgga ggtgagccg 29

<210> 313
<211> 29
<212> DNA
<213> Artificial

<220>
<223> Artificial sequence

<400> 313
ggaattaccc ccgctcgcgc ggggaaaag 29

<210> 314
<211> 28
<212> DNA
<213> Artificial

<220>
<223> Artificial sequence

<400> 314
tttctaaact gcctatacgg cattgaag 28

<210> 315
<211> 28
<212> DNA
<213> Artificial

<220>
<223> Artificial sequence

<400> 315
gttcatggcg gcatacgcca tttagaaa 28

<210> 316

<211> 28
<212> DNA
<213> Artificial

<220>
<223> Artificial sequence

<400> 316
gtgaactgcc gagtaggtag ctgataat 28

<210> 317
<211> 28
<212> DNA
<213> Artificial

<220>
<223> Artificial sequence

<400> 317
tttctgagct gcctactcgg cagtgaac 28

<210> 318
<211> 28
<212> DNA
<213> Artificial

<220>
<223> Artificial sequence

<400> 318
gttatccgcc gcacaggtgg cttagaaa 28

<210> 319
<211> 28
<212> DNA
<213> Artificial

<220>
<223> Artificial sequence

<400> 319
tttctaagcc atctacacga tggtgaac 28

<210> 320
<211> 28
<212> DNA
<213> Artificial

<220>
<223> Artificial sequence

<400> 320
cttctcaatc gcctacgcgg cgataaac 28

<210> 321
<211> 28
<212> DNA
<213> Artificial

<220>
<223> Artificial sequence

<400> 321
tttctaagct gcctgtgcgg cagtgaac 28

<210> 322
<211> 28
<212> DNA
<213> Artificial

<220>
<223> Artificial sequence

<400> 322
gttaaccgcc acgcaggcgg cttagaaa 28

<210> 323
<211> 28
<212> DNA
<213> Artificial

<220>
<223> Artificial sequence

<400> 323
gttcgtcatc gcatagatga tttagaaa 28

<210> 324
<211> 28
<212> DNA
<213> Artificial

<220>
<223> Artificial sequence

<400> 324
gtttgccgcc gtgcaggcgg cttagaaa 28

<210> 325
<211> 28
<212> DNA
<213> Artificial

<220>
<223> Artificial sequence

<400> 325
gctccccgcc ggataggcgg ctgagaga 28

<210> 326
<211> 28
<212> DNA
<213> Artificial

<220>
<223> Artificial sequence

<400> 326
attctcagct gcctatgcgg caggtcac 28

<210> 327
<211> 28
<212> DNA
<213> Artificial

<220>
<223> Artificial sequence

<400> 327
gctggccgcc gcataggcgg ctgagaag 28

<210> 328
<211> 28
<212> DNA
<213> Artificial

<220>
<223> Artificial sequence

<400> 328
cttctaagcc gcccacgtgg cggcgaac 28

<210> 329
<211> 24
<212> DNA
<213> Artificial

<220>
<223> Artificial sequence

<400> 329
tcagctgcct attcggcggt tcac 24

<210> 330
<211> 28
<212> DNA
<213> Artificial

<220>
<223> Artificial sequence

<400> 330
cctcgctgcc gcacaggcag ctcagaaa 28

<210> 331
<211> 28
<212> DNA
<213> Artificial

<220>
<223> Artificial sequence

<400> 331
gttatccgcc gtgtaggcgg tttagaag 28

<210> 332
<211> 28
<212> DNA
<213> Artificial

<220>
<223> Artificial sequence

<400> 332
gtgaaccgcc gtgtaggcgg cttagaaa 28

<210> 333
<211> 28
<212> DNA
<213> Artificial

<220>
<223> Artificial sequence

<400> 333
cttcaccacc gcacaggtgg cttagaaa 28

<210> 334
<211> 28
<212> DNA
<213> Artificial

<220>
<223> Artificial sequence

<400> 334
tttctaaacc atctatgcga tggcgaac 28

<210> 335
<211> 28
<212> DNA
<213> Artificial

<220>
<223> Artificial sequence

<400> 335
gtgaactgcc ggataggcag ctcagaaa 28

<210> 336
<211> 36
<212> DNA
<213> Artificial

<220>
<223> Artificial sequence

<400> 336
gcttcaatga ggccgaggcg tgaagccccg gaagac 36

<210> 337

<211> 36
<212> DNA
<213> Artificial

<220>
<223> Artificial sequence

<400> 337
attgttccgg tcttccaggc cggacttcat tgagac 36

<210> 338
<211> 37
<212> DNA
<213> Artificial

<220>
<223> Artificial sequence

<400> 338
gcttcaatgg ggccggagcg aatatgctcc ggaaaat 37

<210> 339
<211> 36
<212> DNA
<213> Artificial

<220>
<223> Artificial sequence

<400> 339
gtctcttccg cttatgcgga agactttcat tgaggc 36

<210> 340
<211> 36
<212> DNA
<213> Artificial

<220>
<223> Artificial sequence

<400> 340
cttgcatacg tcaaaacgta tgcacttcat tgagga 36

<210> 341
<211> 36
<212> DNA
<213> Artificial

<220>
<223> Artificial sequence

<400> 341
gcctcaacga agggctcccc tggaaagggg agcgat 36

<210> 342
<211> 36
<212> DNA
<213> Artificial

<220>
<223>  Artificial sequence

<400>  342
ccctcaatga aaggccgtcc cataaaggac ggcgac                                36

<210>  343
<211>  36
<212>  DNA
<213>  Artificial

<220>
<223>  Artificial sequence

<400>  343
aattccgcga ccgcgcggtc gcggccccat tgaagc                                36

<210>  344
<211>  36
<212>  DNA
<213>  Artificial

<220>
<223>  Artificial sequence

<400>  344
atctccgaag tctcggcttc ggagcttcat tgaggg                                36

<210>  345
<211>  36
<212>  DNA
<213>  Artificial

<220>
<223>  Artificial sequence

<400>  345
gccgcaacgg agcccgctca ttacgagcgg gatgac                                36

<210>  346
<211>  36
<212>  DNA
<213>  Artificial

<220>
<223>  Artificial sequence

<400>  346
gccgcaatgg agcccgcccc tgaaggccgg gagaac                                36

<210>  347
<211>  36
<212>  DNA
<213>  Artificial

<220>
<223>  Artificial sequence

<400> 347
gagcctcagg cccaaaagcc tgagcccaat tgaagc 36

<210> 348
<211> 30
<212> DNA
<213> Artificial

<220>
<223> Artificial sequence

<400> 348
gcctcaatga aagtcccctc ccgaaggagg 30

<210> 349
<211> 36
<212> DNA
<213> Artificial

<220>
<223> Artificial sequence

<400> 349
gcctcggctt aactgccgag gcaacttcgt tgaggc 36

<210> 350
<211> 36
<212> DNA
<213> Artificial

<220>
<223> Artificial sequence

<400> 350
gcttcaatgg agccatgtct tttcagacat ggtaag 36

<210> 351
<211> 36
<212> DNA
<213> Artificial

<220>
<223> Artificial sequence

<400> 351
gcttcaatga ggccccggca aaatgcccgg ggaaac 36

<210> 352
<211> 36
<212> DNA
<213> Artificial

<220>
<223> Artificial sequence

<400> 352
gcctcaatgg tgtccggcca aaatgaccgg attaat 36

<210> 353
<211> 36
<212> DNA
<213> Artificial

<220>
<223> Artificial sequence

<400> 353
ctttgatgca gccacggcgg ttatcgccat ggacac 36

<210> 354
<211> 37
<212> DNA
<213> Artificial

<220>
<223> Artificial sequence

<400> 354
gtgtcgccgg ggcttcggcc ccggccttcg ttgagcg 37

<210> 355
<211> 36
<212> DNA
<213> Artificial

<220>
<223> Artificial sequence

<400> 355
gcctcaatga agggcagccc ccgaaagggc tgcaac 36

<210> 356
<211> 34
<212> DNA
<213> Artificial

<220>
<223> Artificial sequence

<400> 356
gccgcaatgg tccccgctga tcagcgggga ggac 34

<210> 357
<211> 36
<212> DNA
<213> Artificial

<220>
<223> Artificial sequence

<400> 357
ctctccgtcg tcctcggacg acggcttcgt tgaagc 36

<210> 358

<211> 36
<212> DNA
<213> Artificial

<220>
<223> Artificial sequence

<400> 358
ctttccgctg atctcgatca gcggccccgt tgaagc 36

<210> 359
<211> 36
<212> DNA
<213> Artificial

<220>
<223> Artificial sequence

<400> 359
gcttcaatgg agccgcagtc tcacagccgc ggaaac 36

<210> 360
<211> 28
<212> DNA
<213> Artificial

<220>
<223> Artificial sequence

<400> 360
cctctacccc cgcatatgcg gggcaaac 28

<210> 361
<211> 28
<212> DNA
<213> Artificial

<220>
<223> Artificial sequence

<400> 361
cctctacccc cgcgtgcgcg gggctgac 28

<210> 362
<211> 36
<212> DNA
<213> Artificial

<220>
<223> Artificial sequence

<400> 362
gtcgcaatgg agcctgacct ttgaggtcag gaaaag 36

<210> 363
<211> 36
<212> DNA
<213> Artificial

<220>
<223> Artificial sequence

<400> 363
gcttcaatga ggcccgggcg tttcggcccg gaaggc 36

<210> 364
<211> 36
<212> DNA
<213> Artificial

<220>
<223> Artificial sequence

<400> 364
gcttcaatgg ggccgccgac gatctcggcg gaaaac 36

<210> 365
<211> 36
<212> DNA
<213> Artificial

<220>
<223> Artificial sequence

<400> 365
gttttccggg gcataatgcc ccggcctcat tgaagc 36

<210> 366
<211> 36
<212> DNA
<213> Artificial

<220>
<223> Artificial sequence

<400> 366
gcttcaatgg ggccgcggcc agatggccgc ggaaac 36

<210> 367
<211> 36
<212> DNA
<213> Artificial

<220>
<223> Artificial sequence

<400> 367
gcctcaatgg agcccggtca tgaagaccgg gaatac 36

<210> 368
<211> 35
<212> DNA
<213> Artificial

<220>
<223> Artificial sequence

<400> 368
cctcaatgaa agtccctaca aaaaggagag gaaat 35

<210> 369
<211> 35
<212> DNA
<213> Artificial

<220>
<223> Artificial sequence

<400> 369
cttcaacggg gccgcgctca ttcgagcgcg gatgg 35

<210> 370
<211> 36
<212> DNA
<213> Artificial

<220>
<223> Artificial sequence

<400> 370
gtttccacgg ctgaaaagcc gtggccccat tgaagc 36

<210> 371
<211> 36
<212> DNA
<213> Artificial

<220>
<223> Artificial sequence

<400> 371
gcctcaatga agtgcagttc ccgaaggaac tgcaac 36

<210> 372
<211> 37
<212> DNA
<213> Artificial

<220>
<223> Artificial sequence

<400> 372
gcttcaatgg ggcccggctt tgtgagccgg gagtggg 37

<210> 373
<211> 36
<212> DNA
<213> Artificial

<220>
<223> Artificial sequence

<400> 373
gcttcaacgg agcccaggcc cgtagacctg ggtgac 36

<210> 374
<211> 36
<212> DNA
<213> Artificial

<220>
<223> Artificial sequence

<400> 374
gcttcaatga ggccgcggcg gatggccggg gaaaac 36

<210> 375
<211> 37
<212> DNA
<213> Artificial

<220>
<223> Artificial sequence

<400> 375
gttgcatcca agcttcacag cttggctacg ttgcagg 37

<210> 376
<211> 37
<212> DNA
<213> Artificial

<220>
<223> Artificial sequence

<400> 376
gttttccacg gctaaccagc cgtggcccca ttgaagc 37

<210> 377
<211> 36
<212> DNA
<213> Artificial

<220>
<223> Artificial sequence

<400> 377
gcttcaatgg ggccgcgggt gtgaacccgc ggatag 36

<210> 378
<211> 36
<212> DNA
<213> Artificial

<220>
<223> Artificial sequence

<400> 378
gcttcaatga ggccgcagct cttcgctgcg gatcac 36

<210> 379

<211> 36
<212> DNA
<213> Artificial

<220>
<223> Artificial sequence

<400> 379
ctttccgcgc tcttcggagc gcggccccgt tgaagc 36

<210> 380
<211> 35
<212> DNA
<213> Artificial

<220>
<223> Artificial sequence

<400> 380
gtctcatccc tcccgcgggc gggcttcgtt gaagg 35

<210> 381
<211> 37
<212> DNA
<213> Artificial

<220>
<223> Artificial sequence

<400> 381
gcctcaacga agggtccgcg accgaagtcg cggacgg 37

<210> 382
<211> 36
<212> DNA
<213> Artificial

<220>
<223> Artificial sequence

<400> 382
attcctgagc tattaagctc aggacttcat tgagga 36

<210> 383
<211> 36
<212> DNA
<213> Artificial

<220>
<223> Artificial sequence

<400> 383
gttttccgca gcgttacgct gcggccccat tgaagc 36

<210> 384
<211> 36
<212> DNA
<213> Artificial

<220>
<223> Artificial sequence

<400> 384
gtctccgcgc gcgaaagcga gcggcttcgt tgaagc 36

<210> 385
<211> 36
<212> DNA
<213> Artificial

<220>
<223> Artificial sequence

<400> 385
gtcgccccgc tcttcgcgac cgggctccat tgcggc 36

<210> 386
<211> 36
<212> DNA
<213> Artificial

<220>
<223> Artificial sequence

<400> 386
gccgcaatgg agcccagctt ttggagctgg gaaaag 36

<210> 387
<211> 36
<212> DNA
<213> Artificial

<220>
<223> Artificial sequence

<400> 387
ccctcaatga aagtcacctg ttctcacagg tgagac 36

<210> 388
<211> 36
<212> DNA
<213> Artificial

<220>
<223> Artificial sequence

<400> 388
acctcaatga aaggctgcga ccgaagccgc agcgac 36

<210> 389
<211> 36
<212> DNA
<213> Artificial

<220>
<223> Artificial sequence

<400> 389
gtcgccgccc ctcagcgggc ggcccttcat cgaggc 36

<210> 390
<211> 36
<212> DNA
<213> Artificial

<220>
<223> Artificial sequence

<400> 390
gcttcaattg ggcctcgacc tcgcggtcga ggaaac 36

<210> 391
<211> 36
<212> DNA
<213> Artificial

<220>
<223> Artificial sequence

<400> 391
attttagtac ctaaacaaat tacgtgactg taaaac 36

<210> 392
<211> 36
<212> DNA
<213> Artificial

<220>
<223> Artificial sequence

<400> 392
gttttagtac tctctaaatt ttcgtaacaa taaaac 36

<210> 393
<211> 36
<212> DNA
<213> Artificial

<220>
<223> Artificial sequence

<400> 393
gtttcagtac cattcaatat aacactactg taaaac 36

<210> 394
<211> 35
<212> DNA
<213> Artificial

<220>
<223> Artificial sequence

<400> 394
gtttgagagt gatgtaatct tatatagtga ctaat 35

<210> 395
<211> 36
<212> DNA
<213> Artificial

<220>
<223> Artificial sequence

<400> 395
gttttaccac tatctggaat tacatcactc tcaaac 36

<210> 396
<211> 36
<212> DNA
<213> Artificial

<220>
<223> Artificial sequence

<400> 396
gttttagaag gatgttaaat caataaggtt aaaccc 36

<210> 397
<211> 36
<212> DNA
<213> Artificial

<220>
<223> Artificial sequence

<400> 397
gttgtgttac cctcgtaatt tttgctatct aacaac 36

<210> 398
<211> 36
<212> DNA
<213> Artificial

<220>
<223> Artificial sequence

<400> 398
gttttagagc tgtgctgttt cgaatggttc caaaac 36

<210> 399
<211> 36
<212> DNA
<213> Artificial

<220>
<223> Artificial sequence

<400> 399
gtttgagaat ggtgtagttt catatggtag tcaaac 36

<210> 400

<211> 36
<212> DNA
<213> Artificial

<220>
<223> Artificial sequence

<400> 400
gttttattac catcaaaatt tacacaactc tcaaac 36

<210> 401
<211> 36
<212> DNA
<213> Artificial

<220>
<223> Artificial sequence

<400> 401
gttttggtac cctctcagtt tttggtatag aaaaac 36

<210> 402
<211> 36
<212> DNA
<213> Artificial

<220>
<223> Artificial sequence

<400> 402
gtttgagagt agtgttatct tataaaactc tctcac 36

<210> 403
<211> 35
<212> DNA
<213> Artificial

<220>
<223> Artificial sequence

<400> 403
gtttgagagt tgtgttgttt tagataggtc taaac 35

<210> 404
<211> 36
<212> DNA
<213> Artificial

<220>
<223> Artificial sequence

<400> 404
gctcttgaac tgattgattc gacatctacc tgagac 36

<210> 405
<211> 36
<212> DNA
<213> Artificial

<220>
<223>  Artificial sequence

<400>  405
gtttgagagc tttgttaatt cagaaagatg taaaac                                36

<210>  406
<211>  36
<212>  DNA
<213>  Artificial

<220>
<223>  Artificial sequence

<400>  406
gtttgagagt agtgtcatat ccaatccatc tcaaac                                36

<210>  407
<211>  36
<212>  DNA
<213>  Artificial

<220>
<223>  Artificial sequence

<400>  407
gtttgagacc cctatgaaat tacactgctc tcaaac                                36

<210>  408
<211>  32
<212>  DNA
<213>  Artificial

<220>
<223>  Artificial sequence

<400>  408
cttgtgtatt caaaataaca atactctcaa ac                                    32

<210>  409
<211>  36
<212>  DNA
<213>  Artificial

<220>
<223>  Artificial sequence

<400>  409
gtttgagagt gttgtcaaat aggagtcaga ccaatc                                36

<210>  410
<211>  36
<212>  DNA
<213>  Artificial

<220>
<223>  Artificial sequence

<400> 410
gttttagagt tgtgttattt tgaacggtta caaaac 36

<210> 411
<211> 34
<212> DNA
<213> Artificial

<220>
<223> Artificial sequence

<400> 411
gttttagtac tctgtaattt ttcgctatga taaa 34

<210> 412
<211> 36
<212> DNA
<213> Artificial

<220>
<223> Artificial sequence

<400> 412
ggatgaaacc ccattaatct gacatacatc tgaagc 36

<210> 413
<211> 36
<212> DNA
<213> Artificial

<220>
<223> Artificial sequence

<400> 413
gttgtacagt tacttaaatc ttgagagtac aaaaac 36

<210> 414
<211> 36
<212> DNA
<213> Artificial

<220>
<223> Artificial sequence

<400> 414
gttttgtaga attcaaaatt tacatatctc tcaaac 36

<210> 415
<211> 36
<212> DNA
<213> Artificial

<220>
<223> Artificial sequence

<400> 415
gtttcagatg cctgtcagat caatgacttt gaccac 36

<210> 416
<211> 36
<212> DNA
<213> Artificial

<220>
<223> Artificial sequence

<400> 416
gtttgattcc cctacgaatt tgcactgctc tcaaac 36

<210> 417
<211> 36
<212> DNA
<213> Artificial

<220>
<223> Artificial sequence

<400> 417
gtttgagaat gatgtaaaaa tgtatggtac tcaaac 36

<210> 418
<211> 36
<212> DNA
<213> Artificial

<220>
<223> Artificial sequence

<400> 418
gttttactac tctctaaaat tacattggtc taaaac 36

<210> 419
<211> 36
<212> DNA
<213> Artificial

<220>
<223> Artificial sequence

<400> 419
gtttcttacc catcataatt tacaaggttc tcaaac 36

<210> 420
<211> 36
<212> DNA
<213> Artificial

<220>
<223> Artificial sequence

<400> 420
gttttacaac tatctgaaat tacatcactc tcaaac 36

<210> 421

<211> 36
<212> DNA
<213> Artificial

<220>
<223> Artificial sequence

<400> 421
gtttgagagt tatgtaattt catataggtc taaaac 36

<210> 422
<211> 36
<212> DNA
<213> Artificial

<220>
<223> Artificial sequence

<400> 422
gtcttattct agcaaaaaat catatggtaa caaaac 36

<210> 423
<211> 36
<212> DNA
<213> Artificial

<220>
<223> Artificial sequence

<400> 423
ggttcttaac taattagaat gacgtacttc taaaac 36

<210> 424
<211> 36
<212> DNA
<213> Artificial

<220>
<223> Artificial sequence

<400> 424
gtttgagagt aatgttattt tagatagata taaaac 36

<210> 425
<211> 36
<212> DNA
<213> Artificial

<220>
<223> Artificial sequence

<400> 425
gtttgattac cagtcagaat gtcactgctc caaaac 36

<210> 426
<211> 36
<212> DNA
<213> Artificial

<220>
<223> Artificial sequence

<400> 426
gtttcagaag gatgttaaat caataaggtt aagatc 36

<210> 427
<211> 36
<212> DNA
<213> Artificial

<220>
<223> Artificial sequence

<400> 427
gttttagtac tctgtaattt taggtataag tgaaac 36

<210> 428
<211> 36
<212> DNA
<213> Artificial

<220>
<223> Artificial sequence

<400> 428
gttttagact acataagaat gataccacac caaaac 36

<210> 429
<211> 36
<212> DNA
<213> Artificial

<220>
<223> Artificial sequence

<400> 429
gtttgagaac tatgtaaatt atgctggtag caaaac 36

<210> 430
<211> 36
<212> DNA
<213> Artificial

<220>
<223> Artificial sequence

<400> 430
gttttactac cttactgatt tacaaggttc tcaaac 36

<210> 431
<211> 36
<212> DNA
<213> Artificial

<220>
<223> Artificial sequence

<400> 431
gttttagaag gttgttaaat cagtaagttg aaaaac 36

<210> 432
<211> 36
<212> DNA
<213> Artificial

<220>
<223> Artificial sequence

<400> 432
gtcttggatg agtgtcagat cagtagttcc gagtac 36

<210> 433
<211> 36
<212> DNA
<213> Artificial

<220>
<223> Artificial sequence

<400> 433
gttcaactat accgaaaaac gggagagtac cgaaac 36

<210> 434
<211> 36
<212> DNA
<213> Artificial

<220>
<223> Artificial sequence

<400> 434
gttaaagccc agtcttaaat tacacaactc tcaaac 36

<210> 435
<211> 36
<212> DNA
<213> Artificial

<220>
<223> Artificial sequence

<400> 435
gttttagaag tatgttaaat caatgaggtt tagaac 36

<210> 436
<211> 36
<212> DNA
<213> Artificial

<220>
<223> Artificial sequence

<400> 436
gttttactgt ttctaaattc tttaaggtac aaaaac 36

<210> 437
<211> 36
<212> DNA
<213> Artificial

<220>
<223> Artificial sequence

<400> 437
gttattgctt acacaagtat tgtacagtgc taaaac 36

<210> 438
<211> 36
<212> DNA
<213> Artificial

<220>
<223> Artificial sequence

<400> 438
gtttttgtgc tgtacaattt cttattagag taaaac 36

<210> 439
<211> 36
<212> DNA
<213> Artificial

<220>
<223> Artificial sequence

<400> 439
gttttagaag agtatcaaat caatgagtag ttcaac 36

<210> 440
<211> 36
<212> DNA
<213> Artificial

<220>
<223> Artificial sequence

<400> 440
gttctgctac catcgaaatt tttgctaggc tacaac 36

<210> 441
<211> 36
<212> DNA
<213> Artificial

<220>
<223> Artificial sequence

<400> 441
gttttagagg tttacagaat tacactactc tcaaac 36

<210> 442

<211> 36
<212> DNA
<213> Artificial

<220>
<223> Artificial sequence

<400> 442
gtttgagagt attgttaaat ttgaattatc ttcatc 36

<210> 443
<211> 36
<212> DNA
<213> Artificial

<220>
<223> Artificial sequence

<400> 443
gttctaaaca ttattgattt gaagtacatc taaaac 36

<210> 444
<211> 36
<212> DNA
<213> Artificial

<220>
<223> Artificial sequence

<400> 444
gttcataaca tcattgatat gacatccagc tgaaac 36

<210> 445
<211> 36
<212> DNA
<213> Artificial

<220>
<223> Artificial sequence

<400> 445
gttttagaat tgtgttattt tagatagtaa taaaac 36

<210> 446
<211> 36
<212> DNA
<213> Artificial

<220>
<223> Artificial sequence

<400> 446
gatacagatt cttggtaaat ttgaataatg tgaatg 36

<210> 447
<211> 36
<212> DNA
<213> Artificial

<220>
<223> Artificial sequence

<400> 447
gttcttaacc ttattgattt gtcactcaac taaaac 36

<210> 448
<211> 36
<212> DNA
<213> Artificial

<220>
<223> Artificial sequence

<400> 448
gttttacgat aacttaattc tttcaggtac taaaat 36

<210> 449
<211> 36
<212> DNA
<213> Artificial

<220>
<223> Artificial sequence

<400> 449
gttttagaac tatgttattt tgaatggtaa caaaac 36

<210> 450
<211> 36
<212> DNA
<213> Artificial

<220>
<223> Artificial sequence

<400> 450
gtttaaccac catagtgatt tacatcattc tcaaac 36

<210> 451
<211> 36
<212> DNA
<213> Artificial

<220>
<223> Artificial sequence

<400> 451
gttcttaacc ctattgattt accaagattc taaagc 36

<210> 452
<211> 36
<212> DNA
<213> Artificial

<220>
<223> Artificial sequence

<400> 452
gttttagaag gttgtctatt caataaggtt taaccc 36

<210> 453
<211> 36
<212> DNA
<213> Artificial

<220>
<223> Artificial sequence

<400> 453
gtttaaaagt aattaaagat agcactactc ccaaac 36

<210> 454
<211> 36
<212> DNA
<213> Artificial

<220>
<223> Artificial sequence

<400> 454
gtttgagagc cttgttattt tgtaattctc ttaaac 36

<210> 455
<211> 36
<212> DNA
<213> Artificial

<220>
<223> Artificial sequence

<400> 455
attttggtac tctctcatct tttggtataa ggaaac 36

<210> 456
<211> 36
<212> DNA
<213> Artificial

<220>
<223> Artificial sequence

<400> 456
gttttagcac tatgattatt tagaaagaag taaaac 36

<210> 457
<211> 36
<212> DNA
<213> Artificial

<220>
<223> Artificial sequence

<400> 457
gttttactac cacttgggat tacactactc tcaaac 36

<210> 458
<211> 36
<212> DNA
<213> Artificial

<220>
<223> Artificial sequence

<400> 458
gatctaaacc tcattgatct aacaatcatc taaaac 36

<210> 459
<211> 36
<212> DNA
<213> Artificial

<220>
<223> Artificial sequence

<400> 459
gttctactac tacataattt tacagggtac cataac 36

<210> 460
<211> 36
<212> DNA
<213> Artificial

<220>
<223> Artificial sequence

<400> 460
gtttgagaac cttgtaattt tgtaagggtg taaaac 36

<210> 461
<211> 36
<212> DNA
<213> Artificial

<220>
<223> Artificial sequence

<400> 461
gctcttaact atattgattc gacactcttc taaaac 36

<210> 462
<211> 36
<212> DNA
<213> Artificial

<220>
<223> Artificial sequence

<400> 462
gtttgagaac cttgtaaatt acgatatctg tcaagc 36

<210> 463

<211> 36
<212> DNA
<213> Artificial

<220>
<223> Artificial sequence

<400> 463
gattagtcac tatataagat tacatcactc tcaaac 36

<210> 464
<211> 36
<212> DNA
<213> Artificial

<220>
<223> Artificial sequence

<400> 464
gttctaaacc tgtttgatat gactactatt caagac 36

<210> 465
<211> 36
<212> DNA
<213> Artificial

<220>
<223> Artificial sequence

<400> 465
gttttagatg agtggtaaat ctagaaggtc aaaagc 36

<210> 466
<211> 36
<212> DNA
<213> Artificial

<220>
<223> Artificial sequence

<400> 466
gttttagaat actcaaaatt tacagttaac tcaaac 36

<210> 467
<211> 36
<212> DNA
<213> Artificial

<220>
<223> Artificial sequence

<400> 467
gtagtagaac catctggaat gacatagttc caaaac 36

<210> 468
<211> 36
<212> DNA
<213> Artificial

<220>
<223> Artificial sequence

<400> 468
gtttgaaatc cttccgtatt aacaagactc tcaaac 36

<210> 469
<211> 36
<212> DNA
<213> Artificial

<220>
<223> Artificial sequence

<400> 469
gttttactac cttatgaaat tacactactc tcaaac 36

<210> 470
<211> 36
<212> DNA
<213> Artificial

<220>
<223> Artificial sequence

<400> 470
gtattctaat atctatattc aacataccta gcaaac 36

<210> 471
<211> 36
<212> DNA
<213> Artificial

<220>
<223> Artificial sequence

<400> 471
gttttcgagc tatataagac aacactgctc tcaaac 36

<210> 472
<211> 36
<212> DNA
<213> Artificial

<220>
<223> Artificial sequence

<400> 472
gcttgagagt attgtaaaac cagattcctc tctaac 36

<210> 473
<211> 36
<212> DNA
<213> Artificial

<220>
<223> Artificial sequence

<400> 473
gtttaagtac tatatgaatt tacactactc tcaaac 36

<210> 474
<211> 36
<212> DNA
<213> Artificial

<220>
<223> Artificial sequence

<400> 474
gttttatcac attaagaatt tacaaagtac taaaac 36

<210> 475
<211> 35
<212> DNA
<213> Artificial

<220>
<223> Artificial sequence

<400> 475
gtttgagaac attcaattca acatgactct aaaac 35

<210> 476
<211> 36
<212> DNA
<213> Artificial

<220>
<223> Artificial sequence

<400> 476
gttttagagc tatgctgttt tgaatggtcc caaaac 36

<210> 477
<211> 36
<212> DNA
<213> Artificial

<220>
<223> Artificial sequence

<400> 477
gcttcaatac tacattcttc taacaactac tgaaac 36

<210> 478
<211> 37
<212> DNA
<213> Artificial

<220>
<223> Artificial sequence

<400> 478
gcgacacttt acaacaatat cgcttagcta atgaaac 37

<210> 479
<211> 37
<212> DNA
<213> Artificial

<220>
<223> Artificial sequence

<400> 479
gtttcaattg tacaaactta catccttcaa ctgaaac 37

<210> 480
<211> 37
<212> DNA
<213> Artificial

<220>
<223> Artificial sequence

<400> 480
ccaataatcc ctcatctaaa aatccaacca ctgaaac 37

<210> 481
<211> 37
<212> DNA
<213> Artificial

<220>
<223> Artificial sequence

<400> 481
gcaacacttt atagcaaatc cgcttagcct gtgaaac 37

<210> 482
<211> 37
<212> DNA
<213> Artificial

<220>
<223> Artificial sequence

<400> 482
gttgcaaatt ataccacaaa ccaactcggt ttgaaac 37

<210> 483
<211> 37
<212> DNA
<213> Artificial

<220>
<223> Artificial sequence

<400> 483
gtttcagtga ttgagtaatt ggcaaaggca gtgtagg 37

<210> 484

<211> 37
<212> DNA
<213> Artificial

<220>
<223> Artificial sequence

<400> 484
gcagagatca taacgctacg agctatagca ctgaaac 37

<210> 485
<211> 37
<212> DNA
<213> Artificial

<220>
<223> Artificial sequence

<400> 485
gtttcagttg ctgaattatt tggtaaacta ctgttag 37

<210> 486
<211> 37
<212> DNA
<213> Artificial

<220>
<223> Artificial sequence

<400> 486
ctaacaaatt ataactagac acaacttagt ttgaaac 37

<210> 487
<211> 36
<212> DNA
<213> Artificial

<220>
<223> Artificial sequence

<400> 487
gtttcagttg ttgttgaaag aatgtacgat tgaagc 36

<210> 488
<211> 37
<212> DNA
<213> Artificial

<220>
<223> Artificial sequence

<400> 488
ccaacacatc ataacctagt aagccagcaa ctgaaac 37

<210> 489
<211> 47
<212> DNA
<213> Artificial

<220>
<223> Artificial sequence

<400> 489
gttgtattta ccaatgcaaa gatactaatt ttaaagctaa tcacaac 47

<210> 490
<211> 47
<212> DNA
<213> Artificial

<220>
<223> Artificial sequence

<400> 490
gttgtgattt gcttgaaaac gactaccttt gtggtatcta aaacaac 47

<210> 491
<211> 47
<212> DNA
<213> Artificial

<220>
<223> Artificial sequence

<400> 491
gttgtgattt gctttcaaat tagtatcttt gaaccattgg gaacagc 47

<210> 492
<211> 47
<212> DNA
<213> Artificial

<220>
<223> Artificial sequence

<400> 492
cttgtgatta ctcacataaa aataagaatt tgaaagcaat tcacaac 47

<210> 493
<211> 44
<212> DNA
<213> Artificial

<220>
<223> Artificial sequence

<400> 493
gttgtggttt gctttcaaat tgaacattga tgatattaac caac 44

<210> 494
<211> 46
<212> DNA
<213> Artificial

<220>
<223> Artificial sequence

<400> 494
gctgggaatt ataagtaaaa atacaatttt gaaagcaatt cacaac 46

<210> 495
<211> 46
<212> DNA
<213> Artificial

<220>
<223> Artificial sequence

<400> 495
actgggaata gctttcagat ttagtatttt tgttgcaacg cacagc 46

<210> 496
<211> 32
<212> DNA
<213> Artificial

<220>
<223> Artificial sequence

<400> 496
caaaactaca aaatttgaaa gcaattcaca ac 32

<210> 497
<211> 47
<212> DNA
<213> Artificial

<220>
<223> Artificial sequence

<400> 497
gttgtgaatt gctttcaaat tttagtatct ttgtgataga tcacaac 47

<210> 498
<211> 36
<212> DNA
<213> Artificial

<220>
<223> Artificial sequence

<400> 498
gttttagagc tgtgttgttt cgaatggttc caaaac 36

<210> 499
<211> 36
<212> DNA
<213> Artificial

<220>
<223> Artificial sequence

<400> 499
attttaccat aaagaaattt aaaaagggac taaaac 36

<210> 500
<211> 36
<212> DNA
<213> Artificial

<220>
<223> Artificial sequence

<400> 500
gtttttgtac tctcaagatt taagtaactg tacaac 36

<210> 501
<211> 36
<212> DNA
<213> Artificial

<220>
<223> Artificial sequence

<400> 501
gttttatgat gactcaattc tttcaggtac taaaac 36

<210> 502
<211> 37
<212> DNA
<213> Artificial

<220>
<223> Artificial sequence

<400> 502
gttgtagctc cctctctcac cccggatagc tacaatt 37

<210> 503
<211> 36
<212> DNA
<213> Artificial

<220>
<223> Artificial sequence

<400> 503
gttgtagctc cctttctcat ttcgcagtgc tacaat 36

<210> 504
<211> 47
<212> DNA
<213> Artificial

<220>
<223> Artificial sequence

<400> 504
gctgtgacta atcacgaaga taataatttt tgaaagcaat tcacaac 47

<210> 505

<211> 36
<212> DNA
<213> Artificial

<220>
<223> Artificial sequence

<400> 505
gttgccgctg gaccgcgatt tctgaaatgc tatgct 36

<210> 506
<211> 36
<212> DNA
<213> Artificial

<220>
<223> Artificial sequence

<400> 506
atttagtaa ctggaagatt tgagttagtg taaaac 36

<210> 507
<211> 36
<212> DNA
<213> Artificial

<220>
<223> Artificial sequence

<400> 507
gttttagtac ctggagaaaa taagttatcg taaaac 36

<210> 508
<211> 36
<212> DNA
<213> Artificial

<220>
<223> Artificial sequence

<400> 508
agcttatcag ttcagaaatc gcggtccagc cggaac 36

<210> 509
<211> 36
<212> DNA
<213> Artificial

<220>
<223> Artificial sequence

<400> 509
gatctatttt agctgaaaac tgaaggaatc aatagc 36

<210> 510
<211> 36
<212> DNA
<213> Artificial

<220>
<223> Artificial sequence

<400> 510
aatatcgtgt tttctaatct ttaatcaaac cacaac 36

<210> 511
<211> 47
<212> DNA
<213> Artificial

<220>
<223> Artificial sequence

<400> 511
gttgtaaatt gctttcaatt ttccgtaact ttgtgattag ttacaac 47

<210> 512
<211> 36
<212> DNA
<213> Artificial

<220>
<223> Artificial sequence

<400> 512
atcatatcat accaagattt aaagaggaat tatggc 36

<210> 513
<211> 36
<212> DNA
<213> Artificial

<220>
<223> Artificial sequence

<400> 513
attgtagccg atggggaacc ggggtccagc cgcaac 36

<210> 514
<211> 36
<212> DNA
<213> Artificial

<220>
<223> Artificial sequence

<400> 514
gttgtaacag attagaaaat gcggtcaagc cgcaac 36

<210> 515
<211> 36
<212> DNA
<213> Artificial

<220>
<223> Artificial sequence

<400> 515
agtgtatccg agcgacggtt cccgacgaac cacagc 36

<210> 516
<211> 36
<212> DNA
<213> Artificial

<220>
<223> Artificial sequence

<400> 516
gctggggttc agttctcaaa aaccctgata gacttc 36

<210> 517
<211> 36
<212> DNA
<213> Artificial

<220>
<223> Artificial sequence

<400> 517
gccgtggctt ccctaccgat ttccccgtgg taggct 36

<210> 518
<211> 46
<212> DNA
<213> Artificial

<220>
<223> Artificial sequence

<400> 518
gttgtgaatt gctttcagaa ctttaatttt agtgatgatt cacagt 46

<210> 519
<211> 36
<212> DNA
<213> Artificial

<220>
<223> Artificial sequence

<400> 519
gttccggcca gtgcgcatat cccagtgatc tagaat 36

<210> 520
<211> 36
<212> DNA
<213> Artificial

<220>
<223> Artificial sequence

<400> 520
caagcttatc aagaagggtg aatgctaatt cccagc 36

<210> 521
<211> 36
<212> DNA
<213> Artificial

<220>
<223> Artificial sequence

<400> 521
agtttagctg ttcagaattc ggggtccagc cgcaac 36

<210> 522
<211> 47
<212> DNA
<213> Artificial

<220>
<223> Artificial sequence

<400> 522
gttgtgattt gctttcaaat ttttatcttt gacatattaa aaacagc 47

<210> 523
<211> 36
<212> DNA
<213> Artificial

<220>
<223> Artificial sequence

<400> 523
gtcatagttc ccctgagatt atcgctgtgg tataat 36

<210> 524
<211> 36
<212> DNA
<213> Artificial

<220>
<223> Artificial sequence

<400> 524
gttttgcctt gaatccaaaa taaggcacag tacaat 36

<210> 525
<211> 36
<212> DNA
<213> Artificial

<220>
<223> Artificial sequence

<400> 525
gttatagttc ctagtaaatt ctcgatatgc tataat 36

<210> 526

<211> 36
<212> DNA
<213> Artificial

<220>
<223> Artificial sequence

<400> 526
gttccggtca gagctcaaat cccaatctgc taaact 36

<210> 527
<211> 36
<212> DNA
<213> Artificial

<220>
<223> Artificial sequence

<400> 527
atttaccat ataaacaatt aataataggc taaaac 36

<210> 528
<211> 36
<212> DNA
<213> Artificial

<220>
<223> Artificial sequence

<400> 528
aggttagcag atcgggaatt gcgctctggc tacaac 36

<210> 529
<211> 47
<212> DNA
<213> Artificial

<220>
<223> Artificial sequence

<400> 529
gttgtgcagt atcacaaaga tacagttaaa tgtaagcaat tcacaac 47

<210> 530
<211> 30
<212> DNA
<213> Artificial

<220>
<223> Artificial sequence

<400> 530
gtttccatcc ctcatagtca cttctaaaac 30

<210> 531
<211> 37
<212> DNA
<213> Artificial

<220>
<223> Artificial sequence

<400> 531
gtcacaatcc ttattttaat agaatattct ttgcaat 37

<210> 532
<211> 36
<212> DNA
<213> Artificial

<220>
<223> Artificial sequence

<400> 532
gtcagaaaga atgccccgat tagaggggat tgagac 36

<210> 533
<211> 32
<212> DNA
<213> Artificial

<220>
<223> Artificial sequence

<400> 533
gttttcgtcc ccatgattgg ggatcttttt tt 32

<210> 534
<211> 35
<212> DNA
<213> Artificial

<220>
<223> Artificial sequence

<400> 534
gtttccgtcc ccttacgggg attatggttc ttaac 35

<210> 535
<211> 37
<212> DNA
<213> Artificial

<220>
<223> Artificial sequence

<400> 535
gtcaaaaagt agattccact aaaacaagga ttgaaac 37

<210> 536
<211> 33
<212> DNA
<213> Artificial

<220>
<223> Artificial sequence

<400> 536
gtttcaatcc ttgttttgat agactgttgc aac 33

<210> 537
<211> 36
<212> DNA
<213> Artificial

<220>
<223> Artificial sequence

<400> 537
gaaacaccca tgccctgatt tcaaagggat tgagac 36

<210> 538
<211> 35
<212> DNA
<213> Artificial

<220>
<223> Artificial sequence

<400> 538
gtcgtaatcc cttctaaatc aggtctaatt ctttc 35

<210> 539
<211> 35
<212> DNA
<213> Artificial

<220>
<223> Artificial sequence

<400> 539
gttttaatag catcaccccg caaggggacg gaaac 35

<210> 540
<211> 36
<212> DNA
<213> Artificial

<220>
<223> Artificial sequence

<400> 540
gttctcgtcc cctccacggg gttctcttat ctcaat 36

<210> 541
<211> 36
<212> DNA
<213> Artificial

<220>
<223> Artificial sequence

<400> 541
gtcttaatcc cctagtgatg gggctttgtt tttaat 36

<210> 542
<211> 35
<212> DNA
<213> Artificial

<220>
<223> Artificial sequence

<400> 542
gtttccgtcc ccttgcgggg ttctggtact taaat 35

<210> 543
<211> 27
<212> DNA
<213> Artificial

<220>
<223> Artificial sequence

<400> 543
gttgcatccc cgaaatggga ttaaaac 27

<210> 544
<211> 36
<212> DNA
<213> Artificial

<220>
<223> Artificial sequence

<400> 544
gttctcgtcc cctattactg gggataatct aatacc 36

<210> 545
<211> 34
<212> DNA
<213> Artificial

<220>
<223> Artificial sequence

<400> 545
atttgaaaaa cactccccgt gaggggacgg aaac 34

<210> 546
<211> 36
<212> DNA
<213> Artificial

<220>
<223> Artificial sequence

<400> 546
attagaagat agagaacccc agtaggggac ggaaac 36

<210> 547

<211> 36
<212> DNA
<213> Artificial

<220>
<223> Artificial sequence

<400> 547
aatcagagaa taccccgtat aaaaggggac gagaac 36

<210> 548
<211> 35
<212> DNA
<213> Artificial

<220>
<223> Artificial sequence

<400> 548
gtttatgaat aagatccccg aaaggggacg gaaac 35

<210> 549
<211> 32
<212> DNA
<213> Artificial

<220>
<223> Artificial sequence

<400> 549
gttaccgtcc ccttgcgggg gtaagttgtt tc 32

<210> 550
<211> 36
<212> DNA
<213> Artificial

<220>
<223> Artificial sequence

<400> 550
gtcttaatcc cctttagatc gggtcagcct tcaaac 36

<210> 551
<211> 35
<212> DNA
<213> Artificial

<220>
<223> Artificial sequence

<400> 551
gaatgaagag acctgaacta caaagggatt acgac 35

<210> 552
<211> 36
<212> DNA
<213> Artificial

<220>
<223> Artificial sequence

<400> 552
gttctcgtct ccttctagcg gagataagtt gttagc 36

<210> 553
<211> 36
<212> DNA
<213> Artificial

<220>
<223> Artificial sequence

<400> 553
attgagatat aagaaccccg gggaggggac gaaaac 36

<210> 554
<211> 31
<212> DNA
<213> Artificial

<220>
<223> Artificial sequence

<400> 554
aaaaaaataa tcccctaatg gggacggaaa c 31

<210> 555
<211> 36
<212> DNA
<213> Artificial

<220>
<223> Artificial sequence

<400> 555
attaaaaacg aaggacccct ttaaggggac ggaaac 36

<210> 556
<211> 35
<212> DNA
<213> Artificial

<220>
<223> Artificial sequence

<400> 556
gttaaattaa ataatcccct gatggggacg gaaac 35

<210> 557
<211> 37
<212> DNA
<213> Artificial

<220>
<223> Artificial sequence

<400> 557
gtcttaatcc ttgttttgct ggaatatggt ttctgac 37

<210> 558
<211> 33
<212> DNA
<213> Artificial

<220>
<223> Artificial sequence

<400> 558
gtttcgatgc tccatgtttt gagctttttt tat 33

<210> 559
<211> 31
<212> DNA
<213> Artificial

<220>
<223> Artificial sequence

<400> 559
atattaatcg caccatttaa ggaattgaaa c 31

<210> 560
<211> 36
<212> DNA
<213> Artificial

<220>
<223> Artificial sequence

<400> 560
gtttccgtct cctctcggag ttatattctc tcttat 36

<210> 561
<211> 36
<212> DNA
<213> Artificial

<220>
<223> Artificial sequence

<400> 561
gttaaaatca agccctgaaa taaaagggat tgagac 36

<210> 562
<211> 34
<212> DNA
<213> Artificial

<220>
<223> Artificial sequence

<400> 562
ttaaattaaa tgatccccat taggggacgg aaac 34

<210> 563
<211> 34
<212> DNA
<213> Artificial

<220>
<223> Artificial sequence

<400> 563
gtttccgtcc cctctttcgg ggatgttttt tatt 34

<210> 564
<211> 33
<212> DNA
<213> Artificial

<220>
<223> Artificial sequence

<400> 564
gtcgctccct tcacggggag cgtggattga aat 33

<210> 565
<211> 32
<212> DNA
<213> Artificial

<220>
<223> Artificial sequence

<400> 565
gttatcgtcc ccttgcgggg ataagctgca at 32

<210> 566
<211> 32
<212> DNA
<213> Artificial

<220>
<223> Artificial sequence

<400> 566
atttcaatcc acgaggctgt ataagcctcg ac 32

<210> 567
<211> 31
<212> DNA
<213> Artificial

<220>
<223> Artificial sequence

<400> 567
gaaccaaccc atccccaagc ggggacgaaa a 31

<210> 568

<211> 31
<212> DNA
<213> Artificial

<220>
<223> Artificial sequence

<400> 568
gttgaatatt gttcttatta aggattaacg c 31

<210> 569
<211> 25
<212> DNA
<213> Artificial

<220>
<223> Artificial sequence

<400> 569
ttaaatacat cttatgttac tgttc 25

<210> 570
<211> 31
<212> DNA
<213> Artificial

<220>
<223> Artificial sequence

<400> 570
gttagatatc atacttatta aggattaacg c 31

<210> 571
<211> 30
<212> DNA
<213> Artificial

<220>
<223> Artificial sequence

<400> 571
gttttcgtac agtatctgtt gatatttatc 30

<210> 572
<211> 37
<212> DNA
<213> Artificial

<220>
<223> Artificial sequence

<400> 572
gtcttaatcc ttgttgtaat ggaatatact taataat 37

<210> 573
<211> 30
<212> DNA
<213> Artificial

<220>
<223> Artificial sequence

<400> 573
gtttgtagcc ttccctttgg ggattgaaac 30

<210> 574
<211> 30
<212> DNA
<213> Artificial

<220>
<223> Artificial sequence

<400> 574
gtttttagcc tacctataag ggattgaaac 30

<210> 575
<211> 30
<212> DNA
<213> Artificial

<220>
<223> Artificial sequence

<400> 575
gtttcaattc ctcataggta cgttcaaaac 30

<210> 576
<211> 30
<212> DNA
<213> Artificial

<220>
<223> Artificial sequence

<400> 576
gtttcaattc tatgatagtc cgattaaaag 30

<210> 577
<211> 30
<212> DNA
<213> Artificial

<220>
<223> Artificial sequence

<400> 577
gtttaaatag aaacatactg taatgtaaat 30

<210> 578
<211> 36
<212> DNA
<213> Artificial

<220>
<223> Artificial sequence

<400> 578
attaagaaaa taaaagcccc gataggggac gaaaac 36

<210> 579
<211> 27
<212> DNA
<213> Artificial

<220>
<223> Artificial sequence

<400> 579
gtattatcca cacgaacaag gattgaa 27

<210> 580
<211> 37
<212> DNA
<213> Artificial

<220>
<223> Artificial sequence

<400> 580
gtcttaatcc ttgttttagt ggaagttagt ctgtgag 37

<210> 581
<211> 29
<212> DNA
<213> Artificial

<220>
<223> Artificial sequence

<400> 581
gtctccatcc cacacggtgg gcttagaac 29

<210> 582
<211> 28
<212> DNA
<213> Artificial

<220>
<223> Artificial sequence

<400> 582
cttccatact aactagtact tcttaaac 28

<210> 583
<211> 28
<212> DNA
<213> Artificial

<220>
<223> Artificial sequence

<400> 583
tttctaaact acctatgcgg cagttaac 28

<210> 584
<211> 36
<212> DNA
<213> Artificial

<220>
<223> Artificial sequence

<400> 584
gtcgcaatcc ccttacgggg ctaaatcgct tgcaac 36

<210> 585
<211> 27
<212> DNA
<213> Artificial

<220>
<223> Artificial sequence

<400> 585
gttttcgccc ccttacgggg atggtac 27

<210> 586
<211> 25
<212> DNA
<213> Artificial

<220>
<223> Artificial sequence

<400> 586
ctttcaattc cttttgggat gcaac 25

<210> 587
<211> 37
<212> DNA
<213> Artificial

<220>
<223> Artificial sequence

<400> 587
atttgaataa cagaccttat taagaaggga ttaagac 37

<210> 588
<211> 36
<212> DNA
<213> Artificial

<220>
<223> Artificial sequence

<400> 588
gtcgtcagac ccaaaacccc gagaggggac ggaaac 36

<210> 589

<211> 37
<212> DNA
<213> Artificial

<220>
<223> Artificial sequence

<400> 589
gtcgaaagac attgccctgt tccaaaggga ttgagac 37

<210> 590
<211> 37
<212> DNA
<213> Artificial

<220>
<223> Artificial sequence

<400> 590
atttaaacaa atttgacccg atcaagggga ttgaaac 37

<210> 591
<211> 36
<212> DNA
<213> Artificial

<220>
<223> Artificial sequence

<400> 591
gtctgaatga ttccctgatt tagaagggat taagac 36

<210> 592
<211> 36
<212> DNA
<213> Artificial

<220>
<223> Artificial sequence

<400> 592
gtttccgtct ccttacggag tatcattcat tcttat 36

<210> 593
<211> 36
<212> DNA
<213> Artificial

<220>
<223> Artificial sequence

<400> 593
gtaagaatgg aatgacctgc ttgaggggat tgagac 36

<210> 594
<211> 36
<212> DNA
<213> Artificial

<220>
<223> Artificial sequence

<400> 594
atagaaataa aaaaatcccc gtgaggggac ggaaac 36

<210> 595
<211> 37
<212> DNA
<213> Artificial

<220>
<223> Artificial sequence

<400> 595
gtttccgtcc cctctcgagg tgactggggt gtcttac 37

<210> 596
<211> 36
<212> DNA
<213> Artificial

<220>
<223> Artificial sequence

<400> 596
gtcgcaatcc cttttaaatc aggtcaagtt tcctac 36

<210> 597
<211> 37
<212> DNA
<213> Artificial

<220>
<223> Artificial sequence

<400> 597
gtcttaatcc cttcttaata aggtctctgg tttaaat 37

<210> 598
<211> 36
<212> DNA
<213> Artificial

<220>
<223> Artificial sequence

<400> 598
gttagaacgc tgccctgaat taaaagggat tgagac 36

<210> 599
<211> 36
<212> DNA
<213> Artificial

<220>
<223> Artificial sequence

<400> 599
gtcacaatcc ttgttttagt ggacagtttt tgcaat 36

<210> 600
<211> 36
<212> DNA
<213> Artificial

<220>
<223> Artificial sequence

<400> 600
attcggagca agatccacta aaacaaggat tgaaac 36

<210> 601
<211> 37
<212> DNA
<213> Artificial

<220>
<223> Artificial sequence

<400> 601
gtctgaaaag acctaatccc cgcaagggga ctgaaac 37

<210> 602
<211> 33
<212> DNA
<213> Artificial

<220>
<223> Artificial sequence

<400> 602
gttgcaactt acaccccaca aggggacgga aac 33

<210> 603
<211> 36
<212> DNA
<213> Artificial

<220>
<223> Artificial sequence

<400> 603
attgagaaat gtagctcccc gataggggac ggaaac 36

<210> 604
<211> 35
<212> DNA
<213> Artificial

<220>
<223> Artificial sequence

<400> 604
gtcgcaaaca aaaagccccg taaggggatt gcaac 35

<210>  605
<211>  36
<212>  DNA
<213>  Artificial

<220>
<223>  Artificial sequence

<400>  605
gatataaacc taattacctc gagaggggac ggaaac                                36

<210>  606
<211>  36
<212>  DNA
<213>  Artificial

<220>
<223>  Artificial sequence

<400>  606
gataattacc tatctccgag ttaaggagac gaaaac                                36

<210>  607
<211>  37
<212>  DNA
<213>  Artificial

<220>
<223>  Artificial sequence

<400>  607
gtttcagtcc ccttccttac ggggtaacgg ccatctc                               37

<210>  608
<211>  35
<212>  DNA
<213>  Artificial

<220>
<223>  Artificial sequence

<400>  608
gtcgcaatcc cttcgtaaat caggtcaatt ctttc                                 35

<210>  609
<211>  36
<212>  DNA
<213>  Artificial

<220>
<223>  Artificial sequence

<400>  609
gttctcgtcc cctattcttc ggggtagtta tcgatc                                36

<210>  610

<211> 36
<212> DNA
<213> Artificial

<220>
<223> Artificial sequence

<400> 610
gtctcaatcc cttttagaac agggctaggt tagaac 36

<210> 611
<211> 36
<212> DNA
<213> Artificial

<220>
<223> Artificial sequence

<400> 611
gtcttaatcc ttattatact ggaagtactc tatgag 36

<210> 612
<211> 37
<212> DNA
<213> Artificial

<220>
<223> Artificial sequence

<400> 612
gtcgcaatcc cctgaaaacg ggtctcgtcc ctgcaag 37

<210> 613
<211> 37
<212> DNA
<213> Artificial

<220>
<223> Artificial sequence

<400> 613
gtccttacgg acgctccctg actgaaggga ttaagac 37

<210> 614
<211> 37
<212> DNA
<213> Artificial

<220>
<223> Artificial sequence

<400> 614
gtcgcaatcc cttattcatc aggtctctgt ttccaac 37

<210> 615
<211> 36
<212> DNA
<213> Artificial

<220>
<223> Artificial sequence

<400> 615
attaaaaaga attgaactcc gaaaggagac ggaaac 36

<210> 616
<211> 36
<212> DNA
<213> Artificial

<220>
<223> Artificial sequence

<400> 616
gtttccgtcc cttacagggg ttattcgttt tttaat 36

<210> 617
<211> 30
<212> DNA
<213> Artificial

<220>
<223> Artificial sequence

<400> 617
tacctatcta cctcgagagg ggccggaaac 30

<210> 618
<211> 33
<212> DNA
<213> Artificial

<220>
<223> Artificial sequence

<400> 618
atttcaacct tatccccgca aggggacgga aac 33

<210> 619
<211> 35
<212> DNA
<213> Artificial

<220>
<223> Artificial sequence

<400> 619
gtttccgtcc cctaatgggg atcatttaat ttaac 35

<210> 620
<211> 37
<212> DNA
<213> Artificial

<220>
<223> Artificial sequence

<400> 620
gtcttaatcc ttgttttaat ggaagatact ctctgag 37

<210> 621
<211> 36
<212> DNA
<213> Artificial

<220>
<223> Artificial sequence

<400> 621
cttcaaaaaa ccaaaatccc cgcgagggga ttgaaa 36

<210> 622
<211> 36
<212> DNA
<213> Artificial

<220>
<223> Artificial sequence

<400> 622
gtttccgtcc ccttatgggg tttcttattt tcaaat 36

<210> 623
<211> 25
<212> DNA
<213> Artificial

<220>
<223> Artificial sequence

<400> 623
gtatcagaaa agaatgaatt gaaag 25

<210> 624
<211> 31
<212> DNA
<213> Artificial

<220>
<223> Artificial sequence

<400> 624
tgtttcaatc cttcttaggt aagcccaaaa c 31

<210> 625
<211> 36
<212> DNA
<213> Artificial

<220>
<223> Artificial sequence

<400> 625
ccttacctat taggtcaaat aggattagtt ggaaac 36

<210> 626
<211> 36
<212> DNA
<213> Artificial

<220>
<223> Artificial sequence

<400> 626
gtcttaatcc ctttgatatc agggctcggt tcggac 36

<210> 627
<211> 36
<212> DNA
<213> Artificial

<220>
<223> Artificial sequence

<400> 627
gtctctattt attagagaaa ttaattgaat ggaaac 36

<210> 628
<211> 36
<212> DNA
<213> Artificial

<220>
<223> Artificial sequence

<400> 628
gtcccgctcg ccgtgatgcc ggaaggcgtt gagcac 36

<210> 629
<211> 35
<212> DNA
<213> Artificial

<220>
<223> Artificial sequence

<400> 629
gtttccattc tattagcttc ctcctaagag aaaag 35

<210> 630
<211> 37
<212> DNA
<213> Artificial

<220>
<223> Artificial sequence

<400> 630
ataagaatag gtaatccagt aaaataagga ttgaaac 37

<210> 631

<211> 36
<212> DNA
<213> Artificial

<220>
<223> Artificial sequence

<400> 631
gtcttaatcc cttagaaatc agggagcgtt tcagac 36

<210> 632
<211> 36
<212> DNA
<213> Artificial

<220>
<223> Artificial sequence

<400> 632
gtagcgactt tcattagaaa gaaaaggtgt tgaaac 36

<210> 633
<211> 36
<212> DNA
<213> Artificial

<220>
<223> Artificial sequence

<400> 633
ccccgcttcc ctctgggaag tggaatcaat ggaaac 36

<210> 634
<211> 35
<212> DNA
<213> Artificial

<220>
<223> Artificial sequence

<400> 634
gttcaaacct tccccgattt ctaggggatt aagac 35

<210> 635
<211> 32
<212> DNA
<213> Artificial

<220>
<223> Artificial sequence

<400> 635
gttcaactac ctttcatata aggaattaag ac 32

<210> 636
<211> 36
<212> DNA
<213> Artificial

<220>
<223>  Artificial sequence

<400>  636
gtttcaatgg ggccacggct tttcagccat ggatac                                  36


<210>  637
<211>  37
<212>  DNA
<213>  Artificial

<220>
<223>  Artificial sequence

<400>  637
gtctcgaaat aactacctct catggagaga ggtttac                                 37


<210>  638
<211>  25
<212>  DNA
<213>  Artificial

<220>
<223>  Artificial sequence

<400>  638
gcagaatcta caaaagaatt gaaac                                              25


<210>  639
<211>  35
<212>  DNA
<213>  Artificial

<220>
<223>  Artificial sequence

<400>  639
cttttaactt cttagcaagt ttaattaatg gaaac                                   35


<210>  640
<211>  36
<212>  DNA
<213>  Artificial

<220>
<223>  Artificial sequence

<400>  640
gtcgtaatcc cttcaagtca ggtcattgat tccaat                                  36


<210>  641
<211>  37
<212>  DNA
<213>  Artificial

<220>
<223>  Artificial sequence

<400> 641
gtttcaatcc ttgttttagt ggatcttgct cacgaat 37

<210> 642
<211> 37
<212> DNA
<213> Artificial

<220>
<223> Artificial sequence

<400> 642
gtcgagaagt agcttccact aaaacaagga ttgaaac 37

<210> 643
<211> 35
<212> DNA
<213> Artificial

<220>
<223> Artificial sequence

<400> 643
atttgagtta cttccagtag aataaggatt aagac 35

<210> 644
<211> 36
<212> DNA
<213> Artificial

<220>
<223> Artificial sequence

<400> 644
gtcttaatcc cttttgtatc agggcagcat tttatc 36

<210> 645
<211> 35
<212> DNA
<213> Artificial

<220>
<223> Artificial sequence

<400> 645
gtcgcaatac tctgcgagta cgattatatc aaaag 35

<210> 646
<211> 37
<212> DNA
<213> Artificial

<220>
<223> Artificial sequence

<400> 646
ctcactatac atacacagtc cgtaaggcta ttaagac 37

<210> 647
<211> 35
<212> DNA
<213> Artificial

<220>
<223> Artificial sequence

<400> 647
gtttccatac gtttaacttt caaagaagtt taaag 35

<210> 648
<211> 30
<212> DNA
<213> Artificial

<220>
<223> Artificial sequence

<400> 648
gtttttagct tacctataag gaattgaaac 30

<210> 649
<211> 37
<212> DNA
<213> Artificial

<220>
<223> Artificial sequence

<400> 649
ggccaaaacc aggccccgaa acgcagggga ttaagac 37

<210> 650
<211> 32
<212> DNA
<213> Artificial

<220>
<223> Artificial sequence

<400> 650
gtcgcactct acatgagtgc gtggattgaa at 32

<210> 651
<211> 32
<212> DNA
<213> Artificial

<220>
<223> Artificial sequence

<400> 651
gtttcaatcc acgctcccgc atagggagcg ac 32

<210> 652

<211> 30
<212> DNA
<213> Artificial

<220>
<223> Artificial sequence

<400> 652
gttgaacatt aacatgagat gtatttaaat 30

<210> 653
<211> 26
<212> DNA
<213> Artificial

<220>
<223> Artificial sequence

<400> 653
ataccttacc tataaggaat tgagac 26

<210> 654
<211> 30
<212> DNA
<213> Artificial

<220>
<223> Artificial sequence

<400> 654
gtatcaatat ctattaggta aggtatctac 30

<210> 655
<211> 30
<212> DNA
<213> Artificial

<220>
<223> Artificial sequence

<400> 655
atttcaatac atctaatgtt tttatttatc 30

<210> 656
<211> 30
<212> DNA
<213> Artificial

<220>
<223> Artificial sequence

<400> 656
gtttcaatcc ctcataggtt cgattcaaac 30

<210> 657
<211> 29
<212> DNA
<213> Artificial

<220>
<223> Artificial sequence

<400> 657
tattaggtca aataggatta gttggaaac 29

<210> 658
<211> 28
<212> DNA
<213> Artificial

<220>
<223> Artificial sequence

<400> 658
tttctaagct gcctgtgcgg cagtgaac 28

<210> 659
<211> 37
<212> DNA
<213> Artificial

<220>
<223> Artificial sequence

<400> 659
gtatctatac atattcagtc agtaagacta tcaagac 37

<210> 660
<211> 36
<212> DNA
<213> Artificial

<220>
<223> Artificial sequence

<400> 660
tcccccactc gctaggggac gcgatcgatt ggaaac 36

<210> 661
<211> 36
<212> DNA
<213> Artificial

<220>
<223> Artificial sequence

<400> 661
gtcttaatcc cttctttatc aggtctgtta ttcaat 36

<210> 662
<211> 37
<212> DNA
<213> Artificial

<220>
<223> Artificial sequence

<400> 662
gattcaatac cagccctgaa ttcaaaggga ttaagac 37

<210> 663
<211> 37
<212> DNA
<213> Artificial

<220>
<223> Artificial sequence

<400> 663
ctttcaatac cctacgacgg gtcgtaccag ttgcaac 37

<210> 664
<211> 36
<212> DNA
<213> Artificial

<220>
<223> Artificial sequence

<400> 664
gttggaaaca tgccctgatt taaaagggat taagac 36

<210> 665
<211> 36
<212> DNA
<213> Artificial

<220>
<223> Artificial sequence

<400> 665
gtcttaatcc ccgattagtg ggcaagtct tccgac 36

<210> 666
<211> 36
<212> DNA
<213> Artificial

<220>
<223> Artificial sequence

<400> 666
ctcgcattga tgacccgttc taaaggggat tgagac 36

<210> 667
<211> 36
<212> DNA
<213> Artificial

<220>
<223> Artificial sequence

<400> 667
ctcgcgccga ctgggttaag tggcttagtt ggaaac 36

<210> 668
<211> 37
<212> DNA
<213> Artificial

<220>
<223> Artificial sequence

<400> 668
ggcggcgccc cctccccgat gagaagggga ttgaaac 37

<210> 669
<211> 37
<212> DNA
<213> Artificial

<220>
<223> Artificial sequence

<400> 669
cttgcgccga tcctttgcga tcggattagt tggaaac 37

<210> 670
<211> 37
<212> DNA
<213> Artificial

<220>
<223> Artificial sequence

<400> 670
gtcttaatcc cttcttaatc aggtcgtttg tttggac 37

<210> 671
<211> 35
<212> DNA
<213> Artificial

<220>
<223> Artificial sequence

<400> 671
atgtagatgt attccagtat aataaggatt aagac 35

<210> 672
<211> 36
<212> DNA
<213> Artificial

<220>
<223> Artificial sequence

<400> 672
gtcaccactc gctggtgaca ccaattgatt ggaaac 36

<210> 673

<211>  37
<212>  DNA
<213>  Artificial

<220>
<223>  Artificial sequence

<400>  673
gaagaaagac ataccctgaa gaagaaggga ttacgac                                37

<210>  674
<211>  36
<212>  DNA
<213>  Artificial

<220>
<223>  Artificial sequence

<400>  674
gtctcaatcc cctctaatcg gggcattcgt tctgac                                 36

<210>  675
<211>  36
<212>  DNA
<213>  Artificial

<220>
<223>  Artificial sequence

<400>  675
ctccccactc gctggggaaa ttaattgaat ggaaac                                 36

<210>  676
<211>  36
<212>  DNA
<213>  Artificial

<220>
<223>  Artificial sequence

<400>  676
gtttccattc attacgcttc acgaattaga agcaag                                 36

<210>  677
<211>  36
<212>  DNA
<213>  Artificial

<220>
<223>  Artificial sequence

<400>  677
ctcagaaacc cccgcccgct aatagggctt tgtgac                                 36

<210>  678
<211>  30
<212>  DNA
<213>  Artificial

<220>
<223> Artificial sequence

<400> 678
gtttttagcc tacctataag gaattgaaac 30

<210> 679
<211> 37
<212> DNA
<213> Artificial

<220>
<223> Artificial sequence

<400> 679
gtttcaatcc ttgttttagt ggatcttgct cacgaat 37

<210> 680
<211> 37
<212> DNA
<213> Artificial

<220>
<223> Artificial sequence

<400> 680
gtttctattc gctgagtcgc gagacgttca gcggaac 37

<210> 681
<211> 37
<212> DNA
<213> Artificial

<220>
<223> Artificial sequence

<400> 681
gtcagaaagc accgagcgcc ataaggtgca ttaagac 37

<210> 682
<211> 35
<212> DNA
<213> Artificial

<220>
<223> Artificial sequence

<400> 682
gtttaaaatg actcgccccg taaggggacg gaaac 35

<210> 683
<211> 36
<212> DNA
<213> Artificial

<220>
<223> Artificial sequence

<400> 683
gtttagaagg acactaaccc cgaaggggat tgaaac 36

<210> 684
<211> 37
<212> DNA
<213> Artificial

<220>
<223> Artificial sequence

<400> 684
ctcatagagt atcttccatt aaaacaagga ttaagac 37

<210> 685
<211> 36
<212> DNA
<213> Artificial

<220>
<223> Artificial sequence

<400> 685
gttacgctta cacttacccc gcaaggggat ggaaac 36

<210> 686
<211> 35
<212> DNA
<213> Artificial

<220>
<223> Artificial sequence

<400> 686
gtttccatcc ccgtgagggg taaatgattt aaaac 35

<210> 687
<211> 25
<212> DNA
<213> Artificial

<220>
<223> Artificial sequence

<400> 687
gatgaatccc aaaagggatt gaaag 25

<210> 688
<211> 35
<212> DNA
<213> Artificial

<220>
<223> Artificial sequence

<400> 688
gttgcaacag tacttaccgt ttacggtatt gaaac 35

<210> 689
<211> 35
<212> DNA
<213> Artificial

<220>
<223> Artificial sequence

<400> 689
gtattagtac tgcccggttt agacgggatt aagac 35

<210> 690
<211> 37
<212> DNA
<213> Artificial

<220>
<223> Artificial sequence

<400> 690
gtttcaatcc gcttcgaggt tcgcggtggt ttcagac 37

<210> 691
<211> 33
<212> DNA
<213> Artificial

<220>
<223> Artificial sequence

<400> 691
gtttcaattc ccgcaatgcg ggtaagatac atc 33

<210> 692
<211> 36
<212> DNA
<213> Artificial

<220>
<223> Artificial sequence

<400> 692
attgcatgca atccataccg cttgcggtat tgaaac 36

<210> 693
<211> 33
<212> DNA
<213> Artificial

<220>
<223> Artificial sequence

<400> 693
gctacaattc gcgccccttg cggggactgt aac 33

<210> 694

<211> 33
<212> DNA
<213> Artificial

<220>
<223> Artificial sequence

<400> 694
gtctctttct gtatggagag agtggattga aat 33

<210> 695
<211> 33
<212> DNA
<213> Artificial

<220>
<223> Artificial sequence

<400> 695
gatgcagccc gctcccctca cggggacggc aac 33

<210> 696
<211> 33
<212> DNA
<213> Artificial

<220>
<223> Artificial sequence

<400> 696
atttcaatcc acacagcctt cgcaggctgt gac 33

<210> 697
<211> 37
<212> DNA
<213> Artificial

<220>
<223> Artificial sequence

<400> 697
gtgtcaatgc ccttactcgg gctattccca tttctac 37

<210> 698
<211> 32
<212> DNA
<213> Artificial

<220>
<223> Artificial sequence

<400> 698
atttcaatcc acgaggcaac ataagcctcg ac 32

<210> 699
<211> 36
<212> DNA
<213> Artificial

<220>
<223> Artificial sequence

<400> 699
gtcttaatcc ccttaaatcg ggtcatgttg ttaaat 36

<210> 700
<211> 30
<212> DNA
<213> Artificial

<220>
<223> Artificial sequence

<400> 700
gtaagtacct tacctataag gaatggaaac 30

<210> 701
<211> 30
<212> DNA
<213> Artificial

<220>
<223> Artificial sequence

<400> 701
gtttttaccc tacctatgag gaattgaaac 30

<210> 702
<211> 29
<212> DNA
<213> Artificial

<220>
<223> Artificial sequence

<400> 702
gtcggacatt acctatgagg aattgatac 29

<210> 703
<211> 30
<212> DNA
<213> Artificial

<220>
<223> Artificial sequence

<400> 703
gttgcgatcc ctccaggggt gatcagcgac 30

<210> 704
<211> 30
<212> DNA
<213> Artificial

<220>
<223> Artificial sequence

<400> 704
gttgcgaccc ctcttagggg cgatgaggac 30

<210> 705
<211> 30
<212> DNA
<213> Artificial

<220>
<223> Artificial sequence

<400> 705
atttaaatac atctcatgtt aatgttcaac 30

<210> 706
<211> 37
<212> DNA
<213> Artificial

<220>
<223> Artificial sequence

<400> 706
gtttcaatcc ttattttact ggattctctt cttttat 37

<210> 707
<211> 37
<212> DNA
<213> Artificial

<220>
<223> Artificial sequence

<400> 707
gtccggcagc atgtgccctc aatcaaggga ttaagac 37

<210> 708
<211> 37
<212> DNA
<213> Artificial

<220>
<223> Artificial sequence

<400> 708
gtttcaatcc ttgttttagt ggatcttgct cacgaat 37

<210> 709
<211> 36
<212> DNA
<213> Artificial

<220>
<223> Artificial sequence

<400> 709
gtctgaacca ccccctcacc gcgaggtgat tgaaac 36

<210> 710
<211> 37
<212> DNA
<213> Artificial

<220>
<223> Artificial sequence

<400> 710
gttggaaacc aagtaccgcg atatagcgga tggaaac 37

<210> 711
<211> 37
<212> DNA
<213> Artificial

<220>
<223> Artificial sequence

<400> 711
gtacgagtgc atcatccatt aaaacaagga ttaagac 37

<210> 712
<211> 35
<212> DNA
<213> Artificial

<220>
<223> Artificial sequence

<400> 712
gtttccgtcc ccttgcgggg aaatggtagt taaac 35

<210> 713
<211> 37
<212> DNA
<213> Artificial

<220>
<223> Artificial sequence

<400> 713
gtgtcaatgc cctcaacggg cttctactca tttctac 37

<210> 714
<211> 37
<212> DNA
<213> Artificial

<220>
<223> Artificial sequence

<400> 714
gtctcaatcc ccttctcatc ggggaggcgg cgccgcc 37

<210> 715

<211> 37
<212> DNA
<213> Artificial

<220>
<223> Artificial sequence

<400> 715
gttgcaccac atacaaagct gcgtgcggca ttgaaac 37

<210> 716
<211> 37
<212> DNA
<213> Artificial

<220>
<223> Artificial sequence

<400> 716
gtttcaatcc ttgttttagt ggaagctact tctcgac 37

<210> 717
<211> 37
<212> DNA
<213> Artificial

<220>
<223> Artificial sequence

<400> 717
atttgattag aatttccccg atctagggga ttgaaac 37

<210> 718
<211> 37
<212> DNA
<213> Artificial

<220>
<223> Artificial sequence

<400> 718
gtttccatac gccttatggc gtgaccttgt ttcggac 37

<210> 719
<211> 36
<212> DNA
<213> Artificial

<220>
<223> Artificial sequence

<400> 719
gtcttaatcc cctacaaagc ggggctcctt tctgac 36

<210> 720
<211> 37
<212> DNA
<213> Artificial

<220>
<223> Artificial sequence

<400> 720
gtcttaatcc ttgttgtagt ggacaatggt ctcgtac 37

<210> 721
<211> 35
<212> DNA
<213> Artificial

<220>
<223> Artificial sequence

<400> 721
gtcttaatcc cttttaaatc agggctggtt cagac 35

<210> 722
<211> 37
<212> DNA
<213> Artificial

<220>
<223> Artificial sequence

<400> 722
gtattgaagg ttatccattt ataataaggt ttaaaac 37

<210> 723
<211> 36
<212> DNA
<213> Artificial

<220>
<223> Artificial sequence

<400> 723
gtaccgtgac attaagtcct tctcttgttt cataac 36

<210> 724
<211> 36
<212> DNA
<213> Artificial

<220>
<223> Artificial sequence

<400> 724
gtaccgtgac attaagtcct tctcttgttt cataac 36

<210> 725
<211> 35
<212> DNA
<213> Artificial

<220>
<223> Artificial sequence

<400> 725
atagatatag acagaagctt ttaatgtgat gggac 35

<210> 726
<211> 37
<212> DNA
<213> Artificial

<220>
<223> Artificial sequence

<400> 726
gtttcaatcc gcttcctcta gcggcatccc tcaatag 37

<210> 727
<211> 36
<212> DNA
<213> Artificial

<220>
<223> Artificial sequence

<400> 727
cttgaagact aaaggaagga attaatgtca cggtac 36

<210> 728
<211> 37
<212> DNA
<213> Artificial

<220>
<223> Artificial sequence

<400> 728
gttggtgcat cagcccggaa ttatgatgtt ttggtac 37

<210> 729
<211> 36
<212> DNA
<213> Artificial

<220>
<223> Artificial sequence

<400> 729
gtaccgtgac atcaaaaatt cctgccgcct ttcaac 36

<210> 730
<211> 36
<212> DNA
<213> Artificial

<220>
<223> Artificial sequence

<400> 730
gttccgtgac attaagggtt tatcggctgg ggcaat 36

<210> 731
<211> 30
<212> DNA
<213> Artificial

<220>
<223> Artificial sequence

<400> 731
gttaaaatca gaccattgtg ggattgaaag 30

<210> 732
<211> 37
<212> DNA
<213> Artificial

<220>
<223> Artificial sequence

<400> 732
gttgcagaag tcgatctagt aaaacaagga ttgaaac 37

<210> 733
<211> 38
<212> DNA
<213> Artificial

<220>
<223> Artificial sequence

<400> 733
gttggaaaca gagacctgat gaataaggga ttgcgact 38

<210> 734
<211> 30
<212> DNA
<213> Artificial

<220>
<223> Artificial sequence

<400> 734
ctttcaatcc cacaatggtc tgattttaac 30

<210> 735
<211> 37
<212> DNA
<213> Artificial

<220>
<223> Artificial sequence

<400> 735
gtttcaatcc ttgttttgct ggaagctgtt tttcaac 37

<210> 736

<211> 33
<212> DNA
<213> Artificial

<220>
<223> Artificial sequence

<400> 736
gaaaggaact aatcctcgga aggggattga aac 33

<210> 737
<211> 37
<212> DNA
<213> Artificial

<220>
<223> Artificial sequence

<400> 737
ctgataaaat tacccttgat gaataaagga ttgcgac 37

<210> 738
<211> 33
<212> DNA
<213> Artificial

<220>
<223> Artificial sequence

<400> 738
caatgcattg cctattgata atgctatcga tcc 33

<210> 739
<211> 33
<212> DNA
<213> Artificial

<220>
<223> Artificial sequence

<400> 739
caatgcattg cctattgata atgctatcga tcc 33

<210> 740
<211> 33
<212> DNA
<213> Artificial

<220>
<223> Artificial sequence

<400> 740
caatgcatta ccctaaagta atgctactgg cac 33

<210> 741
<211> 33
<212> DNA
<213> Artificial

<220>
<223> Artificial sequence

<400> 741
caatgcatta ccctaaagta atgctactgg cac 33

<210> 742
<211> 29
<212> DNA
<213> Artificial

<220>
<223> Artificial sequence

<400> 742
gtatttcccg cgtgcgcggg ggtgagcgg 29

<210> 743
<211> 29
<212> DNA
<213> Artificial

<220>
<223> Artificial sequence

<400> 743
gtatttcccg cgtgcgcggg ggtgagcgg 29

<210> 744
<211> 29
<212> DNA
<213> Artificial

<220>
<223> Artificial sequence

<400> 744
gtgcgccccg cgtgagcggg ggttgaagg 29

<210> 745
<211> 29
<212> DNA
<213> Artificial

<220>
<223> Artificial sequence

<400> 745
gtgcgccccg cgtgagcggg ggttgaagg 29

<210> 746
<211> 37
<212> DNA
<213> Artificial

<220>
<223> Artificial sequence

<400> 746
gttgtaataa gctgtaaagt gttgcggctt tggaaac 37

<210> 747
<211> 37
<212> DNA
<213> Artificial

<220>
<223> Artificial sequence

<400> 747
gttgtaataa gctgtaaagt gttgcggctt tggaaac 37

<210> 748
<211> 28
<212> DNA
<213> Artificial

<220>
<223> Artificial sequence

<400> 748
cgataacccc cgctcgcgcg gggcagac 28

<210> 749
<211> 28
<212> DNA
<213> Artificial

<220>
<223> Artificial sequence

<400> 749
cgataacccc cgctcgcgcg gggcagac 28

<210> 750
<211> 23
<212> DNA
<213> Artificial

<220>
<223> Artificial sequence

<400> 750
cttctaaatc acctatgcgg tga 23

<210> 751
<211> 23
<212> DNA
<213> Artificial

<220>
<223> Artificial sequence

<400> 751
cttctaaatc acctatgcgg tga 23

<210> 752
<211> 23
<212> DNA
<213> Artificial

<220>
<223> Artificial sequence

<400> 752
ctttaacccc cgtaggtacg ggg 23

<210> 753
<211> 23
<212> DNA
<213> Artificial

<220>
<223> Artificial sequence

<400> 753
ctttaacccc cgcaggtacg ggg 23

<210> 754
<211> 36
<212> DNA
<213> Artificial

<220>
<223> Artificial sequence

<400> 754
gttccaacca cgcccccaca gggacgtgct ttccac 36

<210> 755
<211> 36
<212> DNA
<213> Artificial

<220>
<223> Artificial sequence

<400> 755
gttccaacca cgcccccaca gggacgtgct ttccac 36

<210> 756
<211> 36
<212> DNA
<213> Artificial

<220>
<223> Artificial sequence

<400> 756
gctcaagcca ccctgtaatt gggtggttac tagaac 36

<210> 757

<211>  36
<212>  DNA
<213>  Artificial

<220>
<223>  Artificial sequence

<400>  757
gctcaagcca ccctgtaatt gggtggttac tagaac    36

<210>  758
<211>  36
<212>  DNA
<213>  Artificial

<220>
<223>  Artificial sequence

<400>  758
ggtggaagaa ctgccccaag ggtagtggt tggaac    36

<210>  759
<211>  36
<212>  DNA
<213>  Artificial

<220>
<223>  Artificial sequence

<400>  759
ggtggaagaa ctgccccaag ggtagtggt tggaac    36

<210>  760
<211>  28
<212>  DNA
<213>  Artificial

<220>
<223>  Artificial sequence

<400>  760
cggttatccg cgcccgcgta ggcgcgac    28

<210>  761
<211>  29
<212>  DNA
<213>  Artificial

<220>
<223>  Artificial sequence

<400>  761
gtattccccc cgcatgcggg ggttatcgg    29

<210>  762
<211>  29
<212>  DNA
<213>  Artificial

<220>
<223> Artificial sequence

<400> 762
gtcacgcccc tgcgggcgtg ggagaaccg 29

<210> 763
<211> 28
<212> DNA
<213> Artificial

<220>
<223> Artificial sequence

<400> 763
gtgagtcgcc gtgtaggcgg ttgaaagt 28

<210> 764
<211> 27
<212> DNA
<213> Artificial

<220>
<223> Artificial sequence

<400> 764
gttttccccc gcgcacgcgg ggacgac 27

<210> 765
<211> 28
<212> DNA
<213> Artificial

<220>
<223> Artificial sequence

<400> 765
gtgtgccgcc gtgtaggtgg cttgaagt 28

<210> 766
<211> 31
<212> DNA
<213> Artificial

<220>
<223> Artificial sequence

<400> 766
ctgtctcctc cgcgcacgcg cgggtgaacc g 31

<210> 767
<211> 26
<212> DNA
<213> Artificial

<220>
<223> Artificial sequence

<400> 767
gccgtcgttt gaggcggcac gacaac 26

<210> 768
<211> 28
<212> DNA
<213> Artificial

<220>
<223> Artificial sequence

<400> 768
ggttcacccc cgcgtacgcg gggaatac 28

<210> 769
<211> 27
<212> DNA
<213> Artificial

<220>
<223> Artificial sequence

<400> 769
acccgctggc tggaagtatt ccccccg 27

<210> 770
<211> 29
<212> DNA
<213> Artificial

<220>
<223> Artificial sequence

<400> 770
ccgataaccc ccgcacacag gggaaacac 29

<210> 771
<211> 29
<212> DNA
<213> Artificial

<220>
<223> Artificial sequence

<400> 771
gtctctcccg tgcgcgggag ggttaacgg 29

<210> 772
<211> 29
<212> DNA
<213> Artificial

<220>
<223> Artificial sequence

<400> 772
ccgataaccc ccgcatgcgg ggggaatac 29

<210> 773
<211> 29
<212> DNA
<213> Artificial

<220>
<223> Artificial sequence

<400> 773
ccactcaccc gtgcatgcac gggaaatac 29

<210> 774
<211> 25
<212> DNA
<213> Artificial

<220>
<223> Artificial sequence

<400> 774
gtatgctccg caggcgcgga gatgt 25

<210> 775
<211> 24
<212> DNA
<213> Artificial

<220>
<223> Artificial sequence

<400> 775
gatgatctac tatagaattg aaag 24

<210> 776
<211> 40
<212> DNA
<213> Artificial

<220>
<223> Artificial sequence

<400> 776
cggcctcgca acacgcagac cctactggtc tggttgcgag 40

<210> 777
<211> 36
<212> DNA
<213> Artificial

<220>
<223> Artificial sequence

<400> 777
ccagtaaggg tagctcagca ccctataagc tgggac 36

<210> 778

<211> 24
<212> DNA
<213> Artificial

<220>
<223> Artificial sequence

<400> 778
ctttcaactc catgtaatgg attc 24

<210> 779
<211> 30
<212> DNA
<213> Artificial

<220>
<223> Artificial sequence

<400> 779
gtttcaattc tcctagagtc ttattgcaac 30

<210> 780
<211> 35
<212> DNA
<213> Artificial

<220>
<223> Artificial sequence

<400> 780
gttgcaacag tgcttaccgt ttacggtatt gaaac 35

<210> 781
<211> 35
<212> DNA
<213> Artificial

<220>
<223> Artificial sequence

<400> 781
gttgcaacag tgcttaccgt ttacggtatt gaaac 35

<210> 782
<211> 29
<212> DNA
<213> Artificial

<220>
<223> Artificial sequence

<400> 782
cttctaaacc tacataggat atttcaaac 29

<210> 783
<211> 29
<212> DNA
<213> Artificial

<220>
<223>  Artificial sequence

<400>  783
cttctaaacc tacataggat atttcaaac                                    29

<210>  784
<211>  30
<212>  DNA
<213>  Artificial

<220>
<223>  Artificial sequence

<400>  784
gttctcacaa cctatagtta taatccaaac                                   30

<210>  785
<211>  29
<212>  DNA
<213>  Artificial

<220>
<223>  Artificial sequence

<400>  785
cttctaaacc tacataggat atttcaaac                                    29

<210>  786
<211>  29
<212>  DNA
<213>  Artificial

<220>
<223>  Artificial sequence

<400>  786
cttctaaacc tacataggat atttcaaac                                    29

<210>  787
<211>  30
<212>  DNA
<213>  Artificial

<220>
<223>  Artificial sequence

<400>  787
gtttagttgc tacctttgag ggattgaaac                                   30

<210>  788
<211>  30
<212>  DNA
<213>  Artificial

<220>
<223>  Artificial sequence

<400> 788
gtttttactc tacctataag ggattgaaac 30

<210> 789
<211> 35
<212> DNA
<213> Artificial

<220>
<223> Artificial sequence

<400> 789
gttgcaacag tgcttaccgt ttacggtatt gaaac 35

<210> 790
<211> 30
<212> DNA
<213> Artificial

<220>
<223> Artificial sequence

<400> 790
gtttcaaagc cagatagcta ggatgggaag 30

<210> 791
<211> 35
<212> DNA
<213> Artificial

<220>
<223> Artificial sequence

<400> 791
gttgcaacag tgcttaccgt ttacggtatt gaaac 35

<210> 792
<211> 35
<212> DNA
<213> Artificial

<220>
<223> Artificial sequence

<400> 792
gtttcaatac cgtaaacggt aagcactgtt gcaac 35

<210> 793
<211> 35
<212> DNA
<213> Artificial

<220>
<223> Artificial sequence

<400> 793
gttgcaacag tgcttaccgt ttacggtatt gaaac 35

<210> 794
<211> 29
<212> DNA
<213> Artificial

<220>
<223> Artificial sequence

<400> 794
cttctaaacc tacataggat atttcaaac 29

<210> 795
<211> 30
<212> DNA
<213> Artificial

<220>
<223> Artificial sequence

<400> 795
cttttaaccc ctcacagaag gtatctgcac 30

<210> 796
<211> 36
<212> DNA
<213> Artificial

<220>
<223> Artificial sequence

<400> 796
agtttcaata ccgtaaacgg taagcactgt tgcaac 36

<210> 797
<211> 35
<212> DNA
<213> Artificial

<220>
<223> Artificial sequence

<400> 797
gttgcaacag tgcttaccgt ttacggtatt gaaac 35

<210> 798
<211> 35
<212> DNA
<213> Artificial

<220>
<223> Artificial sequence

<400> 798
gtttcaatac cgtaaacggt aagcactgtt gcaac 35

<210> 799

<211> 35
<212> DNA
<213> Artificial

<220>
<223> Artificial sequence

<400> 799
gtttcaatac cgtaaacggt aagcactgtt gcaac 35

<210> 800
<211> 37
<212> DNA
<213> Artificial

<220>
<223> Artificial sequence

<400> 800
ctttttgtat cctccaacgg atcgaatagg ttgcaac 37

<210> 801
<211> 35
<212> DNA
<213> Artificial

<220>
<223> Artificial sequence

<400> 801
gttgcaacag tgcttaccgt ttacggtatt gaaac 35

<210> 802
<211> 35
<212> DNA
<213> Artificial

<220>
<223> Artificial sequence

<400> 802
gttgcaacag tgcttaccgt ttacggtatt gaaac 35

<210> 803
<211> 23
<212> DNA
<213> Artificial

<220>
<223> Artificial sequence

<400> 803
gtttccacct ataaggggtt gta 23

<210> 804
<211> 36
<212> DNA
<213> Artificial

<220>
<223> Artificial sequence

<400> 804
caagcttatc aagaagggtg aatgctaatt cccagc 36

<210> 805
<211> 30
<212> DNA
<213> Artificial

<220>
<223> Artificial sequence

<400> 805
gttctaaact tccactacat tgtttaaaac 30

<210> 806
<211> 29
<212> DNA
<213> Artificial

<220>
<223> Artificial sequence

<400> 806
gttttatacc ttatttgaat agttaaaag 29

<210> 807
<211> 30
<212> DNA
<213> Artificial

<220>
<223> Artificial sequence

<400> 807
gttctaaact tccactacat tgtttaaaac 30

<210> 808
<211> 30
<212> DNA
<213> Artificial

<220>
<223> Artificial sequence

<400> 808
gttttaaaca atgtagtgga tgtttagaac 30

<210> 809
<211> 30
<212> DNA
<213> Artificial

<220>
<223> Artificial sequence

<400> 809
gttctaaact tctacgtata gtatctaaac 30

<210> 810
<211> 30
<212> DNA
<213> Artificial

<220>
<223> Artificial sequence

<400> 810
gttttaaaca atgtagtgga tgtttagaac 30

<210> 811
<211> 30
<212> DNA
<213> Artificial

<220>
<223> Artificial sequence

<400> 811
gttttaaata atacaatgaa agtttagaac 30

<210> 812
<211> 30
<212> DNA
<213> Artificial

<220>
<223> Artificial sequence

<400> 812
gttttaaata aagtattgga agtttagaac 30

<210> 813
<211> 30
<212> DNA
<213> Artificial

<220>
<223> Artificial sequence

<400> 813
gttttaaaca atgtagtgga tgtttagaac 30

<210> 814
<211> 30
<212> DNA
<213> Artificial

<220>
<223> Artificial sequence

<400> 814
gttttaaata aagtattgta agtttagaac 30

<210> 815
<211> 30
<212> DNA
<213> Artificial

<220>
<223> Artificial sequence

<400> 815
gttctaaact tccactacat tgtttaaaac 30

<210> 816
<211> 30
<212> DNA
<213> Artificial

<220>
<223> Artificial sequence

<400> 816
gttctaaact tccactacat tgtttaaaac 30

<210> 817
<211> 30
<212> DNA
<213> Artificial

<220>
<223> Artificial sequence

<400> 817
gttctaaaca tccactacat tgtttaaaac 30

<210> 818
<211> 30
<212> DNA
<213> Artificial

<220>
<223> Artificial sequence

<400> 818
gtatggatat actacataga agtttagaac 30

<210> 819
<211> 30
<212> DNA
<213> Artificial

<220>
<223> Artificial sequence

<400> 819
gttctaaaca tccactacat tatttaaaac 30

<210> 820

<211> 30
<212> DNA
<213> Artificial

<220>
<223> Artificial sequence

<400> 820
gttctaaact ttcattgtat tatttaaaac 30

<210> 821
<211> 29
<212> DNA
<213> Artificial

<220>
<223> Artificial sequence

<400> 821
ttctaaacat ccactacatt gtttaaaac 29

<210> 822
<211> 30
<212> DNA
<213> Artificial

<220>
<223> Artificial sequence

<400> 822
gttttaaaca atgtattgga agtttagaac 30

<210> 823
<211> 30
<212> DNA
<213> Artificial

<220>
<223> Artificial sequence

<400> 823
gttctaaacc ttcattacat tgtttaaaac 30

<210> 824
<211> 30
<212> DNA
<213> Artificial

<220>
<223> Artificial sequence

<400> 824
gttctaaact tccaatactt tatttaaaac 30

<210> 825
<211> 30
<212> DNA
<213> Artificial

<220>
<223> Artificial sequence

<400> 825
gttctaaact tccactacat tgtttaaaac 30

<210> 826
<211> 30
<212> DNA
<213> Artificial

<220>
<223> Artificial sequence

<400> 826
gttttaaaca atgtagtgga tgtttagaac 30

<210> 827
<211> 30
<212> DNA
<213> Artificial

<220>
<223> Artificial sequence

<400> 827
gttctaaact tccaatacat tgtttaaaac 30

<210> 828
<211> 30
<212> DNA
<213> Artificial

<220>
<223> Artificial sequence

<400> 828
gttctaaaca tccactacat tgtttaaaac 30

<210> 829
<211> 30
<212> DNA
<213> Artificial

<220>
<223> Artificial sequence

<400> 829
gttttaaata aagtattgga agtttagaac 30

<210> 830
<211> 36
<212> DNA
<213> Artificial

<220>
<223> Artificial sequence

<400> 830
atctacaata gtagaaatta tatggctttt atagcc 36

<210> 831
<211> 36
<212> DNA
<213> Artificial

<220>
<223> Artificial sequence

<400> 831
atctacaata gtagaaatta tttaagcctt atagcc 36

<210> 832
<211> 36
<212> DNA
<213> Artificial

<220>
<223> Artificial sequence

<400> 832
atctacaata gtagaaatta tctttatgtt ctagac 36

<210> 833
<211> 36
<212> DNA
<213> Artificial

<220>
<223> Artificial sequence

<400> 833
atctgcaaaa gcagaaattt tcttgtgtta ctcaac 36

<210> 834
<211> 36
<212> DNA
<213> Artificial

<220>
<223> Artificial sequence

<400> 834
gtctataaga cgtatttaat ttctactttt gtagat 36

<210> 835
<211> 36
<212> DNA
<213> Artificial

<220>
<223> Artificial sequence

<400> 835
atctacaata gtagaaatta ttagggatta ctaacc 36

<210> 836
<211> 36
<212> DNA
<213> Artificial

<220>
<223> Artificial sequence

<400> 836
atctaccgaa gtagaaattg ttagttacta tttgac 36

<210> 837
<211> 33
<212> DNA
<213> Artificial

<220>
<223> Artificial sequence

<400> 837
tctagccaca tattaatttc tactgttgta gat 33

<210> 838
<211> 36
<212> DNA
<213> Artificial

<220>
<223> Artificial sequence

<400> 838
atctacacta gtagaaatta tttcttatta tttaac 36

<210> 839
<211> 36
<212> DNA
<213> Artificial

<220>
<223> Artificial sequence

<400> 839
atctacaata gtagaaattt tctttaacct ctaggc 36

<210> 840
<211> 36
<212> DNA
<213> Artificial

<220>
<223> Artificial sequence

<400> 840
atctacaata gtagaaatta cattggctta atagcc 36

<210> 841

<211> 36
<212> DNA
<213> Artificial

<220>
<223> Artificial sequence

<400> 841
ctctaaaacc tatataaaat ttctactttt gtagat 36

<210> 842
<211> 35
<212> DNA
<213> Artificial

<220>
<223> Artificial sequence

<400> 842
tctaacggac ttttaaaatt tctacttttg tagat 35

<210> 843
<211> 36
<212> DNA
<213> Artificial

<220>
<223> Artificial sequence

<400> 843
gttgatactg tgagcgaaat gtctactaag gtagat 36

<210> 844
<211> 35
<212> DNA
<213> Artificial

<220>
<223> Artificial sequence

<400> 844
tctgacagta tatgttaatt tctactctcg tagat 35

<210> 845
<211> 35
<212> DNA
<213> Artificial

<220>
<223> Artificial sequence

<400> 845
ctttataaga acctaagaat ttctactgtt gtaga 35

<210> 846
<211> 36
<212> DNA
<213> Artificial

<220>
<223> Artificial sequence

<400> 846
gtctaaagac cgaagtaaat ttctactctt gtagat 36

<210> 847
<211> 36
<212> DNA
<213> Artificial

<220>
<223> Artificial sequence

<400> 847
gtttaataac cttaaataat ttctactttt gtagat 36

<210> 848
<211> 35
<212> DNA
<213> Artificial

<220>
<223> Artificial sequence

<400> 848
tctaatccat atattagatt tctactttcg tagat 35

<210> 849
<211> 35
<212> DNA
<213> Artificial

<220>
<223> Artificial sequence

<400> 849
cttaatagct gatataaatt tctactgttg tagat 35

<210> 850
<211> 36
<212> DNA
<213> Artificial

<220>
<223> Artificial sequence

<400> 850
gcctaatacc tctataaaat ttctactttt gtagat 36

<210> 851
<211> 34
<212> DNA
<213> Artificial

<220>
<223> Artificial sequence

<400> 851
ttaatattat taaagaattt ctactgttgt agat 34

<210> 852
<211> 34
<212> DNA
<213> Artificial

<220>
<223> Artificial sequence

<400> 852
atctacacta gtagaaattc tgaatgagtt ttag 34

<210> 853
<211> 35
<212> DNA
<213> Artificial

<220>
<223> Artificial sequence

<400> 853
tttaaaaata atatttaatt tctactgttg tagat 35

<210> 854
<211> 36
<212> DNA
<213> Artificial

<220>
<223> Artificial sequence

<400> 854
atctacaata gtagaaatta aaaagggtat ctagac 36

<210> 855
<211> 35
<212> DNA
<213> Artificial

<220>
<223> Artificial sequence

<400> 855
gtctaaagca ctattaaatt tctactattg tagat 35

<210> 856
<211> 36
<212> DNA
<213> Artificial

<220>
<223> Artificial sequence

<400> 856
gttataagga acaaataaat gcctgctgtt gcaggc 36

<210> 857
<211> 36
<212> DNA
<213> Artificial

<220>
<223> Artificial sequence

<400> 857
gtctaacgac cttttaaatt tctactgttt gtagat 36

<210> 858
<211> 36
<212> DNA
<213> Artificial

<220>
<223> Artificial sequence

<400> 858
gtcggatcgc tgagcgagcg atctgagaag tggcac 36

<210> 859
<211> 36
<212> DNA
<213> Artificial

<220>
<223> Artificial sequence

<400> 859
atcgcagtcg ccgcaattcc gggggcgtg aggcgg 36

<210> 860
<211> 35
<212> DNA
<213> Artificial

<220>
<223> Artificial sequence

<400> 860
tctagacata caggtggaaa ggtgagagta aagac 35

<210> 861
<211> 25
<212> DNA
<213> Artificial

<220>
<223> Artificial sequence

<400> 861
gtctttactc tcacctttcc acctg 25

<210> 862

<211> 37
<212> DNA
<213> Artificial

<220>
<223> Artificial sequence

<400> 862
atttgaaggt atctccgata agtaaaacgc atcaaag 37

<210> 863
<211> 25
<212> DNA
<213> Artificial

<220>
<223> Artificial sequence

<400> 863
ttttacatac cccctctcat gggat 25

<210> 864
<211> 32
<212> DNA
<213> Artificial

<220>
<223> Artificial sequence

<400> 864
gttgcaactt acgcataggt gtaaaatacg ag 32

<210> 865
<211> 32
<212> DNA
<213> Artificial

<220>
<223> Artificial sequence

<400> 865
gttgcaatct gtatgctcag gtatactgcg ag 32

<210> 866
<211> 31
<212> DNA
<213> Artificial

<220>
<223> Artificial sequence

<400> 866
gttttgaatg aactatgtag aatgtgaatt t 31

<210> 867
<211> 32
<212> DNA
<213> Artificial

<220>
<223>  Artificial sequence

<400>  867
gttgcaataa acgtatgtgc atgaggtgtg ag                                  32

<210>  868
<211>  32
<212>  DNA
<213>  Artificial

<220>
<223>  Artificial sequence

<400>  868
gatatatctt gtatgcatat gtaggttgtg ag                                  32

<210>  869
<211>  30
<212>  DNA
<213>  Artificial

<220>
<223>  Artificial sequence

<400>  869
gtttaagaat aacaatagtt gtatttaaat                                  30

<210>  870
<211>  30
<212>  DNA
<213>  Artificial

<220>
<223>  Artificial sequence

<400>  870
gttaaaacat aacaatagat gtattgaaat                                  30

<210>  871
<211>  30
<212>  DNA
<213>  Artificial

<220>
<223>  Artificial sequence

<400>  871
gcttaaacat atcctttgtt gattctcaac                                  30

<210>  872
<211>  29
<212>  DNA
<213>  Artificial

<220>
<223>  Artificial sequence

<400> 872
gttttatatt aactatgtgg aatgtaaat 29

<210> 873
<211> 30
<212> DNA
<213> Artificial

<220>
<223> Artificial sequence

<400> 873
atttgattag aactatattg gaatgtaaat 30

<210> 874
<211> 30
<212> DNA
<213> Artificial

<220>
<223> Artificial sequence

<400> 874
gggatcttcc tcgtgtgcgt gggaagaaca 30

<210> 875
<211> 29
<212> DNA
<213> Artificial

<220>
<223> Artificial sequence

<400> 875
gtctgccccg cgtgtgcatg ggtggttcc 29

<210> 876
<211> 29
<212> DNA
<213> Artificial

<220>
<223> Artificial sequence

<400> 876
gttttatatt aactatgtga tatgtaaat 29

<210> 877
<211> 37
<212> DNA
<213> Artificial

<220>
<223> Artificial sequence

<400> 877
gttccaatta atcttaagcc ctattaggga ttgaaac 37

<210> 878
<211> 29
<212> DNA
<213> Artificial

<220>
<223> Artificial sequence

<400> 878
gggctcatcc ccgcgcatac ggggaaaac 29

<210> 879
<211> 29
<212> DNA
<213> Artificial

<220>
<223> Artificial sequence

<400> 879
gtttacacac tacatagtca ttataaaac 29

<210> 880
<211> 29
<212> DNA
<213> Artificial

<220>
<223> Artificial sequence

<400> 880
gtcggccccg cgcacgcggg gatgagccc 29

<210> 881
<211> 29
<212> DNA
<213> Artificial

<220>
<223> Artificial sequence

<400> 881
cggtcagccc ctgcgcgccc gggccggac 29

<210> 882
<211> 28
<212> DNA
<213> Artificial

<220>
<223> Artificial sequence

<400> 882
ggtcgctccc cacgcgtacg gaccggac 28

<210> 883

<211>  37
<212>  DNA
<213>  Artificial

<220>
<223>  Artificial sequence

<400>  883
gtttcaatcc cattgctagg attcattaat aagaaac                                37

<210>  884
<211>  37
<212>  DNA
<213>  Artificial

<220>
<223>  Artificial sequence

<400>  884
gttgcagtgc ccagctcagg ggcttgaaga ctgagac                                37

<210>  885
<211>  36
<212>  DNA
<213>  Artificial

<220>
<223>  Artificial sequence

<400>  885
ggtgcggcag ccggtagaac tgcggcttcc gacacc                                 36

<210>  886
<211>  36
<212>  DNA
<213>  Artificial

<220>
<223>  Artificial sequence

<400>  886
cctacaatac ctaagaaatc cgtcctaagt tgacgg                                 36

<210>  887
<211>  25
<212>  DNA
<213>  Artificial

<220>
<223>  Artificial sequence

<400>  887
gtgtcgtagg aagacacatt ttcta                                             25

<210>  888
<211>  25
<212>  DNA
<213>  Artificial

<220>
<223>  Artificial sequence

<400>  888
gtttcaataa ccattccagc tatgg                                             25

<210>  889
<211>  38
<212>  DNA
<213>  Artificial

<220>
<223>  Artificial sequence

<400>  889
ctttcagccc acctattcgt gggaggctga tcgcaaca                               38

<210>  890
<211>  37
<212>  DNA
<213>  Artificial

<220>
<223>  Artificial sequence

<400>  890
ctttcaaccc acccagtacc tggagggttg ttgtcac                                37

<210>  891
<211>  37
<212>  DNA
<213>  Artificial

<220>
<223>  Artificial sequence

<400>  891
ctttcaatcc ctagagaagg tattttctta tttcaac                                37

<210>  892
<211>  37
<212>  DNA
<213>  Artificial

<220>
<223>  Artificial sequence

<400>  892
gttgcaaccg tccttccagc aatgggaggg ttgaaag                                37

<210>  893
<211>  37
<212>  DNA
<213>  Artificial

<220>
<223>  Artificial sequence

<400> 893
ctttcaatcg cccccttgcc gcaaacctgg atgaaac 37

<210> 894
<211> 37
<212> DNA
<213> Artificial

<220>
<223> Artificial sequence

<400> 894
ctttcaaccc acccctagcc gggatggttg ttgaaac 37

<210> 895
<211> 37
<212> DNA
<213> Artificial

<220>
<223> Artificial sequence

<400> 895
ctttcaaccc gcctcaagct gggaggggta ttgaaac 37

<210> 896
<211> 37
<212> DNA
<213> Artificial

<220>
<223> Artificial sequence

<400> 896
ctttcaatcc ctagagaagg tattttctta tttcaac 37

<210> 897
<211> 38
<212> DNA
<213> Artificial

<220>
<223> Artificial sequence

<400> 897
agtttcatca accctcctga tgtgggatgg gttgaaag 38

<210> 898
<211> 37
<212> DNA
<213> Artificial

<220>
<223> Artificial sequence

<400> 898
gtttcaacaa ccctcacggt aggcggtgag ttgaaag 37

<210> 899
<211> 37
<212> DNA
<213> Artificial

<220>
<223> Artificial sequence

<400> 899
gtcacaacaa ccctccaggt actaggcggg ttgaaag 37

<210> 900
<211> 37
<212> DNA
<213> Artificial

<220>
<223> Artificial sequence

<400> 900
gtcgcaatcg ccctcccaga atagggtggg ttgaaag 37

<210> 901
<211> 36
<212> DNA
<213> Artificial

<220>
<223> Artificial sequence

<400> 901
gatatagacc accccaatat cgaaggggac taaaac 36

<210> 902
<211> 36
<212> DNA
<213> Artificial

<220>
<223> Artificial sequence

<400> 902
gttttagtcc ccttcgtttt tggggttatc tatatc 36

<210> 903
<211> 36
<212> DNA
<213> Artificial

<220>
<223> Artificial sequence

<400> 903
gttgtagttc cctttcattt tgggatcatt cacaac 36

<210> 904

<211> 35
<212> DNA
<213> Artificial

<220>
<223> Artificial sequence

<400> 904
gttaatgaaa aaagcccgac atagcgggca atcac 35

<210> 905
<211> 34
<212> DNA
<213> Artificial

<220>
<223> Artificial sequence

<400> 905
gtaagactac ctcaaaacga aagaggacta aaac 34

<210> 906
<211> 37
<212> DNA
<213> Artificial

<220>
<223> Artificial sequence

<400> 906
gttattgccc attatctcgg gctcttctca tttctac 37

<210> 907
<211> 31
<212> DNA
<213> Artificial

<220>
<223> Artificial sequence

<400> 907
cttattgctc actatatcga gctgtattca c 31

<210> 908
<211> 24
<212> DNA
<213> Artificial

<220>
<223> Artificial sequence

<400> 908
ttcgatattg ggtagtcta tatc 24

<210> 909
<211> 37
<212> DNA
<213> Artificial

<220>
<223> Artificial sequence

<400> 909
gtctcaggcg gtcgtcagaa agaccgtcat gcagaac 37

<210> 910
<211> 37
<212> DNA
<213> Artificial

<220>
<223> Artificial sequence

<400> 910
ggttcagtcc gccgtcgtct tggcggtgat gtgaggc 37

<210> 911
<211> 36
<212> DNA
<213> Artificial

<220>
<223> Artificial sequence

<400> 911
gttaaaagag aatagcccga catagtgggc aataac 36

<210> 912
<211> 36
<212> DNA
<213> Artificial

<220>
<223> Artificial sequence

<400> 912
gagaaacatc acccccaaat ggaggggac tgcacc 36

<210> 913
<211> 36
<212> DNA
<213> Artificial

<220>
<223> Artificial sequence

<400> 913
gttgtgttta cccttcaaaa taagggcagt tacaac 36

<210> 914
<211> 36
<212> DNA
<213> Artificial

<220>
<223> Artificial sequence

<400> 914
gttgtagata cccttcaaaa taagggcttt cccaac 36

<210> 915
<211> 36
<212> DNA
<213> Artificial

<220>
<223> Artificial sequence

<400> 915
gttgtgatta cccttcaaaa tgtgatattt cccaac 36

<210> 916
<211> 36
<212> DNA
<213> Artificial

<220>
<223> Artificial sequence

<400> 916
gttgtcatac ccatccaaac gataggcttc tacaac 36

<210> 917
<211> 36
<212> DNA
<213> Artificial

<220>
<223> Artificial sequence

<400> 917
gttgtatcag cctttagttg gaaaggtaga cacaac 36

<210> 918
<211> 36
<212> DNA
<213> Artificial

<220>
<223> Artificial sequence

<400> 918
gttgttttta cctttcaaaa agaaggcaga tgcaac 36

<210> 919
<211> 36
<212> DNA
<213> Artificial

<220>
<223> Artificial sequence

<400> 919
gttgtattaa cctttagttt gaaaggcaaa aacaac 36

<210> 920
<211> 36
<212> DNA
<213> Artificial

<220>
<223> Artificial sequence

<400> 920
gttgtgtgta cccttcgaat agagggtaga tccaac 36

<210> 921
<211> 36
<212> DNA
<213> Artificial

<220>
<223> Artificial sequence

<400> 921
gttgtctcca cccttctaac taagggtatt cccaac 36

<210> 922
<211> 36
<212> DNA
<213> Artificial

<220>
<223> Artificial sequence

<400> 922
gttgggactg ctctcatttt gaagggtatt cacaac 36

<210> 923
<211> 37
<212> DNA
<213> Artificial

<220>
<223> Artificial sequence

<400> 923
gttggtactg tacacgtttt tggtgtgcaa tcacaac 37

<210> 924
<211> 36
<212> DNA
<213> Artificial

<220>
<223> Artificial sequence

<400> 924
gttgtagttg ctctcggttt ggagggtata aacaac 36

<210> 925

<211> 36
<212> DNA
<213> Artificial

<220>
<223> Artificial sequence

<400> 925
gctgttgaag ccttcatttt gaagggtaat tacagc 36

<210> 926
<211> 30
<212> DNA
<213> Artificial

<220>
<223> Artificial sequence

<400> 926
gactaaatcc aagtagattg gaattttaac 30

<210> 927
<211> 36
<212> DNA
<213> Artificial

<220>
<223> Artificial sequence

<400> 927
gttttagact agtgcaaatt cgcaccagtg tagtag 36

<210> 928
<211> 36
<212> DNA
<213> Artificial

<220>
<223> Artificial sequence

<400> 928
gttttaagac cttgtaaatt tacaatcctt tcaatc 36

<210> 929
<211> 36
<212> DNA
<213> Artificial

<220>
<223> Artificial sequence

<400> 929
gaactacacc cgtgcaaaaa tgcaggggtc taaaac 36

<210> 930
<211> 90
<212> DNA
<213> Artificial

<220>
<223> Artificial sequence

<400> 930
aucuaaaauu auaaauguac caaauaauua augcucugua aucauuuaaa aguauuuuga 60

acggaccucu guuugacacg ucugaauaac 90

<210> 931
<211> 88
<212> DNA
<213> Artificial

<220>
<223> Artificial sequence

<400> 931
ucagaaugca ucccaacauu cuauacacug aaaucauaga aaaucacguu uguggcccga 60

ccaacugcuu cggcaugucg gguuuuuu 88

<210> 932
<211> 129
<212> DNA
<213> Artificial

<220>
<223> Artificial sequence

<400> 932
uuaauuacau ucuuuuaaca acgaagucgc cuucgggcga gcugaaauca auuugauuaa 60

auauuagauc cggcuacuga ggucuuugac cuuauccgga uuaacgaaga gccuccgagg 120

aggcuuuuu 129

<210> 933
<211> 111
<212> DNA
<213> Artificial

<220>
<223> Artificial sequence

<400> 933
uuagagauca uaacgcuaug agcuauagga aaucaccuuc gggugagcug aaauccccua 60

aagcuaagau ugaauccggc cacuaucuau uaguagauau ccggauauuc u 111

<210> 934
<211> 100
<212> DNA
<213> Artificial

<220>
<223> Artificial sequence

<400> 934

uaaauuagaa aucaucuaaa uuucgauacc cugaaaucaa caaaauuaaa gauugaaucg 60

uuuuucuaug cucgucuuaa uagcgagcau auaacgauuu 100

<210> 935
<211> 108
<212> DNA
<213> Artificial

<220>
<223> Artificial sequence

<400> 935
uuguuagaau guucccgcaa cacuuuauag caaauccguu cgaugccuug aaaucaucaa 60

aaagauauaa uagacccgcc cacuguauug uacauggcgg gacuuuuu 108

<210> 936
<211> 99
<212> DNA
<213> Artificial

<220>
<223> Artificial sequence

<400> 936
guuuuacuuc uuucuaaaua aacauaguua aguuaaaaca agcuuaaagc gucaauguaa 60

uauuuuauua acacccuacu gugucagugg gguuuuuuu 99

<210> 937
<211> 154
<212> DNA
<213> Artificial

<220>
<223> Artificial sequence

<400> 937
auuucaaaau auucccccuu uacauuuuuc aaaagaaaau guacgcuaag aguguuacua 60

cucuguaaca uuacauuggu acguuaaaau aagcuuaaag cguaaaaguu ggcccuauga 120

ggucuccgcc aucgacuucg ucgguggcuu uuuu 154

<210> 938
<211> 104
<212> DNA
<213> Artificial

<220>
<223> Artificial sequence

<400> 938
aacuacguug gaacuauucg aaacaacaca gccaaaagau uuuucuuuug aguuaaaaua 60

ugguuaucca uaaucaguua ugcgcaccga uucggugcuu uuuu 104

<210> 939
<211> 90
<212> DNA
<213> Artificial

<220>
<223> Artificial sequence

<400> 939
auuguuagua uucaaaauaa cauagcaagu uaaaauaagg cuuuguccgu uaucaacuuu 60

uaauuaagua gcgcuguuuc ggcgcuuuuu 90

<210> 940
<211> 89
<212> DNA
<213> Artificial

<220>
<223> Artificial sequence

<400> 940
guuggaacca uucaaaacag cauagcaagu uaaaauaagg cuaguccguu aucaacuuga 60

aaaaguggca ccgagucggu gcuuuuuuu 89

<210> 941
<211> 96
<212> DNA
<213> Artificial

<220>
<223> Artificial sequence

<400> 941
uugugguuug aaaccauucg aaacaacaca gcgaguuaaa auaaggcuua guccguacuc 60

aacuugaaaa gguggcaccg auucggugu uuuuuu 96

<210> 942
<211> 102
<212> DNA
<213> Artificial

<220>
<223> Artificial sequence

<400> 942
guuggaauca uucgaaacaa cacagcaagu uaaaauaagg cagugauuuu uaauccaguc 60

cguacacaac uugaaaaagu gcgcaccgau ucggugcuuu uu 102

<210> 943
<211> 120
<212> DNA
<213> Artificial

<220>
<223> Artificial sequence

<400> 943
cgucuugauu accagucagg acagcacugc gagucaaaau acggcuuugc caaacuugcc 60

ucccuucgga ggcgucucgu aggagacaau uugaagcccc uuuaggggcu ucauuuuucu 120

<210> 944
<211> 89
<212> DNA
<213> Artificial

<220>
<223> Artificial sequence

<400> 944
guuuuacuau uucuagauuc uuuaagaucu acaaaaauaa ggauuuauuc cgaauuuacc 60

accuauuuua auuaauaggu gguuuuuuu 89

<210> 945
<211> 146
<212> DNA
<213> Artificial

<220>
<223> Artificial sequence

<400> 945
uguugagacg acauccucaa caacuugaau ugauugaucu gacaucuacg aguugagauc 60

aaacaaagcu ucagcugagu uucaauuucu gagcccaugu ugggccauac auaugccacc 120

cgagugcaaa ucggguggcu uuuuuu 146

<210> 946
<211> 144
<212> DNA
<213> Artificial

<220>
<223> Artificial sequence

<400> 946
ggauuguuug gucgcaaucc augaucaagg ucauugaccu gacaggcaua aauugaaaua 60

aagcaagguu ucgaccaagc uucagaaggu uuuauaccug gccuuauggc ugugaggcuc 120

ccgauaaugu cgggagccuc uuuu 144

<210> 947
<211> 167
<212> DNA
<213> Artificial

<220>
<223> Artificial sequence

<400> 947
uugggauuga ucaucccaaa caucauuggg uucuaccuca uugaucugac acacagcauu 60

gaaguaaagc aagauuaauu ucaagcuuaa uuuucuucac auuuuaugug cagaagggcu 120

uaugcccaca auacauaaaa aguccgcauu cacuugcgga cuuuuau 167

<210> 948
<211> 149
<212> DNA
<213> Artificial

<220>
<223> Artificial sequence

<400> 948
caugguuagc uaccauacaa gcaagaauug uuuagcuaac uauucuugcu aggaagaacc 60

cauuaaucug acauacaggg uuaaaguaac gcaagggcuu cagcccaagc uucaugaacu 120

uuuaaaaagu uggccuuaug gccuuuuuu 149

<210> 949
<211> 116
<212> DNA
<213> Artificial

<220>
<223> Artificial sequence

<400> 949
uaaucucagu uuaauaccua uaugagauua caucaugagu ucaaauaaaa guuuacucaa 60

aucgcccgaa agagcccaca uugguggacu aaacaaaucu ucggauuugu uuuuuu 116

<210> 950
<211> 129
<212> DNA
<213> Artificial

<220>
<223> Artificial sequence

<400> 950
auaaguaauc caauuaguuu uggagguuua cagaauuaca cuacgaguuc aaauacaaau 60

uuauuuacaa ugccuucggg ccacccgacg uaggguauca ucucaauucu ucugaauugg 120

gauuuuuuu 129

<210> 951
<211> 120
<212> DNA
<213> Artificial

<220>
<223> Artificial sequence

<400> 951
gaaauugucu uauaccagua agauaauuua cauaguaagu ucaaacaagc uuuuagcgaa 60

auuaccgcuu ugcggauuca cauuguguga aguuaacucu cgaaagagag uuuuuucuuu 120

<210> 952
<211> 121
<212> DNA
<213> Artificial

<220>
<223> Artificial sequence

<400> 952
auaucauuau cauugauuua caaggugagu ucaaacaagg auuuauccgu aauugauugc 60

ucgcauugug cgacauuuuc uuauguaaau cgugaagucg gacuuucgac uucuuuuuuu 120

u 121

<210> 953
<211> 106
<212> DNA
<213> Artificial

<220>
<223> Artificial sequence

<400> 953
guugacuacc auaugagauu acacuacacg guucaaauaa agaauuuuuc uaaucgccca 60

augggcccau auugauaugg augaaacucg cuuagcgagu uuuuuu 106

<210> 954
<211> 115
<212> DNA
<213> Artificial

<220>
<223> Artificial sequence

<400> 954
auuuaagauc caucuuaaau uacacaacga guucaaauaa gaauucauca aaaucguccc 60

uuuugggacc gcucauugug gagcaucaag gcuuaacaug guuaagccuu uuuuu 115

<210> 955
<211> 84
<212> DNA
<213> Artificial

<220>
<223> Artificial sequence

<400> 955
guacuuauac cuaaaauuac agaaucuacu gaaacaagac aauaugucgu guuuauccca 60

ucaauuuauu gguggauuu uuuu 84

<210> 956
<211> 92

<212> DNA
<213> Artificial

<220>
<223> Artificial sequence

<400> 956
ugaucauaau cuagcaaaag uuuauaugau cuaacaaaac aaggguuuau cccggaauca 60

aguuccaagu auaugcuugg agcuuuuucu uu 92

<210> 957
<211> 109
<212> DNA
<213> Artificial

<220>
<223> Artificial sequence

<400> 957
uguaagggac gccuuacaca guuacuuaaa ucuugcagaa gcuacaaaga uaaggcuuca 60

ugccgaaauc aacacccugu cauuuuaugg cagggguguuu ucguuauuu 109

<210> 958
<211> 130
<212> DNA
<213> Artificial

<220>
<223> Artificial sequence

<400> 958
uguagucgac ggacuaccgu guuugacgaa acacgucuuu aauaauuuua cugauaagaa 60

auuauugaga aucuacaaaa auaaggcauc uugccgaauu uaccgcccua cauauguagg 120

gcgguuuuuu 130

<210> 959
<211> 95
<212> DNA
<213> Artificial

<220>
<223> Artificial sequence

<400> 959
uguagagaaa auuuuauagu cacguaaauu uuucagaucu acuaaaacaa ggcuuuaugc 60

cgaaaucagg agcaccgacg ggugcuccuu uuuuu 95

<210> 960
<211> 110
<212> DNA
<213> Artificial

<220>
<223> Artificial sequence

<400> 960
uguauuucga aauacagaug uacaguuaag aauacauaag aaugauacau cacuaaaaaa 60

aggcuuuaug ccguaacuac uacuuauuuu caaaauaagu aguuuuuuuu 110

<210> 961
<211> 72
<212> DNA
<213> Artificial

<220>
<223> Artificial sequence

<400> 961
cuaguaagaa auugucgcac aaaaauaaga cgcauuaugc ugucgaauuu ccccaccuag 60

ugggguuuuu uu 72

<210> 962
<211> 92
<212> DNA
<213> Artificial

<220>
<223> Artificial sequence

<400> 962
auuauugcuu acacaauuau ugucgugcua aaauaaggcg cuguuaaugc agcugccgca 60

uccgccagag cauuuaugcu cuggcuuuuu uu 92

<210> 963
<211> 115
<212> DNA
<213> Artificial

<220>
<223> Artificial sequence

<400> 963
uauauauuac uuacuuaaca aaauaauugu acgauuccaa aauaaggcgc uuauguaaga 60

ugcaauaaug cacuuauaua agcugccgua aacgccgagg uaacucgguu uuuuu 115

<210> 964
<211> 98
<212> DNA
<213> Artificial

<220>
<223> Artificial sequence

<400> 964
aguacaaauu aauuauuguu uacccaaaua uuguacaucc uaaaucaagg cgcuuaauug 60

cugccguaau ugcugaaagc guagcuuuca guuuuuuu 98

<210> 965
<211> 92
<212> DNA
<213> Artificial

<220>
<223> Artificial sequence

<400> 965
cgucgucgcu gcgcgaaaug gcugaguagg cagcggcuau aauaaggggu guggaggcau 60

ccugcgaagu ucuacucuac ggaguaucuu cu 92

<210> 966
<211> 73
<212> DNA
<213> Artificial

<220>
<223> Artificial sequence

<400> 966
aagaaauuua aaaagggacu aaaauaaaga guuugcggga cucugcgggg uuacaauccc 60

cuaaaaccgc uuu 73

<210> 967
<211> 96
<212> DNA
<213> Artificial

<220>
<223> Artificial sequence

<400> 967
uaugggaaau cggaagggaa gccacggcaa ggugguuuca uagaaaucac ugaaggauua 60

cccucgucac agaaaugugg cggggggauu ccuauu 96

<210> 968
<211> 125
<212> DNA
<213> Artificial

<220>
<223> Artificial sequence

<400> 968
uaagaucaua ucacagcaau gaucuuaggg uuacuaugau aagggcuuuc uacuuuaggg 60

guagagaugu cccgcggcgu uggggaucgc cuauugcccu uaaagggcac uccccauuuu 120

aauuu 125

<210> 969
<211> 107
<212> DNA
<213> Artificial

<220>
<223> Artificial sequence

<400> 969
uuaaucagga acuagguaua gcauaucgag aguuuaacua guuacuauaa caaggcauua 60

agccguaaag uaucccuau guucauuuga accuaggggu aucuuuu 107

<210> 970
<211> 100
<212> DNA
<213> Artificial

<220>
<223> Artificial sequence

<400> 970
auggcauauc ggagccugaa uuguugcuau aauaaggugc uggguuuagc ccagaccgcc 60

aaguuaaccc cggcauuuau ugcuggggua ucuuguuuuu 100

<210> 971
<211> 103
<212> DNA
<213> Artificial

<220>
<223> Artificial sequence

<400> 971
cgauuguggu uauccggggu gagagccguu gcugcaauaa ggaggggucg caagaccccg 60

uccguaccca aaagccuggc agggaaaccu gucaggcuuu uuu 103

<210> 972
<211> 111
<212> DNA
<213> Artificial

<220>
<223> Artificial sequence

<400> 972
acauauuguc gcacugcgaa augagaaccg uugcuacaau aaggccgucu gaaaagaugu 60

gccgcaacgc ucugccccuu aaagcuucug cuuuaagggg caucguuuau u 111

<210> 973
<211> 110
<212> DNA
<213> Artificial

<220>
<223> Artificial sequence

<400> 973
gcauauuguu gcacugcgaa augagagacg uugcuacaau aaggcuucug aaaagaauga 60

ccguaacgcu cugccccuug ugauucuuaa uugcaagggg caucguuuuu 110

<210> 974
<211> 99
<212> DNA
<213> Artificial

<220>
<223> Artificial sequence

<400> 974
cuaagagaau uauaucauac caagugauaa uuagguuauu acaauaaggu aagaaaccua 60

aaagcucuaa ucccauucuu cggaauggga uuaucuuuu 99

<210> 975
<211> 105
<212> DNA
<213> Artificial

<220>
<223> Artificial sequence

<400> 975
gcgagggaua ucauaccaca ucaaggcuug cgagguugcu augauaaggc aacaggccgc 60

aaagcacuga cccgcauucc aaugaaugcg ggucaucuac uuuuu 105

<210> 976
<211> 123
<212> DNA
<213> Artificial

<220>
<223> Artificial sequence

<400> 976
aucacagggu gccauuacca gagaugguag cacgguggaa cggaccggca ccuacaggac 60

aagugaucau acacgugaca gccgccuccc ccgccccagu ggccaagggg aggcggcuuu 120

ucu 123

<210> 977
<211> 105
<212> DNA
<213> Artificial

<220>
<223> Artificial sequence

<400> 977
gacuuuccag cagaucggga auugcgcuuu gcuacuaaca agcugaaucc guuaggagua 60

aaugcaccaa augagagggc cggcuuuugc cggcccuuug cuuuu 105

<210> 978
<211> 87
<212> DNA

<213> Artificial

<220>
<223> Artificial sequence

<400> 978
cgucuagcaa ggaacgcggc guggccucuc cguuaacaag gcacaugcca ccagaucgag 60

gcgggcuccg guccgccucu uugcuuu 87

<210> 979
<211> 90
<212> DNA
<213> Artificial

<220>
<223> Artificial sequence

<400> 979
cacucaguuc acugggauau gcgcucugac cgcuaacaag cugaaagaug caccaaaugg 60

aaagccccgc augcggggcu uucgucuuuu 90

<210> 980
<211> 109
<212> DNA
<213> Artificial

<220>
<223> Artificial sequence

<400> 980
gccauuaaua auugauugca auaacacucu ggugauugag gagccuaugg uuaacaagug 60

gguuuccugc acaaaucuaa gagcugccuc cgggcggcuc uuuugcuuu 109

<210> 981
<211> 93
<212> DNA
<213> Artificial

<220>
<223> Artificial sequence

<400> 981
uggaaauucg auggggaucg ggggguccagc cguuaacaug uucccuucgg ggagcacgaa 60

augcggggcg ggccacgguc cgccccuuuu uuu 93

<210> 982
<211> 83
<212> DNA
<213> Artificial

<220>
<223> Artificial sequence

<400> 982
guucagaauu cgcggucgag ccguuaacaa gcucgaagaa gcaccacauu aaaacgcguc 60

cugcggggcg cguuucuuu uuu 83

<210> 983
<211> 125
<212> DNA
<213> Artificial

<220>
<223> Artificial sequence

<400> 983
uagcaaaucg agaggcgguc gcuuuucgca agcaaauuga ccccuugugc gggcucggca 60

ucccaagguc agcugccggu uauuaucgaa aaggcccacc gcaagcagcg cgugggccuu 120

uuuuu 125

<210> 984
<211> 107
<212> DNA
<213> Artificial

<220>
<223> Artificial sequence

<400> 984
uucugućcca uuuguuguga uuugcuuuug cacagcaucc uuuggacaac uuguucuuug 60

aggauauuaa aaccaaccua ucuguuuaag auagucaaua ucuuuuu 107

<210> 985
<211> 158
<212> DNA
<213> Artificial

<220>
<223> Artificial sequence

<400> 985
uuaaugugua aauauaaaaa aauuuaucga aaaauacaau aguauuaaaa aauuauaugu 60

auauuuguca acacaaauuu gaaagcaaau cacaauaagg auuauuccgu ugugaaaaca 120

uuuggaaggg ggaguauuua uacuccucgu ucuuuuuu 158

<210> 986
<211> 94
<212> DNA
<213> Artificial

<220>
<223> Artificial sequence

<400> 986
cguugauuaa acaaaucaau uuuuacaucu uaucacagca aggcuauaug ccgaaggaug 60

uaauccuaua cucccgcuuc ggugggaguu uuuu 94

<210> 987
<211> 112
<212> DNA
<213> Artificial

<220>
<223> Artificial sequence

<400> 987
auguuuuaua uauuugcagc augauuaaua uuucuaaucu uuaaucuuau cacaauaagg 60

cuauaugccg uagaugaaaa ucuuuagucc ugcuucggug ggacuuuuuu uu 112

<210> 988
<211> 121
<212> DNA
<213> Artificial

<220>
<223> Artificial sequence

<400> 988
guuuguuuua ucagaaauaa guuguauauu ugcacucaga uacacaguga agacuuuuca 60

caacaaggcu auaagccgaa gauuuucuug uacccugcgg ucaaccacag ggucuuuuuu 120

u 121

<210> 989
<211> 80
<212> DNA
<213> Artificial

<220>
<223> Artificial sequence

<400> 989
guuauaucuu aacaaaaacc agcgauuauc ucuaauaaga cuuaagucgc aaaaugcucc 60

cuauuuuggg agcuuuuuuu 80

<210> 990
<211> 75
<212> DNA
<213> Artificial

<220>
<223> Artificial sequence

<400> 990
gagacaggcu accuagcaag accccuucgu ggggucgcau ucuucacccc cucgcagcag 60

cgaggggguu cguuu 75

<210> 991
<211> 109
<212> DNA
<213> Artificial

<220>
<223> Artificial sequence

<400> 991
acaaaacauc ugaacaucac uuuaacuccc aacggauuca agacaaaauu ugaaaugcaa 60

accgauuuuc cugacugcca gccagucaca ccgguaacaa aagcauuuu 109

<210> 992
<211> 100
<212> DNA
<213> Artificial

<220>
<223> Artificial sequence

<400> 992
guuguaacag gguaggguuu uuugaggggu cuuaaaauca agaacuguua caacaguucc 60

auucuagggc ccaucuucgg acgggccuca gccuuuuuuu 100

<210> 993
<211> 151
<212> DNA
<213> Artificial

<220>
<223> Artificial sequence

<400> 993
gucuaaagga cagaauuuuu caacgggugu gccaauggcc acuuuccagg uggcaaagcc 60

cguugaacuu cucaaaaaga acgcucgcuc aguguucuga ccuuucgagc gccuguucag 120

ggcgaaaacc cugggaggcg cucgaagcau a 151

<210> 994
<211> 159
<212> DNA
<213> Artificial

<220>
<223> Artificial sequence

<400> 994
gucuagagga cagaauuuuu caacgggugu gccaauggcc acuuuccagg uggcaaagcc 60

cguugagcuu cucaaaaaga acgcucgcuc aguguucuga ccuuucgagc gccuguucag 120

ggcgaaaacc cugggaggcg cucgaaucau aggugggac 159

<210> 995
<211> 107
<212> DNA
<213> Artificial

<220>
<223> Artificial sequence

<400> 995
cuggacgaug ucucuuuuau uucuuuuuuc uuggaucuga guacgagcac ccacauugga 60

cauuucgcau ggugggugcu cguacuauag guaaaacaaa ccuuuuu 107

<210> 996
<211> 75
<212> DNA
<213> Artificial

<220>
<223> Artificial sequence

<400> 996
gcuggagacg uuuuuugaaa cggcgagugc ugcggauagc gaguuucucu uggggaggcg 60

cucgcggcca cuuuu 75

<210> 997
<211> 94
<212> DNA
<213> Artificial

<220>
<223> Artificial sequence

<400> 997
accauaucca gguguugauu guaaacacuu agcgggugaa auuauauaug uuuguaauau 60

cuucacuauc caaaguuauc ucugguuuug guuu 94

<210> 998
<211> 87
<212> DNA
<213> Artificial

<220>
<223> Artificial sequence

<400> 998
auuuguuuau ugaaagaagc cuagacguua ggguucgcgu gcauguuagg cuccagcagg 60

uaccuccguu auaaccuaac cucguuu 87

<210> 999
<211> 80
<212> DNA
<213> Artificial

<220>
<223> Artificial sequence

<400> 999
agucccagcg acuaugucgu auggacgaag cgcuuauuua ucggagauag cuccgggugc 60

aaacucggag cuguuuuuuu 80

<210> 1000
<211> 75
<212> DNA
<213> Artificial

<220>
<223> Artificial sequence

<400> 1000
ccagcgacua ugucguaugg guaaagcgcu uauuuaucgg agaguucugg augcaaaccc 60

agagcuguuu uuuua 75

<210> 1001
<211> 122
<212> DNA
<213> Artificial

<220>
<223> Artificial sequence

<400> 1001
gagacgcaau accuugcgau cagagcggcg cgauguauac ucgcagcgag acaggcuacc 60

uagcaagacc ccuucguggg gucgcauucu ucaccccuc gcagcagcga gggggguucgu 120

uu 122

<210> 1002
<211> 104
<212> DNA
<213> Artificial

<220>
<223> Artificial sequence

<400> 1002
aauaauaaua aaaaacgaug cccccugagg aagucccagg aggcagagcg uugcgagaaa 60

uuuaucucuu auuguaacaa cguuuuucau uucgcagugc cauu 104

<210> 1003
<211> 79
<212> DNA
<213> Artificial

<220>
<223> Artificial sequence

<400> 1003
ggauucaaga caaaauuuga aaugcaaacc gauuuuccug acugccagcc agucacaccg 60

guaacaaaag cauuuuuuu 79

<210> 1004
<211> 84
<212> DNA
<213> Artificial

<220>
<223> Artificial sequence

<400> 1004
gggucacuaa uuugaagcaa aucacaauaa ggacuauucc guugugaaaa cauuuaggau 60

ggggcauuug ccucguccuu uuuu 84

<210> 1005
<211> 128
<212> DNA
<213> Artificial

<220>
<223> Artificial sequence

<400> 1005
ugaucaaggu cauugaccug acaggcauaa auugaaauaa agcaagguuu cgaccaagcu 60

ucagaagguu uuauaccugg ccuuauggcu gugaggcucc cgauaauguc gggagccucu 120

uuucauuu 128

<210> 1006
<211> 136
<212> DNA
<213> Artificial

<220>
<223> Artificial sequence

<400> 1006
uggcuuuauc aagaauggug agaccagucc gaugguggau gaggaucugc ucuuuuucau 60

uacgaaaucc auaaaaagga augacaucau gcaauucaac auauccauag cuguauugca 120

ugaugucauu ccuuuu 136

<210> 1007
<211> 129
<212> DNA
<213> Artificial

<220>
<223> Artificial sequence

<400> 1007
ggugucuagg uccuugaccu gauaggcaua uauugaaaua aagcaagacc cuugggucaa 60

gcuucagaag guuuuagacc uggccuuaug gccaugggc ucccggcagc gccgggagcc 120

ccucuuucu 129

<210> 1008
<211> 90
<212> DNA
<213> Artificial

<220>

<223> Artificial sequence

<400> 1008
uauaaucuca uaagaaauuu aaaaagggac uaaaauaaag aguuugcggg acucugcggg 60

guuacaaucc ccuaaaaccg cuuuuaaaau 90

<210> 1009
<211> 82
<212> DNA
<213> Artificial

<220>
<223> Artificial sequence

<400> 1009
auuugaaagc aacucacaau aaggauuauu ccguugugaa aacaucuaaa ggagccccua 60

uaguaauaua gggguuguuu uu 82

<210> 1010
<211> 87
<212> DNA
<213> Artificial

<220>
<223> Artificial sequence

<400> 1010
uagaacugaa ccucaguaag cauuggcucg uuuccaaugu ugauugcucc gccggugcuc 60

cuuauuuuua agggcgccgg cuuucuu 87

<210> 1011
<211> 83
<212> DNA
<213> Artificial

<220>
<223> Artificial sequence

<400> 1011
uacaauuuug aagcaauuca caauaaggau uauuccguug ugaaaacauu uaaagcggcc 60

ucuaaagggu cgcuuuuuua uuu 83

<210> 1012
<211> 85
<212> DNA
<213> Artificial

<220>
<223> Artificial sequence

<400> 1012
guagauucaa aauuuacauu caaguucaaa uaaagauuuu aucuaaauug ccuucgggcc 60

uggaucggug ucgguccauu uuuuu 85

<210> 1013
<211> 123
<212> DNA
<213> Artificial

<220>
<223> Artificial sequence

<400> 1013
ggcaauucgg uagggaaacc acgguaacag aauuaccgua agguuuuuuc ugugaaggau 60

caucccucgc uugggcaacc aggcgggga aauuccucgu ucgggccaau cagcccuuca 120

uuu 123

<210> 1014
<211> 109
<212> DNA
<213> Artificial

<220>
<223> Artificial sequence

<400> 1014
aguuuugguc uguucaaaau aacacaacga guuaaaauaa agcuuaugcu uaaaugcugg 60

uuauccaguu gucauuuaga ugacgugcau cacuucggug augcuuuuu 109

<210> 1015
<211> 126
<212> DNA
<213> Artificial

<220>
<223> Artificial sequence

<400> 1015
caucuaaaca uuauugauuu aacauacugu guugagauca aacggagcuu uuuagcgaug 60

uuuauccuuu ugagccauua uauggccaua cauauuauaa aaagugcccc uaaaagggca 120

cuuuuu 126

<210> 1016
<211> 104
<212> DNA
<213> Artificial

<220>
<223> Artificial sequence

<400> 1016
cuaguggagu uauacaaaac aacauuagcg aaguuaaaau aagguuuaau ccguaaucaa 60

caaguuuuau cuugcgcgcg ccguuucggc gcuuuuuuug uuuu 104

<210> 1017
<211> 88

<212> DNA
<213> Artificial

<220>
<223> Artificial sequence

<400> 1017
uacguggagu cauacaaagc cacauagcaa guuaaaauaa gguuuuaucc guaaucaauc 60

augggcgcug uuucggcgcc uauuuuuu 88

<210> 1018
<211> 103
<212> DNA
<213> Artificial

<220>
<223> Artificial sequence

<400> 1018
ccuguuuaac aucauugauu uaacauccug guuuaaaguc aaacaaggca guaaugccaa 60

guucuuacuu augacucacg uaugugagca uacauauaaa aaa 103

<210> 1019
<211> 139
<212> DNA
<213> Artificial

<220>
<223> Artificial sequence

<400> 1019
auguuugacc uuauugauuu aacauccugu guuaaaauca agcaaagcgc uuugcgcgga 60

guuucaacuu uugacccauu auaugggcau uacauaagcg aaaggaacca uucuucaguu 120

ggagaauggu uccuuuuuu 139

<210> 1020
<211> 102
<212> DNA
<213> Artificial

<220>
<223> Artificial sequence

<400> 1020
cggauagaca ucauuguuuu gaacuacugg uuuaaaguca aacaagguag caauaccaag 60

uucuuacuua ugacccauuu auaugggcau acauacaaua aa 102

<210> 1021
<211> 81
<212> DNA
<213> Artificial

<220>
<223> Artificial sequence

<400> 1021
guuuuauuga uaggaaaaug auugagaauc uacaaagaua aggcuuuaug ccgaaaugac 60

ugccuuuaug gcaguuuuuu u 81

<210> 1022
<211> 124
<212> DNA
<213> Artificial

<220>
<223> Artificial sequence

<400> 1022
uugaacugau ugauucgaca ucuacacguu gagaucaaac aaagcuucgg cugaguuuca 60

auuuuugagc ccauguuggg ccauacauac cgccauccgg gaagauaccg gguggcauuu 120

uuuu 124

<210> 1023
<211> 124
<212> DNA
<213> Artificial

<220>
<223> Artificial sequence

<400> 1023
uugaacugau ugauucgaca ucuacacguu gagaucaaac aaagcuucgg cugaguuuca 60

auuuuugagc ccauguuggg ccauacauac cgccauccgg gaagauaccg gguggcauuu 120

uuuu 124

<210> 1024
<211> 109
<212> DNA
<213> Artificial

<220>
<223> Artificial sequence

<400> 1024
cguuuugaac ugauugauuc gacaucuaca cguugagauc aaacaaagcc ucggcugagu 60

uucaauuucu gagcccaugu ugggccauac auaccgccau ccgggaaaa 109

<210> 1025
<211> 124
<212> DNA
<213> Artificial

<220>
<223> Artificial sequence

<400> 1025
uugaacugau ugauucgaca ucuacacguu gagaucaaac aaagcuucgg cugaguuuca 60

auuuuugagc ccauguuggg ccauacauac cgccauccgg gaagauaccg gguggcauuu 120

uuuu 124

<210> 1026
<211> 124
<212> DNA
<213> Artificial

<220>
<223> Artificial sequence

<400> 1026
uugaacugau ugauucgaca ucuacacguu gagaucaaac aaagcuucgg cugaguuuca 60

auuuuugagc ccauguuggg ccauacauac cgccauccgg gaagauaccg gguggcauuu 120

uuuu 124

<210> 1027
<211> 124
<212> DNA
<213> Artificial

<220>
<223> Artificial sequence

<400> 1027
uugaacugau ugauucgaca ucuacacguu gagaucaaac aaagcuucgg cugaguuuca 60

auuuuugagc ccauguuggg ccauacauac cgccauccgg gaagauaccg gguggcauuu 120

uuuu 124

<210> 1028
<211> 124
<212> DNA
<213> Artificial

<220>
<223> Artificial sequence

<400> 1028
uugaacugau ugauucgaca ucuacacguu gagaucaaac aaagcuucgg cugaguuuca 60

auuuuugagc ccauguuggg ccauacauac cgccauccgg gaagauaccg gguggcauuu 120

uuuu 124

<210> 1029
<211> 121
<212> DNA
<213> Artificial

<220>
<223> Artificial sequence

<400> 1029

uugaacugau ugauucgaca ucuacacguu gagaucaaac aaagccucgg cugaguuuca 60

auuucugagc ccauguuggg ccauacauac cgccauccgg gaaaacccgg augguuuuuu 120

u 121

<210> 1030
<211> 121
<212> DNA
<213> Artificial

<220>
<223> Artificial sequence

<400> 1030
uugaacugau ugauucgaca ucuacacguu gagaucaaac aaagcuucgg cugaguuuca 60

auuuuugagc ccauguuggg ccauacauac gccauccggg aaaacucggg uggcuuuuuu 120

u 121

<210> 1031
<211> 109
<212> DNA
<213> Artificial

<220>
<223> Artificial sequence

<400> 1031
cguuuugaac ugauugauuc gacaucuaca cguugagauc aaacaaagcc ucggcugagu 60

uucaauuucu gagcccaugu ugggccauac auaccgccau ccgggaaaa 109

<210> 1032
<211> 121
<212> DNA
<213> Artificial

<220>
<223> Artificial sequence

<400> 1032
uugaacugau ugauucgaca ucuacacguu gagaucaaac aaagcuucgg cugaguuuca 60

auuuuugagc ccauguuggg ccauacauac gccauccggg aaaacccgug uggcuuuuuu 120

u 121

<210> 1033
<211> 121
<212> DNA
<213> Artificial

<220>
<223> Artificial sequence

<400> 1033
uugaacugau ugauucgaca ucuacacguu gagaucaaac aaagcuucgg cugaguuuca 60

auuuuugagc ccauguuggg ccauacauac gccauccggg aaaacccggg uggcuuuuuu 120

u 121

<210> 1034
<211> 124
<212> DNA
<213> Artificial

<220>
<223> Artificial sequence

<400> 1034
uugaacugau ugauucgaca ucuacacguu gagaucaaac aaagcuucgg cugaguuuca 60

auuuuugagc ccauguuggg ccauacauac cgccauccgg gaagauaccg gguggcauuu 120

uuuu 124

<210> 1035
<211> 124
<212> DNA
<213> Artificial

<220>
<223> Artificial sequence

<400> 1035
uugaacugau ugauucgaca ucuacacguu gagaucaaac aaagcuucgg cugaguuuca 60

auuuuugagc ccauguuggg ccauacauac cgccauccgg gaagauaccg gguggcauuu 120

uuuu 124

<210> 1036
<211> 102
<212> DNA
<213> Artificial

<220>
<223> Artificial sequence

<400> 1036
guuuuaagac cuaucgagaa caacacacuu aaguucaaau aaaaauuuau ucuauugacc 60

agaaauggca gcccgcugug ggcauuuuaa cuucauuuuu uu 102

<210> 1037
<211> 98
<212> DNA
<213> Artificial

<220>
<223> Artificial sequence

<400> 1037
aaucuugaau cuauugaucu gacauacagc gucagaauaa aacaaggcca ucggccaagu 60

uuacagcgcu cuuuuugaga guggggcuua ugcccaua 98

<210> 1038
<211> 67
<212> DNA
<213> Artificial

<220>
<223> Artificial sequence

<400> 1038
uaucuuaacu uuauugaaau gacacucugu uaaaaucaaa caaggcuucg gccaaguuuu 60

cccuuuu 67

<210> 1039
<211> 113
<212> DNA
<213> Artificial

<220>
<223> Artificial sequence

<400> 1039
cuaucuaaac uuauugauuu aacacucuga guuaaaauca aacaaggcgu aagccaaguu 60

ucaucuugug agucccuaug gaccauaaag gcgcuauuga ucuucgugau caa 113

<210> 1040
<211> 112
<212> DNA
<213> Artificial

<220>
<223> Artificial sequence

<400> 1040
acucuuaacc uuauugaucu aacaaucaua auuuaaaauc aagcaaugcg uuuuaguacg 60

cagaguuuca acacuugucc cgagcuaucg agggacuuuu uuuauguaaa aa 112

<210> 1041
<211> 113
<212> DNA
<213> Artificial

<220>
<223> Artificial sequence

<400> 1041
aucgaaauac ucauugauuu gauacucuga guuaaaauca aacaaggucu ucggaccaag 60

uuuucuauuc uaagccucuu augaggccuu uuuuuaugcc uucaaaccaa aaa 113

<210> 1042
<211> 113
<212> DNA
<213> Artificial

<220>
<223> Artificial sequence

<400> 1042
aucgaaauac ucauugauuu gauacucuga guuaaaauca aacaaggucu ucggaccaag 60

uuuucuauuc uaagccucuu augaggccuu uuuuuaugcc uucaaaccaa aaa 113

<210> 1043
<211> 96
<212> DNA
<213> Artificial

<220>
<223> Artificial sequence

<400> 1043
guuaaaccuu auugaauaga caaccugauu uaaaaucaag caaagcucuu cggaguggag 60

uuuauacauu ugucccgcgu auugcgggcu uuuuuu 96

<210> 1044
<211> 109
<212> DNA
<213> Artificial

<220>
<223> Artificial sequence

<400> 1044
acguuugacc uuauugaaua gacaaccuga uuuaaaauca agcaaagcuc uucggagcgg 60

aguuuauaca uuugucccgu guauugcggg cuuuuuuuau gcaacaaaa 109

<210> 1045
<211> 97
<212> DNA
<213> Artificial

<220>
<223> Artificial sequence

<400> 1045
acguuagacc uuauugaaua gacaaccuga uuuaaaauca agcaaagcuc uucggagugg 60

aguuuauaca uuugucccgc guauugcggg cuuuuuu 97

<210> 1046
<211> 97
<212> DNA
<213> Artificial

<220>
<223> Artificial sequence

<400> 1046
ugggauguca uugucugaau caaugauuga aaaauauaac gcucaccgug aucacuugaa 60

gcuacucaaa cagcucgagg auaagauuga cgauccg 97

<210> 1047
<211> 90
<212> DNA
<213> Artificial

<220>
<223> Artificial sequence

<400> 1047
guuuuacuau uucuagauuc uuuaagaucu acaaaaauaa ggauuuauuc cgaauuuacc 60

accuauuuua auuaauaggu gguuuuuuuu 90

<210> 1048
<211> 102
<212> DNA
<213> Artificial

<220>
<223> Artificial sequence

<400> 1048
gguucguaag uaaauagaaa gacauacuua gauuaaaaua aacaauaaug uuaaaugguc 60

guaaggcugc ucccgcgugg gggcuuuuuu uuugccuuuu uu 102

<210> 1049
<211> 106
<212> DNA
<213> Artificial

<220>
<223> Artificial sequence

<400> 1049
acauucgaga cuacugaucu aacacucaua gucaagauca aacgaguggu uuuccacgag 60

uuauuacuuu ugagccaugu uauucuggcc auacauauaa uaaaaa 106

<210> 1050
<211> 106
<212> DNA
<213> Artificial

<220>
<223> Artificial sequence

<400> 1050
acauucgaga cuacugaucu aacacucaua gucaagauca aacgaguggu uuuccacgag 60

uuauuacuuu ugagccaugu uauucuggcc auacauauaa uaaaaa 106

<210> 1051
<211> 168
<212> DNA
<213> Artificial

<220>
<223>  Artificial sequence

<400>  1051
guucuuaaca uuauucaaau gacauacuga guugaaauca aacaaggauu uauuccaagu    60

uuuauauuuu gaaccucuuu uugaggucag accucuuucu uaauugaaag agguuuuuuu    120

guugcaauuu uuaacaauau uuguaauagu agugguagaa guuuguuu    168

<210>  1052
<211>  125
<212>  DNA
<213>  Artificial

<220>
<223>  Artificial sequence

<400>  1052
uucauuaacc uuauugauuu gacauacuga guuaaaauaa aacaaugcgu aagcaaaguu    60

uacacuuauu agccauuuau aguuggcaau acauaaagaa agaccuaggu cauaugaccu    120

agguc    125

<210>  1053
<211>  106
<212>  DNA
<213>  Artificial

<220>
<223>  Artificial sequence

<400>  1053
accgauaaac ucauugauuu gauacucugg guuaaaauca aacgaaaccu ucggguuaag    60

uuuuuacuuc ugagccucgu augaggccuu uuuuuaugcc uucauu    106

<210>  1054
<211>  90
<212>  DNA
<213>  Artificial

<220>
<223>  Artificial sequence

<400>  1054
guuuuacuau uucuagauuc uuuaagaucu acaaaaauaa ggauuuauuc cgaauuuacc    60

accuauuuua auuaauaggu gguuuuuuuu    90

<210>  1055
<211>  112
<212>  DNA
<213>  Artificial

<220>
<223>  Artificial sequence

<400> 1055
acacuaaacu uuauugaucu aacaaccaga uuuaaaauca agcaaugcau cuuuugaugc 60

aaaguuucaa uacuuguccc gagcuaucga gggacuuuuu uuaugcaaaa aa 112

<210> 1056
<211> 92
<212> DNA
<213> Artificial

<220>
<223> Artificial sequence

<400> 1056
gaaauacuca uugauuugau acucugaguu aaaaucaaac aaggucuuug gaccaaguuu 60

ucuauucuga gccucuuaug aggccuuuuu uu 92

<210> 1057
<211> 138
<212> DNA
<213> Artificial

<220>
<223> Artificial sequence

<400> 1057
ccgcuugauc uuacugaauu gucacuaaga guuaaaguca gacaaggugg uuuaccacca 60

aguuuuaucu uaugagcccg uuaucgagcc auacauaucu uguuagaguc cuuugcugaa 120

ugcgucagcg aaggacuc 138

<210> 1058
<211> 112
<212> DNA
<213> Artificial

<220>
<223> Artificial sequence

<400> 1058
acucuaaacu uuauugaucu aacaaccaga uuuaaaauca agcaaugcau cuuuugaugc 60

aaaguuucaa cacuuguccc gagcuaucga gggacuuuuu uuauacaaaa aa 112

<210> 1059
<211> 112
<212> DNA
<213> Artificial

<220>
<223> Artificial sequence

<400> 1059
acucuaaacu uuauugaucu aacaaccaga uuuaaaauca agcaaugcau cuuuugaugc 60

aaaguuucaa cacuuguccc gagcuaucga gggacuuuuu uuauacaaaa aa 112

<210> 1060
<211> 83
<212> DNA
<213> Artificial

<220>
<223> Artificial sequence

<400> 1060
guuuuguuga uaggaaaaug auugagaauc uacaaagaua aggcuuuaug ccgaaauuac 60

ugccuuuuug gcaguuuuuu uuu 83

<210> 1061
<211> 141
<212> DNA
<213> Artificial

<220>
<223> Artificial sequence

<400> 1061
ggauuaacau uauugguuua acgucuuguu uuaaaaucaa acaaaacacu uuaguguuga 60

guuuuaacuu uugacccacu uuauuagugg gcaguacaua aaaaaggaau cacucuuaua 120

augaggagug auuccuuuuu u 141

<210> 1062
<211> 115
<212> DNA
<213> Artificial

<220>
<223> Artificial sequence

<400> 1062
acauuucaau uuacugauuu aacaaccuua uuuuuaaaau caagcaaggc uuucgggccg 60

aguuucaca uguguaccgc uuauagcggu cuuuuuuuuu gcaguaaaua aaaaa 115

<210> 1063
<211> 100
<212> DNA
<213> Artificial

<220>
<223> Artificial sequence

<400> 1063
acauuucaau uuacugauuu aacaaccuua uuuuuaaaau caagcaaggc uuucgggccg 60

aguuucaca uguguaccgc uuauagcggu cuuuuuuuuu 100

<210> 1064
<211> 112
<212> DNA

<213> Artificial

<220>
<223> Artificial sequence

<400> 1064
caucuaaacu uuauugaucu aacaaccaga uuuaaaauca agcaaugcau cuuuugaugc 60

aaaguuucaa uauuuguccc acguuaucga gggacuuuuu uuauacaaaa aa 112

<210> 1065
<211> 94
<212> DNA
<213> Artificial

<220>
<223> Artificial sequence

<400> 1065
cauuuuaauc ucauuagaau gacguacuga guuaaaauca aacaaagccu uaugcugagu 60

uuuaucuuuu gagcccuuaa gggccauaca uaua 94

<210> 1066
<211> 82
<212> DNA
<213> Artificial

<220>
<223> Artificial sequence

<400> 1066
guuuuauuga uaggaaaaug auugagaauc ugcaaagaua aggcuuuaug ccgaaauuac 60

ugccucuuag gcaguuuuuu uu 82

<210> 1067
<211> 92
<212> DNA
<213> Artificial

<220>
<223> Artificial sequence

<400> 1067
gcucuuaagu aauugagaug acuuacugag uuaaaauaaa cauuaaaguu aaaugaccuu 60

cuggucggcu cccgcguggg ggcuauuuuu uu 92

<210> 1068
<211> 104
<212> DNA
<213> Artificial

<220>
<223> Artificial sequence

<400> 1068
aggaauaaca ucauugauuu aacauccaga guuaaaguca aacaaugcau ucugcaaagu 60

uauuucuucu gacccauuua uuaaugggca auacauauua aaaa 104

<210> 1069
<211> 104
<212> DNA
<213> Artificial

<220>
<223> Artificial sequence

<400> 1069
aggaauaaca ucauugauuu aacauccaga guuaaaguca aacaaugcau ucugcaaagu 60

uauuucuucu gacccauuua uuaaugggca auacauauua aaaa 104

<210> 1070
<211> 140
<212> DNA
<213> Artificial

<220>
<223> Artificial sequence

<400> 1070
cuguuugacc uuauagauuu accacucaga guugaaauca aacaagaugc uuuagcauca 60

aguuucccau ugugagcccg uuaucggugc cauacauauu aaaacgacca agccaccuuu 120

ucgugacuug gucguuuuuu 140

<210> 1071
<211> 90
<212> DNA
<213> Artificial

<220>
<223> Artificial sequence

<400> 1071
guuuuacugu uucuaaaucu uugaagaucu acaaaaauaa ggauuuauuc cgaauuuacc 60

accuauuuua aaaaauaggu gguuuuuuuu 90

<210> 1072
<211> 96
<212> DNA
<213> Artificial

<220>
<223> Artificial sequence

<400> 1072
guuuuacugu uacuaaauuc uugagaaccu acuaaaauaa ggauuuauuc cgaauuuacc 60

accuauuuuu uaauaggugg uuuuuuuuua caaaaa 96

<210> 1073

<211> 96
<212> DNA
<213> Artificial

<220>
<223> Artificial sequence

<400> 1073
uuguucaaac uacaugaaau gacguacuga guuaaaauca acauuuaugu uaaauagucu 60

caauugacug gcucccgcgu gggggcuauu uuuuuu 96

<210> 1074
<211> 136
<212> DNA
<213> Artificial

<220>
<223> Artificial sequence

<400> 1074
cacaucgaau ucauugauuu gauacucuga guuaaaauca agcaaggcgc aagccaaguu 60

uaaucuucug aguccuguau gggaccauac auaaagagcg uuauugauuu cauuaucaau 120

aacgcucuau uuuauu 136

<210> 1075
<211> 140
<212> DNA
<213> Artificial

<220>
<223> Artificial sequence

<400> 1075
auguuugacc uuauugauuu aacauccugu guuaaaauca agcaaugcgc uuugcgcgga 60

guuuccacuu uugacccauu auaugggcau uacauaagcu aaaggaacca uucuucgauu 120

uggagaaugg uuccuuuuuu 140

<210> 1076
<211> 103
<212> DNA
<213> Artificial

<220>
<223> Artificial sequence

<400> 1076
auguuugacc uuauugauuu aacauccugu guuaaaauca agcaaugcgc uuugcgcgga 60

guuuccacuu uugacccauu auaugggcau uacauaagcu aaa 103

<210> 1077
<211> 110
<212> DNA
<213> Artificial

<220>
<223> Artificial sequence

<400> 1077
auuauaacau cuauguauuu gaaauguauu aaugcaucaa aaucuuagau cacaauuucu 60

auaauauacg uaaaacagca aauuuuacuu uauaagaauu uuuauauuuu 110

<210> 1078
<211> 109
<212> DNA
<213> Artificial

<220>
<223> Artificial sequence

<400> 1078
cguuuugaac ugauugauuc gacaucuaca cguugagauc aaacaaagcc ucggcugagu 60

uucaauuucu gagcccaugu ugggccauac auaccgccau ccgggaaaa 109

<210> 1079
<211> 108
<212> DNA
<213> Artificial

<220>
<223> Artificial sequence

<400> 1079
cguuuugaac ugauugauuc gacaucuaca cguugagauc aaacaaagcu ucggcugagu 60

uucaauuuuu gagcccaugu ugggccauac auacgccauc cgggaaaa 108

<210> 1080
<211> 69
<212> DNA
<213> Artificial

<220>
<223> Artificial sequence

<400> 1080
acuuaauaac cucauugauu ugauauucag aguuaaaauc aaacaaggcu uaugccaagu 60

uuucucuuu 69

<210> 1081
<211> 140
<212> DNA
<213> Artificial

<220>
<223> Artificial sequence

<400> 1081
auguuugacc uuauugauuu aacauccugu guuaaaauca agcaaugcgc uuugcgcgga 60

guuuccacuu uugacccauu auaugggcau uacauaagcg aaaggaacca uucuucgauu 120

uggagaaugg uuccuuuuuu 140

<210> 1082
<211> 117
<212> DNA
<213> Artificial

<220>
<223> Artificial sequence

<400> 1082
guucuaaacc uucuugauau gacuacuagc gucaagauca aacaaggcau uuugccgagu 60

uucaccuuuu gaguccguau ucggaccauu acauauguau caagaugucg cccauuu 117

<210> 1083
<211> 109
<212> DNA
<213> Artificial

<220>
<223> Artificial sequence

<400> 1083
acacuugacc uuauugauuu aauguccuuu auuugaaauc aaacaacacc auucauggug 60

aaguuuucac accuguccau ccuauauaua uggacaccua cauaaaaaa 109

<210> 1084
<211> 117
<212> DNA
<213> Artificial

<220>
<223> Artificial sequence

<400> 1084
guucuaaacc uucuugauau gacuacuagc gucaagauca aacaaggcau uuugccgagu 60

uucaccuuuu gaguccguau ucggaccauu acauauguau caagaugucg cccauuu 117

<210> 1085
<211> 103
<212> DNA
<213> Artificial

<220>
<223> Artificial sequence

<400> 1085
guucuaaacc uucuugauau gacuacuagc gucaagauca aacaaggcau uuugccgagu 60

uucaccuuuu gaguccguau ucggaccauu acauauguau caa 103

<210> 1086
<211> 106

<212> DNA
<213> Artificial

<220>
<223> Artificial sequence

<400> 1086
acaaucucaa cauuucaauu uacugauuua acaaccugau uuaaaaucaa gcaaggucuu 60

cggaccgagu uuucacauuu guaccgcuua uagcggucuu uuuuuu 106

<210> 1087
<211> 93
<212> DNA
<213> Artificial

<220>
<223> Artificial sequence

<400> 1087
auugaaagau ucauugauuu gauacucuga guuaaaauca aacaaaccuu cgggugaguu 60

uuuacuucug agccucguau ggggccuuuu uuu 93

<210> 1088
<211> 109
<212> DNA
<213> Artificial

<220>
<223> Artificial sequence

<400> 1088
cuacuaaaua uuauugauuu gaaguacaga guuaaaauca aacaagugcu ucagcacaag 60

uuucuacuuu ugagucaguu aucugaccaa uacauaaaag ggaauuaaa 109

<210> 1089
<211> 110
<212> DNA
<213> Artificial

<220>
<223> Artificial sequence

<400> 1089
caacuugaau ugauugaucu gacaucuacg aguugagauc aaacaaagcu ucagcugagu 60

uucaauuucu gagcccaugu ugggccauac auaugccacc cgagugcaaa 110

<210> 1090
<211> 110
<212> DNA
<213> Artificial

<220>
<223> Artificial sequence

<400> 1090

caacuugaau ugauugaucu gacaucuacg aguugagauc aaacaaagcu ucagcugagu 60

uucaauuucu gagcccaugu ugggccauac auaugccacc cgagugcaaa 110

<210> 1091
<211> 143
<212> DNA
<213> Artificial

<220>
<223> Artificial sequence

<400> 1091
acuuuugacc cuauugaucu gacauacagc guuaaaauca agcaaggcuu ucgagccaag 60

uuuaaaucuu uggguucgcu auucggaccg uacauaguaa aagggcguca ucgaguucaa 120

auucgauggc gcccuuuagu uuu 143

<210> 1092
<211> 102
<212> DNA
<213> Artificial

<220>
<223> Artificial sequence

<400> 1092
acuuuugacc cuauugaucu gacauacagc guuaaaauca agcaaggcuu ucgagccaag 60

uuuaaaucuu ugguucgcu auucggaccg uacauaguaa aa 102

<210> 1093
<211> 103
<212> DNA
<213> Artificial

<220>
<223> Artificial sequence

<400> 1093
acguaaaaca ucauugauau gacauccaga aguugaaauc aagcaagacu uaaagucaag 60

cuucuacuuu ugauucacgu uuuguggaca guacauauug aca 103

<210> 1094
<211> 95
<212> DNA
<213> Artificial

<220>
<223> Artificial sequence

<400> 1094
accgaaauac ucauugauuu gauacucuga guuaaaauca aacaaggucu uuugaccaag 60

uuuucuauuc ugagccucuu augaggccuu uuuuu 95

<210> 1095
<211> 119
<212> DNA
<213> Artificial

<220>
<223> Artificial sequence

<400> 1095
gguuuugacc ucauugauuu aacauucuga guugaaauca aacaaggcuu uuauagcuga 60

guuuuucuuu uugauccaua uauauggacu auacauaaaa aggagucauc ugacuccuu 119

<210> 1096
<211> 99
<212> DNA
<213> Artificial

<220>
<223> Artificial sequence

<400> 1096
auuuuaaucu cauuagaaug acguacugag uuaaaaucaa acaaggucuu gugccaaguu 60

uuaucuucug agcccuuaag ggccauacau auaagaaaa 99

<210> 1097
<211> 99
<212> DNA
<213> Artificial

<220>
<223> Artificial sequence

<400> 1097
auuuuaaucu cauuagaaug acguacugag uuaaaaucaa acaaggucuu gugccaaguu 60

uuaucuucug agcccuuaag ggccauacau auaagaaaa 99

<210> 1098
<211> 128
<212> DNA
<213> Artificial

<220>
<223> Artificial sequence

<400> 1098
acguuuaacc uauuugaucu gacauucaag guuaaaauca aacaaggcuu cggccaaguu 60

uuauucuuag ggcucgcuau cgagccauac auaaaaacac acccguuucg gcgggugugu 120

uuucuuuu 128

<210> 1099
<211> 129
<212> DNA
<213> Artificial

<220>
<223> Artificial sequence

<400> 1099
acgcuugacu cuauugauuu gucacugaaa guuaaaauca agcaaggccu ugugccaagu 60

uuuacuuuuu gagcccggua ucgagccaua cauagucggc ccuuccaguc auguggaagg 120

gccguuuuu 129

<210> 1100
<211> 86
<212> DNA
<213> Artificial

<220>
<223> Artificial sequence

<400> 1100
guuuuacuau uacuaaauuc uuaaggaucu acaaaaauaa ggauuuauuc cgaauuuacc 60

accuacuugu uaggugguuu uuuuuu 86

<210> 1101
<211> 167
<212> DNA
<213> Artificial

<220>
<223> Artificial sequence

<400> 1101
auguucuaag uaauugagau gacuuagaga auuaaaauca acauuaaagu aaaucgauca 60

uuuugauugg gcccaauggg gccuauuuuu uugcucugaa uuuuuagucg auauguuuaa 120

uugauuuaaa uaauccgguu ggugauucau aaauaaaauc aguuuuu 167

<210> 1102
<211> 143
<212> DNA
<213> Artificial

<220>
<223> Artificial sequence

<400> 1102
cugcuugacc uucuagauuu accacucagu guuaaaauca aacaagauac ugcgguauca 60

aguuuuccuu uuugagcucg uuaucgagcc aauacauaau uaaacgacca acucacuggc 120

aguccaauga guuggucauu uuu 143

<210> 1103
<211> 121
<212> DNA
<213> Artificial

<220>

<223>  Artificial sequence

<400>  1103
uugaacugau ugauucgaca ucuacacguu gagaucaaac aaagccucgg cugaguuuca       60

auuucugagc ccauguuggg ccauacauac cgccauccgg gaaaacccgg augguuuuuu      120

u                                                                      121

<210>  1104
<211>  140
<212>  DNA
<213>  Artificial

<220>
<223>  Artificial sequence

<400>  1104
gguuuaaccu cauugaucug auacacaaua uugaaguaac gcaaggcuuu augccaagcu       60

uaauuacuca ucaagagugg ccuuauggcc guacauaaaa ucccguguug uuacaaaaca      120

gcacgggauu ucuuuuuauu                                                  140

<210>  1105
<211>  145
<212>  DNA
<213>  Artificial

<220>
<223>  Artificial sequence

<400>  1105
guauagaaac cauugaucug acauacaaag uugaaguaac gcaaggcguc agccaagcuu       60

auugacucuu uacgaguggc ccuauggccg uacauaaaau cccguguugc cuuugcaaca      120

cgggauuuau cuuuauucau ccuuu                                            145

<210>  1106
<211>  91
<212>  DNA
<213>  Artificial

<220>
<223>  Artificial sequence

<400>  1106
uuguuaguau ucaaaauaac auagcaaguu aaaauaaggc uuuguccguu aucaacuuuu       60

aauuaaguag cgcuguuucg gcgcuuuuuu u                                      91

<210>  1107
<211>  94
<212>  DNA
<213>  Artificial

<220>
<223>  Artificial sequence

<400> 1107
auuauugcuu acacaauuau ugucgugcua aaauaaggcg cuguuaaugc agcugccgca 60

uccgccagag cauuuaugcu cuggcuuuuu auuu 94

<210> 1108
<211> 89
<212> DNA
<213> Artificial

<220>
<223> Artificial sequence

<400> 1108
auugucgcac ugcgaaauga gaaccguugc uacaauaagg ccgucugaaa agaugugccg 60

caacgcucug ccccuuaaag cuucugcuu 89

<210> 1109
<211> 143
<212> DNA
<213> Artificial

<220>
<223> Artificial sequence

<400> 1109
ggguucuacc ucauugaucu gacacacagc auugaaguaa agcaagauua auuucaagcu 60

uaauuuucuu cacauuuuau gugcagaagg gcuuaugccc acaauacaua aaaaguccgc 120

auucacuugc ggacuuuuau uuu 143

<210> 1110
<211> 143
<212> DNA
<213> Artificial

<220>
<223> Artificial sequence

<400> 1110
uauuuuaacu auauugauuu gacgcucuga guuaaaauca aacaaagcuu cggcugaguu 60

uuaucuuuug aggccgcuau cggcccauac auauuuuauu caagaaaagc uauugacggu 120

uaucaaacca ccaauagcuu uuu 143

<210> 1111
<211> 140
<212> DNA
<213> Artificial

<220>
<223> Artificial sequence

<400> 1111
acuuuuaacc uuauugauuu aacauccuga uuugaaauca aacaaugcac uuugugcgga 60

guuuauccau uugucccagc uaucugggca auacauagug aaagaggaac cgacccuuaa 120

ugggacggcu ccucuuuuuu 140

<210> 1112
<211> 140
<212> DNA
<213> Artificial

<220>
<223> Artificial sequence

<400> 1112
acuuuuaacc uuauugauuu aacauccuga uuugaaauca aacaaugcac uuugugcgga 60

guuuauccau uugucccagc uaucugggca auacauagug aaagaggaac cgacccuuaa 120

ugggacggcu ccucuuuuuu 140

<210> 1113
<211> 149
<212> DNA
<213> Artificial

<220>
<223> Artificial sequence

<400> 1113
uauuuuaacu auauugauuu gacgcucugg guuaaaauca aacaaagcuu uaugcugagu 60

uuuaucuuuu gaguccgcua ucggaccaua cauaauuuua uuaagaaaag cuauugaagg 120

uuauuaaacc gacaauagcu uuucuuuuu 149

<210> 1114
<211> 137
<212> DNA
<213> Artificial

<220>
<223> Artificial sequence

<400> 1114
acaauugaau uuacuucuuu aacauccuaa uuugaaauca agcaaugcac uucggugcgg 60

aguuuacaca uuugucgucu uuauaggcgc guacauaaag aaaagaggaa cguuccuuaa 120

uggaaguucc ucuuuuu 137

<210> 1115
<211> 79
<212> DNA
<213> Artificial

<220>
<223> Artificial sequence

<400> 1115

uacuaauuuu aaaguauuca caauaaggau uuuuccguug ugagaacauu agguugccuc 60

guccuuauaa cggggcuuu 79

<210> 1116
<211> 74
<212> DNA
<213> Artificial

<220>
<223> Artificial sequence

<400> 1116
agauuuuuaa gcaagucaca auaaggauua uuccguugug aaaacaaaca gagcggggca 60

acucgcucuu uuuu 74

<210> 1117
<211> 78
<212> DNA
<213> Artificial

<220>
<223> Artificial sequence

<400> 1117
auaccuaaaa uuacagaauc uacuaaaaca aggcaaaaug ccguguuuau cucgucaacu 60

uguuggcgag auuuuuuu 78

<210> 1118
<211> 95
<212> DNA
<213> Artificial

<220>
<223> Artificial sequence

<400> 1118
cuuguacuua uaccuaaaau uacagaaucu acugaaacaa gacaauaugu cguguuuauc 60

ccaucaauuu auuggugga uuuuuuuaug uuuuu 95

<210> 1119
<211> 119
<212> DNA
<213> Artificial

<220>
<223> Artificial sequence

<400> 1119
uuguuggaac uauucgaaac agcacagcgc guuaaaauaa ggcagugauu uuuuaaucca 60

guccguauuc agcuugaaaa agugagcacc gauucggugc uuuuuuuuau gucauuguu 119

<210> 1120
<211> 119

<212> DNA
<213> Artificial

<220>
<223> Artificial sequence

<400> 1120
uuguuggagc uauucgaaac aacacagcga guuaaaauaa ggcuuugucc guacacaacu 60

uguaaaagug gcacccgauu cgggugcguu uuuuugugcc cuuacaaaua auuucaaca 119

<210> 1121
<211> 102
<212> DNA
<213> Artificial

<220>
<223> Artificial sequence

<400> 1121
cuugcacagu uacuuaaauc uugcugagcc uacaaagaua aggcuucaug ccgaaaucaa 60

gcacccccgu uuuuaacgag gggugcuuuu cguuauguaa aa 102

<210> 1122
<211> 110
<212> DNA
<213> Artificial

<220>
<223> Artificial sequence

<400> 1122
cuuacacagu uacuuaaauc uugcagaagc uacaaagaua aggcuucaug ccgaaaucaa 60

cacccuaucu auuauaagau aggguguuuu cguuuuauag aggagauuuu 110

<210> 1123
<211> 119
<212> DNA
<213> Artificial

<220>
<223> Artificial sequence

<400> 1123
uuguuggaac uauucgaaac aacacagcaa guuaaaauaa ggcuuugucc guacacaacu 60

ugaaaaagug cgcaccgauu cggugcuuuu uauuuuauua uacuaacuca uuguacucc 119

<210> 1124
<211> 119
<212> DNA
<213> Artificial

<220>
<223> Artificial sequence

<400> 1124

uuguuggaac cauucaaaac agcauagcaa aguuaaaaua aggcuagucc guuaucaacu 60

ugaaaaagug gcaccgaguc ggugcuuuuu uugauacuuc uauucuacuc ugacugcaa 119

<210> 1125
<211> 85
<212> DNA
<213> Artificial

<220>
<223> Artificial sequence

<400> 1125
auucacauua uucaaguuua cuacagauuc uguauuaaga uacaaaaucg uaagacuggc 60

ugccuuaagc agcuuuuuuu auuuu 85

<210> 1126
<211> 105
<212> DNA
<213> Artificial

<220>
<223> Artificial sequence

<400> 1126
cuuacacagu uacuuaaauc uugcagaagc uacaaagaua aggcuucaug ccgaaaucaa 60

cacccugucu augacggggu guuuucgugu uguaaaggag uuuuu 105

<210> 1127
<211> 119
<212> DNA
<213> Artificial

<220>
<223> Artificial sequence

<400> 1127
auguuggaau cauucgaaac aacacagcaa guuaaaauaa ggcuuugucc guacucaacu 60

uaaaaagugc gcaccgauuc ggugcuuuuu uauuucaauc aagaacacaa aaaaacagc 119

<210> 1128
<211> 119
<212> DNA
<213> Artificial

<220>
<223> Artificial sequence

<400> 1128
uuguuggaac cauucaaaac aacguagcaa guuaaaauaa ggcuaguccg uuaucaacuu 60

gaaaaagugg caccgagucg gugcuuuuug uaucuuuauu uuaccuuguc aauaaacua 119

<210> 1129
<211> 119

<212> DNA
<213> Artificial

<220>
<223> Artificial sequence

<400> 1129
ccuauggaac uauucaauac agcauagcaa aguuaaaaua aggcuuuguc cguaaucaac 60

cugaaaaggg gagcaccgaa ucggugcuuu uuuacuuucu accaacucua ccaauacuu 119

<210> 1130
<211> 99
<212> DNA
<213> Artificial

<220>
<223> Artificial sequence

<400> 1130
cuuacacagu uacuuaaauc uugcagaagc uacaaagaua aggcuucaug ccgaauucaa 60

cacccuguca uuuauggcgg gguguuuucg auuuguaaa 99

<210> 1131
<211> 109
<212> DNA
<213> Artificial

<220>
<223> Artificial sequence

<400> 1131
cuugcacggu uacuuaaauc uugcagagcc uacaaagaua aggcuucaug ccgaauucaa 60

gcaccccaug uuuacauggg gugcuuuucg uuguguaaaa aggagagua 109

<210> 1132
<211> 106
<212> DNA
<213> Artificial

<220>
<223> Artificial sequence

<400> 1132
cuuacacagu uacuuaaauc uugcagaagc uacaaagaua aggcuucaug ccgaaaucaa 60

cacccugucu augacggggu guuuucgugu uguaaaggag uuuuua 106

<210> 1133
<211> 119
<212> DNA
<213> Artificial

<220>
<223> Artificial sequence

<400> 1133

uuguuggaac uauucgaaac aacacaguga guuaaaauaa ggcuuugucc guacacaacu 60

uauaaaagug gcacccgauu cggaugcauu uuuuauuugu gauucaaaua gauauaaca 119

<210> 1134
<211> 119
<212> DNA
<213> Artificial

<220>
<223> Artificial sequence

<400> 1134
ucguuggaac uauucgaaac aacacagcaa aguuaaaaua aggcuuuguc cguacacaac 60

uugaaaaagu gcgcaccgau ucggugcuuu uuuguuuuac uauacuaauu uauucuacu 119

<210> 1135
<211> 119
<212> DNA
<213> Artificial

<220>
<223> Artificial sequence

<400> 1135
guguuggaau cauucgaaac aacacagcaa guuaaaauaa ggcagugauu uuuaauccag 60

uccguacaca acuugaaaaa gugcgcaccg auucggugcu uuuuuauuug cuuuacugu 119

<210> 1136
<211> 100
<212> DNA
<213> Artificial

<220>
<223> Artificial sequence

<400> 1136
cuugcacagu uacuuaaauc uugcagagcc uacaaagaua aggcuuuaug ccgaaaucaa 60

gcaccccguu uauacgaggu gcuuuucguu uuaaauaaaa 100

<210> 1137
<211> 99
<212> DNA
<213> Artificial

<220>
<223> Artificial sequence

<400> 1137
cuuacacagu uacuuaaauc uugcagaagc uacaaagaua aggcuucaug ccgaauucaa 60

cacccuguca uuuauggcgg gguguuuucg uuuuguaaa 99

<210> 1138
<211> 119

<212> DNA
<213> Artificial

<220>
<223> Artificial sequence

<400> 1138
gguuugaaac cauucgaaac aauacagcaa aguuaaaaua aggcuuaguc cguauucaac 60

uugagaaagu ggcaccgauu cggugcuuuu uugcugcaca aagagucccu uggaauuau 119

<210> 1139
<211> 119
<212> DNA
<213> Artificial

<220>
<223> Artificial sequence

<400> 1139
uuguuggaac uauucgaaac aacacagcaa guuaaaauaa ggcuuugucc guacacaacu 60

ugaaaaagug cgcaccgauu cggugcuuuu uuaauuacua caggugcuuu uuuaauuac 119

<210> 1140
<211> 100
<212> DNA
<213> Artificial

<220>
<223> Artificial sequence

<400> 1140
cuuacacagu uacuuaaauc uugcagaagc uacaaagaua aggcuucaug ccgaaaucaa 60

cacccuguca uuuuauggca gggguuuuc guuauuuaaa 100

<210> 1141
<211> 119
<212> DNA
<213> Artificial

<220>
<223> Artificial sequence

<400> 1141
gguuugaaac cauucgaaac aacacagcga guuaaaauaa ggcuuagucc guacucaacu 60

ugaaaaggug gcaccgauuc gguguuuuuu uuauacacaa agagucccuu ggaaucauc 119

<210> 1142
<211> 102
<212> DNA
<213> Artificial

<220>
<223> Artificial sequence

<400> 1142

cuuacacagu uacuuaaauc uugcagaagc uacaaagaua aggcuucaug ccgaaaucaa 60

cacccuguca uuuuauggca ggguguuuuc guuauuuaaa aa 102

<210> 1143
<211> 79
<212> DNA
<213> Artificial

<220>
<223> Artificial sequence

<400> 1143
cucacuggga uaugcgcucu ggacgcuaac aagccgagag augcggcgcc aaauggaaag 60

ccccgcagug cggggcuuu 79

<210> 1144
<211> 108
<212> DNA
<213> Artificial

<220>
<223> Artificial sequence

<400> 1144
uuauagccgc ugccuacuca gccauuucgc gcagcgacga cgauuauaug uuagauuuuu 60

uuaaaaaaua cuaaaauuug gagcgaagcu uaugauugaa cggauuuu 108

<210> 1145
<211> 135
<212> DNA
<213> Artificial

<220>
<223> Artificial sequence

<400> 1145
accaaggttg ttctaataaa atattgaaaa tattgtttat tagttcctct ttaagtttct 60

ttaaatagtc ggtattttgt tatgaattat gatatcttac tattgtttta tttgatgaaa 120

aattttctta aaaca 135

<210> 1146
<211> 328
<212> PRT
<213> Pseudomonas aeruginosa

<400> 1146

Gly Ser Phe Thr Met Asp Asp Ile Ser Pro Ser Glu Leu Lys Thr Ile
1               5                   10                  15

Leu His Ser Lys Arg Ala Asn Leu Tyr Tyr Leu Gln His Cys Arg Val
            20                  25                  30

```
Leu Val Asn Gly Gly Arg Val Glu Tyr Val Thr Asp Glu Gly Arg His
        35                  40                  45

Ser His Tyr Trp Asn Ile Pro Ile Ala Asn Thr Thr Ser Leu Leu Leu
    50                  55                  60

Gly Thr Gly Thr Ser Ile Thr Gln Ala Ala Met Arg Glu Leu Ala Arg
65                  70                  75                  80

Ala Gly Val Leu Val Gly Phe Cys Gly Gly Gly Gly Thr Pro Leu Phe
                85                  90                  95

Ser Ala Asn Glu Val Asp Val Glu Val Ser Trp Leu Thr Pro Gln Ser
            100                 105                 110

Glu Tyr Arg Pro Thr Glu Tyr Leu Gln Arg Trp Val Gly Phe Trp Phe
        115                 120                 125

Asp Glu Glu Lys Arg Leu Val Ala Ala Arg His Phe Gln Arg Ala Arg
    130                 135                 140

Leu Glu Arg Ile Arg His Ser Trp Leu Glu Asp Arg Val Leu Arg Asp
145                 150                 155                 160

Ala Gly Phe Ala Val Asp Ala Thr Ala Leu Ala Val Ala Val Glu Asp
                165                 170                 175

Ser Ala Arg Ala Leu Glu Gln Ala Pro Asn His Glu His Leu Leu Thr
            180                 185                 190

Glu Glu Ala Arg Leu Ser Lys Arg Leu Phe Lys Leu Ala Ala Gln Ala
        195                 200                 205

Thr Arg Tyr Gly Glu Phe Val Arg Ala Lys Arg Gly Ser Gly Gly Asp
    210                 215                 220

Pro Ala Asn Arg Phe Leu Asp His Gly Asn Tyr Leu Ala Tyr Gly Leu
225                 230                 235                 240

Ala Ala Thr Ala Thr Trp Val Leu Gly Ile Pro His Gly Leu Ala Val
                245                 250                 255

Leu His Gly Lys Thr Arg Arg Gly Gly Leu Val Phe Asp Val Ala Asp
            260                 265                 270

Leu Ile Lys Asp Ser Leu Ile Leu Pro Gln Ala Phe Leu Ser Ala Met
        275                 280                 285
```

Arg Gly Asp Glu Glu Gln Asp Phe Arg Gln Ala Cys Leu Asp Asn Leu
290 295 300

Ser Arg Ala Gln Ala Leu Asp Phe Met Ile Asp Thr Leu Lys Asp Val
305 310 315 320

Ala Gln Arg Ser Thr Val Ser Ala
325

<210> 1147
<211> 326
<212> PRT
<213> Thermotoga maritima

<400> 1147

Arg Glu Leu Tyr Phe Gln Gly Met Glu Ser Val Tyr Leu Phe Ser Ser
1 5 10 15

Gly Thr Leu Lys Arg Lys Ala Asn Thr Ile Cys Leu Glu Thr Glu Ser
20 25 30

Gly Arg Lys Tyr Ile Pro Val Glu Asn Val Met Asp Ile Lys Val Phe
35 40 45

Gly Glu Val Asp Leu Asn Lys Arg Phe Leu Glu Phe Leu Ser Gln Lys
50 55 60

Arg Ile Pro Ile His Phe Phe Asn Arg Glu Gly Tyr Tyr Val Gly Thr
65 70 75 80

Phe Tyr Pro Arg Glu Tyr Leu Asn Ser Gly Phe Leu Ile Leu Lys Gln
85 90 95

Ala Glu His Tyr Ile Asn Gln Glu Lys Arg Met Leu Ile Ala Arg Glu
100 105 110

Ile Val Ser Arg Ser Phe Gln Asn Met Val Asp Phe Leu Lys Lys Arg
115 120 125

Lys Val Arg Ala Asp Ser Leu Thr Arg Tyr Lys Lys Lys Ala Glu Glu
130 135 140

Ala Ser Asn Val Ser Glu Leu Met Gly Ile Glu Gly Asn Ala Arg Glu
145 150 155 160

Glu Tyr Tyr Ser Met Ile Asp Ser Leu Val Ser Asp Glu Arg Phe Arg
165 170 175

Ile Glu Lys Arg Thr Arg Arg Pro Pro Lys Asn Phe Ala Asn Thr Leu
180 185 190

Ile Ser Phe Gly Asn Ser Leu Leu Tyr Thr Thr Val Leu Ser Leu Ile
195 200 205

Tyr Gln Thr His Leu Asp Pro Arg Ile Gly Tyr Leu His Glu Thr Asn
210 215 220

Phe Arg Arg Phe Ser Leu Asn Leu Asp Ile Ala Glu Leu Phe Lys Pro
225 230 235 240

Ala Val Val Asp Arg Leu Phe Leu Asn Leu Val Asn Thr Arg Gln Ile
245 250 255

Asn Glu Lys His Phe Asp Glu Ile Ser Glu Gly Leu Met Leu Asn Asp
260 265 270

Glu Gly Lys Ser Leu Phe Val Lys Asn Tyr Glu Gln Ala Leu Arg Glu
275 280 285

Thr Val Phe His Lys Lys Leu Asn Arg Tyr Val Ser Met Arg Ser Leu
290 295 300

Ile Lys Met Glu Leu His Lys Leu Glu Lys His Leu Ile Gly Glu Gln
305 310 315 320

Val Phe Gly Ser Glu Glu
325

<210> 1148
<211> 315
<212> PRT
<213> Aquifex aeolicus

<400> 1148

Gly Arg Val Tyr Tyr Ile Asn Ser His Gly Thr Leu Ser Arg His Glu
1 5 10 15

Asn Thr Leu Arg Phe Glu Asn Ala Glu Val Lys Lys Asp Ile Pro Val
20 25 30

Glu Asp Val Glu Glu Ile Phe Val Phe Ala Glu Leu Ser Leu Asn Thr
35 40 45

Lys Leu Leu Asn Phe Leu Ala Ser Lys Gly Ile Pro Leu His Phe Phe
50 55 60

```
Asn Tyr Tyr Gly Tyr Tyr Thr Gly Thr Phe Tyr Pro Arg Glu Ser Ser
65                  70                  75                  80

Val Ser Gly His Leu Leu Ile Lys Gln Val Glu His Tyr Leu Asp Ala
                85                  90                  95

Gln Lys Arg Leu Tyr Leu Ala Lys Ser Phe Val Ile Gly Ser Ile Leu
            100                 105                 110

Asn Leu Glu Tyr Val Tyr Lys Ile Ser Ala Asp Thr Tyr Leu Asn Lys
        115                 120                 125

Val Lys Glu Thr Asn Ser Ile Pro Glu Leu Met Ser Val Glu Ala Glu
    130                 135                 140

Phe Arg Lys Leu Cys Tyr Lys Lys Leu Glu Glu Val Thr Gly Trp Glu
145                 150                 155                 160

Leu Glu Lys Arg Thr Lys Arg Pro Pro Gln Asn Pro Leu Asn Ala Leu
                165                 170                 175

Ile Ser Phe Gly Asn Ser Leu Thr Tyr Ala Lys Val Leu Gly Glu Ile
            180                 185                 190

Tyr Lys Thr Gln Leu Asn Pro Thr Val Ser Tyr Leu His Glu Pro Ser
        195                 200                 205

Thr Lys Arg Phe Ser Leu Ser Leu Asp Val Ala Glu Val Phe Lys Pro
    210                 215                 220

Ile Phe Val Asp Asn Leu Ile Ile Arg Leu Ile Gln Glu Asn Lys Ile
225                 230                 235                 240

Asp Lys Thr His Phe Ser Thr Glu Leu Asn Met Thr Phe Leu Asn Glu
                245                 250                 255

Ile Gly Arg Lys Val Phe Leu Lys Ala Phe Asn Glu Leu Leu Glu Thr
            260                 265                 270

Thr Ile Phe Tyr Pro Lys Leu Asn Arg Lys Val Ser His Arg Thr Leu
        275                 280                 285

Ile Lys Leu Glu Leu Tyr Lys Leu Ile Lys His Leu Leu Glu Glu Glu
    290                 295                 300

Val Tyr Leu Pro Leu Asn Tyr Gly Gly Leu Lys
```

305 310 315

<210> 1149
<211> 101
<212> PRT
<213> Sulfolobus solfataricus

<400> 1149

Gly Ala Met Leu Tyr Leu Ile Phe Tyr Asp Ile Thr Asp Asp Asn Leu
1 5 10 15

Arg Asn Arg Val Ala Glu Phe Leu Lys Lys Lys Gly Leu Asp Arg Ile
20 25 30

Gln Tyr Ser Val Phe Met Gly Asp Leu Asn Ser Ser Arg Leu Lys Asp
35 40 45

Val Glu Ala Gly Leu Lys Ile Ile Gly Asn Arg Lys Lys Leu Gln Glu
50 55 60

Asp Glu Arg Phe Phe Ile Leu Ile Val Pro Ile Thr Glu Asn Gln Phe
65 70 75 80

Arg Glu Arg Ile Val Ile Gly Tyr Ser Gly Ser Glu Arg Glu Glu Lys
85 90 95

Ser Asn Val Val Trp
100

<210> 1150
<211> 101
<212> PRT
<213> Sulfolobus solfataricus

<400> 1150

Met Ala Met Leu Tyr Leu Ile Phe Tyr Asp Ile Thr Asp Asp Asn Leu
1 5 10 15

Arg Asn Arg Val Ala Glu Phe Leu Lys Lys Lys Gly Leu Asp Arg Ile
20 25 30

Gln Tyr Ser Val Phe Met Gly Asp Leu Asn Ser Ser Arg Leu Lys Asp
35 40 45

Val Glu Ala Gly Leu Lys Ile Ile Gly Asn Arg Lys Lys Leu Gln Glu
50 55 60

Asp Glu Arg Phe Phe Ile Leu Ile Val Pro Ile Thr Glu Asn Gln Phe
65 70 75 80

Arg Glu Arg Ile Val Ile Gly Tyr Ser Gly Ser Glu Arg Glu Glu Lys
85 90 95

Ser Asn Val Val Trp
100

<210> 1151
<211> 91
<212> PRT
<213> Sulfolobus solfataricus

<400> 1151

Phe Gln Gly Met Lys Leu Leu Val Val Tyr Asp Val Ser Asp Asp Ser
1 5 10 15

Lys Arg Asn Lys Leu Ala Asn Asn Leu Lys Lys Leu Gly Leu Glu Arg
20 25 30

Ile Gln Arg Ser Ala Phe Glu Gly Asp Met Asp Ser Gln Arg Met Lys
35 40 45

Asp Leu Val Arg Val Val Lys Leu Ile Val Asp Thr Asn Thr Asp Ile
50 55 60

Val His Ile Ile Pro Leu Gly Ile Arg Asp Trp Glu Arg Arg Ile Val
65 70 75 80

Ile Gly Arg Glu Gly Leu Glu Glu Trp Leu Val
85 90

<210> 1152
<211> 103
<212> PRT
<213> Escherichia coli

<400> 1152

Met Ser Met Leu Val Val Val Thr Glu Asn Val Pro Pro Arg Leu Arg
1 5 10 15

Gly Arg Leu Ala Ile Trp Leu Leu Glu Val Arg Ala Gly Val Tyr Val
20 25 30

Gly Asp Val Ser Ala Lys Ile Arg Glu Met Ile Trp Glu Gln Ile Ala
35 40 45

Gly Leu Ala Glu Glu Gly Asn Val Val Met Ala Trp Ala Thr Asn Thr
50 55 60

Glu Thr Gly Phe Glu Phe Gln Thr Phe Gly Leu Asn Arg Arg Thr Pro
65 70 75 80

Val Asp Leu Asp Gly Leu Arg Leu Val Ser Phe Leu Pro Val Gly Ser
85 90 95

Ser Glu Asn Leu Tyr Phe Gln
100

<210> 1153
<211> 305
<212> PRT
<213> Escherichia coli

<400> 1153

Met Thr Trp Leu Pro Leu Asn Pro Ile Pro Leu Lys Asp Arg Val Ser
1 5 10 15

Met Ile Phe Leu Gln Tyr Gly Gln Ile Asp Val Ile Asp Gly Ala Phe
20 25 30

Val Leu Ile Asp Lys Thr Gly Ile Arg Thr His Ile Pro Val Gly Ser
35 40 45

Val Ala Cys Ile Met Leu Glu Pro Gly Thr Arg Val Ser His Ala Ala
50 55 60

Val Arg Leu Ala Ala Gln Val Gly Thr Leu Leu Val Trp Val Gly Glu
65 70 75 80

Ala Gly Val Arg Val Tyr Ala Ser Gly Gln Pro Gly Gly Ala Arg Ser
85 90 95

Asp Lys Leu Leu Tyr Gln Ala Lys Leu Ala Leu Asp Glu Asp Leu Arg
100 105 110

Leu Lys Val Val Arg Lys Met Phe Glu Leu Arg Phe Gly Glu Pro Ala
115 120 125

Pro Ala Arg Arg Ser Val Glu Gln Leu Arg Gly Ile Glu Gly Ser Arg
130 135 140

Val Arg Ala Thr Tyr Ala Leu Leu Ala Lys Gln Tyr Gly Val Thr Trp
145 150 155 160

Asn Gly Arg Arg Tyr Asp Pro Lys Asp Trp Glu Lys Gly Asp Thr Ile
165 170 175

Asn Gln Cys Ile Ser Ala Ala Thr Ser Cys Leu Tyr Gly Val Thr Glu
180 185 190

Ala Ala Ile Leu Ala Ala Gly Tyr Ala Pro Ala Ile Gly Phe Val His
195 200 205

Thr Gly Lys Pro Leu Ser Phe Val Tyr Asp Ile Ala Asp Ile Ile Lys
210 215 220

Phe Asp Thr Val Val Pro Lys Ala Phe Glu Ile Ala Arg Arg Asn Pro
225 230 235 240

Gly Glu Pro Asp Arg Glu Val Arg Leu Ala Cys Arg Asp Ile Phe Arg
245 250 255

Ser Ser Lys Thr Leu Ala Lys Leu Ile Pro Leu Ile Glu Asp Val Leu
260 265 270

Ala Ala Gly Glu Ile Gln Pro Pro Ala Pro Pro Glu Asp Ala Gln Pro
275 280 285

Val Ala Ile Pro Leu Pro Val Ser Leu Gly Asp Ala Gly His Arg Ser
290 295 300

Ser
305

<210> 1154
<211> 964
<212> PRT
<213> Thermobifida fusca

<400> 1154

Met Gly Ser Ser His His His His His His Ser Ser Gly Leu Val Pro
1 5 10 15

Arg Gly Ser His Met Pro Glu His Asp Ser Thr Asp Asp Lys His Gly
20 25 30

Ile Pro Pro Leu Asp Leu Arg Phe Trp Ala Lys Glu Arg Gly Leu Arg
35 40 45

Gly Lys Thr Tyr Pro Leu Val Cys His Ser Leu Asp Ala Ala Ala Ala
50 55 60

Ala Leu Val Leu Trp Asn Glu Tyr Leu Ser Pro Gly Leu Arg Asp Thr
65 70 75 80

```
Ile Ala Ser Ser Met Glu Thr Asp Glu Glu His Ala Gly His Cys Ile
                85                  90                  95

Ala Phe Trp Ala Gly Leu His Asp Ile Gly Lys Leu Thr Arg Glu Phe
            100                 105                 110

Gln Gln Gln Ile Ala Ile Asp Leu Ser Ala Tyr Pro Gly Glu Glu Leu
        115                 120                 125

Ser Gly Glu Gln Arg Ser His Ala Ala Ala Thr Gly Lys Trp Leu Pro
    130                 135                 140

Phe Ala Leu Pro Ser Leu Gly Tyr Pro Asn Gly Gly Leu Val Thr Gly
145                 150                 155                 160

Leu Val Ala Gln Met Leu Gly Gly His His Gly Thr Phe His Pro His
                165                 170                 175

Pro Ser Phe Gln Ser Arg Asn Pro Leu Ala Glu Phe Gly Phe Ser Ser
            180                 185                 190

Pro His Trp Glu Lys Gln Arg His Ala Leu Leu His Ala Val Phe Asp
        195                 200                 205

Ala Thr Gly Arg Pro Thr Pro Pro Asp Met Leu Asp Gly Pro Thr Ala
    210                 215                 220

Ser Val Val Cys Gly Leu Val Ile Leu Ala Asp Trp Leu Val Ser Gln
225                 230                 235                 240

Glu Asp Phe Leu Leu Glu Arg Leu Thr Ser Leu Pro Ala Asp Gly Ser
                245                 250                 255

Ala Ser Ala Leu Arg Ala His Phe Glu Thr Ser Leu Arg Arg Ile Pro
            260                 265                 270

Ser Leu Leu Asp Ala Ala Gly Leu Arg Pro Ile Thr Val Pro Pro Ala
        275                 280                 285

Thr Phe Thr Glu Ser Phe Pro His Leu Ser Lys Pro Asn Gly Leu Gln
    290                 295                 300

Ala Ser Leu Ala Lys His Leu Pro Cys Leu Cys Thr Gly Pro Gly Leu
305                 310                 315                 320

Val Leu Ile Thr Ala Pro Met Gly Glu Gly Lys Thr Glu Ala Ala Tyr
                325                 330                 335
```

His Val Ala Asp Leu Leu Gly Lys Ala Thr Gly Arg Pro Gly Arg Phe
340 345 350

Leu Ala Leu Pro Thr Met Ala Thr Ala Asp Gln Met His Thr Arg Leu
355 360 365

Lys Glu Tyr Ala Arg Tyr Arg Val Glu Asn Thr Asp Leu Pro Arg Ser
370 375 380

Ser Thr Leu Ala Leu Leu His Ser Met Ala Trp Leu Asn Pro Asp Tyr
385 390 395 400

Ala Pro Ala Asp Leu Pro Gly Val Ser Lys Val Leu Ser Asn Leu Gly
405 410 415

His Arg Asp Pro Phe Ala Ala Thr Asp Trp Leu Met Gly Arg Lys Arg
420 425 430

Gly Leu Leu Ala Pro Trp Ala Val Gly Thr Ile Asp Gln Ala Leu Met
435 440 445

Ala Val Leu Arg Ala Lys His Asn Ala Leu Arg Leu Phe Gly Leu Ala
450 455 460

Gly Lys Val Val Val Val Asp Glu Ala His Ala Val Asp Pro Tyr Met
465 470 475 480

Gln Val Leu Leu Glu Gln Leu Leu Arg Trp Leu Gly Thr Leu Asp Val
485 490 495

Pro Val Val Leu Leu Ser Ala Thr Leu His His Ser Ile Ala Asn Ser
500 505 510

Leu Val Lys Ala Tyr Leu Glu Gly Ala Arg Gly Arg Arg Trp Asn Arg
515 520 525

Ser Glu Pro Gln Pro Val Ser Glu Val Ser Tyr Pro Gly Trp Leu His
530 535 540

Val Asp Ala Arg Ile Gly Lys Val Thr Arg Ser Ser Asp Val Asp Pro
545 550 555 560

Leu Pro Ile Ala Thr Thr Pro Arg Lys Pro Leu Glu Val Arg Leu Val
565 570 575

Asp Val Pro Val Lys Glu Gly Ala Leu Asn Arg Ser Thr Val Leu Ala

580 585 590

Lys Glu Leu Thr Pro Leu Val Lys Gln Gly Gly Cys Ala Ala Ile Ile
595 600 605

Cys Thr Thr Val Ala Glu Ala Gln Gly Val Tyr Asp Leu Leu Ser Gln
610 615 620

Trp Phe Ala Thr Leu Gly Glu Asp Ala Pro Asp Leu Tyr Leu Leu His
625 630 635 640

Ser Arg Phe Pro Asn Arg Gln Arg Thr Glu Ile Thr Ala Thr Ile Val
645 650 655

Asp Leu Phe Gly Lys Glu Gly Ala Gln Ser Gly Arg Arg Pro Thr Arg
660 665 670

Gly Ala Val Leu Val Ala Thr Gln Val Val Glu Gln Ser Leu Asp Leu
675 680 685

Asp Val Asp Leu Met Ile Ser Asp Leu Ala Pro Val Ser Leu Leu Leu
690 695 700

Gln Arg Ala Gly Arg Cys Trp Arg His Glu His Leu Gly Ile Ile Asn
705 710 715 720

Arg Pro Gln Trp Ala Lys Gln Pro Glu Leu Val Val Leu Thr Pro Glu
725 730 735

Gln Asn Gly Asp Ala Asp Arg Ala Pro Trp Phe Pro Arg Ser Trp Thr
740 745 750

Ser Val Tyr Pro Leu Ala Leu Leu Gln Arg Thr Tyr Thr Leu Leu Arg
755 760 765

Arg Arg Asn Gly Ala Pro Val Gln Ile Pro Glu Asp Val Gln Gln Leu
770 775 780

Val Asp Asp Val Tyr Asp Asp Asp Ser Leu Ala Glu Asp Leu Glu Ala
785 790 795 800

Asp Met Glu Arg Met Gly Glu Glu Leu Ala Gln Arg Gly Leu Ala Arg
805 810 815

Asn Ala Val Ile Pro Asp Pro Asp Asp Ala Glu Asp Asn Leu Asn Gly
820 825 830

Leu Thr Glu Phe Ser Phe Asp Val Asp Glu His Val Leu Ala Thr Arg
        835                 840                 845

Phe Gly Ala Gly Ser Val Arg Val Leu Cys Tyr Tyr Val Asp Thr Ala
    850                 855                 860

Gly Asn Arg Trp Leu Asp Pro Glu Cys Thr Val Glu Phe Pro Glu Gln
865                 870                 875                 880

Gly Thr Gly Arg Glu Gly Arg Phe Thr Met Ala Asp Cys Arg Asp Leu
                885                 890                 895

Val Ala Arg Thr Ile Pro Val Arg Met Gly Pro Trp Ala Ser Gln Leu
            900                 905                 910

Thr Glu Asp Asn His Pro Pro Glu Ala Trp Arg Glu Ser Phe Tyr Leu
        915                 920                 925

Arg Asp Leu Val Leu Ile Pro Gln Arg Val Thr Asp Glu Gly Ala Val
    930                 935                 940

Leu Pro Thr Glu Thr Gly Gly Arg Glu Trp Leu Leu Asp Pro Cys Lys
945                 950                 955                 960

Gly Leu Ile Phe

<210> 1155
<211> 964
<212> PRT
<213> Thermobifida fusca

<400> 1155

Met Gly Ser Ser His His His His His His Ser Ser Gly Leu Val Pro
1               5                   10                  15

Arg Gly Ser His Met Pro Glu His Asp Ser Thr Asp Asp Lys His Gly
            20                  25                  30

Ile Pro Pro Leu Asp Leu Arg Phe Trp Ala Lys Glu Arg Gly Leu Arg
        35                  40                  45

Gly Lys Thr Tyr Pro Leu Val Cys His Ser Leu Asp Ala Ala Ala Ala
    50                  55                  60

Ala Leu Val Leu Trp Asn Glu Tyr Leu Ser Pro Gly Leu Arg Asp Thr
65                  70                  75                  80

```
Ile Ala Ser Ser Met Glu Thr Asp Glu Glu His Ala Gly His Cys Ile
                85                  90                  95

Ala Phe Trp Ala Gly Leu His Asp Ile Gly Lys Leu Thr Arg Glu Phe
            100                 105                 110

Gln Gln Gln Ile Ala Ile Asp Leu Ser Ala Tyr Pro Gly Glu Glu Leu
        115                 120                 125

Ser Gly Glu Gln Arg Ser His Ala Ala Ala Thr Gly Lys Trp Leu Pro
    130                 135                 140

Phe Ala Leu Pro Ser Leu Gly Tyr Pro Asn Gly Gly Leu Val Thr Gly
145                 150                 155                 160

Leu Val Ala Gln Met Leu Gly Gly His His Gly Thr Phe His Pro His
                165                 170                 175

Pro Ser Phe Gln Ser Arg Asn Pro Leu Ala Glu Phe Gly Phe Ser Ser
            180                 185                 190

Pro His Trp Glu Lys Gln Arg His Ala Leu Leu His Ala Val Phe Asp
        195                 200                 205

Ala Thr Gly Arg Pro Thr Pro Pro Asp Met Leu Asp Gly Pro Thr Ala
    210                 215                 220

Ser Val Val Cys Gly Leu Val Ile Leu Ala Asp Trp Leu Val Ser Gln
225                 230                 235                 240

Glu Asp Phe Leu Leu Glu Arg Leu Thr Ser Leu Pro Ala Asp Gly Ser
                245                 250                 255

Ala Ser Ala Leu Arg Ala His Phe Glu Thr Ser Leu Arg Arg Ile Pro
            260                 265                 270

Ser Leu Leu Asp Ala Ala Gly Leu Arg Pro Ile Thr Val Pro Pro Ala
        275                 280                 285

Thr Phe Thr Glu Ser Phe Pro His Leu Ser Lys Pro Asn Gly Leu Gln
    290                 295                 300

Ala Ser Leu Ala Lys His Leu Pro Cys Leu Cys Thr Gly Pro Gly Leu
305                 310                 315                 320

Val Leu Ile Thr Ala Pro Met Gly Glu Gly Lys Thr Glu Ala Ala Tyr
                325                 330                 335
```

His Val Ala Asp Leu Leu Gly Lys Ala Thr Gly Arg Pro Gly Arg Phe
340 345 350

Leu Ala Leu Pro Thr Met Ala Thr Ala Asp Gln Met His Thr Arg Leu
355 360 365

Lys Glu Tyr Ala Arg Tyr Arg Val Glu Asn Thr Asp Leu Pro Arg Ser
370 375 380

Ser Thr Leu Ala Leu Leu His Ser Met Ala Trp Leu Asn Pro Asp Tyr
385 390 395 400

Ala Pro Ala Asp Leu Pro Gly Val Ser Lys Val Leu Ser Asn Leu Gly
405 410 415

His Arg Asp Pro Phe Ala Ala Thr Asp Trp Leu Met Gly Arg Lys Arg
420 425 430

Gly Leu Leu Ala Pro Trp Ala Val Gly Thr Ile Asp Gln Ala Leu Met
435 440 445

Ala Val Leu Arg Ala Lys His Asn Ala Leu Arg Leu Phe Gly Leu Ala
450 455 460

Gly Lys Val Val Val Val Asp Glu Ala His Ala Val Asp Pro Tyr Met
465 470 475 480

Gln Val Leu Leu Glu Gln Leu Leu Arg Trp Leu Gly Thr Leu Asp Val
485 490 495

Pro Val Val Leu Leu Ser Ala Thr Leu His His Ser Ile Ala Asn Ser
500 505 510

Leu Val Lys Ala Tyr Leu Glu Gly Ala Arg Gly Arg Arg Trp Asn Arg
515 520 525

Ser Glu Pro Gln Pro Val Ser Glu Val Ser Tyr Pro Gly Trp Leu His
530 535 540

Val Asp Ala Arg Ile Gly Lys Val Thr Arg Ser Ser Asp Val Asp Pro
545 550 555 560

Leu Pro Ile Ala Thr Thr Pro Arg Lys Pro Leu Glu Val Arg Leu Val
565 570 575

Asp Val Pro Val Lys Glu Gly Ala Leu Asn Arg Ser Thr Val Leu Ala
580 585 590

Lys Glu Leu Thr Pro Leu Val Lys Gln Gly Gly Cys Ala Ala Ile Ile
595 600 605

Cys Thr Thr Val Ala Glu Ala Gln Gly Val Tyr Asp Leu Leu Ser Gln
610 615 620

Trp Phe Ala Thr Leu Gly Glu Asp Ala Pro Asp Leu Tyr Leu Leu His
625 630 635 640

Ser Arg Phe Pro Asn Arg Gln Arg Thr Glu Ile Thr Ala Thr Ile Val
645 650 655

Asp Leu Phe Gly Lys Glu Gly Ala Gln Ser Gly Arg Arg Pro Thr Arg
660 665 670

Gly Ala Val Leu Val Ala Thr Gln Val Val Glu Gln Ser Leu Asp Leu
675 680 685

Asp Val Asp Leu Met Ile Ser Asp Leu Ala Pro Val Ser Leu Leu Leu
690 695 700

Gln Arg Ala Gly Arg Cys Trp Arg His Glu His Leu Gly Ile Ile Asn
705 710 715 720

Arg Pro Gln Trp Ala Lys Gln Pro Glu Leu Val Val Leu Thr Pro Glu
725 730 735

Gln Asn Gly Asp Ala Asp Arg Ala Pro Trp Phe Pro Arg Ser Trp Thr
740 745 750

Ser Val Tyr Pro Leu Ala Leu Leu Gln Arg Thr Tyr Thr Leu Leu Arg
755 760 765

Arg Arg Asn Gly Ala Pro Val Gln Ile Pro Glu Asp Val Gln Gln Leu
770 775 780

Val Asp Asp Val Tyr Asp Asp Asp Ser Leu Ala Glu Asp Leu Glu Ala
785 790 795 800

Asp Met Glu Arg Met Gly Glu Glu Leu Ala Gln Arg Gly Leu Ala Arg
805 810 815

Asn Ala Val Ile Pro Asp Pro Asp Asp Ala Glu Asp Asn Leu Asn Gly
820 825 830

Leu Thr Glu Phe Ser Phe Asp Val Asp Glu His Val Leu Ala Thr Arg

835 840 845

Phe Gly Ala Gly Ser Val Arg Val Leu Cys Tyr Tyr Val Asp Thr Ala
850 855 860

Gly Asn Arg Trp Leu Asp Pro Glu Cys Thr Val Glu Phe Pro Glu Gln
865 870 875 880

Gly Thr Gly Arg Glu Gly Arg Phe Thr Met Ala Asp Cys Arg Asp Leu
885 890 895

Val Ala Arg Thr Ile Pro Val Arg Met Gly Pro Trp Ala Ser Gln Leu
900 905 910

Thr Glu Asp Asn His Pro Pro Glu Ala Trp Arg Glu Ser Phe Tyr Leu
915 920 925

Arg Asp Leu Val Leu Ile Pro Gln Arg Val Thr Asp Glu Gly Ala Val
930 935 940

Leu Pro Thr Glu Thr Gly Gly Arg Glu Trp Leu Leu Asp Pro Cys Lys
945 950 955 960

Gly Leu Ile Phe

<210> 1156
<211> 964
<212> PRT
<213> Thermobifida fusca

<400> 1156

Met Gly Ser Ser His His His His His His Ser Ser Gly Leu Val Pro
1 5 10 15

Arg Gly Ser His Met Pro Glu His Asp Ser Thr Asp Asp Lys His Gly
20 25 30

Ile Pro Pro Leu Asp Leu Arg Phe Trp Ala Lys Glu Arg Gly Leu Arg
35 40 45

Gly Lys Thr Tyr Pro Leu Val Cys His Ser Leu Asp Ala Ala Ala Ala
50 55 60

Ala Leu Val Leu Trp Asn Glu Tyr Leu Ser Pro Gly Leu Arg Asp Thr
65 70 75 80

Ile Ala Ser Ser Met Glu Thr Asp Glu Glu His Ala Gly His Cys Ile

85 90 95

Ala Phe Trp Ala Gly Leu His Asp Ile Gly Lys Leu Thr Arg Glu Phe
100 105 110

Gln Gln Gln Ile Ala Ile Asp Leu Ser Ala Tyr Pro Gly Glu Glu Leu
115 120 125

Ser Gly Glu Gln Arg Ser His Ala Ala Ala Thr Gly Lys Trp Leu Pro
130 135 140

Phe Ala Leu Pro Ser Leu Gly Tyr Pro Asn Gly Gly Leu Val Thr Gly
145 150 155 160

Leu Val Ala Gln Met Leu Gly Gly His His Gly Thr Phe His Pro His
165 170 175

Pro Ser Phe Gln Ser Arg Asn Pro Leu Ala Glu Phe Gly Phe Ser Ser
180 185 190

Pro His Trp Glu Lys Gln Arg His Ala Leu Leu His Ala Val Phe Asp
195 200 205

Ala Thr Gly Arg Pro Thr Pro Pro Asp Met Leu Asp Gly Pro Thr Ala
210 215 220

Ser Val Val Cys Gly Leu Val Ile Leu Ala Asp Trp Leu Val Ser Gln
225 230 235 240

Glu Asp Phe Leu Leu Glu Arg Leu Thr Ser Leu Pro Ala Asp Gly Ser
245 250 255

Ala Ser Ala Leu Arg Ala His Phe Glu Thr Ser Leu Arg Arg Ile Pro
260 265 270

Ser Leu Leu Asp Ala Ala Gly Leu Arg Pro Ile Thr Val Pro Pro Ala
275 280 285

Thr Phe Thr Glu Ser Phe Pro His Leu Ser Lys Pro Asn Gly Leu Gln
290 295 300

Ala Ser Leu Ala Lys His Leu Pro Cys Leu Cys Thr Gly Pro Gly Leu
305 310 315 320

Val Leu Ile Thr Ala Pro Met Gly Glu Gly Lys Thr Glu Ala Ala Tyr
325 330 335

His Val Ala Asp Leu Leu Gly Lys Ala Thr Gly Arg Pro Gly Arg Phe
340 345 350

Leu Ala Leu Pro Thr Met Ala Thr Ala Asp Gln Met His Thr Arg Leu
355 360 365

Lys Glu Tyr Ala Arg Tyr Arg Val Glu Asn Thr Asp Leu Pro Arg Ser
370 375 380

Ser Thr Leu Ala Leu Leu His Ser Met Ala Trp Leu Asn Pro Asp Tyr
385 390 395 400

Ala Pro Ala Asp Leu Pro Gly Val Ser Lys Val Leu Ser Asn Leu Gly
405 410 415

His Arg Asp Pro Phe Ala Ala Thr Asp Trp Leu Met Gly Arg Lys Arg
420 425 430

Gly Leu Leu Ala Pro Trp Ala Val Gly Thr Ile Asp Gln Ala Leu Met
435 440 445

Ala Val Leu Arg Ala Lys His Asn Ala Leu Arg Leu Phe Gly Leu Ala
450 455 460

Gly Lys Val Val Val Val Asp Glu Ala His Ala Val Asp Pro Tyr Met
465 470 475 480

Gln Val Leu Leu Glu Gln Leu Leu Arg Trp Leu Gly Thr Leu Asp Val
485 490 495

Pro Val Val Leu Leu Ser Ala Thr Leu His His Ser Ile Ala Asn Ser
500 505 510

Leu Val Lys Ala Tyr Leu Glu Gly Ala Arg Gly Arg Arg Trp Asn Arg
515 520 525

Ser Glu Pro Gln Pro Val Ser Glu Val Ser Tyr Pro Gly Trp Leu His
530 535 540

Val Asp Ala Arg Ile Gly Lys Val Thr Arg Ser Ser Asp Val Asp Pro
545 550 555 560

Leu Pro Ile Ala Thr Thr Pro Arg Lys Pro Leu Glu Val Arg Leu Val
565 570 575

Asp Val Pro Val Lys Glu Gly Ala Leu Asn Arg Ser Thr Val Leu Ala
580 585 590

```
Lys Glu Leu Thr Pro Leu Val Lys Gln Gly Gly Cys Ala Ala Ile Ile
        595                 600                 605

Cys Thr Thr Val Ala Glu Ala Gln Gly Val Tyr Asp Leu Leu Ser Gln
    610                 615                 620

Trp Phe Ala Thr Leu Gly Glu Asp Ala Pro Asp Leu Tyr Leu Leu His
625                 630                 635                 640

Ser Arg Phe Pro Asn Arg Gln Arg Thr Glu Ile Thr Ala Thr Ile Val
                645                 650                 655

Asp Leu Phe Gly Lys Glu Gly Ala Gln Ser Gly Arg Arg Pro Thr Arg
            660                 665                 670

Gly Ala Val Leu Val Ala Thr Gln Val Val Glu Gln Ser Leu Asp Leu
        675                 680                 685

Asp Val Asp Leu Met Ile Ser Asp Leu Ala Pro Val Ser Leu Leu Leu
    690                 695                 700

Gln Arg Ala Gly Arg Cys Trp Arg His Glu His Leu Gly Ile Ile Asn
705                 710                 715                 720

Arg Pro Gln Trp Ala Lys Gln Pro Glu Leu Val Val Leu Thr Pro Glu
                725                 730                 735

Gln Asn Gly Asp Ala Asp Arg Ala Pro Trp Phe Pro Arg Ser Trp Thr
            740                 745                 750

Ser Val Tyr Pro Leu Ala Leu Leu Gln Arg Thr Tyr Thr Leu Leu Arg
        755                 760                 765

Arg Arg Asn Gly Ala Pro Val Gln Ile Pro Glu Asp Val Gln Gln Leu
    770                 775                 780

Val Asp Asp Val Tyr Asp Asp Asp Ser Leu Ala Glu Asp Leu Glu Ala
785                 790                 795                 800

Asp Met Glu Arg Met Gly Glu Glu Leu Ala Gln Arg Gly Leu Ala Arg
                805                 810                 815

Asn Ala Val Ile Pro Asp Pro Asp Asp Ala Glu Asp Asn Leu Asn Gly
            820                 825                 830

Leu Thr Glu Phe Ser Phe Asp Val Asp Glu His Val Leu Ala Thr Arg
        835                 840                 845
```

Phe Gly Ala Gly Ser Val Arg Val Leu Cys Tyr Tyr Val Asp Thr Ala
850 855 860

Gly Asn Arg Trp Leu Asp Pro Glu Cys Thr Val Glu Phe Pro Glu Gln
865 870 875 880

Gly Thr Gly Arg Glu Gly Arg Phe Thr Met Ala Asp Cys Arg Asp Leu
885 890 895

Val Ala Arg Thr Ile Pro Val Arg Met Gly Pro Trp Ala Ser Gln Leu
900 905 910

Thr Glu Asp Asn His Pro Pro Glu Ala Trp Arg Glu Ser Phe Tyr Leu
915 920 925

Arg Asp Leu Val Leu Ile Pro Gln Arg Val Thr Asp Glu Gly Ala Val
930 935 940

Leu Pro Thr Glu Thr Gly Gly Arg Glu Trp Leu Leu Asp Pro Cys Lys
945 950 955 960

Gly Leu Ile Phe

<210> 1157
<211> 964
<212> PRT
<213> Thermobifida fusca

<400> 1157

Met Gly Ser Ser His His His His His His Ser Ser Gly Leu Val Pro
1 5 10 15

Arg Gly Ser His Met Pro Glu His Asp Ser Thr Asp Asp Lys His Gly
20 25 30

Ile Pro Pro Leu Asp Leu Arg Phe Trp Ala Lys Glu Arg Gly Leu Arg
35 40 45

Gly Lys Thr Tyr Pro Leu Val Cys His Ser Leu Asp Ala Ala Ala Ala
50 55 60

Ala Leu Val Leu Trp Asn Glu Tyr Leu Ser Pro Gly Leu Arg Asp Thr
65 70 75 80

Ile Ala Ser Ser Met Glu Thr Asp Glu Glu His Ala Gly His Cys Ile
85 90 95

Ala Phe Trp Ala Gly Leu His Asp Ile Gly Lys Leu Thr Arg Glu Phe
100 105 110

Gln Gln Gln Ile Ala Ile Asp Leu Ser Ala Tyr Pro Gly Glu Glu Leu
115 120 125

Ser Gly Glu Gln Arg Ser His Ala Ala Ala Thr Gly Lys Trp Leu Pro
130 135 140

Phe Ala Leu Pro Ser Leu Gly Tyr Pro Asn Gly Gly Leu Val Thr Gly
145 150 155 160

Leu Val Ala Gln Met Leu Gly Gly His His Gly Thr Phe His Pro His
165 170 175

Pro Ser Phe Gln Ser Arg Asn Pro Leu Ala Glu Phe Gly Phe Ser Ser
180 185 190

Pro His Trp Glu Lys Gln Arg His Ala Leu Leu His Ala Val Phe Asp
195 200 205

Ala Thr Gly Arg Pro Thr Pro Pro Asp Met Leu Asp Gly Pro Thr Ala
210 215 220

Ser Val Val Cys Gly Leu Val Ile Leu Ala Asp Trp Leu Val Ser Gln
225 230 235 240

Glu Asp Phe Leu Leu Glu Arg Leu Thr Ser Leu Pro Ala Asp Gly Ser
245 250 255

Ala Ser Ala Leu Arg Ala His Phe Glu Thr Ser Leu Arg Arg Ile Pro
260 265 270

Ser Leu Leu Asp Ala Ala Gly Leu Arg Pro Ile Thr Val Pro Pro Ala
275 280 285

Thr Phe Thr Glu Ser Phe Pro His Leu Ser Lys Pro Asn Gly Leu Gln
290 295 300

Ala Ser Leu Ala Lys His Leu Pro Cys Leu Cys Thr Gly Pro Gly Leu
305 310 315 320

Val Leu Ile Thr Ala Pro Met Gly Glu Gly Lys Thr Glu Ala Ala Tyr
325 330 335

His Val Ala Asp Leu Leu Gly Lys Ala Thr Gly Arg Pro Gly Arg Phe

340 345 350

Leu Ala Leu Pro Thr Met Ala Thr Ala Asp Gln Met His Thr Arg Leu
355 360 365

Lys Glu Tyr Ala Arg Tyr Arg Val Glu Asn Thr Asp Leu Pro Arg Ser
370 375 380

Ser Thr Leu Ala Leu Leu His Ser Met Ala Trp Leu Asn Pro Asp Tyr
385 390 395 400

Ala Pro Ala Asp Leu Pro Gly Val Ser Lys Val Leu Ser Asn Leu Gly
405 410 415

His Arg Asp Pro Phe Ala Ala Thr Asp Trp Leu Met Gly Arg Lys Arg
420 425 430

Gly Leu Leu Ala Pro Trp Ala Val Gly Thr Ile Asp Gln Ala Leu Met
435 440 445

Ala Val Leu Arg Ala Lys His Asn Ala Leu Arg Leu Phe Gly Leu Ala
450 455 460

Gly Lys Val Val Val Val Asp Glu Ala His Ala Val Asp Pro Tyr Met
465 470 475 480

Gln Val Leu Leu Glu Gln Leu Leu Arg Trp Leu Gly Thr Leu Asp Val
485 490 495

Pro Val Val Leu Leu Ser Ala Thr Leu His His Ser Ile Ala Asn Ser
500 505 510

Leu Val Lys Ala Tyr Leu Glu Gly Ala Arg Gly Arg Arg Trp Asn Arg
515 520 525

Ser Glu Pro Gln Pro Val Ser Glu Val Ser Tyr Pro Gly Trp Leu His
530 535 540

Val Asp Ala Arg Ile Gly Lys Val Thr Arg Ser Ser Asp Val Asp Pro
545 550 555 560

Leu Pro Ile Ala Thr Thr Pro Arg Lys Pro Leu Glu Val Arg Leu Val
565 570 575

Asp Val Pro Val Lys Glu Gly Ala Leu Asn Arg Ser Thr Val Leu Ala
580 585 590

Lys Glu Leu Thr Pro Leu Val Lys Gln Gly Gly Cys Ala Ala Ile Ile
595 600 605

Cys Thr Thr Val Ala Glu Ala Gln Gly Val Tyr Asp Leu Leu Ser Gln
610 615 620

Trp Phe Ala Thr Leu Gly Glu Asp Ala Pro Asp Leu Tyr Leu Leu His
625 630 635 640

Ser Arg Phe Pro Asn Arg Gln Arg Thr Glu Ile Thr Ala Thr Ile Val
645 650 655

Asp Leu Phe Gly Lys Glu Gly Ala Gln Ser Gly Arg Arg Pro Thr Arg
660 665 670

Gly Ala Val Leu Val Ala Thr Gln Val Val Glu Gln Ser Leu Asp Leu
675 680 685

Asp Val Asp Leu Met Ile Ser Asp Leu Ala Pro Val Ser Leu Leu Leu
690 695 700

Gln Arg Ala Gly Arg Cys Trp Arg His Glu His Leu Gly Ile Ile Asn
705 710 715 720

Arg Pro Gln Trp Ala Lys Gln Pro Glu Leu Val Val Leu Thr Pro Glu
725 730 735

Gln Asn Gly Asp Ala Asp Arg Ala Pro Trp Phe Pro Arg Ser Trp Thr
740 745 750

Ser Val Tyr Pro Leu Ala Leu Leu Gln Arg Thr Tyr Thr Leu Leu Arg
755 760 765

Arg Arg Asn Gly Ala Pro Val Gln Ile Pro Glu Asp Val Gln Gln Leu
770 775 780

Val Asp Asp Val Tyr Asp Asp Asp Ser Leu Ala Glu Asp Leu Glu Ala
785 790 795 800

Asp Met Glu Arg Met Gly Glu Glu Leu Ala Gln Arg Gly Leu Ala Arg
805 810 815

Asn Ala Val Ile Pro Asp Pro Asp Asp Ala Glu Asp Asn Leu Asn Gly
820 825 830

Leu Thr Glu Phe Ser Phe Asp Val Asp Glu His Val Leu Ala Thr Arg
835 840 845

Phe Gly Ala Gly Ser Val Arg Val Leu Cys Tyr Tyr Val Asp Thr Ala
850 855 860

Gly Asn Arg Trp Leu Asp Pro Glu Cys Thr Val Glu Phe Pro Glu Gln
865 870 875 880

Gly Thr Gly Arg Glu Gly Arg Phe Thr Met Ala Asp Cys Arg Asp Leu
885 890 895

Val Ala Arg Thr Ile Pro Val Arg Met Gly Pro Trp Ala Ser Gln Leu
900 905 910

Thr Glu Asp Asn His Pro Pro Glu Ala Trp Arg Glu Ser Phe Tyr Leu
915 920 925

Arg Asp Leu Val Leu Ile Pro Gln Arg Val Thr Asp Glu Gly Ala Val
930 935 940

Leu Pro Thr Glu Thr Gly Gly Arg Glu Trp Leu Leu Asp Pro Cys Lys
945 950 955 960

Gly Leu Ile Phe

<210> 1158
<211> 244
<212> PRT
<213> Methanocaldococcus jannaschii

<400> 1158

Met Ile Leu Gly Glu Ile Met Glu Val Leu Ala Phe Lys Asn Gln Ser
1 5 10 15

Leu Ile Asp His Val Asn Asp Met Val Lys Tyr Trp Glu Arg Ile Lys
20 25 30

Tyr Arg Tyr Leu Lys Thr Ile Lys Arg Ala Leu Glu Ala Leu Asn Ile
35 40 45

Lys Leu Asp Ile Glu Lys Val Asp Glu Phe Met Lys Ile Leu Ile Lys
50 55 60

Leu His Asp Ile Gly Lys Ala Ser Lys Ile Tyr Gln Arg Ala Ile Ile
65 70 75 80

Asn Asp Gln Glu Lys Leu Met Gly Phe Arg His Glu Leu Val Ser Ala
85 90 95

Tyr Tyr Thr Tyr His Ile Leu Leu Lys Lys Phe Gly Asp Lys Asn Leu
100 105 110

Ala Phe Ile Gly Ala Leu Thr Val Met Leu His His Glu Pro Ile Ile
115 120 125

Met Gly Gln Ile Arg Asn Leu Lys Lys Lys Glu Leu Thr Ala Glu Val
130 135 140

Val Leu Asp Lys Leu Lys Lys Phe Asp Gly Met Ile Glu Asp Phe Glu
145 150 155 160

Asp Leu Ile Lys Lys Leu Ile Gly Tyr Ser Ile Gly Asp Ile Ile Lys
165 170 175

Asn Asp Ser Asn Lys Asp Asp Ile Ile Arg Phe Val Ile Glu Met Ser
180 185 190

Val Arg Ala Arg His Thr Pro Asn Ser Glu Lys Leu Arg Phe Ile Val
195 200 205

Gly Thr Leu Leu Leu Pro Leu Val Met Cys Asp Tyr Lys Gly Ala Glu
210 215 220

Ser Arg Glu Gly Lys Ala Pro Lys Phe Ala Glu Val Leu Glu Val Glu
225 230 235 240

Ser Tyr Val Ile

<210> 1159
<211> 265
<212> PRT
<213> Thermus thermophilus

<400> 1159

Met Ser His His His His His His Ser Met Ala Ala Leu Ala Leu Trp
1 5 10 15

Ala Lys Ser Gly Asn Pro Phe His Pro Leu Leu Ala His Met Leu Asp
20 25 30

Thr Ala Ala Val Ala Leu Ala Val Leu Arg Met Glu Pro Pro Arg Thr
35 40 45

Arg Ala Leu Tyr Ala Glu Asp Trp Gly Leu Pro Glu Glu Gly Ala Leu
50 55 60

Ala Trp Ala Ala Ala Leu Val Gly Leu His Asp Leu Gly Lys Ala Ser
65 70 75 80

Pro Val Phe Gln Ala Gly Trp Glu Glu Gly Lys Glu Arg Val Gln Arg
85 90 95

Ala Gly Leu Pro Phe Gly Glu Leu Leu Asp Trp Val Ala His Gly Val
100 105 110

Phe Thr Glu Leu Phe Leu Arg Arg Leu Leu Lys Glu Lys Gly Leu Pro
115 120 125

Glu Arg Ala Ala Asn Asp Leu Ala Ala Ala Leu Gly Ala His His Gly
130 135 140

Phe Pro Ala Asn Ala Glu Glu Lys Ser Arg Ala Arg Arg His Leu Arg
145 150 155 160

Thr Glu Asp Pro Leu Trp Lys Glu Ala Arg Arg Trp Leu Leu Glu Glu
165 170 175

Val Phe Arg Arg Leu Gly Ala Pro Leu Pro Pro Ser Gln Gly Asn Gly
180 185 190

Glu Ala Arg Pro Glu Ala Val Leu Arg Val Met Ala Leu Ala Ser Phe
195 200 205

Ala Asp Trp Val Ala Ser Asp Pro Ser Leu Phe Pro Tyr Gly Arg Asp
210 215 220

Pro Arg Arg Gly Asp Tyr Leu Lys Glu Ala Leu Arg Leu Ala Gln Glu
225 230 235 240

Ala Leu Asn Arg Leu Gly Trp Pro Ala Phe Ala Lys Ala Gln Arg Arg
245 250 255

Glu Phe Gly Glu Leu Phe Pro Tyr Ile
260 265

<210> 1160
<211> 206
<212> PRT
<213> Sulfolobus solfataricus

<400> 1160

Tyr Phe Gln Gly Met Ile Thr Glu Phe Leu Leu Lys Lys Lys Leu Glu
1 5 10 15

Glu His Leu Ser His Val Lys Glu Glu Asn Thr Ile Tyr Val Thr Asp
20 25 30

Leu Val Arg Cys Pro Arg Arg Val Arg Tyr Glu Ser Glu Tyr Lys Glu
35 40 45

Leu Ala Ile Ser Gln Val Tyr Ala Pro Ser Ala Ile Leu Gly Asp Ile
50 55 60

Leu His Leu Gly Leu Glu Ser Val Leu Lys Gly Asn Phe Asn Ala Glu
65 70 75 80

Thr Glu Val Glu Thr Leu Arg Glu Ile Asn Val Gly Gly Lys Val Tyr
85 90 95

Lys Ile Lys Gly Arg Ala Asp Ala Ile Ile Arg Asn Asp Asn Gly Lys
100 105 110

Ser Ile Val Ile Glu Ile Lys Thr Ser Arg Ser Asp Lys Gly Leu Pro
115 120 125

Leu Ile His His Lys Met Gln Leu Gln Ile Tyr Leu Trp Leu Phe Ser
130 135 140

Ala Glu Lys Gly Ile Leu Val Tyr Ile Thr Pro Asp Arg Ile Ala Glu
145 150 155 160

Tyr Glu Ile Asn Glu Pro Leu Asp Glu Ala Thr Ile Val Arg Leu Ala
165 170 175

Glu Asp Thr Ile Met Leu Gln Asn Ser Pro Arg Phe Asn Trp Glu Cys
180 185 190

Lys Tyr Cys Ile Phe Ser Val Ile Cys Pro Ala Lys Leu Thr
195 200 205

<210> 1161
<211> 235
<212> PRT
<213> Mannheimia succiniciproducens

<400> 1161

Met Ser Leu Ala Asn Arg Ile Arg Leu His Ile Trp Gly Asp Tyr Ala
1 5 10 15

Cys Phe Thr Arg Pro Glu Met Lys Val Glu Arg Val Ser Tyr Asp Val
20 25 30

Ile Thr Pro Ser Ala Ala Arg Gly Ile Leu Ser Ala Ile His Trp Lys
35 40 45

Pro Ala Ile Asn Trp Val Ile Asp Lys Ile Tyr Val Leu Lys Pro Ile
50 55 60

Arg Phe Glu Ser Val Arg Arg Asn Glu Leu Gly Ala Lys Ile Ser Glu
65 70 75 80

Ser Lys Val Ser Gly Ala Met Lys Arg Lys Ser Val Ala Asp Leu Tyr
85 90 95

Thr Val Ile Glu Asp Asp Arg Gln Gln Arg Ala Ala Thr Val Leu Lys
100 105 110

Asp Val Ala Tyr Val Ile Glu Ala His Ala Val Met Thr Ser Lys Ala
115 120 125

Gly Val Asp Glu Asn Thr Thr Lys His Ile Glu Met Phe Lys Arg Arg
130 135 140

Ala Leu Lys Gly Gln Cys Phe Gln Gln Pro Cys Met Gly Val Arg Glu
145 150 155 160

Phe Pro Ala His Phe Ala Leu Ile Asp Asp Asn Asp Pro Leu Pro Leu
165 170 175

Ser Gln Leu Ser Glu Ser Glu Phe Asn Arg Asp Leu Gly Trp Met Leu
180 185 190

His Asp Ile Asp Phe Glu His Gly Asn Thr Pro His Phe Phe Arg Ala
195 200 205

Glu Leu Lys Asn Gly Val Ile Asp Val Pro Pro Phe Tyr Ala Glu Glu
210 215 220

Val Lys Arg Glu Gly His His His His His His
225 230 235

<210> 1162
<211> 226
<212> PRT
<213> Xanthomonas oryzae pv. Oryzae

<400> 1162

Gly His Met Ser Tyr Gly Val Arg Leu His Val Trp Gly Glu Arg Ala
1 5 10 15

Leu Phe Thr Arg Pro Glu Met Lys Val Glu Arg Val Ser Tyr Asp Ile
20 25 30

Ile Thr Pro Ser Ala Ala Arg Gly Ile Leu Glu Ala Ile His Trp Lys
35 40 45

Pro Ala Ile Arg Trp Val Val Asp Ser Ile Gln Val Leu Lys Pro Ile
50 55 60

Cys Phe Glu Ser Ile Arg Arg Asn Glu Val Gly Ser Lys Leu Ser Ala
65 70 75 80

Ala Ser Ile Ser Lys Ala Ile Lys Ala Gly Arg Thr Asp Glu Leu Val
85 90 95

Lys Tyr Val Glu Glu Asp Arg Gln Gln Arg Ala Ala Thr Val Leu Arg
100 105 110

Glu Val Gly Tyr Ile Ile Ala Ala His Phe Glu Met Thr Asp Lys Ala
115 120 125

Gly Pro Asp Asp Asn Val Gly Lys His Leu Asp Ile Phe Asn Arg Arg
130 135 140

Ala Arg Arg Gly Gln Cys Phe Gln Ala Pro Cys Leu Gly Thr Arg Glu
145 150 155 160

Phe Pro Ala Ser Phe Ala Leu Leu Gly Asp Asp Asp Thr Pro Pro Ala
165 170 175

Ser Asp Pro Ala Leu Ser Gly Glu Arg Asp Leu Gly Trp Met Leu His
180 185 190

Asp Ile Asp Phe Ala Asp Gly Met Thr Pro Arg Phe Phe Arg Ala Arg
195 200 205

Met Val Asp Gly Leu Val Ala Val Pro Pro Pro Gln Asp Gly Gly Val
210 215 220

Arg Ala
225

<210> 1163
<211> 244
<212> PRT
<213> Streptococcus pyogenes serotype

<400> 1163

Gly His Met Tyr Arg Ser Arg Asp Phe Tyr Val Arg Val Ser Gly Gln
1               5                   10                  15

Arg Ala Leu Phe Thr Asn Pro Ala Thr Lys Gly Gly Ser Glu Arg Ser
            20                  25                  30

Ser Tyr Ser Val Pro Thr Arg Gln Ala Leu Asn Gly Ile Val Asp Ala
        35                  40                  45

Ile Tyr Tyr Lys Pro Thr Phe Thr Asn Ile Val Thr Glu Val Lys Val
    50                  55                  60

Ile Asn Gln Ile Gln Thr Glu Leu Gln Gly Val Arg Ala Leu Leu His
65                  70                  75                  80

Asp Tyr Ser Ala Asp Leu Ser Tyr Val Ser Tyr Leu Ser Asp Val Val
                85                  90                  95

Tyr Leu Ile Lys Phe His Phe Val Trp Asn Glu Asp Arg Lys Asp Leu
            100                 105                 110

Asn Ser Asp Arg Leu Pro Ala Lys His Glu Ala Ile Met Glu Arg Ser
        115                 120                 125

Ile Arg Lys Gly Gly Arg Arg Asp Val Phe Leu Gly Thr Arg Glu Cys
    130                 135                 140

Leu Gly Leu Val Asp Asp Ile Ser Gln Glu Glu Tyr Glu Thr Thr Val
145                 150                 155                 160

Ser Tyr Tyr Asn Gly Val Asn Ile Asp Leu Gly Ile Met Phe His Ser
                165                 170                 175

Phe Ala Tyr Pro Lys Asp Lys Lys Thr Pro Leu Lys Ser Tyr Phe Thr
            180                 185                 190

Lys Thr Val Met Lys Asn Gly Val Ile Thr Phe Lys Ala Gln Ser Glu
        195                 200                 205

Cys Asp Ile Val Asn Thr Leu Ser Ser Tyr Ala Phe Lys Ala Pro Glu
    210                 215                 220

Glu Ile Lys Ser Val Asn Asp Glu Cys Met Glu Tyr Asp Ala Met Glu
225                 230                 235                 240

Lys Gly Glu Asn

<210> 1164
<211> 191
<212> PRT
<213> pseudomonas aeruginosa

<400> 1164

Gly Ser Phe Thr Met Asp His Tyr Leu Asp Ile Arg Leu Arg Pro Asp
1 5 10 15

Pro Glu Phe Pro Pro Ala Gln Leu Met Cys Val Leu Phe Gly Lys Leu
20 25 30

His Gln Ala Leu Val Ala Gln Gly Gly Asp Arg Ile Gly Val Ser Phe
35 40 45

Pro Asp Leu Asp Glu Ser Arg Ser Arg Leu Gly Glu Arg Leu Arg Ile
50 55 60

His Ala Ser Ala Asp Asp Leu Arg Ala Leu Leu Ala Arg Pro Trp Leu
65 70 75 80

Glu Gly Leu Arg Asp His Leu Gln Phe Gly Glu Pro Ala Val Val Pro
85 90 95

His Pro Thr Pro Tyr Arg Gln Val Ser Arg Val Gln Ala Lys Ser Asn
100 105 110

Pro Glu Arg Leu Arg Arg Arg Leu Met Arg Arg His Asp Leu Ser Glu
115 120 125

Glu Glu Ala Arg Lys Arg Ile Pro Asp Thr Val Ala Arg Thr Leu Asp
130 135 140

Leu Pro Phe Val Thr Leu Arg Ser Gln Ser Thr Gly Gln His Phe Arg
145 150 155 160

Leu Phe Ile Arg His Gly Pro Leu Gln Val Thr Ala Glu Glu Gly Gly
165 170 175

Phe Thr Cys Tyr Gly Leu Ser Lys Gly Gly Phe Val Pro Trp Phe
180 185 190

<210> 1165
<211> 211
<212> PRT
<213> Thermus thermophilus

<400> 1165

Met Trp Leu Thr Lys Leu Val Leu Asn Pro Ala Ser Arg Ala Ala Arg
1               5                   10                  15

Arg Asp Leu Ala Asn Pro Tyr Glu Met His Arg Thr Leu Ser Lys Ala
            20                  25                  30

Val Ser Arg Ala Leu Glu Glu Gly Arg Glu Arg Leu Leu Trp Arg Leu
        35                  40                  45

Glu Pro Ala Arg Gly Leu Glu Pro Pro Val Val Leu Val Gln Thr Leu
    50                  55                  60

Thr Glu Pro Asp Trp Ser Val Leu Asp Glu Gly Tyr Ala Gln Val Phe
65                  70                  75                  80

Pro Pro Lys Pro Phe His Pro Ala Leu Lys Pro Gly Gln Arg Leu Arg
                85                  90                  95

Phe Arg Leu Arg Ala Asn Pro Ala Lys Arg Leu Ala Ala Thr Gly Lys
            100                 105                 110

Arg Val Ala Leu Lys Thr Pro Ala Glu Lys Val Ala Trp Leu Glu Arg
        115                 120                 125

Arg Leu Glu Glu Gly Gly Phe Arg Leu Leu Glu Gly Glu Arg Gly Pro
    130                 135                 140

Trp Val Gln Ile Leu Gln Asp Thr Phe Leu Glu Val Arg Arg Lys Lys
145                 150                 155                 160

Asp Gly Glu Glu Ala Gly Lys Leu Leu Gln Val Gln Ala Val Leu Phe
                165                 170                 175

Glu Gly Arg Leu Glu Val Val Asp Pro Glu Arg Ala Leu Ala Thr Leu
            180                 185                 190

Arg Arg Gly Val Gly Pro Gly Lys Ala Leu Gly Leu Gly Leu Leu Ser
        195                 200                 205

Val Ala Pro
    210

<210>  1166
<211>  273
<212>  PRT
<213>  Pyrococcus furiosus

<400>  1166

Met Ala His His His His His His Gly Ser Arg Phe Leu Ile Arg Leu
1               5                   10                  15

Val Pro Glu Asp Lys Asp Arg Ala Phe Lys Val Pro Tyr Asn His Gln
            20                  25                  30

Tyr Tyr Leu Gln Gly Leu Ile Tyr Asn Ala Ile Lys Ser Ser Asn Pro
        35                  40                  45

Lys Leu Ala Thr Tyr Leu His Glu Val Lys Gly Pro Lys Leu Phe Thr
    50                  55                  60

Tyr Ser Leu Phe Met Ala Glu Lys Arg Glu His Pro Lys Gly Leu Pro
65                  70                  75                  80

Tyr Phe Leu Gly Tyr Lys Lys Gly Phe Phe Tyr Phe Ser Thr Cys Val
                85                  90                  95

Pro Glu Ile Ala Glu Ala Leu Val Asn Gly Leu Leu Met Asn Pro Glu
            100                 105                 110

Val Arg Leu Trp Asp Glu Arg Phe Tyr Leu His Glu Ile Lys Val Leu
        115                 120                 125

Arg Glu Pro Lys Lys Phe Asn Gly Ser Thr Phe Val Thr Leu Ser Pro
    130                 135                 140

Ile Ala Val Thr Val Val Arg Lys Gly Lys Ser Tyr Asp Val Pro Pro
145                 150                 155                 160

Met Glu Lys Glu Phe Tyr Ser Ile Ile Lys Asp Asp Leu Gln Asp Lys
                165                 170                 175

Tyr Val Met Ala Tyr Gly Asp Lys Pro Pro Ser Glu Phe Glu Met Glu
            180                 185                 190

Val Leu Ile Ala Lys Pro Lys Arg Phe Arg Ile Lys Pro Gly Ile Tyr
        195                 200                 205

Gln Thr Ala Trp His Leu Val Phe Arg Ala Tyr Gly Asn Asp Asp Leu
    210                 215                 220

Leu Lys Val Gly Tyr Glu Val Gly Phe Gly Glu Lys Asn Ser Leu Gly
225                 230                 235                 240

Phe Gly Met Val Lys Val Glu Gly Asn Lys Thr Thr Lys Glu Ala Glu
                245                 250                 255

Glu Gln Glu Lys Ile Thr Phe Asn Ser Arg Glu Glu Leu Lys Thr Gly
260 265 270

Val

<210> 1167
<211> 328
<212> PRT
<213> Sulfolobus solfataricus

<400> 1167

Met His His His His His His Met Ile Ser Gly Ser Val Arg Phe Leu
1 5 10 15

Val Asn Leu Glu Ser Leu Asn Gly Val Glu Ser Ile Gly Asn Leu Thr
20 25 30

Lys His Arg Thr Ala Pro Val Val Leu Lys Thr Ser Thr Gly Tyr Leu
35 40 45

Val Arg Tyr Val Pro Val Ile Ser Gly Glu Ala Leu Ala His Ala Tyr
50 55 60

Gln Ala Ser Leu Val Asp Ile Ala Lys Lys Glu Gly Leu Pro Val Gly
65 70 75 80

Ser Leu Ser Ser Gln Tyr Glu Phe Ile Lys Phe Ser Thr Asp Glu Ala
85 90 95

Leu Lys Ile Glu Gly Ile Lys Glu Pro Lys Asp Tyr Asn Asp Ala Arg
100 105 110

Arg Phe Glu Val Glu Val Met Leu Lys Asp Val Ile Ala Asp Val Gly
115 120 125

Gly Phe Met Tyr Ala Gly Gly Ala Pro Val Arg Arg Thr Ser Arg Ile
130 135 140

Lys Leu Gly Tyr Met Ile Pro Ala Leu Arg Gly Asp Glu Ile Pro Ala
145 150 155 160

Gln Leu Glu Ala Gln Phe His Val Arg Phe Ser Asn Lys Pro Val Ser
165 170 175

Gly Ser Gln Ala Ile Phe Asn Val Glu Val Ser Ser Ala Leu Tyr Thr
180 185 190

Phe Ser Phe Glu Leu Asp Glu Asp Leu Ile Ala Val Pro Ser Thr Phe
195 200 205

Gly Glu Lys Val Lys Gly Glu Glu Glu Leu Glu Arg Gln Lys Ala Lys
210 215 220

Arg Val Lys Ser Ala Ile Lys Ala Leu Tyr Ser Leu Leu Ser Gly Asn
225 230 235 240

Phe Gly Gly Lys Arg Ser Arg Phe Leu Pro Ser Met Lys Leu Met Ser
245 250 255

Leu Val Val Thr Lys Thr Asp Phe Pro Phe Met Pro Glu Pro Ala His
260 265 270

Asp Asp Asp Tyr Ile Lys Thr Thr Ile Met Arg Leu Gly Lys Ala Lys
275 280 285

Gly Val Leu Asn Gly Asn Leu Ala Lys Ala Tyr Val Ile Asn Asn Glu
290 295 300

Gly Ile Glu Val Gly Glu Gly Val Thr Val Leu Ser Thr Val Glu Asp
305 310 315 320

Leu Val Val Lys Leu Glu Glu Glu
325

<210> 1168
<211> 351
<212> PRT
<213> Methanopyrus kandleri

<400> 1168

Met Val Gly Ile Gly Gly Thr Ile Thr Leu Val Gly Glu Ile Arg Leu
1 5 10 15

Arg Thr Gly Thr Arg Ile Gly Thr Ser Glu Glu Glu Ile Glu Ile Gly
20 25 30

Gly Leu Asp Asn Pro Val Ile Arg Asp Pro Val Ser Gly Tyr Pro Tyr
35 40 45

Val Pro Gly Ser Ser Leu Lys Gly Arg Ala Arg Ala Leu Phe Glu Leu
50 55 60

Ala Trp Met Lys Ser Arg Glu Ile Glu Pro Asp Val Phe Phe Gly Ala
65 70 75 80

His His Asn Glu Arg His Glu Cys Gly Phe Val Arg Arg Glu Val Tyr
85 90 95

Glu Glu Ala Lys Glu Tyr Leu Arg Glu Asp Pro Pro Trp Leu Glu Asn
100 105 110

Gly Thr Cys Pro Val Cys Arg Ile Phe Gly Ser Ala Gly Asp Gly Ile
115 120 125

Gly Phe Ser Asp Pro Gly Arg Leu Glu Asp Glu Arg Arg Gly Leu Gly
130 135 140

Tyr Asp Pro Tyr Gly Arg Tyr Arg Asp Pro Asn Asp Ala Gln Glu Leu
145 150 155 160

Ser Gly Val Val Asp Val Lys Lys Glu Ala Arg Val Ala Phe Arg Asp
165 170 175

Ala His Pro Thr Thr Tyr Thr Val Asn Asp Val Phe Glu Arg Ala Gly
180 185 190

Glu Pro Thr Glu Val Lys His Glu Asn Ala Ile Asn Arg Val Ser Gly
195 200 205

Glu Ala Asn Pro Arg Ser Met Glu Arg Val Pro Lys Gly Ser Arg Phe
210 215 220

Gly Leu Glu Val Val Tyr Arg Val Glu Asp Gly Glu Glu Leu Glu Ser
225 230 235 240

Asp Leu Lys Tyr Leu Met Ser Ser Leu Lys Leu Val Glu Asp Gln Gly
245 250 255

Ile Gly His Ser Thr Ser Arg Gly Tyr Gly Arg Val Glu Phe Arg Ile
260 265 270

Ala Ala Leu Cys Ala Arg Ser Thr Gly Trp Tyr Leu Asp Pro Gly Ala
275 280 285

Gly Glu Gly Phe Pro Glu Glu Glu Asp Lys Asp Glu Ala Ala Asp Glu
290 295 300

Val Thr Tyr Leu Ser Asp Leu Glu Ala Glu Arg Tyr Glu Ile Val Ile
305 310 315 320

Arg Ala Arg Asp Leu Glu Asp Arg Ala Tyr Leu Arg Pro Glu Glu Trp

325 330 335

Val Glu Arg Leu Asp Glu Val Val Gly Glu Leu Pro Trp Gly Arg
340 345 350

<210> 1169
<211> 506
<212> PRT
<213> thermus thermophilus

<400> 1169

Gly Ala Gly Ser Met Gly Ser Leu Glu Lys Phe Asn Leu Ile Asp Glu
1 5 10 15

Pro Trp Ile Pro Val Leu Lys Gly Gly Arg Val Val Glu Val Gly Ile
20 25 30

Gly Glu Ala Leu Leu Arg Ala His Glu Phe Ala Arg Ile Glu Thr Pro
35 40 45

Ser Pro Leu Glu Glu Ala Val Leu His Arg Leu Leu Leu Ala Val Leu
50 55 60

His Arg Ala Leu Ser Gly Pro Arg Cys Pro Glu Asp Val Leu Asp Trp
65 70 75 80

Trp Arg Lys Gly Gly Phe Pro Gln Asp Pro Ile Arg Asp Tyr Leu Asn
85 90 95

Arg Phe Arg Asp Arg Phe Phe Leu Phe His Pro Glu Ala Pro Phe Leu
100 105 110

Gln Val Ala Asp Leu Pro Glu Glu Asn Pro Leu Pro Trp Ser Lys Leu
115 120 125

Leu Pro Glu Leu Ala Ser Gly Asn Asn Pro Thr Leu Phe Asp His Thr
130 135 140

Thr Glu Glu Asn Leu Pro Lys Ala Thr Tyr Ala Gln Ala Ala Arg Ala
145 150 155 160

Leu Leu Val His Gln Ala Phe Ala Pro Gly Gly Leu Leu Arg Arg Tyr
165 170 175

Gly Val Gly Ser Ala Lys Asp Ala Pro Val Ala Arg Pro Ala Leu Phe
180 185 190

Leu Pro Thr Gly Gln Asn Leu Leu Glu Thr Leu Leu Leu Asn Leu Val

195 200 205

Pro Tyr Thr Pro Glu Asp Asp Ala Pro Ile Trp Glu Val Pro Pro Leu
210 215 220

Arg Leu Gly Asp Leu Glu Gly Ala Arg Thr Lys Trp Pro Leu Thr Gly
225 230 235 240

Arg Thr Arg Val Tyr Thr Trp Pro Ala Arg Gly Val Arg Leu Leu Asp
245 250 255

Glu Gly Asp Gly Val Arg Phe Met Gly Tyr Gly Pro Gly Val Glu Pro
260 265 270

Leu Glu Ala Thr His Arg Asp Pro Met Val Ala Gln Arg Leu Asp Ala
275 280 285

Lys Gly Asn Leu Leu Val Leu Arg Leu Ser Glu Glu Arg Ser Phe Trp
290 295 300

Arg Asp Phe Ser Ala Met Leu Pro Arg Gln Gly Gly Lys Val Ala Ala
305 310 315 320

Thr Leu Glu His Ala Glu Asn Leu Gln Gly Glu Leu Glu Asp Glu Gly
325 330 335

Leu Glu Gly Arg Ile Thr Leu Arg Val Leu Gly Gln Val Ser Asp Gln
340 345 350

Ala Lys Val Leu Asp Ile Arg Arg Glu Val Tyr Pro Leu Pro Ser Gly
355 360 365

Leu Leu Thr Pro Lys Ala Glu Glu Asn Leu Glu Lys Ala Leu Lys Met
370 375 380

Ala Glu Glu Leu Gly Gln Gly Leu Lys His Leu Ala Gln Glu Val Ala
385 390 395 400

Lys Ala Val Val Gly Glu Arg Asp Arg Gly His Gly Arg Ser Pro Tyr
405 410 415

Leu Glu Glu Leu Thr Lys Leu Ala Asn Ser Leu Pro Leu Glu Arg Leu
420 425 430

Tyr Trp His Ala Leu Asp Gly Ala Phe Pro Arg Phe Phe Ala Arg Val
435 440 445

Glu Glu Glu Ala Ser Leu Asp Leu Trp Arg Glu Ala Leu Arg Gly Ala
450 455 460

Ala Leu Glu Ala Trp Lys Ala Thr Arg Arg Phe Leu Gly Thr Gly Ala
465 470 475 480

Arg His Leu Lys Ala Leu Ala Gln Gly Glu Gln Glu Phe Gly Arg Leu
485 490 495

Leu Gly Glu Leu Gly Glu Glu Val Arg Thr
500 505

<210> 1170
<211> 1101
<212> PRT
<213> Actinomyces naeslundii

<400> 1170

Met Trp Tyr Ala Ser Leu Met Ser Ala His His Leu Arg Val Gly Ile
1 5 10 15

Asp Val Gly Thr His Ser Val Gly Leu Ala Thr Leu Arg Val Asp Asp
20 25 30

His Gly Thr Pro Ile Glu Leu Leu Ser Ala Leu Ser His Ile His Asp
35 40 45

Ser Gly Val Gly Lys Glu Gly Lys Lys Asp His Asp Thr Arg Lys Lys
50 55 60

Leu Ser Gly Ile Ala Arg Arg Ala Arg Arg Leu Leu His His Arg Arg
65 70 75 80

Thr Gln Leu Gln Gln Leu Asp Glu Val Leu Arg Asp Leu Gly Phe Pro
85 90 95

Ile Pro Thr Pro Gly Glu Phe Leu Asp Leu Asn Glu Gln Thr Asp Pro
100 105 110

Tyr Arg Val Trp Arg Val Arg Ala Arg Leu Val Glu Glu Lys Leu Pro
115 120 125

Glu Glu Leu Arg Gly Pro Ala Ile Ser Met Ala Val Arg His Ile Ala
130 135 140

Arg His Arg Gly Trp Arg Asn Pro Tyr Ser Lys Val Glu Ser Leu Leu
145 150 155 160

Ser Pro Ala Glu Glu Ser Pro Phe Met Lys Ala Leu Arg Glu Arg Ile
165 170 175

Leu Ala Thr Thr Gly Glu Val Leu Asp Asp Gly Ile Thr Pro Gly Gln
180 185 190

Ala Met Ala Gln Val Ala Leu Thr His Asn Ile Ser Met Arg Gly Pro
195 200 205

Glu Gly Ile Leu Gly Lys Leu His Gln Ser Asp Asn Ala Asn Glu Ile
210 215 220

Arg Lys Ile Cys Ala Arg Gln Gly Val Ser Pro Asp Val Cys Lys Gln
225 230 235 240

Leu Leu Arg Ala Val Phe Lys Ala Asp Ser Pro Arg Gly Ser Ala Val
245 250 255

Ser Arg Val Ala Pro Asp Pro Leu Pro Gly Gln Gly Ser Phe Arg Arg
260 265 270

Ala Pro Lys Cys Asp Pro Glu Phe Gln Arg Phe Arg Ile Ile Ser Ile
275 280 285

Val Ala Asn Leu Arg Ile Ser Glu Thr Lys Gly Glu Asn Arg Pro Leu
290 295 300

Thr Ala Asp Glu Arg Arg His Val Val Thr Phe Leu Thr Glu Asp Ser
305 310 315 320

Gln Ala Asp Leu Thr Trp Val Asp Val Ala Glu Lys Leu Gly Val His
325 330 335

Arg Arg Asp Leu Arg Gly Thr Ala Val His Thr Asp Asp Gly Glu Arg
340 345 350

Ser Ala Ala Arg Pro Pro Ile Asp Ala Thr Asp Arg Ile Met Arg Gln
355 360 365

Thr Lys Ile Ser Ser Leu Lys Thr Trp Trp Glu Glu Ala Asp Ser Glu
370 375 380

Gln Arg Gly Ala Met Ile Arg Tyr Leu Tyr Glu Asp Pro Thr Asp Ser
385 390 395 400

Glu Cys Ala Glu Ile Ile Ala Glu Leu Pro Glu Glu Asp Gln Ala Lys
405 410 415

Leu Asp Ser Leu His Leu Pro Ala Gly Arg Ala Ala Tyr Ser Arg Glu
420 425 430

Ser Leu Thr Ala Leu Ser Asp His Met Leu Ala Thr Thr Asp Asp Leu
435 440 445

His Glu Ala Arg Lys Arg Leu Phe Gly Val Asp Asp Ser Trp Ala Pro
450 455 460

Pro Ala Glu Ala Ile Asn Ala Pro Val Gly Asn Pro Ser Val Asp Arg
465 470 475 480

Thr Leu Lys Ile Val Gly Arg Tyr Leu Ser Ala Val Glu Ser Met Trp
485 490 495

Gly Thr Pro Glu Val Ile His Val Glu His Val Arg Asp Gly Phe Thr
500 505 510

Ser Glu Arg Met Ala Asp Glu Arg Asp Lys Ala Asn Arg Arg Arg Tyr
515 520 525

Asn Asp Asn Gln Glu Ala Met Lys Lys Ile Gln Arg Asp Tyr Gly Lys
530 535 540

Glu Gly Tyr Ile Ser Arg Gly Asp Ile Val Arg Leu Asp Ala Leu Glu
545 550 555 560

Leu Gln Gly Cys Ala Cys Leu Tyr Cys Gly Thr Thr Ile Gly Tyr His
565 570 575

Thr Cys Gln Leu Asp His Ile Val Pro Gln Ala Gly Pro Gly Ser Asn
580 585 590

Asn Arg Arg Gly Asn Leu Val Ala Val Cys Glu Arg Cys Asn Arg Ser
595 600 605

Lys Ser Asn Thr Pro Phe Ala Val Trp Ala Gln Lys Cys Gly Ile Pro
610 615 620

His Val Gly Val Lys Glu Ala Ile Gly Arg Val Arg Gly Trp Arg Lys
625 630 635 640

Gln Thr Pro Asn Thr Ser Ser Glu Asp Leu Thr Arg Leu Lys Lys Glu
645 650 655

Val Ile Ala Arg Leu Arg Arg Thr Gln Glu Asp Pro Glu Ile Asp Glu
660 665 670

```
Arg Ser Met Glu Ser Val Ala Trp Met Ala Asn Glu Leu His His Arg
        675                 680                 685

Ile Ala Ala Ala Tyr Pro Glu Thr Thr Val Met Val Tyr Arg Gly Ser
    690                 695                 700

Ile Thr Ala Ala Ala Arg Lys Ala Ala Gly Ile Asp Ser Arg Ile Asn
705                 710                 715                 720

Leu Ile Gly Glu Lys Gly Arg Lys Asp Arg Ile Asp Arg Arg His His
                725                 730                 735

Ala Val Asp Ala Ser Val Val Ala Leu Met Glu Ala Ser Val Ala Lys
            740                 745                 750

Thr Leu Ala Glu Arg Ser Ser Leu Arg Gly Glu Gln Arg Leu Thr Gly
        755                 760                 765

Lys Glu Gln Thr Trp Lys Gln Tyr Thr Gly Ser Thr Val Gly Ala Arg
    770                 775                 780

Glu His Phe Glu Met Trp Arg Gly His Met Leu His Leu Thr Glu Leu
785                 790                 795                 800

Phe Asn Glu Arg Leu Ala Glu Asp Lys Val Tyr Val Thr Gln Asn Ile
                805                 810                 815

Arg Leu Arg Leu Ser Asp Gly Asn Ala His Thr Val Asn Pro Ser Lys
            820                 825                 830

Leu Val Ser His Arg Leu Gly Asp Gly Leu Thr Val Gln Gln Ile Asp
        835                 840                 845

Arg Ala Cys Thr Pro Ala Leu Trp Cys Ala Leu Thr Arg Glu Lys Asp
    850                 855                 860

Phe Asp Glu Lys Asn Gly Leu Pro Ala Arg Glu Asp Arg Ala Ile Arg
865                 870                 875                 880

Val His Gly His Glu Ile Lys Ser Ser Asp Tyr Ile Gln Val Phe Ser
                885                 890                 895

Lys Arg Lys Lys Thr Asp Ser Asp Arg Asp Glu Thr Pro Phe Gly Ala
            900                 905                 910

Ile Ala Val Arg Gly Gly Phe Val Glu Ile Gly Pro Ser Ile His His
```

915 920 925

Ala Arg Ile Tyr Arg Val Glu Gly Lys Lys Pro Val Tyr Ala Met Leu
930 935 940

Arg Val Phe Thr His Asp Leu Leu Ser Gln Arg His Gly Asp Leu Phe
945 950 955 960

Ser Ala Val Ile Pro Pro Gln Ser Ile Ser Met Arg Cys Ala Glu Pro
965 970 975

Lys Leu Arg Lys Ala Ile Thr Thr Gly Asn Ala Thr Tyr Leu Gly Trp
980 985 990

Val Val Val Gly Asp Glu Leu Glu Ile Asn Val Asp Ser Phe Thr Lys
995 1000 1005

Tyr Ala Ile Gly Arg Phe Leu Glu Asp Phe Pro Asn Thr Thr Arg
1010 1015 1020

Trp Arg Ile Cys Gly Tyr Asp Thr Asn Ser Lys Leu Thr Leu Lys
1025 1030 1035

Pro Ile Val Leu Ala Ala Glu Gly Leu Glu Asn Pro Ser Ser Ala
1040 1045 1050

Val Asn Glu Ile Val Glu Leu Lys Gly Trp Arg Val Ala Ile Asn
1055 1060 1065

Val Leu Thr Lys Val His Pro Thr Val Val Arg Arg Asp Ala Leu
1070 1075 1080

Gly Arg Pro Arg Tyr Ser Ser Arg Ser Asn Leu Pro Thr Ser Trp
1085 1090 1095

Thr Ile Glu
1100

<210> 1171
<211> 1372
<212> PRT
<213> Streptococcus pyogenes

<400> 1171

Gly Ser Gly His Met Asp Lys Lys Tyr Ser Ile Gly Leu Ala Ile Gly
1 5 10 15

Thr Asn Ser Val Gly Trp Ala Val Ile Thr Asp Glu Tyr Lys Val Pro

20 25 30

Ser Lys Lys Phe Lys Val Leu Gly Asn Thr Asp Arg His Ser Ile Lys
35 40 45

Lys Asn Leu Ile Gly Ala Leu Leu Phe Asp Ser Gly Glu Thr Ala Glu
50 55 60

Ala Thr Arg Leu Lys Arg Thr Ala Arg Arg Arg Tyr Thr Arg Arg Lys
65 70 75 80

Asn Arg Ile Leu Tyr Leu Gln Glu Ile Phe Ser Asn Glu Met Ala Lys
85 90 95

Val Asp Asp Ser Phe Phe His Arg Leu Glu Glu Ser Phe Leu Val Glu
100 105 110

Glu Asp Lys Lys His Glu Arg His Pro Ile Phe Gly Asn Ile Val Asp
115 120 125

Glu Val Ala Tyr His Glu Lys Tyr Pro Thr Ile Tyr His Leu Arg Lys
130 135 140

Lys Leu Val Asp Ser Thr Asp Lys Ala Asp Leu Arg Leu Ile Tyr Leu
145 150 155 160

Ala Leu Ala His Met Ile Lys Phe Arg Gly His Phe Leu Ile Glu Gly
165 170 175

Asp Leu Asn Pro Asp Asn Ser Asp Val Asp Lys Leu Phe Ile Gln Leu
180 185 190

Val Gln Thr Tyr Asn Gln Leu Phe Glu Glu Asn Pro Ile Asn Ala Ser
195 200 205

Gly Val Asp Ala Lys Ala Ile Leu Ser Ala Arg Leu Ser Lys Ser Arg
210 215 220

Arg Leu Glu Asn Leu Ile Ala Gln Leu Pro Gly Glu Lys Lys Asn Gly
225 230 235 240

Leu Phe Gly Asn Leu Ile Ala Leu Ser Leu Gly Leu Thr Pro Asn Phe
245 250 255

Lys Ser Asn Phe Asp Leu Ala Glu Asp Ala Lys Leu Gln Leu Ser Lys
260 265 270

Asp Thr Tyr Asp Asp Asp Leu Asp Asn Leu Leu Ala Gln Ile Gly Asp
        275                 280                 285

Gln Tyr Ala Asp Leu Phe Leu Ala Ala Lys Asn Leu Ser Asp Ala Ile
    290                 295                 300

Leu Leu Ser Asp Ile Leu Arg Val Asn Thr Glu Ile Thr Lys Ala Pro
305                 310                 315                 320

Leu Ser Ala Ser Met Ile Lys Arg Tyr Asp Glu His His Gln Asp Leu
                325                 330                 335

Thr Leu Leu Lys Ala Leu Val Arg Gln Gln Leu Pro Glu Lys Tyr Lys
            340                 345                 350

Glu Ile Phe Phe Asp Gln Ser Lys Asn Gly Tyr Ala Gly Tyr Ile Asp
        355                 360                 365

Gly Gly Ala Ser Gln Glu Glu Phe Tyr Lys Phe Ile Lys Pro Ile Leu
    370                 375                 380

Glu Lys Met Asp Gly Thr Glu Glu Leu Leu Val Lys Leu Asn Arg Glu
385                 390                 395                 400

Asp Leu Leu Arg Lys Gln Arg Thr Phe Asp Asn Gly Ser Ile Pro His
                405                 410                 415

Gln Ile His Leu Gly Glu Leu His Ala Ile Leu Arg Arg Gln Glu Asp
            420                 425                 430

Phe Tyr Pro Phe Leu Lys Asp Asn Arg Glu Lys Ile Glu Lys Ile Leu
        435                 440                 445

Thr Phe Arg Ile Pro Tyr Tyr Val Gly Pro Leu Ala Arg Gly Asn Ser
    450                 455                 460

Arg Phe Ala Trp Met Thr Arg Lys Ser Glu Glu Thr Ile Thr Pro Trp
465                 470                 475                 480

Asn Phe Glu Glu Val Val Asp Lys Gly Ala Ser Ala Gln Ser Phe Ile
                485                 490                 495

Glu Arg Met Thr Asn Phe Asp Lys Asn Leu Pro Asn Glu Lys Val Leu
            500                 505                 510

Pro Lys His Ser Leu Leu Tyr Glu Tyr Phe Thr Val Tyr Asn Glu Leu
        515                 520                 525

Thr Lys Val Lys Tyr Val Thr Glu Gly Met Arg Lys Pro Ala Phe Leu
530 535 540

Ser Gly Glu Gln Lys Lys Ala Ile Val Asp Leu Leu Phe Lys Thr Asn
545 550 555 560

Arg Lys Val Thr Val Lys Gln Leu Lys Glu Asp Tyr Phe Lys Lys Ile
565 570 575

Glu Glu Phe Asp Ser Val Glu Ile Ser Gly Val Glu Asp Arg Phe Asn
580 585 590

Ala Ser Leu Gly Thr Tyr His Asp Leu Leu Lys Ile Ile Lys Asp Lys
595 600 605

Asp Phe Leu Asp Asn Glu Glu Asn Glu Asp Ile Leu Glu Asp Ile Val
610 615 620

Leu Thr Leu Thr Leu Phe Glu Asp Arg Glu Met Ile Glu Glu Arg Leu
625 630 635 640

Lys Thr Tyr Ala His Leu Phe Asp Asp Lys Val Met Lys Gln Leu Lys
645 650 655

Arg Arg Arg Tyr Thr Gly Trp Gly Arg Leu Ser Arg Lys Leu Ile Asn
660 665 670

Gly Ile Arg Asp Lys Gln Ser Gly Lys Thr Ile Leu Asp Phe Leu Lys
675 680 685

Ser Asp Gly Phe Ala Asn Arg Asn Phe Met Gln Leu Ile His Asp Asp
690 695 700

Ser Leu Thr Phe Lys Glu Asp Ile Gln Lys Ala Gln Val Ser Gly Gln
705 710 715 720

Gly Asp Ser Leu His Glu His Ile Ala Asn Leu Ala Gly Ser Pro Ala
725 730 735

Ile Lys Lys Gly Ile Leu Gln Thr Val Lys Val Val Asp Glu Leu Val
740 745 750

Lys Val Met Gly Arg His Lys Pro Glu Asn Ile Val Ile Glu Met Ala
755 760 765

Arg Glu Asn Gln Thr Thr Gln Lys Gly Gln Lys Asn Ser Arg Glu Arg
770 775 780

Met Lys Arg Ile Glu Glu Gly Ile Lys Glu Leu Gly Ser Gln Ile Leu
785 790 795 800

Lys Glu His Pro Val Glu Asn Thr Gln Leu Gln Asn Glu Lys Leu Tyr
805 810 815

Leu Tyr Tyr Leu Gln Asn Gly Arg Asp Met Tyr Val Asp Gln Glu Leu
820 825 830

Asp Ile Asn Arg Leu Ser Asp Tyr Asp Val Asp Ala Ile Val Pro Gln
835 840 845

Ser Phe Leu Lys Asp Asp Ser Ile Asp Asn Lys Val Leu Thr Arg Ser
850 855 860

Asp Lys Asn Arg Gly Lys Ser Asp Asn Val Pro Ser Glu Glu Val Val
865 870 875 880

Lys Lys Met Lys Asn Tyr Trp Arg Gln Leu Leu Asn Ala Lys Leu Ile
885 890 895

Thr Gln Arg Lys Phe Asp Asn Leu Thr Lys Ala Glu Arg Gly Gly Leu
900 905 910

Ser Glu Leu Asp Lys Ala Gly Phe Ile Lys Arg Gln Leu Val Glu Thr
915 920 925

Arg Gln Ile Thr Lys His Val Ala Gln Ile Leu Asp Ser Arg Met Asn
930 935 940

Thr Lys Tyr Asp Glu Asn Asp Lys Leu Ile Arg Glu Val Lys Val Ile
945 950 955 960

Thr Leu Lys Ser Lys Leu Val Ser Asp Phe Arg Lys Asp Phe Gln Phe
965 970 975

Tyr Lys Val Arg Glu Ile Asn Asn Tyr His His Ala His Asp Ala Tyr
980 985 990

Leu Asn Ala Val Val Gly Thr Ala Leu Ile Lys Lys Tyr Pro Lys Leu
995 1000 1005

Glu Ser Glu Phe Val Tyr Gly Asp Tyr Lys Val Tyr Asp Val Arg
1010 1015 1020

Lys Met Ile Ala Lys Ser Glu Gln Glu Ile Gly Lys Ala Thr Ala

1025 1030 1035

Lys Tyr Phe Phe Tyr Ser Asn Ile Met Asn Phe Phe Lys Thr Glu
1040 1045 1050

Ile Thr Leu Ala Asn Gly Glu Ile Arg Lys Arg Pro Leu Ile Glu
1055 1060 1065

Thr Asn Gly Glu Thr Gly Glu Ile Val Trp Asp Lys Gly Arg Asp
1070 1075 1080

Phe Ala Thr Val Arg Lys Val Leu Ser Met Pro Gln Val Asn Ile
1085 1090 1095

Val Lys Lys Thr Glu Val Gln Thr Gly Gly Phe Ser Lys Glu Ser
1100 1105 1110

Ile Leu Pro Lys Arg Asn Ser Asp Lys Leu Ile Ala Arg Lys Lys
1115 1120 1125

Asp Trp Asp Pro Lys Lys Tyr Gly Gly Phe Asp Ser Pro Thr Val
1130 1135 1140

Ala Tyr Ser Val Leu Val Val Ala Lys Val Glu Lys Gly Lys Ser
1145 1150 1155

Lys Lys Leu Lys Ser Val Lys Glu Leu Leu Gly Ile Thr Ile Met
1160 1165 1170

Glu Arg Ser Ser Phe Glu Lys Asn Pro Ile Asp Phe Leu Glu Ala
1175 1180 1185

Lys Gly Tyr Lys Glu Val Lys Lys Asp Leu Ile Ile Lys Leu Pro
1190 1195 1200

Lys Tyr Ser Leu Phe Glu Leu Glu Asn Gly Arg Lys Arg Met Leu
1205 1210 1215

Ala Ser Ala Gly Glu Leu Gln Lys Gly Asn Glu Leu Ala Leu Pro
1220 1225 1230

Ser Lys Tyr Val Asn Phe Leu Tyr Leu Ala Ser His Tyr Glu Lys
1235 1240 1245

Leu Lys Gly Ser Pro Glu Asp Asn Glu Gln Lys Gln Leu Phe Val
1250 1255 1260

Glu Gln His Lys His Tyr Leu Asp Glu Ile Ile Glu Gln Ile Ser
1265 1270 1275

Glu Phe Ser Lys Arg Val Ile Leu Ala Asp Ala Asn Leu Asp Lys
1280 1285 1290

Val Leu Ser Ala Tyr Asn Lys His Arg Asp Lys Pro Ile Arg Glu
1295 1300 1305

Gln Ala Glu Asn Ile Ile His Leu Phe Thr Leu Thr Asn Leu Gly
1310 1315 1320

Ala Pro Ala Ala Phe Lys Tyr Phe Asp Thr Thr Ile Asp Arg Lys
1325 1330 1335

Arg Tyr Thr Ser Thr Lys Glu Val Leu Asp Ala Thr Leu Ile His
1340 1345 1350

Gln Ser Ile Thr Gly Leu Tyr Glu Thr Arg Ile Asp Leu Ser Gln
1355 1360 1365

Leu Gly Gly Asp
1370

<210> 1172
<211> 1372
<212> PRT
<213> Streptococcus pyogenes

<400> 1172

Gly Ala Ala Ser Met Asp Lys Lys Tyr Ser Ile Gly Leu Asp Ile Gly
1 5 10 15

Thr Asn Ser Val Gly Trp Ala Val Ile Thr Asp Glu Tyr Lys Val Pro
20 25 30

Ser Lys Lys Phe Lys Val Leu Gly Asn Thr Asp Arg His Ser Ile Lys
35 40 45

Lys Asn Leu Ile Gly Ala Leu Leu Phe Asp Ser Gly Glu Thr Ala Glu
50 55 60

Ala Thr Arg Leu Lys Arg Thr Ala Arg Arg Arg Tyr Thr Arg Arg Lys
65 70 75 80

Asn Arg Ile Cys Tyr Leu Gln Glu Ile Phe Ser Asn Glu Met Ala Lys
85 90 95

Val Asp Asp Ser Phe Phe His Arg Leu Glu Glu Ser Phe Leu Val Glu
100 105 110

Glu Asp Lys Lys His Glu Arg His Pro Ile Phe Gly Asn Ile Val Asp
115 120 125

Glu Val Ala Tyr His Glu Lys Tyr Pro Thr Ile Tyr His Leu Arg Lys
130 135 140

Lys Leu Val Asp Ser Thr Asp Lys Ala Asp Leu Arg Leu Ile Tyr Leu
145 150 155 160

Ala Leu Ala His Met Ile Lys Phe Arg Gly His Phe Leu Ile Glu Gly
165 170 175

Asp Leu Asn Pro Asp Asn Ser Asp Val Asp Lys Leu Phe Ile Gln Leu
180 185 190

Val Gln Thr Tyr Asn Gln Leu Phe Glu Glu Asn Pro Ile Asn Ala Ser
195 200 205

Gly Val Asp Ala Lys Ala Ile Leu Ser Ala Arg Leu Ser Lys Ser Arg
210 215 220

Arg Leu Glu Asn Leu Ile Ala Gln Leu Pro Gly Glu Lys Lys Asn Gly
225 230 235 240

Leu Phe Gly Asn Leu Ile Ala Leu Ser Leu Gly Leu Thr Pro Asn Phe
245 250 255

Lys Ser Asn Phe Asp Leu Ala Glu Asp Ala Lys Leu Gln Leu Ser Lys
260 265 270

Asp Thr Tyr Asp Asp Asp Leu Asp Asn Leu Leu Ala Gln Ile Gly Asp
275 280 285

Gln Tyr Ala Asp Leu Phe Leu Ala Ala Lys Asn Leu Ser Asp Ala Ile
290 295 300

Leu Leu Ser Asp Ile Leu Arg Val Asn Thr Glu Ile Thr Lys Ala Pro
305 310 315 320

Leu Ser Ala Ser Met Ile Lys Arg Tyr Asp Glu His His Gln Asp Leu
325 330 335

Thr Leu Leu Lys Ala Leu Val Arg Gln Gln Leu Pro Glu Lys Tyr Lys
340 345 350

Glu Ile Phe Phe Asp Gln Ser Lys Asn Gly Tyr Ala Gly Tyr Ile Asp
355 360 365

Gly Gly Ala Ser Gln Glu Glu Phe Tyr Lys Phe Ile Lys Pro Ile Leu
370 375 380

Glu Lys Met Asp Gly Thr Glu Glu Leu Leu Val Lys Leu Asn Arg Glu
385 390 395 400

Asp Leu Leu Arg Lys Gln Arg Thr Phe Asp Asn Gly Ser Ile Pro His
405 410 415

Gln Ile His Leu Gly Glu Leu His Ala Ile Leu Arg Arg Gln Glu Asp
420 425 430

Phe Tyr Pro Phe Leu Lys Asp Asn Arg Glu Lys Ile Glu Lys Ile Leu
435 440 445

Thr Phe Arg Ile Pro Tyr Tyr Val Gly Pro Leu Ala Arg Gly Asn Ser
450 455 460

Arg Phe Ala Trp Met Thr Arg Lys Ser Glu Glu Thr Ile Thr Pro Trp
465 470 475 480

Asn Phe Glu Glu Val Val Asp Lys Gly Ala Ser Ala Gln Ser Phe Ile
485 490 495

Glu Arg Met Thr Asn Phe Asp Lys Asn Leu Pro Asn Glu Lys Val Leu
500 505 510

Pro Lys His Ser Leu Leu Tyr Glu Tyr Phe Thr Val Tyr Asn Glu Leu
515 520 525

Thr Lys Val Lys Tyr Val Thr Glu Gly Met Arg Lys Pro Ala Phe Leu
530 535 540

Ser Gly Glu Gln Lys Lys Ala Ile Val Asp Leu Leu Phe Lys Thr Asn
545 550 555 560

Arg Lys Val Thr Val Lys Gln Leu Lys Glu Asp Tyr Phe Lys Lys Ile
565 570 575

Glu Cys Phe Asp Ser Val Glu Ile Ser Gly Val Glu Asp Arg Phe Asn
580 585 590

Ala Ser Leu Gly Thr Tyr His Asp Leu Leu Lys Ile Ile Lys Asp Lys
595 600 605

Asp Phe Leu Asp Asn Glu Glu Asn Glu Asp Ile Leu Glu Asp Ile Val
610 615 620

Leu Thr Leu Thr Leu Phe Glu Asp Arg Glu Met Ile Glu Glu Arg Leu
625 630 635 640

Lys Thr Tyr Ala His Leu Phe Asp Asp Lys Val Met Lys Gln Leu Lys
645 650 655

Arg Arg Arg Tyr Thr Gly Trp Gly Arg Leu Ser Arg Lys Leu Ile Asn
660 665 670

Gly Ile Arg Asp Lys Gln Ser Gly Lys Thr Ile Leu Asp Phe Leu Lys
675 680 685

Ser Asp Gly Phe Ala Asn Arg Asn Phe Met Gln Leu Ile His Asp Asp
690 695 700

Ser Leu Thr Phe Lys Glu Asp Ile Gln Lys Ala Gln Val Ser Gly Gln
705 710 715 720

Gly Asp Ser Leu His Glu His Ile Ala Asn Leu Ala Gly Ser Pro Ala
725 730 735

Ile Lys Lys Gly Ile Leu Gln Thr Val Lys Val Val Asp Glu Leu Val
740 745 750

Lys Val Met Gly Arg His Lys Pro Glu Asn Ile Val Ile Glu Met Ala
755 760 765

Arg Glu Asn Gln Thr Thr Gln Lys Gly Gln Lys Asn Ser Arg Glu Arg
770 775 780

Met Lys Arg Ile Glu Glu Gly Ile Lys Glu Leu Gly Ser Gln Ile Leu
785 790 795 800

Lys Glu His Pro Val Glu Asn Thr Gln Leu Gln Asn Glu Lys Leu Tyr
805 810 815

Leu Tyr Tyr Leu Gln Asn Gly Arg Asp Met Tyr Val Asp Gln Glu Leu
820 825 830

Asp Ile Asn Arg Leu Ser Asp Tyr Asp Val Asp His Ile Val Pro Gln
835 840 845

Ser Phe Leu Lys Asp Asp Ser Ile Asp Asn Lys Val Leu Thr Arg Ser

850 855 860

Asp Lys Asn Arg Gly Lys Ser Asp Asn Val Pro Ser Glu Glu Val Val
865 870 875 880

Lys Lys Met Lys Asn Tyr Trp Arg Gln Leu Leu Asn Ala Lys Leu Ile
885 890 895

Thr Gln Arg Lys Phe Asp Asn Leu Thr Lys Ala Glu Arg Gly Gly Leu
900 905 910

Ser Glu Leu Asp Lys Ala Gly Phe Ile Lys Arg Gln Leu Val Glu Thr
915 920 925

Arg Gln Ile Thr Lys His Val Ala Gln Ile Leu Asp Ser Arg Met Asn
930 935 940

Thr Lys Tyr Asp Glu Asn Asp Lys Leu Ile Arg Glu Val Lys Val Ile
945 950 955 960

Thr Leu Lys Ser Lys Leu Val Ser Asp Phe Arg Lys Asp Phe Gln Phe
965 970 975

Tyr Lys Val Arg Glu Ile Asn Asn Tyr His His Ala His Asp Ala Tyr
980 985 990

Leu Asn Ala Val Val Gly Thr Ala Leu Ile Lys Lys Tyr Pro Lys Leu
995 1000 1005

Glu Ser Glu Phe Val Tyr Gly Asp Tyr Lys Val Tyr Asp Val Arg
1010 1015 1020

Lys Met Ile Ala Lys Ser Glu Gln Glu Ile Gly Lys Ala Thr Ala
1025 1030 1035

Lys Tyr Phe Phe Tyr Ser Asn Ile Met Asn Phe Phe Lys Thr Glu
1040 1045 1050

Ile Thr Leu Ala Asn Gly Glu Ile Arg Lys Arg Pro Leu Ile Glu
1055 1060 1065

Thr Asn Gly Glu Thr Gly Glu Ile Val Trp Asp Lys Gly Arg Asp
1070 1075 1080

Phe Ala Thr Val Arg Lys Val Leu Ser Met Pro Gln Val Asn Ile
1085 1090 1095

Val Lys Lys Thr Glu Val Gln Thr Gly Gly Phe Ser Lys Glu Ser
1100 1105 1110

Ile Leu Pro Lys Arg Asn Ser Asp Lys Leu Ile Ala Arg Lys Lys
1115 1120 1125

Asp Trp Asp Pro Lys Lys Tyr Gly Gly Phe Asp Ser Pro Thr Val
1130 1135 1140

Ala Tyr Ser Val Leu Val Val Ala Lys Val Glu Lys Gly Lys Ser
1145 1150 1155

Lys Lys Leu Lys Ser Val Lys Glu Leu Leu Gly Ile Thr Ile Met
1160 1165 1170

Glu Arg Ser Ser Phe Glu Lys Asn Pro Ile Asp Phe Leu Glu Ala
1175 1180 1185

Lys Gly Tyr Lys Glu Val Lys Lys Asp Leu Ile Ile Lys Leu Pro
1190 1195 1200

Lys Tyr Ser Leu Phe Glu Leu Glu Asn Gly Arg Lys Arg Met Leu
1205 1210 1215

Ala Ser Ala Gly Glu Leu Gln Lys Gly Asn Glu Leu Ala Leu Pro
1220 1225 1230

Ser Lys Tyr Val Asn Phe Leu Tyr Leu Ala Ser His Tyr Glu Lys
1235 1240 1245

Leu Lys Gly Ser Pro Glu Asp Asn Glu Gln Lys Gln Leu Phe Val
1250 1255 1260

Glu Gln His Lys His Tyr Leu Asp Glu Ile Ile Glu Gln Ile Ser
1265 1270 1275

Glu Phe Ser Lys Arg Val Ile Leu Ala Asp Ala Asn Leu Asp Lys
1280 1285 1290

Val Leu Ser Ala Tyr Asn Lys His Arg Asp Lys Pro Ile Arg Glu
1295 1300 1305

Gln Ala Glu Asn Ile Ile His Leu Phe Thr Leu Thr Asn Leu Gly
1310 1315 1320

Ala Pro Ala Ala Phe Lys Tyr Phe Asp Thr Thr Ile Asp Arg Lys
1325 1330 1335

Arg Tyr Thr Ser Thr Lys Glu Val Leu Asp Ala Thr Leu Ile His
1340 1345 1350

Gln Ser Ile Thr Gly Leu Tyr Glu Thr Arg Ile Asp Leu Ser Gln
1355 1360 1365

Leu Gly Gly Asp
1370

<210> 1173
<211> 693
<212> PRT
<213> Pyrococcus furiosus

<400> 1173

Met Arg Gly Ser His His His His His His Gly Met Ala Ser Met Thr
1 5 10 15

Gly Gly Gln Gln Met Gly Arg Asp Leu Tyr Asp Asp Asp Asp Lys Asp
20 25 30

His Pro Phe Thr Ser Val Lys Asp Pro Thr Leu Leu Arg Ile Lys Ile
35 40 45

Val Pro Val Gln Pro Phe Ile Ala Asn Ser Arg Lys Gln Leu Asp Leu
50 55 60

Trp Ala Ser Ser His Leu Leu Ser Met Leu Met Tyr Lys Ala Leu Glu
65 70 75 80

Val Ile Val Asp Lys Phe Gly Pro Glu His Val Ile Tyr Pro Ser Leu
85 90 95

Arg Asp Gln Pro Phe Phe Leu Lys Phe Tyr Leu Gly Glu Asn Ile Gly
100 105 110

Asp Glu Ile Leu Val Ala Asn Leu Pro Asn Lys Ala Leu Ala Ile Val
115 120 125

Ser Gly Lys Glu Ala Glu Lys Ile Glu Glu Glu Ile Lys Lys Arg Ile
130 135 140

Arg Asp Phe Leu Leu Gln Leu Tyr Arg Glu Ala Val Asp Trp Ala Val
145 150 155 160

Glu Asn Gly Val Val Lys Val Asp Arg Ser Glu Lys Asp Ser Met Leu
165 170 175

```
Lys Glu Ala Tyr Leu Lys Ile Val Arg Glu Tyr Phe Thr Val Ser Ile
            180                 185                 190

Thr Trp Val Ser Leu Ser Glu Lys Glu Asp Ile Tyr Gln Val Thr Glu
        195                 200                 205

Asn Ala Gly Leu Ser Asp Glu Asp Val Lys Lys Trp Leu Lys Phe Ala
    210                 215                 220

Glu Lys Lys Glu Asn Ser Arg Val Leu Glu Arg Ile Ala Ile Tyr Pro
225                 230                 235                 240

Leu Leu Val Lys Ile Leu Asp Ser Leu Gly Glu Arg Lys Val Thr Glu
                245                 250                 255

Glu Arg Phe Glu Lys Ser Glu Gln Leu Lys Gly Trp Lys Cys His Val
            260                 265                 270

Cys Gly Glu Asn Leu Ala Ile Phe Gly Asp Met Tyr Asp His Asp Asn
        275                 280                 285

Leu Lys Ser Leu Trp Leu Asp Glu Glu Pro Leu Cys Pro Met Cys Leu
    290                 295                 300

Ile Lys Arg Tyr Tyr Pro Val Trp Ile Arg Ser Lys Thr Gly Gln Lys
305                 310                 315                 320

Ile Arg Phe Glu Ser Val Val Asp Val Ala Leu Leu Tyr Lys Asn Trp
                325                 330                 335

Arg Lys Ile Phe Asp Glu Lys Tyr Gly Lys Asp Leu Val Ser Lys Ala
            340                 345                 350

Arg Glu Val Ser Glu Asp Phe Val Lys Asp Asn Met Leu Val Asp Ser
        355                 360                 365

Asp Leu Tyr Tyr Ser Ser Thr Trp Glu Ser Gly Leu Ser Lys Lys Leu
    370                 375                 380

Lys Asn Lys Lys Glu Ile Asp Glu Glu Lys Val Lys Glu Val Val Asp
385                 390                 395                 400

Phe Leu Asn Ala Ala Tyr Lys Glu Ile Gly Asn Pro Pro Lys Tyr Tyr
                405                 410                 415

Ala Ile Leu Val Met Asp Gly Asp Asp Met Gly Lys Val Ile Ser Gly
            420                 425                 430
```

Glu Val Leu Gly Glu Ile Ser Thr Arg Ile His Pro Asn Ile Arg Asp
435 440 445

Tyr Val Glu Ile Pro Glu Ala Lys Tyr Tyr Ser Thr Pro Gln Val His
450 455 460

Val Ala Ile Ser Gln Ala Leu Ala Asn Phe Ser Ile Arg Glu Val Arg
465 470 475 480

Ser Val Val Lys Asp Glu Gly Leu Leu Ile Tyr Ala Gly Gly Asp Asp
485 490 495

Val Leu Ala Ile Leu Pro Val Asp Lys Ala Leu Glu Val Ala Tyr Lys
500 505 510

Ile Arg Lys Glu Phe Gly Lys Ser Phe Glu Asn Gly Ser Leu Leu Pro
515 520 525

Gly Trp Lys Leu Ser Ala Gly Ile Leu Ile Val His Tyr Lys His Pro
530 535 540

Leu Tyr Asp Ala Leu Glu Lys Ala Arg Asp Leu Leu Asn Asn Lys Ala
545 550 555 560

Lys Asn Val Pro Gly Lys Asp Thr Leu Ala Ile Gly Leu Leu Lys Arg
565 570 575

Ser Gly Ser Tyr Tyr Ile Ser Leu Val Gly Trp Glu Leu Ile Arg Val
580 585 590

Phe Tyr Asn Ser Glu Leu Arg Lys Lys Leu Leu Glu Glu Lys Gly Gly
595 600 605

Val Gly Lys Arg Phe Ile Tyr His Val Leu Arg Glu Val Asp Thr Trp
610 615 620

Pro Lys Val Gly Ile Asp Glu Met Leu Lys Phe Glu Val Ile Arg His
625 630 635 640

Ile Arg Gly Arg Asn Lys Glu Glu Thr Lys Glu Leu Arg Glu Lys Ile
645 650 655

Tyr Gly Glu Ile Lys Asp Leu Leu Glu His Val Arg Gly Asn Asn Glu
660 665 670

Val Glu Lys Val Arg Gly Leu Phe Thr Phe Leu Lys Ile Ile Thr Asp

675 680 685

Ala Glu Val Phe Pro
690

<210> 1174
<211> 686
<212> PRT
<213> Pyrococcus furiosus

<400> 1174

Met Ala Asp Thr Arg Phe Pro Asp His Ala Ile Trp Thr His Leu Asp
1 5 10 15

Leu Thr Ser Ala Leu Ser Val Lys Asp Pro Thr Leu Leu Arg Ile Lys
20 25 30

Ile Val Pro Val Gln Pro Phe Ile Ala Asn Ser Arg Lys Gln Leu Asp
35 40 45

Leu Trp Ala Ser Ser His Leu Leu Ser Met Leu Met Tyr Lys Ala Leu
50 55 60

Glu Val Ile Val Asp Lys Phe Gly Pro Glu His Val Ile Tyr Pro Ser
65 70 75 80

Leu Arg Asp Gln Pro Phe Phe Leu Lys Phe Tyr Leu Gly Glu Asn Ile
85 90 95

Gly Asp Glu Ile Leu Val Ala Asn Leu Pro Asn Lys Ala Leu Ala Ile
100 105 110

Val Ser Gly Lys Glu Ala Glu Lys Ile Glu Glu Glu Ile Lys Lys Arg
115 120 125

Ile Arg Asp Phe Leu Leu Gln Leu Tyr Arg Glu Ala Val Asp Trp Ala
130 135 140

Val Glu Asn Gly Val Val Lys Val Asp Arg Ser Glu Lys Asp Ser Met
145 150 155 160

Leu Lys Glu Ala Tyr Leu Lys Ile Val Arg Glu Tyr Phe Thr Val Ser
165 170 175

Ile Thr Trp Val Ser Leu Ser Glu Lys Glu Asp Ile Tyr Gln Val Thr
180 185 190

Glu Asn Ala Gly Leu Ser Asp Glu Asp Val Lys Lys Trp Leu Lys Phe

195 200 205

Ala Glu Lys Lys Glu Asn Ser Arg Val Leu Glu Arg Ile Ala Ile Tyr
210 215 220

Pro Leu Leu Val Lys Ile Leu Asp Ser Leu Gly Glu Arg Lys Val Thr
225 230 235 240

Glu Glu Arg Phe Glu Lys Ser Glu Gln Leu Lys Gly Trp Lys Cys His
245 250 255

Val Cys Gly Glu Asn Leu Ala Ile Phe Gly Asp Met Tyr Asp His Asp
260 265 270

Asn Leu Lys Ser Leu Trp Leu Asp Glu Glu Pro Leu Cys Pro Met Cys
275 280 285

Leu Ile Lys Arg Tyr Tyr Pro Val Trp Ile Arg Ser Lys Thr Gly Gln
290 295 300

Lys Ile Arg Phe Glu Ser Val Val Asp Val Ala Leu Leu Tyr Lys Asn
305 310 315 320

Trp Arg Lys Ile Phe Asp Glu Lys Tyr Gly Lys Asp Leu Val Ser Lys
325 330 335

Ala Arg Glu Val Ser Glu Asp Phe Val Lys Asp Asn Met Leu Val Asp
340 345 350

Ser Asp Leu Tyr Tyr Ser Ser Thr Trp Glu Ser Gly Leu Ser Lys Lys
355 360 365

Leu Lys Asn Lys Lys Glu Ile Asp Glu Glu Lys Val Lys Glu Val Val
370 375 380

Asp Phe Leu Asn Ala Ala Tyr Lys Glu Ile Gly Asn Pro Pro Lys Tyr
385 390 395 400

Tyr Ala Ile Leu Val Met Asp Gly Asp Asp Met Gly Lys Val Ile Ser
405 410 415

Gly Glu Val Leu Gly Glu Ile Ser Thr Arg Ile His Pro Asn Ile Arg
420 425 430

Asp Tyr Val Glu Ile Pro Glu Ala Lys Tyr Tyr Ser Thr Pro Gln Val
435 440 445

His Val Ala Ile Ser Gln Ala Leu Ala Asn Phe Ser Ile Arg Glu Val
450 455 460

Arg Ser Val Val Lys Asp Glu Gly Leu Leu Ile Tyr Ala Gly Gly Asp
465 470 475 480

Asp Val Leu Ala Ile Leu Pro Val Asp Lys Ala Leu Glu Val Ala Tyr
485 490 495

Lys Ile Arg Lys Glu Phe Gly Lys Ser Phe Glu Asn Gly Ser Leu Leu
500 505 510

Pro Gly Trp Lys Leu Ser Ala Gly Ile Leu Ile Val His Tyr Lys His
515 520 525

Pro Leu Tyr Asp Ala Leu Glu Lys Ala Arg Asp Leu Leu Asn Asn Lys
530 535 540

Ala Lys Asn Val Pro Gly Lys Asp Thr Leu Ala Ile Gly Leu Leu Lys
545 550 555 560

Arg Ser Gly Ser Tyr Tyr Ile Ser Leu Val Gly Trp Glu Leu Ile Arg
565 570 575

Val Phe Tyr Asn Ser Glu Leu Arg Lys Lys Leu Leu Glu Glu Lys Gly
580 585 590

Gly Val Gly Lys Arg Phe Ile Tyr His Val Leu Arg Glu Val Asp Thr
595 600 605

Trp Pro Lys Val Gly Ile Asp Glu Met Leu Lys Phe Glu Val Ile Arg
610 615 620

His Ile Arg Gly Arg Asn Lys Glu Glu Thr Lys Glu Leu Arg Glu Lys
625 630 635 640

Ile Tyr Gly Glu Ile Lys Asp Leu Leu Glu His Val Arg Gly Asn Asn
645 650 655

Glu Val Glu Lys Val Arg Gly Leu Phe Thr Phe Leu Lys Ile Ile Thr
660 665 670

Asp Ala Glu Val Phe Pro Leu Glu His His His His His His
675 680 685

<210> 1175
<211> 169
<212> PRT

<213> Thermus thermophilus

<400> 1175

Met Ser Pro Gly Glu Arg Phe Leu Asp Trp Leu Lys Arg Leu Gln Gly
1 5 10 15

Gln Lys Ala Trp Thr Ala Ala Arg Ala Ala Phe Arg Arg Ser Leu Ala
20 25 30

Phe Pro Pro Gly Ala Tyr Pro Arg Ala Met Pro Tyr Val Glu Pro Phe
35 40 45

Leu Ala Lys Gly Asp Trp Arg Gln Glu Glu Arg Glu Ala His Tyr Leu
50 55 60

Val Ala Ala Leu Tyr Ala Leu Lys Asp Gly Asp His Gln Val Gly Arg
65 70 75 80

Thr Leu Ala Arg Ala Leu Trp Glu Lys Ala Gln Gly Ser Ala Ser Val
85 90 95

Glu Lys Arg Phe Leu Ala Leu Leu Glu Ala Asp Arg Asp Gln Ile Ala
100 105 110

Phe Arg Leu Arg Gln Ala Val Ala Leu Val Glu Gly Gly Ile Asp Phe
115 120 125

Ala Arg Leu Leu Asp Asp Leu Leu Arg Trp Phe Ser Pro Glu Arg His
130 135 140

Val Gln Ala Arg Trp Ala Arg Glu Tyr Tyr Gly Ala Gly Ala Ser Glu
145 150 155 160

Glu Glu Lys Lys Lys Glu Val Glu Ala
165

<210> 1176
<211> 114
<212> PRT
<213> Thermus thermophilus

<400> 1176

Met Arg Thr Arg Ser Gln Val Trp Ala Gln Lys Ala Tyr Glu Lys Val
1 5 10 15

Arg Glu Ala Ala Lys Gly Glu Gly Arg Gly Glu Tyr Arg Asp Met Ala
20 25 30

Leu Lys Leu Pro Val Leu Val Arg Gln Ala Gly Leu Ser Gln Ala Leu
35 40 45

Ala Phe Val Asp Ser Arg Gly Lys Glu Ala His Lys Ala Leu Gly Asn
50 55 60

Asp Leu Ala Gln Val Leu Gly Tyr Arg Asp Leu Arg Glu Leu Ala Glu
65 70 75 80

Ala Ala Arg Glu Ala Glu Leu Leu Gln Tyr Leu Arg Leu Thr Arg Glu
85 90 95

Val Leu Ala Ala Ala Glu Trp Phe Lys Arg Phe Ala Gln Ala Leu Ile
100 105 110

Glu Glu

<210> 1177
<211> 155
<212> PRT
<213> Archaeoglobus fulgidus

<400> 1177

Met Asp Ile Arg Glu Ile Glu Gln Glu Arg Ala Ser Phe Ala Phe Lys
1 5 10 15

Val Val Ser Asp Ile Lys Asp Lys Tyr Ser Gln Asn Lys Lys Val Gln
20 25 30

Gly Lys Tyr Ser Ser Tyr Ala Glu Lys Ala Pro Thr Ile Ile Leu Asn
35 40 45

Asn Gly Leu Gly Ala Thr Leu Ala Phe Phe Leu Ser Lys Leu Glu Lys
50 55 60

Pro Ile Asp Asp Val Asp Tyr Lys Ser Ile Asn Pro Glu Ser Phe Gly
65 70 75 80

Asn Ala Glu Asn Ile Ala Tyr Ala Phe Leu Tyr Lys His Leu Ser Thr
85 90 95

Trp Leu Ala Glu Gly Asn Gly Lys Asp Ser Ala Phe Ser Gly Leu Thr
100 105 110

Asn Gly Glu Asp Pro Leu Lys Tyr Ile Met Glu Lys Thr Ala Ile Asp
115 120 125

Val Ala Ile Ser Thr Glu Glu Ala Leu Ser Ile Leu Asn Trp Ile Lys
130 135 140

Lys Phe Ala Lys Ala Met Leu Glu Glu Glu Leu
145 150 155

<210> 1178
<211> 1302
<212> PRT
<213> Francisella tularensis

<400> 1178

Ser Asn Ala Ser Ile Tyr Gln Glu Phe Val Asn Lys Tyr Ser Leu Ser
1 5 10 15

Lys Thr Leu Arg Phe Glu Leu Ile Pro Gln Gly Lys Thr Leu Glu Asn
20 25 30

Ile Lys Ala Arg Gly Leu Ile Leu Asp Asp Glu Lys Arg Ala Lys Asp
35 40 45

Tyr Lys Lys Ala Lys Gln Ile Ile Asp Lys Tyr His Gln Phe Phe Ile
50 55 60

Glu Glu Ile Leu Ser Ser Val Cys Ile Ser Glu Asp Leu Leu Gln Asn
65 70 75 80

Tyr Ser Asp Val Tyr Phe Lys Leu Lys Lys Ser Asp Asp Asp Asn Leu
85 90 95

Gln Lys Asp Phe Lys Ser Ala Lys Asp Thr Ile Lys Lys Gln Ile Ser
100 105 110

Glu Tyr Ile Lys Asp Ser Glu Lys Phe Lys Asn Leu Phe Asn Gln Asn
115 120 125

Leu Ile Asp Ala Lys Lys Gly Gln Glu Ser Asp Leu Ile Leu Trp Leu
130 135 140

Lys Gln Ser Lys Asp Asn Gly Ile Glu Leu Phe Lys Ala Asn Ser Asp
145 150 155 160

Ile Thr Asp Ile Asp Glu Ala Leu Glu Ile Ile Lys Ser Phe Lys Gly
165 170 175

Trp Thr Thr Tyr Phe Lys Gly Phe His Glu Asn Arg Lys Asn Val Tyr
180 185 190

Ser Ser Asn Asp Ile Pro Thr Ser Ile Ile Tyr Arg Ile Val Asp Asp
195 200 205

Asn Leu Pro Lys Phe Leu Glu Asn Lys Ala Lys Tyr Glu Ser Leu Lys
210 215 220

Asp Lys Ala Pro Glu Ala Ile Asn Tyr Glu Gln Ile Lys Lys Asp Leu
225 230 235 240

Ala Glu Glu Leu Thr Phe Asp Ile Asp Tyr Lys Thr Ser Glu Val Asn
245 250 255

Gln Arg Val Phe Ser Leu Asp Glu Val Phe Glu Ile Ala Asn Phe Asn
260 265 270

Asn Tyr Leu Asn Gln Ser Gly Ile Thr Lys Phe Asn Thr Ile Ile Gly
275 280 285

Gly Lys Phe Val Asn Gly Glu Asn Thr Lys Arg Lys Gly Ile Asn Glu
290 295 300

Tyr Ile Asn Leu Tyr Ser Gln Gln Ile Asn Asp Lys Thr Leu Lys Lys
305 310 315 320

Tyr Lys Met Ser Val Leu Phe Lys Gln Ile Leu Ser Asp Thr Glu Ser
325 330 335

Lys Ser Phe Val Ile Asp Lys Leu Glu Asp Asp Ser Asp Val Val Thr
340 345 350

Thr Met Gln Ser Phe Tyr Glu Gln Ile Ala Ala Phe Lys Thr Val Glu
355 360 365

Glu Lys Ser Ile Lys Glu Thr Leu Ser Leu Leu Phe Asp Asp Leu Lys
370 375 380

Ala Gln Lys Leu Asp Leu Ser Lys Ile Tyr Phe Lys Asn Asp Lys Ser
385 390 395 400

Leu Thr Asp Leu Ser Gln Gln Val Phe Asp Asp Tyr Ser Val Ile Gly
405 410 415

Thr Ala Val Leu Glu Tyr Ile Thr Gln Gln Ile Ala Pro Lys Asn Leu
420 425 430

Asp Asn Pro Ser Lys Lys Glu Gln Glu Leu Ile Ala Lys Lys Thr Glu
435 440 445

Lys Ala Lys Tyr Leu Ser Leu Glu Thr Ile Lys Leu Ala Leu Glu Glu
    450                 455                 460

Phe Asn Lys His Arg Asp Ile Asp Lys Gln Cys Arg Phe Glu Glu Ile
465                 470                 475                 480

Leu Ala Asn Phe Ala Ala Ile Pro Met Ile Phe Asp Glu Ile Ala Gln
                485                 490                 495

Asn Lys Asp Asn Leu Ala Gln Ile Ser Ile Lys Tyr Gln Asn Gln Gly
            500                 505                 510

Lys Lys Asp Leu Leu Gln Ala Ser Ala Glu Asp Asp Val Lys Ala Ile
        515                 520                 525

Lys Asp Leu Leu Asp Gln Thr Asn Asn Leu Leu His Lys Leu Lys Ile
    530                 535                 540

Phe His Ile Ser Gln Ser Glu Asp Lys Ala Asn Ile Leu Asp Lys Asp
545                 550                 555                 560

Glu His Phe Tyr Leu Val Phe Glu Glu Cys Tyr Phe Glu Leu Ala Asn
                565                 570                 575

Ile Val Pro Leu Tyr Asn Lys Ile Arg Asn Tyr Ile Thr Gln Lys Pro
            580                 585                 590

Tyr Ser Asp Glu Lys Phe Lys Leu Asn Phe Glu Asn Ser Thr Leu Ala
        595                 600                 605

Asn Gly Trp Asp Lys Asn Lys Glu Pro Asp Asn Thr Ala Ile Leu Phe
    610                 615                 620

Ile Lys Asp Asp Lys Tyr Tyr Leu Gly Val Met Asn Lys Lys Asn Asn
625                 630                 635                 640

Lys Ile Phe Asp Asp Lys Ala Ile Lys Glu Asn Lys Gly Glu Gly Tyr
                645                 650                 655

Lys Lys Ile Val Tyr Lys Leu Leu Pro Gly Ala Asn Lys Met Leu Pro
            660                 665                 670

Lys Val Phe Phe Ser Ala Lys Ser Ile Lys Phe Tyr Asn Pro Ser Glu
        675                 680                 685

Asp Ile Leu Arg Ile Arg Asn His Ser Thr His Thr Lys Asn Gly Ser
    690                 695                 700

Pro Gln Lys Gly Tyr Glu Lys Phe Glu Phe Asn Ile Glu Asp Cys Arg
705 710 715 720

Lys Phe Ile Asp Phe Tyr Lys Gln Ser Ile Ser Lys His Pro Glu Trp
725 730 735

Lys Asp Phe Gly Phe Arg Phe Ser Asp Thr Gln Arg Tyr Asn Ser Ile
740 745 750

Asp Glu Phe Tyr Arg Glu Val Glu Asn Gln Gly Tyr Lys Leu Thr Phe
755 760 765

Glu Asn Ile Ser Glu Ser Tyr Ile Asp Ser Val Val Asn Gln Gly Lys
770 775 780

Leu Tyr Leu Phe Gln Ile Tyr Asn Lys Asp Phe Ser Ala Tyr Ser Lys
785 790 795 800

Gly Arg Pro Asn Leu His Thr Leu Tyr Trp Lys Ala Leu Phe Asp Glu
805 810 815

Arg Asn Leu Gln Asp Val Val Tyr Lys Leu Asn Gly Glu Ala Glu Leu
820 825 830

Phe Tyr Arg Lys Gln Ser Ile Pro Lys Lys Ile Thr His Pro Ala Lys
835 840 845

Glu Ala Ile Ala Asn Lys Asn Lys Asp Asn Pro Lys Lys Glu Ser Val
850 855 860

Phe Glu Tyr Asp Leu Ile Lys Asp Lys Arg Phe Thr Glu Asp Lys Phe
865 870 875 880

Phe Phe His Cys Pro Ile Thr Ile Asn Phe Lys Ser Ser Gly Ala Asn
885 890 895

Lys Phe Asn Asp Glu Ile Asn Leu Leu Leu Lys Glu Lys Ala Asn Asp
900 905 910

Val His Ile Leu Ser Ile Asp Arg Gly Glu Arg His Leu Ala Tyr Tyr
915 920 925

Thr Leu Val Asp Gly Lys Gly Asn Ile Ile Lys Gln Asp Thr Phe Asn
930 935 940

Ile Ile Gly Asn Asp Arg Met Lys Thr Asn Tyr His Asp Lys Leu Ala

945 950 955 960

Ala Ile Glu Lys Asp Arg Asp Ser Ala Arg Lys Asp Trp Lys Lys Ile
965 970 975

Asn Asn Ile Lys Glu Met Lys Glu Gly Tyr Leu Ser Gln Val Val His
980 985 990

Glu Ile Ala Lys Leu Val Ile Glu Tyr Asn Ala Ile Val Val Phe Gln
995 1000 1005

Asp Leu Asn Phe Gly Phe Lys Arg Gly Arg Phe Lys Val Glu Lys
1010 1015 1020

Gln Val Tyr Gln Lys Leu Glu Lys Met Leu Ile Glu Lys Leu Asn
1025 1030 1035

Tyr Leu Val Phe Lys Asp Asn Glu Phe Asp Lys Thr Gly Gly Val
1040 1045 1050

Leu Arg Ala Tyr Gln Leu Thr Ala Pro Phe Glu Thr Phe Lys Lys
1055 1060 1065

Met Gly Lys Gln Thr Gly Ile Ile Tyr Tyr Val Pro Ala Gly Phe
1070 1075 1080

Thr Ser Lys Ile Cys Pro Val Thr Gly Phe Val Asn Gln Leu Tyr
1085 1090 1095

Pro Lys Tyr Glu Ser Val Ser Lys Ser Gln Glu Phe Phe Ser Lys
1100 1105 1110

Phe Asp Lys Ile Cys Tyr Asn Leu Asp Lys Gly Tyr Phe Glu Phe
1115 1120 1125

Ser Phe Asp Tyr Lys Asn Phe Gly Asp Lys Ala Ala Lys Gly Lys
1130 1135 1140

Trp Thr Ile Ala Ser Phe Gly Ser Arg Leu Ile Asn Phe Arg Asn
1145 1150 1155

Ser Asp Lys Asn His Asn Trp Asp Thr Arg Glu Val Tyr Pro Thr
1160 1165 1170

Lys Glu Leu Glu Lys Leu Leu Lys Asp Tyr Ser Ile Glu Tyr Gly
1175 1180 1185

His Gly Glu Cys Ile Lys Ala Ala Ile Cys Gly Glu Ser Asp Lys
1190 1195 1200

Lys Phe Phe Ala Lys Leu Thr Ser Val Leu Asn Thr Ile Leu Gln
1205 1210 1215

Met Ala Asn Ser Lys Thr Gly Thr Glu Leu Asp Tyr Leu Ile Ser
1220 1225 1230

Pro Val Ala Asp Val Asn Gly Asn Phe Phe Asp Ser Arg Gln Ala
1235 1240 1245

Pro Lys Asn Met Pro Gln Asp Ala Asp Ala Asn Gly Ala Tyr His
1250 1255 1260

Ile Gly Leu Lys Gly Leu Met Leu Leu Gly Arg Ile Lys Asn Asn
1265 1270 1275

Gln Glu Gly Lys Lys Leu Asn Leu Val Ile Lys Asn Glu Glu Tyr
1280 1285 1290

Phe Glu Phe Val Gln Asn Arg Asn Asn
1295 1300

<210> 1179
<211> 1137
<212> PRT
<213> Alicyclobacillus acidoterrestris

<400> 1179

Met Ala Val Lys Ser Ile Lys Val Lys Leu Arg Leu Asp Asp Met Pro
1 5 10 15

Glu Ile Arg Ala Gly Leu Trp Lys Leu His Lys Glu Val Asn Ala Gly
20 25 30

Val Arg Tyr Tyr Thr Glu Trp Leu Ser Leu Leu Arg Gln Glu Asn Leu
35 40 45

Tyr Arg Arg Ser Pro Asn Gly Asp Gly Glu Gln Glu Cys Asp Lys Thr
50 55 60

Ala Glu Glu Cys Lys Ala Glu Leu Leu Glu Arg Leu Arg Ala Arg Gln
65 70 75 80

Val Glu Asn Gly His Arg Gly Pro Ala Gly Ser Asp Asp Glu Leu Leu
85 90 95

```
Gln Leu Ala Arg Gln Leu Tyr Glu Leu Leu Val Pro Gln Ala Ile Gly
            100                 105                 110

Ala Lys Gly Asp Ala Gln Gln Ile Ala Arg Lys Phe Leu Ser Pro Leu
        115                 120                 125

Ala Asp Lys Asp Ala Val Gly Gly Leu Gly Ile Ala Lys Ala Gly Asn
    130                 135                 140

Lys Pro Arg Trp Val Arg Met Arg Glu Ala Gly Glu Pro Gly Trp Glu
145                 150                 155                 160

Glu Glu Lys Glu Lys Ala Glu Thr Arg Lys Ser Ala Asp Arg Thr Ala
                165                 170                 175

Asp Val Leu Arg Ala Leu Ala Asp Phe Gly Leu Lys Pro Leu Met Arg
            180                 185                 190

Val Tyr Thr Asp Ser Glu Met Ser Ser Val Glu Trp Lys Pro Leu Arg
        195                 200                 205

Lys Gly Gln Ala Val Arg Thr Trp Asp Arg Asp Met Phe Gln Gln Ala
    210                 215                 220

Ile Glu Arg Met Met Ser Trp Glu Ser Trp Asn Gln Arg Val Gly Gln
225                 230                 235                 240

Glu Tyr Ala Lys Leu Val Glu Gln Lys Asn Arg Phe Glu Gln Lys Asn
                245                 250                 255

Phe Val Gly Gln Glu His Leu Val His Leu Val Asn Gln Leu Gln Gln
            260                 265                 270

Asp Met Lys Glu Ala Ser Pro Gly Leu Glu Ser Lys Glu Gln Thr Ala
        275                 280                 285

His Tyr Val Thr Gly Arg Ala Leu Arg Gly Ser Asp Lys Val Phe Glu
    290                 295                 300

Lys Trp Gly Lys Leu Ala Pro Asp Ala Pro Phe Asp Leu Tyr Asp Ala
305                 310                 315                 320

Glu Ile Lys Asn Val Gln Arg Arg Asn Thr Arg Arg Phe Gly Ser His
                325                 330                 335

Asp Leu Phe Ala Lys Leu Ala Glu Pro Glu Tyr Gln Ala Leu Trp Arg
            340                 345                 350
```

Glu Asp Ala Ser Phe Leu Thr Arg Tyr Ala Val Tyr Asn Ser Ile Leu
355 360 365

Arg Lys Leu Asn His Ala Lys Met Phe Ala Thr Phe Thr Leu Pro Asp
370 375 380

Ala Thr Ala His Pro Ile Trp Thr Arg Phe Asp Lys Leu Gly Gly Asn
385 390 395 400

Leu His Gln Tyr Thr Phe Leu Phe Asn Glu Phe Gly Glu Arg Arg His
405 410 415

Ala Ile Arg Phe His Lys Leu Leu Lys Val Glu Asn Gly Val Ala Arg
420 425 430

Glu Val Asp Asp Val Thr Val Pro Ile Ser Met Ser Glu Gln Leu Asp
435 440 445

Asn Leu Leu Pro Arg Asp Pro Asn Glu Pro Ile Ala Leu Tyr Phe Arg
450 455 460

Asp Tyr Gly Ala Glu Gln His Phe Thr Gly Glu Phe Gly Gly Ala Lys
465 470 475 480

Ile Gln Cys Arg Arg Asp Gln Leu Ala His Met His Arg Arg Arg Gly
485 490 495

Ala Arg Asp Val Tyr Leu Asn Val Ser Val Arg Val Gln Ser Gln Ser
500 505 510

Glu Ala Arg Gly Glu Arg Arg Pro Pro Tyr Ala Ala Val Phe Arg Leu
515 520 525

Val Gly Asp Asn His Arg Ala Phe Val His Phe Asp Lys Leu Ser Asp
530 535 540

Tyr Leu Ala Glu His Pro Asp Asp Gly Lys Leu Gly Ser Glu Gly Leu
545 550 555 560

Leu Ser Gly Leu Arg Val Met Ser Val Asp Leu Gly Leu Arg Thr Ser
565 570 575

Ala Ser Ile Ser Val Phe Arg Val Ala Arg Lys Asp Glu Leu Lys Pro
580 585 590

Asn Ser Lys Gly Arg Val Pro Phe Phe Phe Pro Ile Lys Gly Asn Asp
595 600 605

```
Asn Leu Val Ala Val His Glu Arg Ser Gln Leu Leu Lys Leu Pro Gly
    610                 615                 620

Glu Thr Glu Ser Lys Asp Leu Arg Ala Ile Arg Glu Glu Arg Gln Arg
625                 630                 635                 640

Thr Leu Arg Gln Leu Arg Thr Gln Leu Ala Tyr Leu Arg Leu Leu Val
                645                 650                 655

Arg Cys Gly Ser Glu Asp Val Gly Arg Arg Glu Arg Ser Trp Ala Lys
            660                 665                 670

Leu Ile Glu Gln Pro Val Asp Ala Ala Asn His Met Thr Pro Asp Trp
        675                 680                 685

Arg Glu Ala Phe Glu Asn Glu Leu Gln Lys Leu Lys Ser Leu His Gly
    690                 695                 700

Ile Cys Ser Asp Lys Glu Trp Met Asp Ala Val Tyr Glu Ser Val Arg
705                 710                 715                 720

Arg Val Trp Arg His Met Gly Lys Gln Val Arg Asp Trp Arg Lys Asp
                725                 730                 735

Val Arg Ser Gly Glu Arg Pro Lys Ile Arg Gly Tyr Ala Lys Asp Val
            740                 745                 750

Val Gly Gly Asn Ser Ile Glu Gln Ile Glu Tyr Leu Glu Arg Gln Tyr
        755                 760                 765

Lys Phe Leu Lys Ser Trp Ser Phe Phe Gly Lys Val Ser Gly Gln Val
    770                 775                 780

Ile Arg Ala Glu Lys Gly Ser Arg Phe Ala Ile Thr Leu Arg Glu His
785                 790                 795                 800

Ile Asp His Ala Lys Glu Asp Arg Leu Lys Lys Leu Ala Asp Arg Ile
                805                 810                 815

Ile Met Glu Ala Leu Gly Tyr Val Tyr Ala Leu Asp Glu Arg Gly Lys
            820                 825                 830

Gly Lys Trp Val Ala Lys Tyr Pro Pro Cys Gln Leu Ile Leu Leu Glu
        835                 840                 845

Glu Leu Ser Glu Tyr Gln Phe Asn Asn Asp Arg Pro Pro Ser Glu Asn
```

850 855 860

Asn Gln Leu Met Gln Trp Ser His Arg Gly Val Phe Gln Glu Leu Ile
865 870 875 880

Asn Gln Ala Gln Val His Asp Leu Leu Val Gly Thr Met Tyr Ala Ala
885 890 895

Phe Ser Ser Arg Phe Asp Ala Arg Thr Gly Ala Pro Gly Ile Arg Cys
900 905 910

Arg Arg Val Pro Ala Arg Cys Thr Gln Glu His Asn Pro Glu Pro Phe
915 920 925

Pro Trp Trp Leu Asn Lys Phe Val Val Glu His Thr Leu Asp Ala Cys
930 935 940

Pro Leu Arg Ala Asp Asp Leu Ile Pro Thr Gly Glu Gly Glu Ile Phe
945 950 955 960

Val Ser Pro Phe Ser Ala Glu Glu Gly Asp Phe His Gln Ile His Ala
965 970 975

Asp Leu Asn Ala Ala Gln Asn Leu Gln Gln Arg Leu Trp Ser Asp Phe
980 985 990

Asp Ile Ser Gln Ile Arg Leu Arg Cys Asp Trp Gly Glu Val Asp Gly
995 1000 1005

Glu Leu Val Leu Ile Pro Arg Leu Thr Gly Lys Arg Thr Ala Asp
1010 1015 1020

Ser Tyr Ser Asn Lys Val Phe Tyr Thr Asn Thr Gly Val Thr Tyr
1025 1030 1035

Tyr Glu Arg Glu Arg Gly Lys Lys Arg Arg Lys Val Phe Ala Gln
1040 1045 1050

Glu Lys Leu Ser Glu Glu Glu Ala Glu Leu Leu Val Glu Ala Asp
1055 1060 1065

Glu Ala Arg Glu Lys Ser Val Val Leu Met Arg Asp Pro Ser Gly
1070 1075 1080

Ile Ile Asn Arg Gly Asn Trp Thr Arg Gln Lys Glu Phe Trp Ser
1085 1090 1095

Met Val Asn Gln Arg Ile Glu Gly Tyr Leu Val Lys Gln Ile Arg
1100 1105 1110

Ser Arg Val Pro Leu Gln Asp Ser Ala Cys Glu Asn Thr Gly Asp
1115 1120 1125

Ile Leu Glu His His His His His His
1130 1135

<210> 1180
<211> 820
<212> PRT
<213> Deltaproteobacteria bacterium

<400> 1180

Met Glu Lys Arg Ile Asn Lys Ile Arg Lys Lys Leu Ser Ala Asp Asn
1 5 10 15

Ala Thr Lys Pro Val Ser Arg Ser Gly Pro Met Lys Thr Leu Leu Val
20 25 30

Arg Val Met Thr Asp Asp Leu Lys Lys Arg Leu Glu Lys Arg Arg Lys
35 40 45

Lys Pro Glu Val Met Pro Gln Val Ile Ser Asn Asn Ala Ala Asn Asn
50 55 60

Leu Arg Met Leu Leu Asp Asp Tyr Thr Lys Met Lys Glu Ala Ile Leu
65 70 75 80

Gln Val Tyr Trp Gln Glu Phe Lys Asp Asp His Val Gly Leu Met Cys
85 90 95

Lys Phe Ala Gln Pro Ala Ser Gly Lys Phe Gly Gln Arg Ala Leu Asp
100 105 110

Phe Tyr Ser Ile His Val Thr Lys Glu Ser Thr His Pro Val Lys Pro
115 120 125

Leu Ala Gln Ile Ala Gly Asn Arg Tyr Ala Ser Gly Pro Val Gly Lys
130 135 140

Ala Leu Ser Asp Ala Cys Met Gly Thr Ile Ala Ser Phe Leu Ser Lys
145 150 155 160

Tyr Gln Asp Ile Ile Ile Glu His Gln Lys Val Val Lys Gly Asn Gln
165 170 175

Lys Arg Leu Glu Ser Leu Arg Glu Leu Ala Gly Lys Glu Asn Leu Glu
180 185 190

Tyr Pro Ser Val Thr Leu Pro Pro Gln Pro His Thr Lys Glu Gly Val
195 200 205

Asp Ala Tyr Asn Glu Val Ile Ala Arg Val Arg Met Trp Val Asn Leu
210 215 220

Asn Leu Trp Gln Lys Leu Lys Leu Ser Arg Asp Asp Ala Lys Pro Leu
225 230 235 240

Leu Arg Leu Lys Gly Phe Pro Ser Phe Pro Val Val Glu Arg Arg Glu
245 250 255

Asn Glu Val Asp Trp Trp Asn Thr Ile Asn Glu Val Lys Lys Leu Ile
260 265 270

Asp Ala Lys Arg Asp Met Gly Arg Val Phe Trp Ser Gly Val Thr Ala
275 280 285

Glu Lys Arg Asn Thr Ile Leu Glu Gly Tyr Asn Tyr Leu Pro Asn Glu
290 295 300

Asn Asp His Lys Lys Arg Glu Gly Ser Leu Glu Asn Pro Lys Lys Pro
305 310 315 320

Ala Lys Arg Gln Phe Gly Asp Leu Leu Leu Tyr Leu Glu Lys Lys Tyr
325 330 335

Ala Gly Asp Trp Gly Lys Val Phe Asp Glu Ala Trp Glu Arg Ile Asp
340 345 350

Lys Lys Ile Ala Gly Leu Thr Ser His Ile Glu Arg Glu Glu Ala Arg
355 360 365

Asn Ala Glu Asp Ala Gln Ser Lys Ala Val Leu Thr Asp Trp Leu Arg
370 375 380

Ala Lys Ala Ser Phe Val Leu Glu Arg Leu Lys Glu Met Asp Glu Lys
385 390 395 400

Glu Phe Tyr Ala Cys Glu Ile Gln Leu Gln Lys Trp Tyr Gly Asp Leu
405 410 415

Arg Gly Asn Pro Phe Ala Val Glu Ala Glu Asn Arg Val Val Asp Ile
420 425 430

Ser Gly Phe Ser Ile Gly Ser Asp Gly His Ser Ile Gln Tyr Arg Asn
435 440 445

Leu Leu Ala Trp Lys Tyr Leu Glu Asn Gly Lys Arg Glu Phe Tyr Leu
450 455 460

Leu Met Asn Tyr Gly Lys Lys Gly Arg Ile Arg Phe Thr Asp Gly Thr
465 470 475 480

Asp Ile Lys Lys Ser Gly Lys Trp Gln Gly Leu Leu Tyr Gly Gly Gly
485 490 495

Lys Ala Lys Val Ile Asp Leu Thr Phe Asp Pro Asp Asp Glu Gln Leu
500 505 510

Ile Ile Leu Pro Leu Ala Phe Gly Thr Arg Gln Gly Arg Glu Phe Ile
515 520 525

Trp Asn Asp Leu Leu Ser Leu Glu Thr Gly Leu Ile Lys Leu Ala Asn
530 535 540

Gly Arg Val Ile Glu Lys Thr Ile Tyr Asn Lys Lys Ile Gly Arg Asp
545 550 555 560

Glu Pro Ala Leu Phe Val Ala Leu Thr Phe Glu Arg Arg Glu Val Val
565 570 575

Asp Pro Ser Asn Ile Lys Pro Val Asn Leu Ile Gly Val Ala Arg Gly
580 585 590

Glu Asn Ile Pro Ala Val Ile Ala Leu Thr Asp Pro Glu Gly Cys Pro
595 600 605

Leu Pro Glu Phe Lys Asp Ser Ser Gly Gly Pro Thr Asp Ile Leu Arg
610 615 620

Ile Gly Glu Gly Tyr Lys Glu Lys Gln Arg Ala Ile Gln Ala Ala Lys
625 630 635 640

Glu Val Glu Gln Arg Arg Ala Gly Gly Tyr Ser Arg Lys Phe Ala Ser
645 650 655

Lys Ser Arg Asn Leu Ala Asp Asp Met Val Arg Asn Ser Ala Arg Asp
660 665 670

Leu Phe Tyr His Ala Val Thr His Asp Ala Val Leu Val Phe Ala Asn
675 680 685

Leu Ser Arg Gly Phe Gly Arg Gln Gly Lys Arg Thr Phe Met Thr Glu
690 695 700

Arg Gln Tyr Thr Lys Met Glu Asp Trp Leu Thr Ala Lys Leu Ala Tyr
705 710 715 720

Glu Gly Leu Thr Ser Lys Thr Tyr Leu Ser Lys Thr Leu Ala Gln Tyr
725 730 735

Thr Ser Lys Thr Cys Ser Asn Cys Gly Phe Arg Phe Ser His Arg Pro
740 745 750

Val Gln Glu Gln Phe Val Cys Leu Asp Cys Gly His Glu Val His Ala
755 760 765

Ala Glu Gln Ala Ala Leu Asn Ile Ala Arg Ser Trp Leu Phe Leu Asn
770 775 780

Ser Asn Ser Thr Glu Phe Lys Ser Tyr Lys Ser Gly Lys Gln Pro Phe
785 790 795 800

Val Gly Ala Trp Gln Ala Phe Tyr Lys Arg Arg Leu Lys Glu Val Trp
805 810 815

Lys Pro Asn Ala
820

<210> 1181
<211> 1160
<212> PRT
<213> Leptotrichia buccalis

<400> 1181

Ser Met Lys Val Thr Lys Val Gly Gly Ile Ser His Lys Lys Tyr Thr
1 5 10 15

Ser Glu Gly Arg Leu Val Lys Ser Glu Ser Glu Glu Asn Arg Thr Asp
20 25 30

Glu Arg Leu Ser Ala Leu Leu Asn Met Arg Leu Asp Met Tyr Ile Lys
35 40 45

Asn Pro Ser Ser Thr Glu Thr Lys Glu Asn Gln Lys Arg Ile Gly Lys
50 55 60

Leu Lys Lys Phe Phe Ser Asn Lys Met Val Tyr Leu Lys Asp Asn Thr
65 70 75 80

Leu Ser Leu Lys Asn Gly Lys Lys Glu Asn Ile Asp Arg Glu Tyr Ser
85 90 95

Glu Thr Asp Ile Leu Glu Ser Asp Val Arg Asp Lys Lys Asn Phe Ala
100 105 110

Val Leu Lys Lys Ile Tyr Leu Asn Glu Asn Val Asn Ser Glu Glu Leu
115 120 125

Glu Val Phe Arg Asn Asp Ile Lys Lys Lys Leu Asn Lys Ile Asn Ser
130 135 140

Leu Lys Tyr Ser Phe Glu Lys Asn Lys Ala Asn Tyr Gln Lys Ile Asn
145 150 155 160

Glu Asn Asn Ile Glu Lys Val Glu Gly Lys Ser Lys Arg Asn Ile Ile
165 170 175

Tyr Asp Tyr Tyr Arg Glu Ser Ala Lys Arg Asp Ala Tyr Val Ser Asn
180 185 190

Val Lys Glu Ala Phe Asp Lys Leu Tyr Lys Glu Glu Asp Ile Ala Lys
195 200 205

Leu Val Leu Glu Ile Glu Asn Leu Thr Lys Leu Glu Lys Tyr Lys Ile
210 215 220

Arg Glu Phe Tyr His Glu Ile Ile Gly Arg Lys Asn Asp Lys Glu Asn
225 230 235 240

Phe Ala Lys Ile Ile Tyr Glu Glu Ile Gln Asn Val Asn Asn Met Lys
245 250 255

Glu Leu Ile Glu Lys Val Pro Asp Met Ser Glu Leu Lys Lys Ser Gln
260 265 270

Val Phe Tyr Lys Tyr Tyr Leu Asp Lys Glu Glu Leu Asn Asp Lys Asn
275 280 285

Ile Lys Tyr Ala Phe Cys His Phe Val Glu Ile Glu Met Ser Gln Leu
290 295 300

Leu Lys Asn Tyr Val Tyr Lys Arg Leu Ser Asn Ile Ser Asn Asp Lys
305 310 315 320

Ile Lys Arg Ile Phe Glu Tyr Gln Asn Leu Lys Lys Leu Ile Glu Asn

325 330 335

Lys Leu Leu Asn Lys Leu Asp Thr Tyr Val Arg Asn Cys Gly Lys Tyr
340 345 350

Asn Tyr Tyr Leu Gln Asp Gly Glu Ile Ala Thr Ser Asp Phe Ile Ala
355 360 365

Arg Asn Arg Gln Asn Glu Ala Phe Leu Arg Asn Ile Ile Gly Val Ser
370 375 380

Ser Val Ala Tyr Phe Ser Leu Arg Asn Ile Leu Glu Thr Glu Asn Glu
385 390 395 400

Asn Asp Ile Thr Gly Arg Met Arg Gly Lys Thr Val Lys Asn Asn Lys
405 410 415

Gly Glu Glu Lys Tyr Val Ser Gly Glu Val Asp Lys Ile Tyr Asn Glu
420 425 430

Asn Lys Lys Asn Glu Val Lys Glu Asn Leu Lys Met Phe Tyr Ser Tyr
435 440 445

Asp Phe Asn Met Asp Asn Lys Asn Glu Ile Glu Asp Phe Phe Ala Asn
450 455 460

Ile Asp Glu Ala Ile Ser Ser Ile Arg His Gly Ile Val His Phe Asn
465 470 475 480

Leu Glu Leu Glu Gly Lys Asp Ile Phe Ala Phe Lys Asn Ile Ala Pro
485 490 495

Ser Glu Ile Ser Lys Lys Met Phe Gln Asn Glu Ile Asn Glu Lys Lys
500 505 510

Leu Lys Leu Lys Ile Phe Arg Gln Leu Asn Ser Ala Asn Val Phe Arg
515 520 525

Tyr Leu Glu Lys Tyr Lys Ile Leu Asn Tyr Leu Lys Arg Thr Arg Phe
530 535 540

Glu Phe Val Asn Lys Asn Ile Pro Phe Val Pro Ser Phe Thr Lys Leu
545 550 555 560

Tyr Ser Arg Ile Asp Asp Leu Lys Asn Ser Leu Gly Ile Tyr Trp Lys
565 570 575

```
Thr Pro Lys Thr Asn Asp Asp Asn Lys Thr Lys Glu Ile Ile Asp Ala
            580                 585                 590

Gln Ile Tyr Leu Leu Lys Asn Ile Tyr Tyr Gly Glu Phe Leu Asn Tyr
        595                 600                 605

Phe Met Ser Asn Asn Gly Asn Phe Phe Glu Ile Ser Lys Glu Ile Ile
    610                 615                 620

Glu Leu Asn Lys Asn Asp Lys Arg Asn Leu Lys Thr Gly Phe Tyr Lys
625                 630                 635                 640

Leu Gln Lys Phe Glu Asp Ile Gln Glu Lys Ile Pro Lys Glu Tyr Leu
                645                 650                 655

Ala Asn Ile Gln Ser Leu Tyr Met Ile Asn Ala Gly Asn Gln Asp Glu
            660                 665                 670

Glu Glu Lys Asp Thr Tyr Ile Asp Phe Ile Gln Lys Ile Phe Leu Lys
        675                 680                 685

Gly Phe Met Thr Tyr Leu Ala Asn Asn Gly Arg Leu Ser Leu Ile Tyr
    690                 695                 700

Ile Gly Ser Asp Glu Glu Thr Asn Thr Ser Leu Ala Glu Lys Lys Gln
705                 710                 715                 720

Glu Phe Asp Lys Phe Leu Lys Lys Tyr Glu Gln Asn Asn Asn Ile Lys
                725                 730                 735

Ile Pro Tyr Glu Ile Asn Glu Phe Leu Arg Glu Ile Lys Leu Gly Asn
            740                 745                 750

Ile Leu Lys Tyr Thr Glu Arg Leu Asn Met Phe Tyr Leu Ile Leu Lys
        755                 760                 765

Leu Leu Asn His Lys Glu Leu Thr Asn Leu Lys Gly Ser Leu Glu Lys
    770                 775                 780

Tyr Gln Ser Ala Asn Lys Glu Glu Ala Phe Ser Asp Gln Leu Glu Leu
785                 790                 795                 800

Ile Asn Leu Leu Asn Leu Asp Asn Asn Arg Val Thr Glu Asp Phe Glu
                805                 810                 815

Leu Glu Ala Asp Glu Ile Gly Lys Phe Leu Asp Phe Asn Gly Asn Lys
            820                 825                 830
```

Val Lys Asp Asn Lys Glu Leu Lys Lys Phe Asp Thr Asn Lys Ile Tyr
835 840 845

Phe Asp Gly Glu Asn Ile Ile Lys His Arg Ala Phe Tyr Asn Ile Lys
850 855 860

Lys Tyr Gly Met Leu Asn Leu Leu Glu Lys Ile Ala Asp Lys Ala Gly
865 870 875 880

Tyr Lys Ile Ser Ile Glu Glu Leu Lys Lys Tyr Ser Asn Lys Lys Asn
885 890 895

Glu Ile Glu Lys Asn His Lys Met Gln Glu Asn Leu His Arg Lys Tyr
900 905 910

Ala Arg Pro Arg Lys Asp Glu Lys Phe Thr Asp Glu Asp Tyr Glu Ser
915 920 925

Tyr Lys Gln Ala Ile Glu Asn Ile Glu Glu Tyr Thr His Leu Lys Asn
930 935 940

Lys Val Glu Phe Asn Glu Leu Asn Leu Leu Gln Gly Leu Leu Leu Arg
945 950 955 960

Ile Leu His Arg Leu Val Gly Tyr Thr Ser Ile Trp Glu Arg Asp Leu
965 970 975

Arg Phe Arg Leu Lys Gly Glu Phe Pro Glu Asn Gln Tyr Ile Glu Glu
980 985 990

Ile Phe Asn Phe Glu Asn Lys Lys Asn Val Lys Tyr Lys Gly Gly Gln
995 1000 1005

Ile Val Glu Lys Tyr Ile Lys Phe Tyr Lys Glu Leu His Gln Asn
1010 1015 1020

Asp Glu Val Lys Ile Asn Lys Tyr Ser Ser Ala Asn Ile Lys Val
1025 1030 1035

Leu Lys Gln Glu Lys Lys Asp Leu Tyr Ile Ala Asn Tyr Ile Ala
1040 1045 1050

Ala Phe Asn Tyr Ile Pro His Ala Glu Ile Ser Leu Leu Glu Val
1055 1060 1065

Leu Glu Asn Leu Arg Lys Leu Leu Ser Tyr Asp Arg Lys Leu Lys
1070 1075 1080

Asn Ala Val Met Lys Ser Val Val Asp Ile Leu Lys Glu Tyr Gly
1085 1090 1095

Phe Val Ala Thr Phe Lys Ile Gly Ala Asp Lys Lys Ile Gly Ile
1100 1105 1110

Gln Thr Leu Glu Ser Glu Lys Ile Val His Leu Lys Asn Leu Lys
1115 1120 1125

Lys Lys Lys Leu Met Thr Asp Arg Asn Ser Glu Glu Leu Cys Lys
1130 1135 1140

Leu Val Lys Ile Met Phe Glu Tyr Lys Met Glu Glu Lys Lys Ser
1145 1150 1155

Glu Asn
1160

<210> 1182
<211> 1440
<212> PRT
<213> Lachnospiraceae bacterium

<400> 1182

Ser Asn Ala Met Lys Ile Ser Lys Val Arg Glu Glu Asn Arg Gly Ala
1 5 10 15

Lys Leu Thr Val Asn Ala Lys Thr Ala Val Val Ser Glu Asn Arg Ser
20 25 30

Gln Glu Gly Ile Leu Tyr Asn Asp Pro Ser Arg Tyr Gly Lys Ser Arg
35 40 45

Lys Asn Asp Glu Asp Arg Asp Arg Tyr Ile Glu Ser Arg Leu Lys Ser
50 55 60

Ser Gly Lys Leu Tyr Arg Ile Phe Asn Glu Asp Lys Asn Lys Arg Glu
65 70 75 80

Thr Asp Glu Leu Gln Trp Phe Leu Ser Glu Ile Val Lys Lys Ile Asn
85 90 95

Arg Arg Asn Gly Leu Val Leu Ser Asp Met Leu Ser Val Asp Asp Arg
100 105 110

Ala Phe Glu Lys Ala Phe Glu Lys Tyr Ala Glu Leu Ser Tyr Thr Asn
115 120 125

Arg Arg Asn Lys Val Ser Gly Ser Pro Ala Phe Glu Thr Cys Gly Val
130 135 140

Asp Ala Ala Thr Ala Glu Arg Leu Lys Gly Ile Ile Ser Glu Thr Asn
145 150 155 160

Phe Ile Asn Arg Ile Lys Asn Asn Ile Asp Asn Lys Val Ser Glu Asp
165 170 175

Ile Ile Asp Arg Ile Ile Ala Lys Tyr Leu Lys Lys Ser Leu Cys Arg
180 185 190

Glu Arg Val Lys Arg Gly Leu Lys Lys Leu Leu Met Asn Ala Phe Asp
195 200 205

Leu Pro Tyr Ser Asp Pro Asp Ile Asp Val Gln Arg Asp Phe Ile Asp
210 215 220

Tyr Val Leu Glu Asp Phe Tyr His Val Arg Ala Lys Ser Gln Val Ser
225 230 235 240

Arg Ser Ile Lys Asn Met Asn Met Pro Val Gln Pro Glu Gly Asp Gly
245 250 255

Lys Phe Ala Ile Thr Val Ser Lys Gly Gly Thr Glu Ser Gly Asn Lys
260 265 270

Arg Ser Ala Glu Lys Glu Ala Phe Lys Lys Phe Leu Ser Asp Tyr Ala
275 280 285

Ser Leu Asp Glu Arg Val Arg Asp Asp Met Leu Arg Arg Met Arg Arg
290 295 300

Leu Val Val Leu Tyr Phe Tyr Gly Ser Asp Asp Ser Lys Leu Ser Asp
305 310 315 320

Val Asn Glu Lys Phe Asp Val Trp Glu Asp His Ala Ala Arg Arg Val
325 330 335

Asp Asn Arg Glu Phe Ile Lys Leu Pro Leu Glu Asn Lys Leu Ala Asn
340 345 350

Gly Lys Thr Asp Lys Asp Ala Glu Arg Ile Arg Lys Asn Thr Val Lys
355 360 365

Glu Leu Tyr Arg Asn Gln Asn Ile Gly Cys Tyr Arg Gln Ala Val Lys

370 375 380

Ala Val Glu Glu Asp Asn Asn Gly Arg Tyr Phe Asp Asp Lys Met Leu
385 390 395 400

Asn Met Phe Phe Ile His Arg Ile Glu Tyr Gly Val Glu Lys Ile Tyr
405 410 415

Ala Asn Leu Lys Gln Val Thr Glu Phe Lys Ala Arg Thr Gly Tyr Leu
420 425 430

Ser Glu Lys Ile Trp Lys Asp Leu Ile Asn Tyr Ile Ser Ile Lys Tyr
435 440 445

Ile Ala Met Gly Lys Ala Val Tyr Asn Tyr Ala Met Asp Glu Leu Asn
450 455 460

Ala Ser Asp Lys Lys Glu Ile Glu Leu Gly Lys Ile Ser Glu Glu Tyr
465 470 475 480

Leu Ser Gly Ile Ser Ser Phe Asp Tyr Glu Leu Ile Lys Ala Glu Glu
485 490 495

Met Leu Gln Arg Glu Thr Ala Val Tyr Val Ala Phe Ala Ala Arg His
500 505 510

Leu Ser Ser Gln Thr Val Glu Leu Asp Ser Glu Asn Ser Asp Phe Leu
515 520 525

Leu Leu Lys Pro Lys Gly Thr Met Asp Lys Asn Asp Lys Asn Lys Leu
530 535 540

Ala Ser Asn Asn Ile Leu Asn Phe Leu Lys Asp Lys Glu Thr Leu Arg
545 550 555 560

Asp Thr Ile Leu Gln Tyr Phe Gly Gly His Ser Leu Trp Thr Asp Phe
565 570 575

Pro Phe Asp Lys Tyr Leu Ala Gly Gly Lys Asp Asp Val Asp Phe Leu
580 585 590

Thr Asp Leu Lys Asp Val Ile Tyr Ser Met Arg Asn Asp Ser Phe His
595 600 605

Tyr Ala Thr Glu Asn His Asn Asn Gly Lys Trp Asn Lys Glu Leu Ile
610 615 620

Ser Ala Met Phe Glu His Glu Thr Glu Arg Met Thr Val Val Met Lys
625 630 635 640

Asp Lys Phe Tyr Ser Asn Asn Leu Pro Met Phe Tyr Lys Asn Asp Asp
645 650 655

Leu Lys Lys Leu Leu Ile Asp Leu Tyr Lys Asp Asn Val Glu Arg Ala
660 665 670

Ser Gln Val Pro Ser Phe Asn Lys Val Phe Val Arg Lys Asn Phe Pro
675 680 685

Ala Leu Val Arg Asp Lys Asp Asn Leu Gly Ile Glu Leu Asp Leu Lys
690 695 700

Ala Asp Ala Asp Lys Gly Glu Asn Glu Leu Lys Phe Tyr Asn Ala Leu
705 710 715 720

Tyr Tyr Met Phe Lys Glu Ile Tyr Tyr Asn Ala Phe Leu Asn Asp Lys
725 730 735

Asn Val Arg Glu Arg Phe Ile Thr Lys Ala Thr Lys Val Ala Asp Asn
740 745 750

Tyr Asp Arg Asn Lys Glu Arg Asn Leu Lys Asp Arg Ile Lys Ser Ala
755 760 765

Gly Ser Asp Glu Lys Lys Lys Leu Arg Glu Gln Leu Gln Asn Tyr Ile
770 775 780

Ala Glu Asn Asp Phe Gly Gln Arg Ile Lys Asn Ile Val Gln Val Asn
785 790 795 800

Pro Asp Tyr Thr Leu Ala Gln Ile Cys Gln Leu Ile Met Thr Glu Tyr
805 810 815

Asn Gln Gln Asn Asn Gly Cys Met Gln Lys Lys Ser Ala Ala Arg Lys
820 825 830

Asp Ile Asn Lys Asp Ser Tyr Gln His Tyr Lys Met Leu Leu Leu Val
835 840 845

Asn Leu Arg Lys Ala Phe Leu Glu Phe Ile Lys Glu Asn Tyr Ala Phe
850 855 860

Val Leu Lys Pro Tyr Lys His Asp Leu Cys Asp Lys Ala Asp Phe Val
865 870 875 880

Pro Asp Phe Ala Lys Tyr Val Lys Pro Tyr Ala Gly Leu Ile Ser Arg
885 890 895

Val Ala Gly Ser Ser Glu Leu Gln Lys Trp Tyr Ile Val Ser Arg Phe
900 905 910

Leu Ser Pro Ala Gln Ala Asn His Met Leu Gly Phe Leu His Ser Tyr
915 920 925

Lys Gln Tyr Val Trp Asp Ile Tyr Arg Arg Ala Ser Glu Thr Gly Thr
930 935 940

Glu Ile Asn His Ser Ile Ala Glu Asp Lys Ile Ala Gly Val Asp Ile
945 950 955 960

Thr Asp Val Asp Ala Val Ile Asp Leu Ser Val Lys Leu Cys Gly Thr
965 970 975

Ile Ser Ser Glu Ile Ser Asp Tyr Phe Lys Asp Asp Glu Val Tyr Ala
980 985 990

Glu Tyr Ile Ser Ser Tyr Leu Asp Phe Glu Tyr Asp Gly Gly Asn Tyr
995 1000 1005

Lys Asp Ser Leu Asn Arg Phe Cys Asn Ser Asp Ala Val Asn Asp
1010 1015 1020

Gln Lys Val Ala Leu Tyr Tyr Asp Gly Glu His Pro Lys Leu Asn
1025 1030 1035

Arg Asn Ile Ile Leu Ser Lys Leu Tyr Gly Glu Arg Arg Phe Leu
1040 1045 1050

Glu Lys Ile Thr Asp Arg Val Ser Arg Ser Asp Ile Val Glu Tyr
1055 1060 1065

Tyr Lys Leu Lys Lys Glu Thr Ser Gln Tyr Gln Thr Lys Gly Ile
1070 1075 1080

Phe Asp Ser Glu Asp Glu Gln Lys Asn Ile Lys Lys Phe Gln Glu
1085 1090 1095

Met Lys Asn Ile Val Glu Phe Arg Asp Leu Met Asp Tyr Ser Glu
1100 1105 1110

Ile Ala Asp Glu Leu Gln Gly Gln Leu Ile Asn Trp Ile Tyr Leu
1115 1120 1125

Arg Glu Arg Asp Leu Met Asn Phe Gln Leu Gly Tyr His Tyr Ala
1130 1135 1140

Cys Leu Asn Asn Asp Ser Asn Lys Gln Ala Thr Tyr Val Thr Leu
1145 1150 1155

Asp Tyr Gln Gly Lys Lys Asn Arg Lys Ile Asn Gly Ala Ile Leu
1160 1165 1170

Tyr Gln Ile Cys Ala Met Tyr Ile Asn Gly Leu Pro Leu Tyr Tyr
1175 1180 1185

Val Asp Lys Asp Ser Ser Glu Trp Thr Val Ser Asp Gly Lys Glu
1190 1195 1200

Ser Thr Gly Ala Lys Ile Gly Glu Phe Tyr Arg Tyr Ala Lys Ser
1205 1210 1215

Phe Glu Asn Thr Ser Asp Cys Tyr Ala Ser Gly Leu Glu Ile Phe
1220 1225 1230

Glu Asn Ile Ser Glu His Asp Asn Ile Thr Glu Leu Arg Asn Tyr
1235 1240 1245

Ile Glu His Phe Arg Tyr Tyr Ser Ser Phe Asp Arg Ser Phe Leu
1250 1255 1260

Gly Ile Tyr Ser Glu Val Phe Asp Arg Phe Phe Thr Tyr Asp Leu
1265 1270 1275

Lys Tyr Arg Lys Asn Val Pro Thr Ile Leu Tyr Asn Ile Leu Leu
1280 1285 1290

Gln His Phe Val Asn Val Arg Phe Glu Phe Val Ser Gly Lys Lys
1295 1300 1305

Met Ile Gly Ile Asp Lys Lys Asp Arg Lys Ile Ala Lys Glu Lys
1310 1315 1320

Glu Cys Ala Arg Ile Thr Ile Arg Glu Lys Asn Gly Val Tyr Ser
1325 1330 1335

Glu Gln Phe Thr Tyr Lys Leu Lys Asn Gly Thr Val Tyr Val Asp
1340 1345 1350

Ala Arg Asp Lys Arg Tyr Leu Gln Ser Ile Ile Arg Leu Leu Phe

1355 1360 1365

Tyr Pro Glu Lys Val Asn Met Asp Glu Met Ile Glu Val Lys Glu
1370 1375 1380

Lys Lys Lys Pro Ser Asp Asn Asn Thr Gly Lys Gly Tyr Ser Lys
1385 1390 1395

Arg Asp Arg Gln Gln Asp Arg Lys Glu Tyr Asp Lys Tyr Lys Glu
1400 1405 1410

Lys Lys Lys Lys Glu Gly Asn Phe Leu Ser Gly Met Gly Gly Asn
1415 1420 1425

Ile Asn Trp Asp Glu Ile Asn Ala Gln Leu Lys Asn
1430 1435 1440

<210> 1183
<211> 1127
<212> PRT
<213> Prevotella buccae

<400> 1183

Met Gln Lys Gln Asp Lys Leu Phe Val Asp Arg Lys Lys Asn Ala Ile
1 5 10 15

Phe Ala Phe Pro Lys Tyr Ile Thr Ile Met Glu Asn Lys Glu Lys Pro
20 25 30

Glu Pro Ile Tyr Tyr Glu Leu Thr Asp Lys His Phe Trp Ala Ala Phe
35 40 45

Leu Asn Leu Ala Arg His Asn Val Tyr Thr Thr Ile Asn His Ile Asn
50 55 60

Arg Arg Leu Glu Ile Ala Glu Leu Lys Asp Asp Gly Tyr Met Met Gly
65 70 75 80

Ile Lys Gly Ser Trp Asn Glu Gln Ala Lys Lys Leu Asp Lys Lys Val
85 90 95

Arg Leu Arg Asp Leu Ile Met Lys His Phe Pro Phe Leu Glu Ala Ala
100 105 110

Ala Tyr Glu Met Thr Asn Ser Lys Ser Pro Asn Asn Lys Glu Gln Arg
115 120 125

Glu Lys Glu Gln Ser Glu Ala Leu Ser Leu Asn Asn Leu Lys Asn Val

130 135 140

Leu Phe Ile Phe Leu Glu Lys Leu Gln Val Leu Arg Asn Tyr Tyr Ser
145 150 155 160

His Tyr Lys Tyr Ser Glu Glu Ser Pro Lys Pro Ile Phe Glu Thr Ser
165 170 175

Leu Leu Lys Asn Met Tyr Lys Val Phe Asp Ala Asn Val Arg Leu Val
180 185 190

Lys Arg Asp Tyr Met His His Glu Asn Ile Asp Met Gln Arg Asp Phe
195 200 205

Thr His Leu Asn Arg Lys Lys Gln Val Gly Arg Thr Lys Asn Ile Ile
210 215 220

Asp Ser Pro Asn Phe His Tyr His Phe Ala Asp Lys Glu Gly Asn Met
225 230 235 240

Thr Ile Ala Gly Leu Leu Phe Phe Val Ser Leu Phe Leu Asp Lys Lys
245 250 255

Asp Ala Ile Trp Met Gln Lys Lys Leu Lys Gly Phe Lys Asp Gly Arg
260 265 270

Asn Leu Arg Glu Gln Met Thr Asn Glu Val Phe Cys Arg Ser Arg Ile
275 280 285

Ser Leu Pro Lys Leu Lys Leu Glu Asn Val Gln Thr Lys Asp Trp Met
290 295 300

Gln Leu Asp Met Leu Asn Glu Leu Val Arg Cys Pro Lys Ser Leu Tyr
305 310 315 320

Glu Arg Leu Arg Glu Lys Asp Arg Glu Ser Phe Lys Val Pro Phe Asp
325 330 335

Ile Phe Ser Asp Asp Tyr Asn Ala Glu Glu Glu Pro Phe Lys Asn Thr
340 345 350

Leu Val Arg His Gln Asp Arg Phe Pro Tyr Phe Val Leu Arg Tyr Phe
355 360 365

Asp Leu Asn Glu Ile Phe Glu Gln Leu Arg Phe Gln Ile Asp Leu Gly
370 375 380

Thr Tyr His Phe Ser Ile Tyr Asn Lys Arg Ile Gly Asp Glu Asp Glu
385 390 395 400

Val Arg His Leu Thr His His Leu Tyr Gly Phe Ala Arg Ile Gln Asp
405 410 415

Phe Ala Pro Gln Asn Gln Pro Glu Glu Trp Arg Lys Leu Val Lys Asp
420 425 430

Leu Asp His Phe Glu Thr Ser Gln Glu Pro Tyr Ile Ser Lys Thr Ala
435 440 445

Pro His Tyr His Leu Glu Asn Glu Lys Ile Gly Ile Lys Phe Cys Ser
450 455 460

Ala His Asn Asn Leu Phe Pro Ser Leu Gln Thr Asp Lys Thr Cys Asn
465 470 475 480

Gly Arg Ser Lys Phe Asn Leu Gly Thr Gln Phe Thr Ala Glu Ala Phe
485 490 495

Leu Ser Val His Glu Leu Leu Pro Met Met Phe Tyr Tyr Leu Leu Leu
500 505 510

Thr Lys Asp Tyr Ser Arg Lys Glu Ser Ala Asp Lys Val Glu Gly Ile
515 520 525

Ile Arg Lys Glu Ile Ser Asn Ile Tyr Ala Ile Tyr Asp Ala Phe Ala
530 535 540

Asn Asn Glu Ile Asn Ser Ile Ala Asp Leu Thr Arg Arg Leu Gln Asn
545 550 555 560

Thr Asn Ile Leu Gln Gly His Leu Pro Lys Gln Met Ile Ser Ile Leu
565 570 575

Lys Gly Arg Gln Lys Asp Met Gly Lys Glu Ala Glu Arg Lys Ile Gly
580 585 590

Glu Met Ile Asp Asp Thr Gln Arg Arg Leu Asp Leu Leu Cys Lys Gln
595 600 605

Thr Asn Gln Lys Ile Arg Ile Gly Lys Arg Asn Ala Gly Leu Leu Lys
610 615 620

Ser Gly Lys Ile Ala Asp Trp Leu Val Asn Asp Met Met Arg Phe Gln
625 630 635 640

Pro Val Gln Lys Asp Gln Asn Asn Ile Pro Ile Asn Asn Ser Lys Ala
645 650 655

Asn Ser Thr Glu Tyr Arg Met Leu Gln Arg Ala Leu Ala Leu Phe Gly
660 665 670

Ser Glu Asn Phe Arg Leu Lys Ala Tyr Phe Asn Gln Met Asn Leu Val
675 680 685

Gly Asn Asp Asn Pro His Pro Phe Leu Ala Glu Thr Gln Trp Glu His
690 695 700

Gln Thr Asn Ile Leu Ser Phe Tyr Arg Asn Tyr Leu Glu Ala Arg Lys
705 710 715 720

Lys Tyr Leu Lys Gly Leu Lys Pro Gln Asn Trp Lys Gln Tyr Gln His
725 730 735

Phe Leu Ile Leu Lys Val Gln Lys Thr Asn Arg Asn Thr Leu Val Thr
740 745 750

Gly Trp Lys Asn Ser Phe Asn Leu Pro Arg Gly Ile Phe Thr Gln Pro
755 760 765

Ile Arg Glu Trp Phe Glu Lys His Asn Asn Ser Lys Arg Ile Tyr Asp
770 775 780

Gln Ile Leu Ser Phe Asp Arg Val Gly Phe Val Ala Lys Ala Ile Pro
785 790 795 800

Leu Tyr Phe Ala Glu Glu Tyr Lys Asp Asn Val Gln Pro Phe Tyr Asp
805 810 815

Tyr Pro Phe Asn Ile Gly Asn Arg Leu Lys Pro Lys Lys Arg Gln Phe
820 825 830

Leu Asp Lys Lys Glu Arg Val Glu Leu Trp Gln Lys Asn Lys Glu Leu
835 840 845

Phe Lys Asn Tyr Pro Ser Glu Lys Lys Lys Thr Asp Leu Ala Tyr Leu
850 855 860

Asp Phe Leu Ser Trp Lys Lys Phe Glu Arg Glu Leu Arg Leu Ile Lys
865 870 875 880

Asn Gln Asp Ile Val Thr Trp Leu Met Phe Lys Glu Leu Phe Asn Met
885 890 895

Ala Thr Val Glu Gly Leu Lys Ile Gly Glu Ile His Leu Arg Asp Ile
900 905 910

Asp Thr Asn Thr Ala Asn Glu Glu Ser Asn Asn Ile Leu Asn Arg Ile
915 920 925

Met Pro Met Lys Leu Pro Val Lys Thr Tyr Glu Thr Asp Asn Lys Gly
930 935 940

Asn Ile Leu Lys Glu Arg Pro Leu Ala Thr Phe Tyr Ile Glu Glu Thr
945 950 955 960

Glu Thr Lys Val Leu Lys Gln Gly Asn Phe Lys Ala Leu Val Lys Asp
965 970 975

Arg Arg Leu Asn Gly Leu Phe Ser Phe Ala Glu Thr Thr Asp Leu Asn
980 985 990

Leu Glu Glu His Pro Ile Ser Lys Leu Ser Val Asp Leu Glu Leu Ile
995 1000 1005

Lys Tyr Gln Thr Thr Arg Ile Ser Ile Phe Glu Met Thr Leu Gly
1010 1015 1020

Leu Glu Lys Lys Leu Ile Asp Lys Tyr Ser Thr Leu Pro Thr Asp
1025 1030 1035

Ser Phe Arg Asn Met Leu Glu Arg Trp Leu Gln Cys Lys Ala Asn
1040 1045 1050

Arg Pro Glu Leu Lys Asn Tyr Val Asn Ser Leu Ile Ala Val Arg
1055 1060 1065

Asn Ala Phe Ser His Asn Gln Tyr Pro Met Tyr Asp Ala Thr Leu
1070 1075 1080

Phe Ala Glu Val Lys Lys Phe Thr Leu Phe Pro Ser Val Asp Thr
1085 1090 1095

Lys Lys Ile Glu Leu Asn Ile Ala Pro Gln Leu Leu Glu Ile Val
1100 1105 1110

Gly Lys Ala Ile Lys Glu Ile Glu Lys Ser Glu Asn Lys Asn
1115 1120 1125

<210> 1184

<211> 1232
<212> PRT
<213> Bergeyella zoohelcum

<400> 1184

Met Glu Asn Lys Thr Ser Leu Gly Asn Asn Ile Tyr Tyr Asn Pro Phe
1 5 10 15

Lys Pro Gln Asp Lys Ser Tyr Phe Ala Gly Tyr Phe Asn Ala Ala Met
20 25 30

Glu Asn Thr Asp Ser Val Phe Arg Glu Leu Gly Lys Arg Leu Lys Gly
35 40 45

Lys Glu Tyr Thr Ser Glu Asn Phe Phe Asp Ala Ile Phe Lys Glu Asn
50 55 60

Ile Ser Leu Val Glu Tyr Glu Arg Tyr Val Lys Leu Leu Ser Asp Tyr
65 70 75 80

Phe Pro Met Ala Arg Leu Leu Asp Lys Lys Glu Val Pro Ile Lys Glu
85 90 95

Arg Lys Glu Asn Phe Lys Lys Asn Phe Lys Gly Ile Ile Lys Ala Val
100 105 110

Arg Asp Leu Arg Asn Phe Tyr Thr His Lys Glu His Gly Glu Val Glu
115 120 125

Ile Thr Asp Glu Ile Phe Gly Val Leu Asp Glu Met Leu Lys Ser Thr
130 135 140

Val Leu Thr Val Lys Lys Lys Lys Val Lys Thr Asp Lys Thr Lys Glu
145 150 155 160

Ile Leu Lys Lys Ser Ile Glu Lys Gln Leu Asp Ile Leu Cys Gln Lys
165 170 175

Lys Leu Glu Tyr Leu Arg Asp Thr Ala Arg Lys Ile Glu Glu Lys Arg
180 185 190

Arg Asn Gln Arg Glu Arg Gly Glu Lys Glu Leu Val Ala Pro Phe Lys
195 200 205

Tyr Ser Asp Lys Arg Asp Asp Leu Ile Ala Ala Ile Tyr Asn Asp Ala
210 215 220

Phe Asp Val Tyr Ile Asp Lys Lys Lys Asp Ser Leu Lys Glu Ser Ser

225 230 235 240

Lys Ala Lys Tyr Asn Thr Lys Ser Asp Pro Gln Gln Glu Glu Gly Asp
245 250 255

Leu Lys Ile Pro Ile Ser Lys Asn Gly Val Val Phe Leu Leu Ser Leu
260 265 270

Phe Leu Thr Lys Gln Glu Ile His Ala Phe Lys Ser Lys Ile Ala Gly
275 280 285

Phe Lys Ala Thr Val Ile Asp Glu Ala Thr Val Ser Glu Ala Thr Val
290 295 300

Ser His Gly Lys Asn Ser Ile Cys Phe Met Ala Thr His Glu Ile Phe
305 310 315 320

Ser His Leu Ala Tyr Lys Lys Leu Lys Arg Lys Val Arg Thr Ala Glu
325 330 335

Ile Asn Tyr Gly Glu Ala Glu Asn Ala Glu Gln Leu Ser Val Tyr Ala
340 345 350

Lys Glu Thr Leu Met Met Gln Met Leu Asp Glu Leu Ser Lys Val Pro
355 360 365

Asp Val Val Tyr Gln Asn Leu Ser Glu Asp Val Gln Lys Thr Phe Ile
370 375 380

Glu Asp Trp Asn Glu Tyr Leu Lys Glu Asn Asn Gly Asp Val Gly Thr
385 390 395 400

Met Glu Glu Glu Gln Val Ile His Pro Val Ile Arg Lys Arg Tyr Glu
405 410 415

Asp Lys Phe Asn Tyr Phe Ala Ile Arg Phe Leu Asp Glu Phe Ala Gln
420 425 430

Phe Pro Thr Leu Arg Phe Gln Val His Leu Gly Asn Tyr Leu His Asp
435 440 445

Ser Arg Pro Lys Glu Asn Leu Ile Ser Asp Arg Arg Ile Lys Glu Lys
450 455 460

Ile Thr Val Phe Gly Arg Leu Ser Glu Leu Glu His Lys Lys Ala Leu
465 470 475 480

Phe Ile Lys Asn Thr Glu Thr Asn Glu Asp Arg Glu His Tyr Trp Glu
485 490 495

Ile Phe Pro Asn Pro Asn Tyr Asp Phe Pro Lys Glu Asn Ile Ser Val
500 505 510

Asn Asp Lys Asp Phe Pro Ile Ala Gly Ser Ile Leu Asp Arg Glu Lys
515 520 525

Gln Pro Val Ala Gly Lys Ile Gly Ile Lys Val Lys Leu Leu Asn Gln
530 535 540

Gln Tyr Val Ser Glu Val Asp Lys Ala Val Lys Ala His Gln Leu Lys
545 550 555 560

Gln Arg Lys Ala Ser Lys Pro Ser Ile Gln Asn Ile Ile Glu Glu Ile
565 570 575

Val Pro Ile Asn Glu Ser Asn Pro Lys Glu Ala Ile Val Phe Gly Gly
580 585 590

Gln Pro Thr Ala Tyr Leu Ser Met Asn Asp Ile His Ser Ile Leu Tyr
595 600 605

Glu Phe Phe Asp Lys Trp Glu Lys Lys Lys Glu Lys Leu Glu Lys Lys
610 615 620

Gly Glu Lys Glu Leu Arg Lys Glu Ile Gly Lys Glu Leu Glu Lys Lys
625 630 635 640

Ile Val Gly Lys Ile Gln Ala Gln Ile Gln Gln Ile Ile Asp Lys Asp
645 650 655

Thr Asn Ala Lys Ile Leu Lys Pro Tyr Gln Asp Gly Asn Ser Thr Ala
660 665 670

Ile Asp Lys Glu Lys Leu Ile Lys Asp Leu Lys Gln Glu Gln Asn Ile
675 680 685

Leu Gln Lys Leu Lys Asp Glu Gln Thr Val Arg Glu Lys Glu Tyr Asn
690 695 700

Asp Phe Ile Ala Tyr Gln Asp Lys Asn Arg Glu Ile Asn Lys Val Arg
705 710 715 720

Asp Arg Asn His Lys Gln Tyr Leu Lys Asp Asn Leu Lys Arg Lys Tyr
725 730 735

Pro Glu Ala Pro Ala Arg Lys Glu Val Leu Tyr Tyr Arg Glu Lys Gly
740 745 750

Lys Val Ala Val Trp Leu Ala Asn Asp Ile Lys Arg Phe Met Pro Thr
755 760 765

Asp Phe Lys Asn Glu Trp Lys Gly Glu Gln His Ser Leu Leu Gln Lys
770 775 780

Ser Leu Ala Tyr Tyr Glu Gln Cys Lys Glu Glu Leu Lys Asn Leu Leu
785 790 795 800

Pro Glu Lys Val Phe Gln His Leu Pro Phe Lys Leu Gly Gly Tyr Phe
805 810 815

Gln Gln Lys Tyr Leu Tyr Gln Phe Tyr Thr Cys Tyr Leu Asp Lys Arg
820 825 830

Leu Glu Tyr Ile Ser Gly Leu Val Gln Gln Ala Glu Asn Phe Lys Ser
835 840 845

Glu Asn Lys Val Phe Lys Lys Val Glu Asn Glu Cys Phe Lys Phe Leu
850 855 860

Lys Lys Gln Asn Tyr Thr His Lys Glu Leu Asp Ala Arg Val Gln Ser
865 870 875 880

Ile Leu Gly Tyr Pro Ile Phe Leu Glu Arg Gly Phe Met Asp Glu Lys
885 890 895

Pro Thr Ile Ile Lys Gly Lys Thr Phe Lys Gly Asn Glu Ala Leu Phe
900 905 910

Ala Asp Trp Phe Arg Tyr Tyr Lys Glu Tyr Gln Asn Phe Gln Thr Phe
915 920 925

Tyr Asp Thr Glu Asn Tyr Pro Leu Val Glu Leu Glu Lys Lys Gln Ala
930 935 940

Asp Arg Lys Arg Lys Thr Lys Ile Tyr Gln Gln Lys Lys Asn Asp Val
945 950 955 960

Phe Thr Leu Leu Met Ala Lys His Ile Phe Lys Ser Val Phe Lys Gln
965 970 975

Asp Ser Ile Asp Gln Phe Ser Leu Glu Asp Leu Tyr Gln Ser Arg Glu
980 985 990

Glu Arg Leu Gly Asn Gln Glu Arg Ala Arg Gln Thr Gly Glu Arg Asn
995 1000 1005

Thr Asn Tyr Ile Trp Asn Lys Thr Val Asp Leu Lys Leu Cys Asp
1010 1015 1020

Gly Lys Ile Thr Val Glu Asn Val Lys Leu Lys Asn Val Gly Asp
1025 1030 1035

Phe Ile Lys Tyr Glu Tyr Asp Gln Arg Val Gln Ala Phe Leu Lys
1040 1045 1050

Tyr Glu Glu Asn Ile Glu Trp Gln Ala Phe Leu Ile Lys Glu Ser
1055 1060 1065

Lys Glu Glu Glu Asn Tyr Pro Tyr Val Val Glu Arg Glu Ile Glu
1070 1075 1080

Gln Tyr Glu Lys Val Arg Arg Glu Glu Leu Leu Lys Glu Val His
1085 1090 1095

Leu Ile Glu Glu Tyr Ile Leu Glu Lys Val Lys Asp Lys Glu Ile
1100 1105 1110

Leu Lys Lys Gly Asp Asn Gln Asn Phe Lys Tyr Tyr Ile Leu Asn
1115 1120 1125

Gly Leu Leu Lys Gln Leu Lys Asn Glu Asp Val Glu Ser Tyr Lys
1130 1135 1140

Val Phe Asn Leu Asn Thr Glu Pro Glu Asp Val Asn Ile Asn Gln
1145 1150 1155

Leu Lys Gln Glu Ala Thr Asp Leu Glu Gln Lys Ala Phe Val Leu
1160 1165 1170

Thr Tyr Ile Ala Asn Lys Phe Ala His Asn Gln Leu Pro Lys Lys
1175 1180 1185

Glu Phe Trp Asp Tyr Cys Gln Glu Lys Tyr Gly Lys Ile Glu Lys
1190 1195 1200

Glu Lys Thr Tyr Ala Glu Tyr Phe Ala Glu Val Phe Lys Lys Glu
1205 1210 1215

Lys Glu Ala Leu Ile Lys Leu Glu His His His His His His

1220 1225 1230